# EUROPEAN PATENT SPECIFICATION

(11) **EP 2 593 425 B1**
(45) Date of publication and mention of the grant of the patent: **17.10.2018**
(21) Application number: 11738902.3
(22) Date of filing: 15.07.2011
(51) Int. Cl.: C07C 237/22, C07C 271/22, C07C 271/44, A61P 43/00, A61K 31/16, A61K 31/18, A61K 31/357, A61K 31/381, A61K 31/40, A61K 31/415, A61K 31/4164, A61K 31/4192, A61K 31/426, A61K 31/435, A61K 31/495, C07C 311/06, C07C 311/19, C07C 317/36, C07C 335/32, C07D 207/10

(54) **THERAPEUTICALLY ACTIVE COMPOSITIONS AND THEIR METHOD OF USE**
WIRKSTOFFZUSAMMENSETZUNGEN UND VERFAHREN ZU IHRER VERWENDUNG
COMPOSITIONS THÉRAPEUTIQUEMENT ACTIVES ET MÉTHODE D'UTILISATION CORRESPONDANTE

(30) Priority: 16.07.2010 US 365072 P
(43) Date of publication of application: 22.05.2013
(73) Proprietor: Agios Pharmaceuticals, Inc., Cambridge, MA 02139 (US)
(72) Inventor: POPOVICI-MULLER, Janeta, Windham, NH 03087 (US); SALITURO, Francesco, G., Marlborough, MA 01752 (US); SAUNDERS, Jeffrey, O., Lincoln, MA 01773 (US); TRAVINS, Jeremy M., Southborough, MA 01772 (US); YAN, Shunqi, Irvine CA 92603 (US)
(74) Representative: Bösl, Raphael Konrad
(86) International application number: PCT/US2011/044254
(87) International publication number: WO 2012/009678

(56) References cited:
- WO-A1-2009/150248

## Description

### BACKGROUND OF INVENTION

Isocitrate dehydrogenases (IDHs) catalyze the oxidative decarboxylation of isocitrate to 2-oxoglutarate (*i*.*e*., α-ketoglutarate). These enzymes belong to two distinct subclasses, one of which utilizes NAD(+) as the electron acceptor and the other NADP(+). Five isocitrate dehydrogenases have been reported: three NAD(+)-dependent isocitrate dehydrogenases, which localize to the mitochondrial matrix, and two NADP(+)-dependent isocitrate dehydrogenases, one of which is mitochondrial and the other predominantly cytosolic. Each NADP(+)-dependent isozyme is a homodimer.

IDH1 (isocitrate dehydrogenase 1 (NADP+), cytosolic) is also known as IDH; IDP; IDCD; IDPC or PICD. The protein encoded by this gene is the NADP(+)-dependent isocitrate dehydrogenase found in the cytoplasm and peroxisomes. It contains the PTS-1 peroxisomal targeting signal sequence. The presence of this enzyme in peroxisomes suggests roles in the regeneration of NADPH for intraperoxisomal reductions, such as the conversion of 2, 4-dienoyl-CoAs to 3-enoyl-CoAs, as well as in peroxisomal reactions that consume 2-oxoglutarate, namely the alpha-hydroxylation of phytanic acid. The cytoplasmic enzyme serves a significant role in cytoplasmic NADPH production.

The human IDH1 gene encodes a protein of 414 amino acids. The nucleotide and amino acid sequences for human IDH1 can be found as GenBank entries NM_005896.2 and NP_005887.2 respectively. The nucleotide and amino acid sequences for IDH1 are also described in, *e.g.,* Nekrutenko et al., Mol. Biol. Evol. 15:1674-1684(1998); Geisbrecht et al., J. Biol. Chem. 274:30527-30533(1999); Wiemann et al., Genome Res. 11:422-435(2001); The MGC Project Team, Genome Res. 14:2121-2127(2004); Lubec et al., Submitted (DEC-2008) to UniProtKB; Kullmann et al., Submitted (JUN-1996) to the EMBL/GenBank/DDBJ databases; and Sjoeblom et al., Science 314:268-274(2006).

Non-mutant, *e.g*., wild type, IDH1 catalyzes the oxidative decarboxylation of isocitrate to α-ketoglutarate thereby reducing NAD⁺ (NADP⁺) to NADP (NADPH), *e.g.,* in the forward reaction:

Isocitrate + NAD⁺ (NADP⁺) → α-KG + CO₂ + NADH (NADPH) + H⁺.

It has been discovered that mutations of IDH1 present in certain cancer cells result in a new ability of the enzyme to catalyze the NAPH-dependent reduction of α-ketoglutarate to *R(*-*)*-2-hydroxyglutarate (2HG). The production of 2HG is believed to contribute to the formation and progression of cancer (Dang, L et al, Nature 2009, 462:739-44).
Compounds which can be used for the treatment of cancers are described e.g. in WO 2009/150248 A1. The inhibition of mutant IDH1 and its neoactivity is therefore a potential therapeutic treatment for cancer. Accordingly, there is an ongoing need for inhibitors of IDH1 mutants having alpha hydroxyl neoactivity.

### SUMMARY OF INVENTION

The present invention is directed to a compound of Formula II: 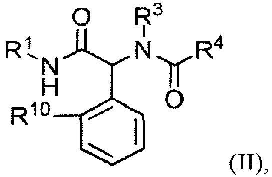 or a pharmaceutically acceptable salt thereof, wherein:
R¹ is selected from cyclohexyl, cyclopentyl, cycloheptyl, 3,3-difluorocyclobutyl, 4,4-difluorocyclohexyl, and bicyclo[2.2.1]heptanyl;
R³ is selected from 3-fluorophenyl, 3-methylphenyl, 3-chlorophenyl, and thien-2-ylmethyl;
R⁴ is selected from 1-(methylmethoxycarbonylamino)ethyl, 1,2,3,4-tetrahydroquinolin-1-yl, 1-ethoxycarbonylpiperidin-2-yl, 1-ethoxycarbonylpyrrolidin-2-yl, 1H-benzimidazol-1-ylmethyl, 1H-indazol-3-ylmethyl, indolin-1-ylmethyl, 1H-indol-3-ylmethyl, 1H-indol-5-ylmethyl, 1H-pyrrolo[2,3-b]pyridine-3-ylmethyl, 1H-pyrrolo[3,2-b]pyridin-3-ylmethyl, 1-methoxycarbonylpiperidin-2-yl, 1-methoxycarbonylpyrrolidin-2-yl, 2-fluoropyridin-3-ylaminomethyl, 2-imino-4-fluoropyridin-1-ylmethyl, 2-methoxyphenylaminomethyl, 2-methyl-1H-benzimidazol-1-ylmethyl, 2-methylimidazol-1-ylmethyl, 2-trifluoromethyl-1H-imidazo)-1-yl, 3-cyanophenylaminomethyl, 3-fluoropyridin-2-ylaminomethyl, 3-methoxyphenylaminomethyl, 4-(1,3,4-oxadiazole-2-yl)phenylaminomethyl, 4-(dimethylaminocarbonyloxy)phenylmethyl, 4,5-dichloroimidazol-1-ylmethyl, 4-cyanophenylaminomethyl, 4-fluorophenylaminomethyl, 4-fluoropyridin-2-ylaminomethyl, 4-hydroxyphenylmethyl, 4-methoxycarbonylmorpholin-3-yl, 4-methoxycarbonylpiperazin-1-ylmethyl, 4-methoxyphenylaminomethyl, 4-methylcarbonyloxyphenylmethyl, 5-fluoropyridin-2-aminomethyl, 5-fluoropyridin-2-oxymethyl, 6-fluoropyridin-3-ylaminomethyl, benzomorpholin-4-ylmethyl, methoxycarbonylaminomethyl, methylmethoxycarbonylaminomethyl, methylphenyl amino methyl, phenylaminomethyl, pyridin-2-oxymethyl, pyridin-2-ylaminomethyl, pyridin-2-yloxymethyl, pyridin-3-oxymethyl, pyridin-3-ylmethyl, pyridin-4-ylmethyl, thiazol-4-ylmethyl, and thien-2-ylmethyl; and
R¹⁰ is selected from methyl, hydrogen, fluoro, chloro, and bromo, wherein:
   when R¹ is cyclopentyl or cyclohexyl, and R³ is thien-2-ylmethyl, then R⁴ is other than thien-2-ylmethyl, 1H-benizimidazol-1-ylmethyl, 1H-indol-3-ylmethyl, or 1H-benzotriazol-1-ylmethyl;
   when R¹ is cyclopentyl, R¹⁰ is hydrogen, and R³ is 3-fluorophenyl, 3-methylphenyl, or 3-chlorophenyl, then R⁴ is other than thien-2-ylmethyl;
   when R¹ is cyclopentyl, R¹⁰ is methyl and R³ is 3-fluorophenyl, then R⁴ is other than thien-2-ylmethyl or 1H-benzotriazol-1-ylmethyl;
   when R¹ is cyclopentyl, R¹⁰ is fluoro and R³ is 3-methylphenyl, then R⁴ is other than thien-2-ylmethyl or 1H-benzotriazol-1-ylmethyl;
   when R¹ is cyclopentyl, R¹⁰ is fluoro and R³ is 3-fluorophenyl, then R⁴ is other than thien-2-ylmethyl;
   when R¹ is cyclohexyl, R¹⁰ is hydrogen, and R³ is 3-methylphenyl, or 3-chlorophenyl, then R⁴ is other than thien-2-ylmethyl; and
   when R¹ is cyclohexyl, R¹⁰ is hydrogen, and R³ is 3-fluorophenyl, then R⁴ is other than 1H-benzotriazol-1-ylmethyl.

Furthermore, the present invention is directed to a compound or a pharmaceutically acceptable salt thereof for use in a method of treating a cancer having an R132X IDH1 mutation, the method comprising administering to a subject a therapeutically effective amount of a compound, wherein the compound is a compound of the present invention.

Furthermore, the present invention is directed to a pharmaceutical composition comprising a compound of the present invention and a pharmaceutically acceptable carrier.

Described herein are compounds for use in methods of treating a cancer characterized by the presence of a mutant allele of IDH1. The compounds for use in methods comprise the step of administering to a subject in need
thereof a compound of formula I, or a pharmaceutically acceptable salt thereof, wherein: 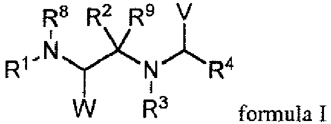
V and W are independently =O or CF₃;
R¹ is selected from C₂-C₆ alkyl, -(C₁-C₃ alkylene)-O-(C₁-C₃ alkyl), carbocyclyl, -(C₁-C₂ alkylene)-(carbocyclyl), aryl, -(C₁-C₂ alkylene)-(aryl), -(C₁-C₂ alkylene)-(heteroaryl), and-(C₁-C₂ alkylene)-(heterocyclyl);
R² is selected from C₄-C₈ alkyl, carbocyclyl, aryl, heterocyclyl, heteroaryl, -(C₁-C₄ alkylene)-(aryl), and -(C₁-C₄ alkylene)-(heteroaryl);
R³ is selected from C₂-C₆, alkyl optionally substituted with =O or -OH; C₂-C₆ alkenyl; -(C₁-C₃ alkylene)-O-(C₁-C₃ alkyl); carbocyclyl; aryl, heterocyclyl, heteroaryl, -(C₁-C₂ alkylene)-(carbocyclyl), -(C₁-C₂ alkylene)-(aryl), -(C₁-C₂ alkylene)-(heterocyclyl), and -(C₁-C₂ alkylene)-(heteroaryl);
R⁴ is selected from -CF₃, -CH₂-O-CH₃ and -R⁵-R⁶-R⁷, wherein:
   R⁵ is selected from a bond; C₁-C₃ straight or branched alkyl wherein one methylene unit in the alkyl of R⁵ is optionally replaced with -O-, -S- or -S(O); and C₂-C₃ alkenyl or alkynyl;
   R⁶ is selected from a bond, -NH-C(O)-, -C(O)-NH-, -NH-S(O)₁₋₂-, -S(O)₁₋₂-NH-, and tetrazolyl;
   R⁷ is a carbocyclyl, aryl, heterocyclyl, or heteroaryl;
   R⁸ is selected from hydrogen and C₁-C₄ alkyl; or R⁸ and R¹ are taken together with the nitrogen atom to form a 5-12 membered heterocyclyl; and
   R⁹ is selected from hydrogen and C₁-C₄ alkyl; or R⁹ and R² are taken together to form a 6-12 membered carbocyclyl or a 5-12 membered heterocyclyl; or
wherein any carbocyclyl, aryl, heterocyclyl or heteroaryl is optionally substituted with one or more substituents.

The compound of formula I inhibits mutant IDH1, particularly mutant IDH1 having alpha hydroxyl neoactivity. Also described herein are pharmaceutical compositions comprising a compound of formula I.

### DETAILED DESCRIPTION OF THE INVENTION

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

### Definitions:

The term "halo" or "halogen" refers to any radical of fluorine, chlorine, bromine or iodine.

The term "alkyl" refers to a hydrocarbon chain that may be a straight chain or branched chain, containing the indicated number of carbon atoms. For example, C₁-C₁₂ alkyl indicates that the group may have from 1 to 12 (inclusive) carbon atoms in it. The term "haloalkyl" refers to an alkyl in which one or more hydrogen atoms are replaced by halo, and includes alkyl moieties in which all hydrogens have been replaced by halo (e.g., perfluoroalkyl). The terms "arylalkyl" or "aralkyl" refer to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

The term "alkylene" refers to a divalent alkyl, e.g., -CH₂-, -CH₂CH₂-, and -CH₂CH₂CH₂-.

The term "alkenyl" refers to a straight or branched hydrocarbon chain containing 2-12 carbon atoms and having one or more double bonds. Examples of alkenyl groups include, but are not limited to, allyl, propenyl, 2-butenyl, 3-hexenyl and 3-octenyl groups. One of the double bond carbons may optionally be the point of attachment of the alkenyl substituent. The term "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl. One of the triple bond carbons may optionally be the point of attachment of the alkynyl substituent.

The term "alkoxy" refers to an -O-alkyl radical. The term "haloalkoxy" refers to an alkoxy in which one or more hydrogen atoms are replaced by halo, and includes alkoxy moieties in which all hydrogens have been replaced by halo (e.g., perfluoroalkoxy).

The term "carbocyclyl" refers to a monocyclic, bicyclic or tricyclic, hydrocarbon ring system that is not fully aromatic, wherein any ring atom capable of substitution can be substituted by one or more substituents. A carbocyclyl can be fully or partially saturated. A bicyclic or tricylic carbocyclyl may contain one (in the case of a bicycle) or up to two (in the case of a tricycle) aromatic rings, as long as at least one ring in the carbocyclyl is non-aromatic. Unless otherwise specified, any ring atom capable of substitution in a carbocyclyl can be substituted by one or more substituents.

The term "aryl" refers to a fully aromatic monocyclic, bicyclic, or tricyclic hydrocarbon ring system. Examples of aryl moieties are phenyl, naphthyl, and anthracenyl. Unless otherwise specified, any ring atom in an aryl can be substituted by one or more substituents.

The term "cycloalkyl" as employed herein refers to a saturated cyclic, bicyclic, tricyclic, or polycyclic hydrocarbon group. Unless otherwise specified, any ring atom can be substituted by one or more substituents. The cycloalkyl groups can contain fused rings. Fused rings are rings that share a common carbon atom. Examples of cycloalkyl moieties include, but are not limited to, cyclopropyl, cyclohexyl, methylcyclohexyl, adamantyl, and norbornyl. Unless otherwise specified, any ring atom can be substituted by one or more substituents.

The term "heterocyclyl" refers to a monocyclic, bicyclic or tricyclic, ring structure that is not fully aromatic and includes one to four heteroatoms independently selected from N, O, or S in one or more of the rings. A heterocyclyl can be fully or partially saturated. A bicyclic or tricylic heterocyclyl may contain one (in the case of a bicycle) or up to two (in the case of a tricycle) aromatic rings, as long as at least one ring in the heterocyclyl is non-aromatic. Unless otherwise specified, any ring atom capable of substitution in a heterocyclyl can be substituted by one or more substituents. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like.

The term "heteroaryl" refers to a monocyclic, bicyclic, or tricyclic ring system having 1-3 heteroatoms if monocyclic, 1-6 heteroatoms if bicyclic, or 1-9 heteroatoms if tricyclic, said heteroatoms independently selected from O, N, or S, wherein each ring in a heteroaryl is fully aromatic. Unless otherwise specified, any ring atom capable of substitution in a heteroaryl can be substituted by one or more substituents. The terms "hetaralkyl" and "heteroaralkyl", as used herein, refers to an alkyl group substituted with a heteroaryl group. The ring heteroatoms of the compounds provided herein include N-O, S(O), and S(O)₂.

The term "substituted" refers to the replacement of a hydrogen atom with another moiety. Typical substituents include alkyl (e.g., Cl, C2, C3, C4, C5, C6, C7, C8, C9, C10, C11, C12 straight or branched chain alkyl), cycloalkyl, haloalkyl (e.g., perfluoroalkyl such as CF₃), aryl, heteroaryl, aralkyl, heteroaralkyl, heterocyclyl, alkenyl, alkynyl, cycloalkenyl, heterocycloalkenyl, alkoxy, haloalkoxy (e.g., perfluoroalkoxy such as OCF₃), halo, hydroxy, carboxy, carboxylate, cyano, nitro, amino, alkyl amino, SO₃H, sulfate, phosphate, methylenedioxy (-O-CH₂-O- wherein oxygens are attached to vicinal atoms), ethylenedioxy, oxo (not a substituent on heteroaryl), thioxo (e.g., C=S) (not a substituent on heteroaryl), imino (alkyl, aryl, aralkyl), S(O)ₙalkyl (where n is 0-2), S(O)ₙ aryl (where n is 0-2), S(O)ₙ heteroaryl (where n is 0-2), S(O)ₙ heterocyclyl (where n is 0-2), amine (mono-, di-, alkyl, cycloalkyl, aralkyl, heteroaralkyl, aryl, heteroaryl, and combinations thereof), ester (alkyl, aralkyl, heteroaralkyl, aryl, heteroaryl), amide (mono-, di-, alkyl, aralkyl, heteroaralkyl, aryl, heteroaryl, and combinations thereof), sulfonamide (mono-, di-, alkyl, aralkyl, heteroaralkyl, and combinations thereof). In one aspect, the substituents on a group are independently any one single, or any subset of the aforementioned substituents. In another aspect, a substituent may itself be substituted with any one of the above substituents.

As used herein, the term "elevated levels of 2HG" means 10%, 20% 30%, 50%, 75%, 100%, 200%, 500% or more 2HG then is present in a subject that does not carry a mutant IDH1 allele. The term "elevated levels of 2HG" may refer to the amount of 2HG within a cell, within a tumor, within an organ comprising a tumor, or within a bodily fluid.

The term "bodily fluid" includes one or more of amniotic fluid surrounding a fetus, aqueous humour, blood (*e.g.,* blood plasma), serum, Cerebrospinal fluid, cerumen, chyme, Cowper's fluid, female ejaculate, interstitial fluid, lymph, breast milk, mucus (*e.g.,* nasal drainage or phlegm), pleural fluid, pus, saliva, sebum, semen, serum, sweat, tears, urine, vaginal secretion, or vomit.

As used herein, the terms "inhibit" or "prevent" include both complete and partial inhibition and prevention. An inhibitor may completely or partially inhibit.

The term "treat" means decrease, suppress, attenuate, diminish, arrest, or stabilize the development or progression of a cancer (e.g., a cancer delineated herein), lessen the severity of the cancer or improve the symptoms associated with the cancer.

As used herein, an amount of a compound effective to treat a disorder, or a "therapeutically effective amount" refers to an amount of the compound which is effective, upon single or multiple dose administration to a subject, in treating a cell, or in curing, alleviating, relieving or improving a subject with a disorder beyond that expected in the absence of such treatment.

As used herein, the term "subject" is intended to include human and non-human animals. Exemplary human subjects include a human patient having a disorder, e.g., a disorder described herein or a normal subject. The term "non-human animals" of the invention includes all vertebrates, e.g., non-mammals (such as chickens, amphibians, reptiles) and mammals, such as non-human primates, domesticated and/or agriculturally useful animals, e.g., sheep, dog, cat, cow, pig, etc.

### Compounds

Provided is a compound having formula A: 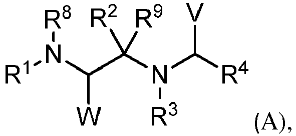 or a pharmaceutically acceptable salt thereof, wherein:
V and W are independently =O or CF₃;
R¹ is selected from C₂-C₆ alkyl, -(C₁-C₃ alkylene)-O-(C₁-C₃ alkyl), carbocyclyl, -(C₁-C₂ alkylene)-(carbocyclyl), aryl, -(C₁-C₂ alkylene)-(aryl), -(C₁-C₂ alkylene)-(heteroaryl), and -(C₁-C₂ alkylene)-(heterocyclyl);
R² is selected from C₄-C₈ alkyl, carbocyclyl, aryl, heterocyclyl, heteroaryl, -(C₁-C₄ alkylene)-(aryl), and -(C₁-C₄ alkylene)-(heteroaryl);
R³ is selected from C₂-C₆ alkyl optionally substituted with =O or -OH; C₂-C₆ alkenyl; -(C₁-C₃ alkylene)-O-(C₁-C₃ alkyl); carbocyclyl; aryl; heterocyclyl; heteroaryl; -(C₁-C₂ alkylene)-(carbocyclyl); -(C₁-C₂ alkylene)-(aryl); -(C₁-C₂ alkylene)-(heterocyclyl); and -(C₁-C₂ alkylene)-(heteroaryl);
R⁴ is selected from -CF₃, -CH₂-O-CH₃, -CH₂Cl, -C(R¹¹)-N(R¹¹)-C(O)-O-(C₁-C₄ alkyl) and -R⁵-R⁶-R⁷, wherein:
   R⁵ is selected from a bond; C₁-C₃ straight or branched alkyl wherein one methylene unit in the alkyl of R⁵ is optionally replaced with -O-, -S-, -S(O)- or -S(O)₂-; and C₂-C₃ alkenyl or alkynyl;
   R⁶ is selected from a bond, -N(R¹¹)-C(O)-, -C(O)-N(R¹¹)-, -N(R¹¹)-S(O)₁₋₂-, -S(O)₁₋₂-N(R¹¹)-, -NH-, -N(C₁-C₃ alkyl)-, and tetrazolyl;
   R⁷ is a carbocyclyl, aryl, heterocyclyl, or heteroaryl;
   R⁸ is selected from hydrogen and C₁-C₄ alkyl; or R⁸ and R¹ are taken together with the nitrogen atom to form a 5-12 membered heterocyclyl;
   R⁹ is selected from hydrogen and C₁-C₄ alkyl; or R⁹ and R² are taken together to form a 6-12 membered carbocyclyl or a 5-12 membered heterocyclyl; and
   each R¹¹ is independently hydrogen or methyl,
wherein any carbocyclyl, aryl, heterocyclyl or heteroaryl is optionally substituted with one or more substituents; and wherein any hydrogen atom is replaced with deuterium.

In one embodiment, the compound has formula I: 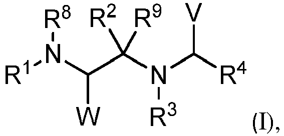 or a pharmaceutically acceptable salt thereof, wherein:
V and W are independently =O or CF₃;
R¹ is selected from C₂-C₆ alkyl, -(C₁-C₃ alkylene)-O-(C₁-C₃ alkyl), carbocyclyl, -(C₁-C₂ alkylene)-(carbocyclyl), aryl, -(C₁-C₂ alkylene)-(aryl), -(C₁-C₂ alkylene)-(heteroaryl), and -(C₁-C₂ alkylene)-(heterocyclyl);
R² is selected from C₄-C₈ alkyl, carbocyclyl, aryl, heterocyclyl, heteroaryl, -(C₁-C₄ alkylene)-(aryl), and -(C₁-C₄ alkylene)-(heteroaryl);
R³ is selected from C₂-C₆ alkyl optionally substituted with =O or -OH; C₂-C₆ alkenyl; -(C₁-C₃ alkylene)-O-(C₁-C₃ alkyl); carbocyclyl; aryl, heterocyclyl, heteroaryl, -(C₁-C₂ alkylene)-(carbocyclyl), -(C₁-C₂ alkylene)-(aryl), -(C₁-C₂ alkylene)-(heterocyclyl), and -(C₁-C₂ alkylene)-(heteroaryl);
R⁴ is selected from -CF₃, -CH₂-O-CH₃ and -R⁵-R⁶-R⁷, wherein:
   R⁵ is selected from a bond; C₁-C₃ straight or branched alkyl wherein one methylene unit in the alkyl of R⁵ is optionally replaced with -O-, -S- or -S(O); and C₂-C₃ alkenyl or alkynyl;
   R⁶ is selected from a bond, -NH-C(O)-, -C(O)-NH-, -NH-S(O)₁₋₂-, -S(O)₁₋₂-NH-, and tetrazolyl;
   R⁷ is a carbocyclyl, aryl, heterocyclyl, or heteroaryl;
   R⁸ is selected from hydrogen and C₁-C₄ alkyl; or R⁸ and R¹ are taken together with the nitrogen atom to form a 5-12 membered heterocyclyl; and
   R⁹ is selected from hydrogen and C₁-C₄ alkyl; or R⁹ and R² are taken together to form a 6-12 membered carbocyclyl or a 5-12 membered heterocyclyl; or
wherein any carbocyclyl, aryl, heterocyclyl or heteroaryl is optionally substituted with one or more substituents.

In one embodiment of formula A or I, V is CF₃ and W is =O. In another embodiment, W is CF₃ and V is =O.

Provided also is a compound having formula I-a, or a pharmaceutically acceptable salt thereof, wherein R¹, R², R³, R⁴, R⁸ and R⁹ are as defined in formula I.

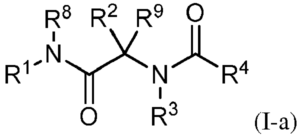

Provided also is a compound having formula I-b, or a pharmaceutically acceptable salt thereof, wherein R¹, R², R³, R⁴, R⁸ and R⁹ are as defined in formula A.

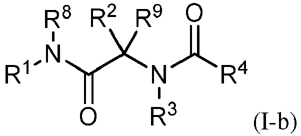

In another embodiment, any carbocyclyl, aryl, heterocyclyl or heteroaryl in formula A, I, I-a or I-b is optionally substituted with one or more substituents independently selected from =O, -C(O)-(C₁-C₃ alkyl), -C(O)-N(R¹⁰)₂, -C(O)-O-(C₁-C₃ alkyl), -C₁-C₄ alkoxy, -C₁-C₄ alkyl, -C₁-C₄ haloalkyl, -C₂-C₄ alkenyl or alkynyl, -C₃-C₈ cycloalkyl, halo, morpholinomethyl, morpholinosulfonyl, morpholinyl, -N(R¹⁰)₂, -NH-C(O)-(C₁-C₃ alkyl), -O-CH₂-C(O)-N(R¹⁰)₂, -OH, -O-phenyl, phenyl, -S(O)₂-piperidin-1-yl, and tetrazolyl; wherein each R¹⁰ is independently selected from hydrogen, C₁-C₃ alkyl, and C₃-C₈ cycloalkyl; and
any cycloalkyl, phenyl or piperidinyl portion of a substituent is optionally further substituted with one or more substituents independently selected from halo, C₁-C₃ alkyl, CF₃, -NH₂, and C₁-C₄ alkoxy.

In another embodiment of Formula A, I, I-a or I-b:
any carbocyclyl, aryl, heterocyclyl or heteroaryl portion of R¹ is optionally substituted with halo, or C₁-C₄ alkoxy;
the carbocyclyl, aryl, heterocyclyl or heteroaryl in R² is optionally substituted with one or more substitutents independently selected from =O, -OH, halo, C₁-C₄ alkyl, C₁-C₄ alkoxy, morpholinyl, -N(R⁸)₂ and -O-CH₂-C(O)-N(R⁸)₂;
any carbocyclyl, aryl, heterocyclyl or heteroaryl in R³ is optionally substituted with one or more substitutents independently selected from -OH, halo, C₁-C₄ alkyl, C₁-C₄ haloalkyl, C₁-C₄ alkoxy, -NH-C(O)-(C₁-C₃ alkyl), -C(O)-(C₁-C₃ alkyl), -C(O)-O-(C₁-C₃ alkyl), tetrazolyl, C₃-C₈ cycloalkyl, phenyl, -O-phenyl, and -S(O)₂-piperidin-1-yl;
any cycloalkyl, phenyl or piperidinyl portion of a substituent of R³ is optionally further substituted with one or more substituents independently selected from halo, C₁-C₃ alkyl, CF₃, -NH₂, and C₁-C₄ alkoxy; and
R⁷ is optionally substituted with one or more substituents independently selected from =O, -OH, halo, C₁-C₄ alkyl, C₂-C₄ alkenyl or alkynyl, C₁-C₄ haloalkyl, -C(O)-N(R⁸)₂, -N(R⁸)₂, C₁-C₄ alkoxy, morpholinomethyl, morpholinosulfonyl, and phenyl, wherein the phenyl substituent of R⁷ is optionally further substituted with one or more substituents independently selected from halo, C₁-C₃ alkyl, CF₃, -NH₂, and C₁-C₄ alkoxy.

In another embodiment of Formula A, I, I-a or I-b, R¹ is piperazinyl, morpholinyl, thiomorpholinyl, tetrahydrothiopyranyl, tetrahydropyranyl, piperidinyl, pyrrolidinyl, or tetrahydrofuranyl, wherein each member of R¹ is optionally substituted.

In another embodiment of Formula A, I, I-a or I-b, R² is selected from carbocyclyl, aryl, heterocyclyl, and heteroaryl, wherein each member of R² is optionally substituted.

In another embodiment of Formula A, I, I-a or I-b, R³ is carbocyclyl; aryl, heterocyclyl, heteroaryl, -(C₁-C₂ alkylene)-(carbocyclyl), -(C₁-C₂ alkylene)-(aryl), -(C₁-C₂ alkylene)-(heterocyclyl), and -(C₁-C₂ alkylene)-(heteroaryl), wherein each member of R³ is optionally substituted.

In another embodiment of Formula A, I, I-a or I-b, R³ is cyclopropyl, cyclopentyl, cyclohexyl or benzyl, wherein each member of R³ is optionally substituted.

In another embodiment of Formula A, I, I-a or I-b, -R⁵-R⁶-R⁷ is not phenyl or N-methyleneisoindoline-1,3-dione.

In another embodiment of Formula A, I, I-a or I-b, R⁶ is not -NHC(O)-.

In another embodiment of Formula A, I, I-a or I-b, R⁸ and R¹ are taken together with the nitrogen atom to form a 5-12 membered heterocyclyl. In one aspect of this embodiment, R² is selected from carbocyclyl, aryl, heterocyclyl, and heteroaryl. In another aspect of this embodiment, -R⁵-R⁶-R⁷ is not phenyl or N-methyleneisoindoline-1,3-dione. In another aspect of this embodiment, R⁶ is not -NHC(O).

In another embodiment of Formula A, I, I-a or I-b, R⁹ is H. In another embodiment, R⁹ is methyl or ethyl.

In another embodiment of Formula A, I, I-a or I-b, R⁹ and R² are taken together to form a 6-12 membered carbocyclyl or a 5-12 membered heterocyclyl, wherein carbocyclyl or heterocyclyl is optionally substituted.

Further, provided is a compound of Formula I-c, or a pharmaceutically acceptable salt thereof. 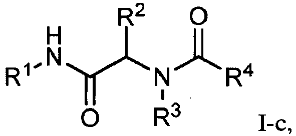 wherein:
R¹ is selected from a C₄-C₇ monocyclic or bicyclic cycloalkyl optionally substituted on a single carbon atom with 1 to 2 fluoro; tetrahydropyranyl, pyrrolidinyl, phenyl, and t-butyl, wherein the phenyl and pyrrolidinyl are optionally substituted;
R² is selected from phenyl, biphenyl, thien-2-yl, and furanyl, wherein R² is optionally substituted;
R³ is selected from phenyl, biphenyl, pyridinyl, thiazolylmethyl, thienylmethyl, cyclohexyl and pyrazolyl, wherein any phenyl, biphenyl, pyridinyl, thiazolyl, thienyl, cyclohexyl or pyrazolyl portion of R³ is optionally substituted; and
R⁴ is as defined in formula A.

In certain embodiments of Formula I-c, R¹ is selected from cyclohexyl, cyclopentyl, cycloheptyl, cyclobutyl, 3,3-difluorocyclobutyl, 4,4,-difluorocyclohexyl, bicyclo[2.2.1]heptanyl, tertahydropyran-3-yl, tertahydropyran-4-yl, 1-t-butoxycarbonylpyrrolidin-3-yl, t-butyl, 2-bromophenyl, 2-methylphenyl, and bicyclo[3.1.0]hexan-3-yl.

In certain embodiments of Formula I-c, R² is selected from phenyl, 2-methylphenyl, 2-fluorphenyl, 2-chlorophenyl, 2-bromophenyl, 2-bromo-5-fluorophenyl, 2,5-dichlorophenyl, 2-fluoro-5-methylphenyl, thien-2-yl, 4-fluorophenyl, 5-bromofuran-2-yl, 3-methylthien-2-yl, 2,4,5-trifluorophenyl, 3-fluoro-5-chlorophenyl, 2,5-difluoro-6-chlorophenyl, 3-chlorophenyl, 3-fluorophenyl, 3-methylphenyl, 2,6-dimethylphenyl, 3-bromopohenyl, 2-ethylphenyl, 2-nitrophenyl, 3'-methoxybiphenyl-3-yl, 2,5-dibromo-6-fluorophenyl, 2-trifluoromethylphenyl, 4-hydoxyphenyl, 3-hydroxyphenyl, 2-hydroxyphenyl, 2-methoxyphenyl, and 2-fluoro-5-methoxyphenyl.

In certain embodiments of Formula I-c, R³ is selected from 3-fluorophenyl, 3-methylphenyl, 3-chlorophenyl, thien-2-ylmethyl, 3-(1-methyl-1H-pyrazol-4-yl)phenyl, 1-methyl-1H-pyrazol-3-yl, 4-chlorophenyl, 3-acetylaminophenyl, 3'-trifluoromethoxy-biphenyl-3-yl, pyridin-3-yl, 4-fluorophenyl, thiazol-2-ylmethyl, cyclohexyl, 2-methylphenyl, 3-fluoro-4-methylphenyl, 2-fluorophenyl, 2-chlorophenyl, 2-bromophenyl, phenyl, 3-bromophenyl, 2-fluorophenyl, 3-chloro-4-methylphenyl, 3-(pyriminidin-5-yl)phenyl, biphenyl-3-yl, 3-trifluoromethylphenyl, 3,4-methylenedioxyphenyl, 3,4-ethylenedioxyphenyl, 3-aminophenyl, 3-ethylcarbonylaminophenyl, 3-t-butoxycarbonylaminophenyl, 3-chloro-4-bromophenyl, 4-methlyphenyl, 3-methoxyphenyl, 3-(1-methyl-1H-pyrazol-5-yl)phenyl, 3-methoxycarbonylaminophenyl, 3-cetylphenyl, 3-(morpholin-4-yl)phenyl, 3,4-difluorophenyl, and 3-(4-t-butoxycarbonylpiperazin-1-yl)phenyl.

In some embodiments, R⁴ is selected from 1-(methylmethoxycarbonylamino)ethyl, 1,2,3,4-tetrahydroquinolin-1-yl, 1-ethoxycarbonylpiperidin-2-yl, 1-ethoxycarbonylpyrrolidin-2-yl, 1H-benzimidazol-1-ylmethyl, 1H-indazol-3-ylmethyl, indolin-1-ylmethyl, 1H-indol-3-ylmethyl, 1H-indol-5-ylmethyl, 1H-pyrrolo[2,3-b]pyridine-3-ylmethyl, 1H-pyrrolo[3,2-b]pyridin-3-ylmethyl, 1-methoxycarbonylpiperidin-2-yl, 1-methoxycarbonylpyrrolidin-2-yl, 2-fluoropyridin-3-ylaminomethyl, 2-imino-4-fluoropyridin-1-ylmethyl, 2-methoxyphenylaminomethyl, 2-methyl-1H-benzimidazol-1-ylmethyl, 2-methylimidazol-1-ylmethyl, 2-trifluoromethyl-1H-imidazol-1-yl, 3-cyanophenylaminomethyl, 3-fluoropyridin-2-ylaminomethyl, 3-methoxyphenylaminomethyl, 4-(1,3,4-oxadiazole-2-yl)phenylaminomethyl, 4-(dimethylaminocarbonyloxy)phenylmethyl, 4,5-dichloroimidazol-1-ylmethyl, 4-cyanophenylaminomethyl, 4-fluorophenylaminomethyl, 4-fluoropyridin-2-ylaminomethyl, 4-hydroxyphenylmethyl, 4-methoxycarbonylmorpholin-3-yl, 4-methoxycarbonylpiperazin-1-ylmethyl, 4-methoxyphenylaminomethyl, 4-methylcarbonyloxyphenylmethyl, 5-fluoropyridin-2-aminomethyl, 5-fluoropyridin-2-oxymethyl, 6-fluoropyridin-3-ylaminomethyl, benzomorpholin-4-ylmethyl, methoxycarbonylaminomethyl, methylmethoxycarbonylaminomethyl, methylphenylaminomethyl, phenylaminomethyl, pyridin-2-oxymethyl, pyridin-2-ylaminomethyl, pyridin-2-yloxymethyl, pyridin-3-oxymethyl, pyridin-3-ylmethyl, pyridin-4-ylmethyl, thiazol-4-ylmethyl, and thien-2-ylmethyl.

### Exemplary compounds of formula I are depicted below in Table 1.

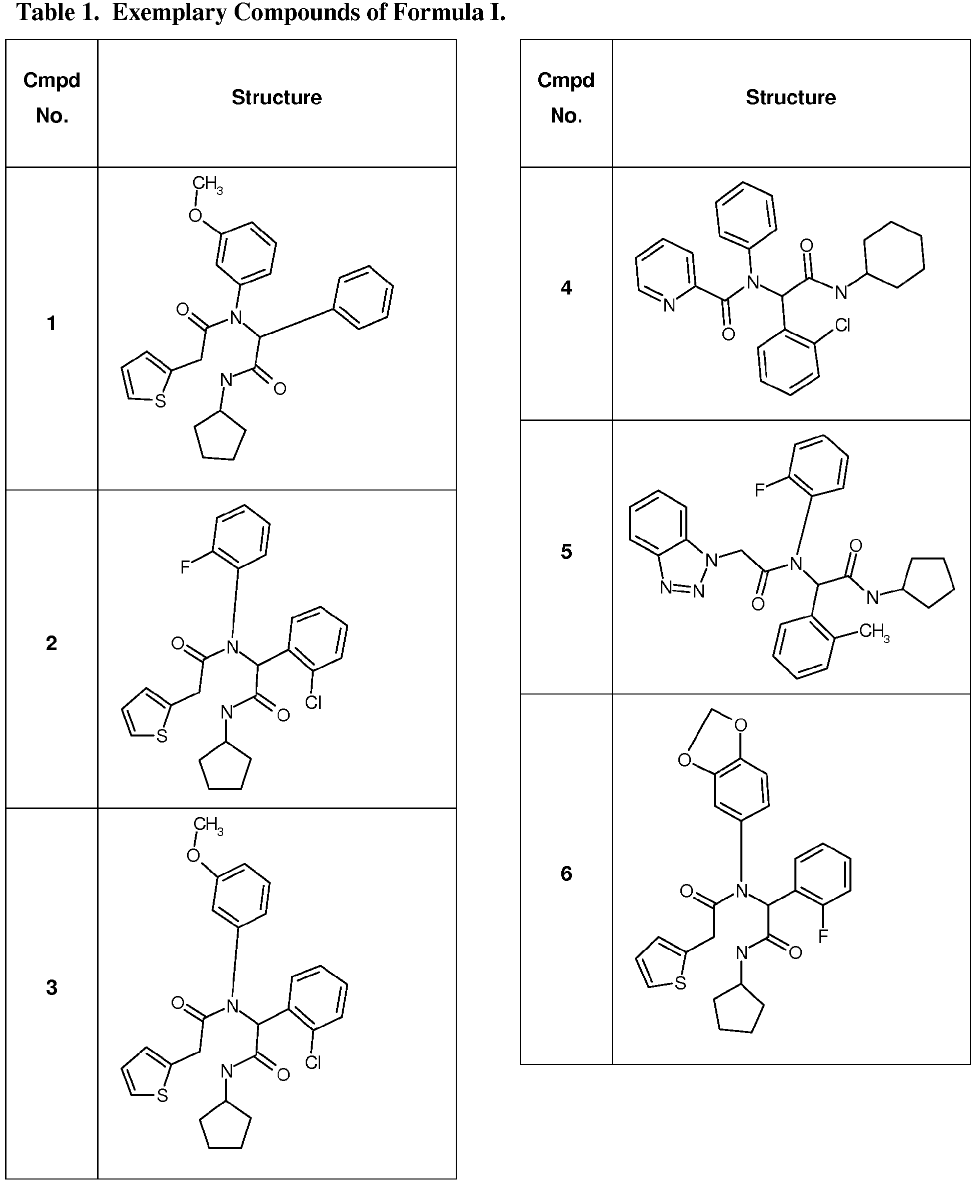

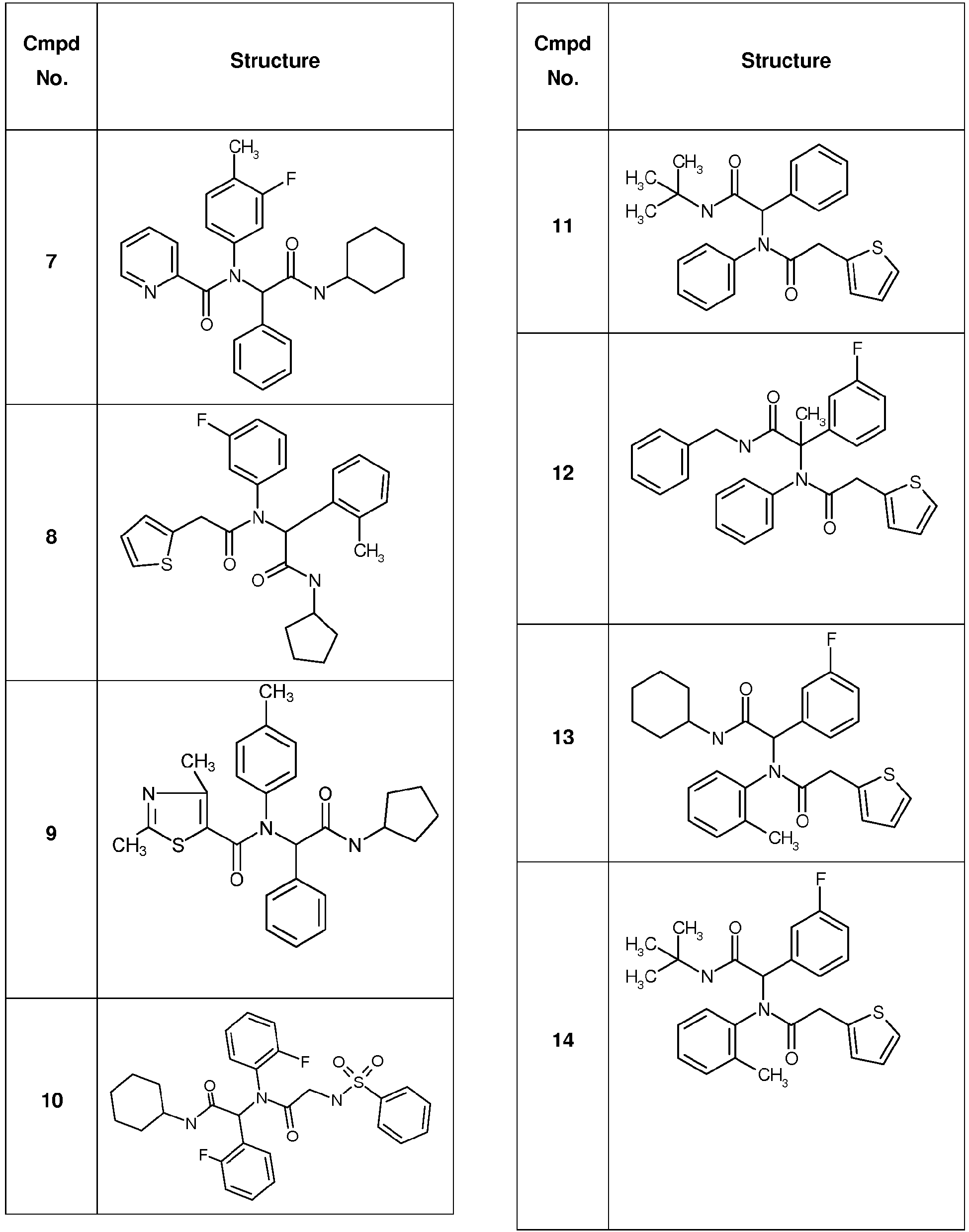

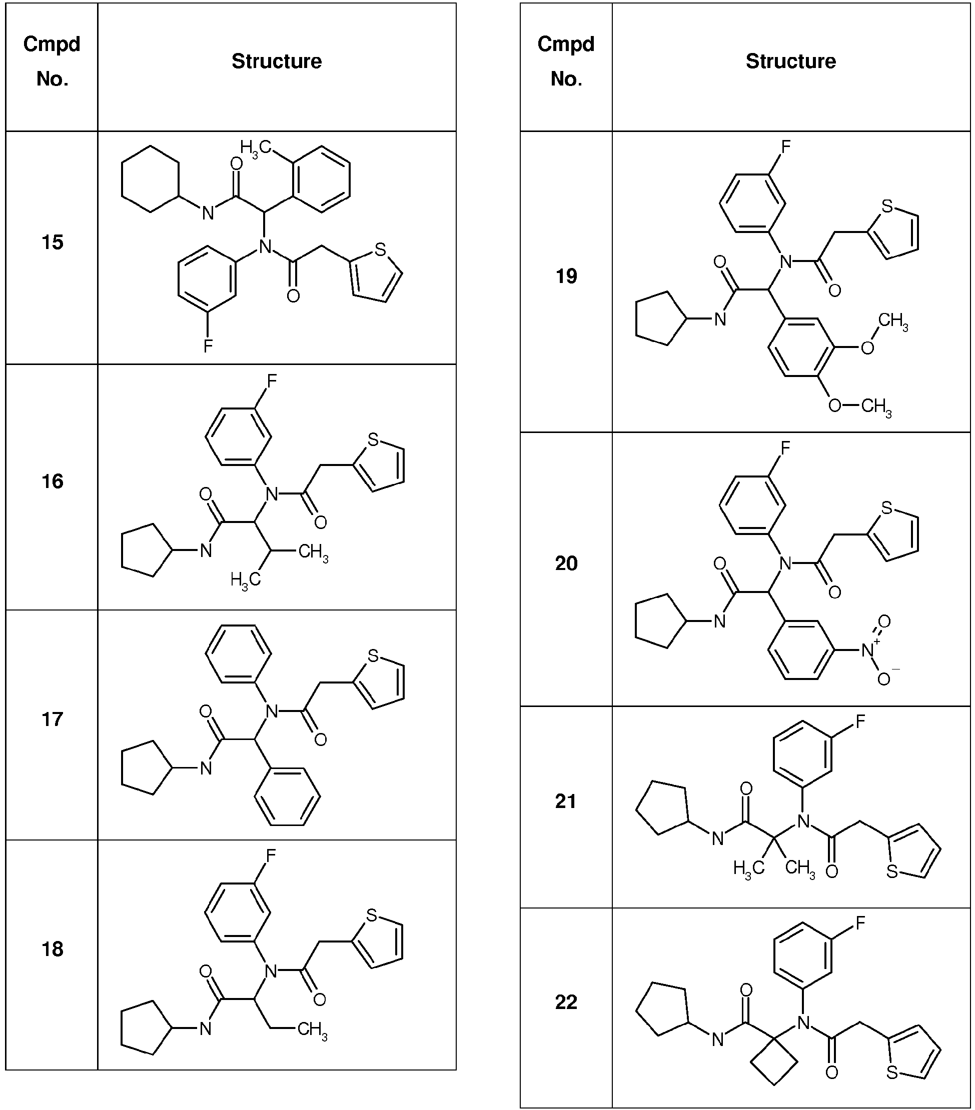

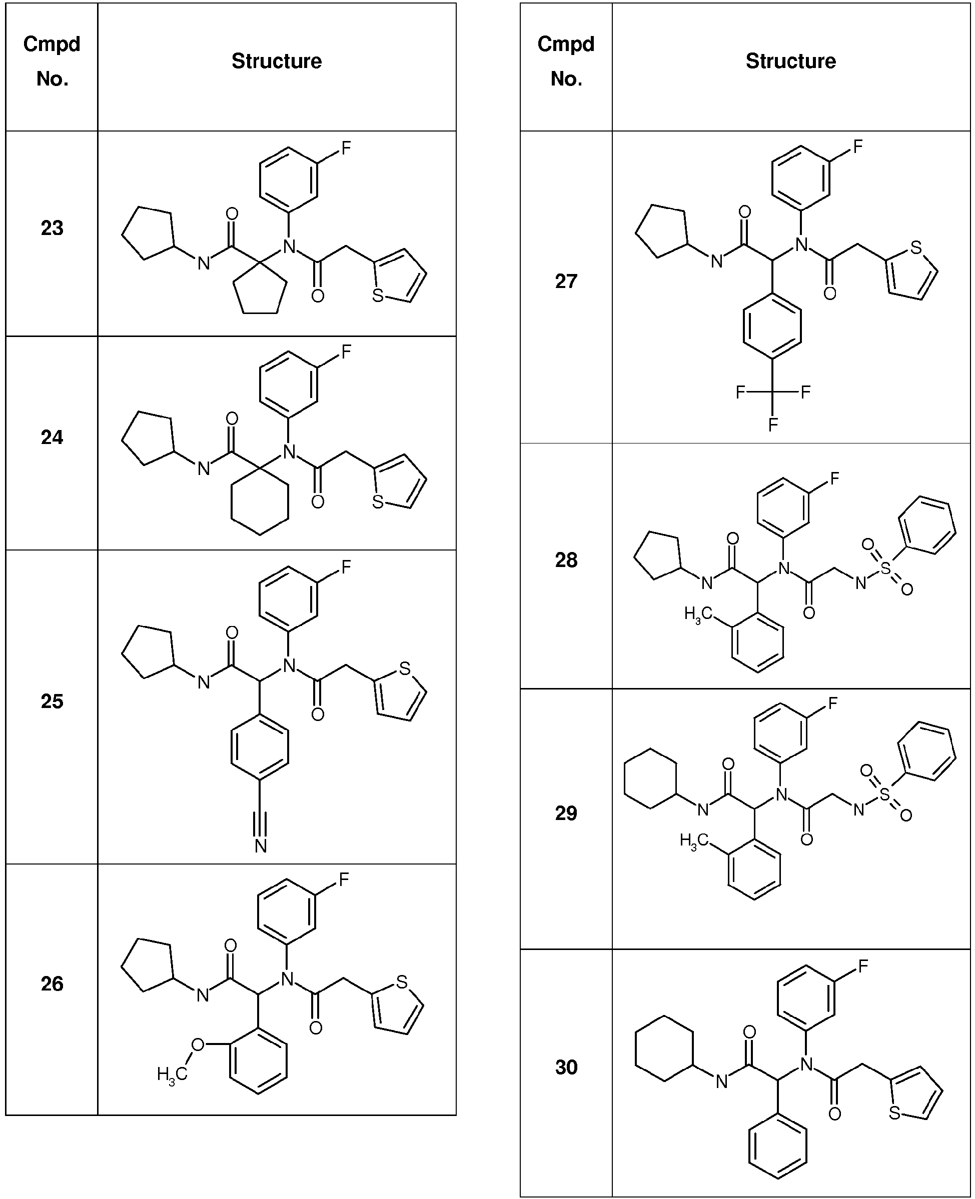

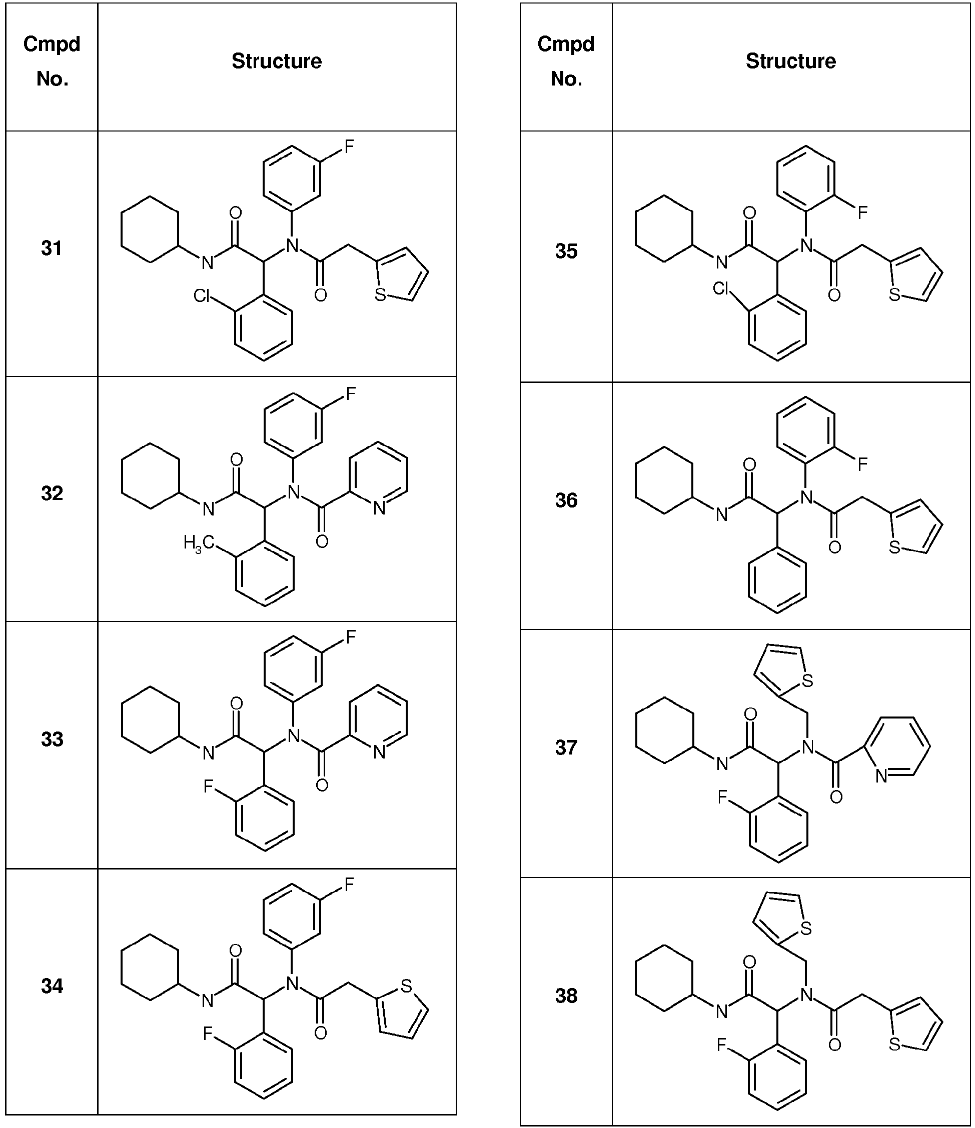

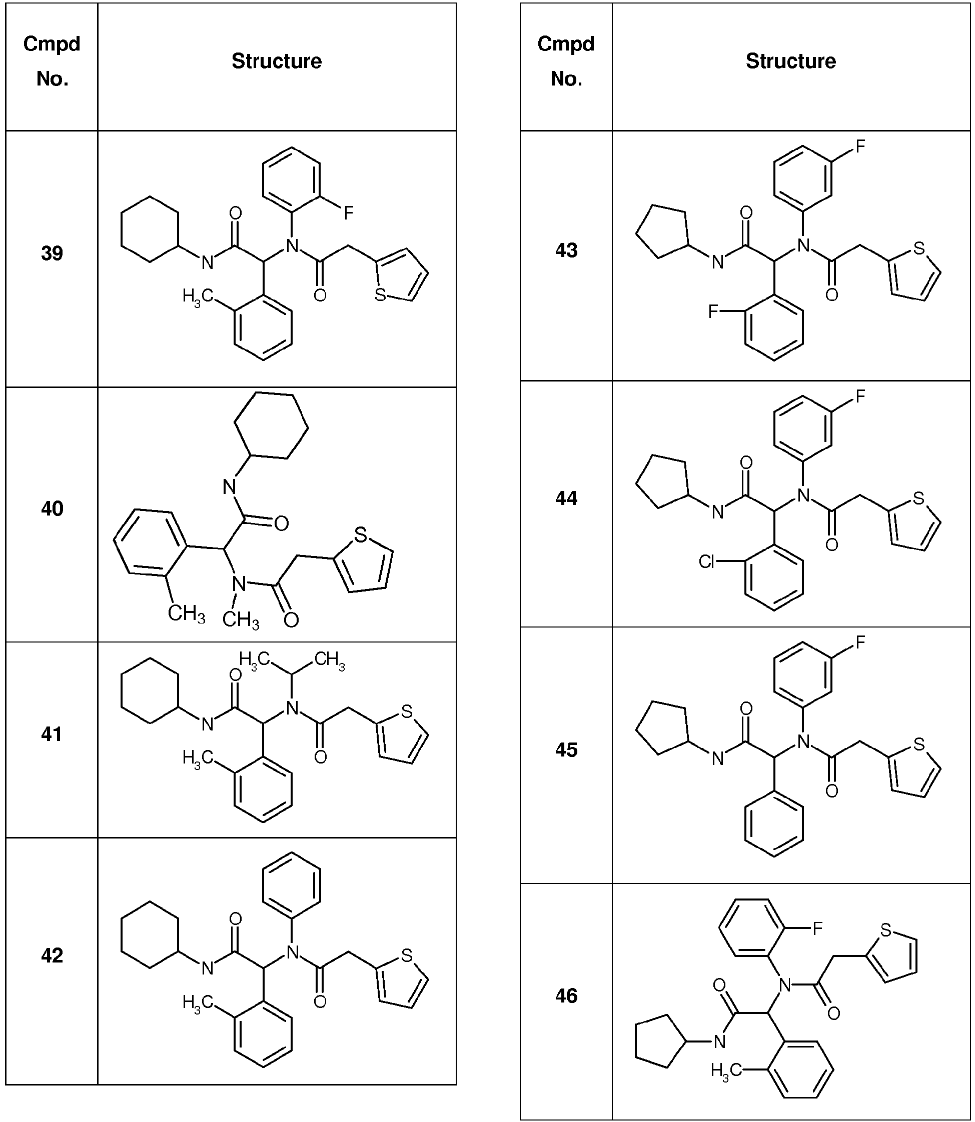

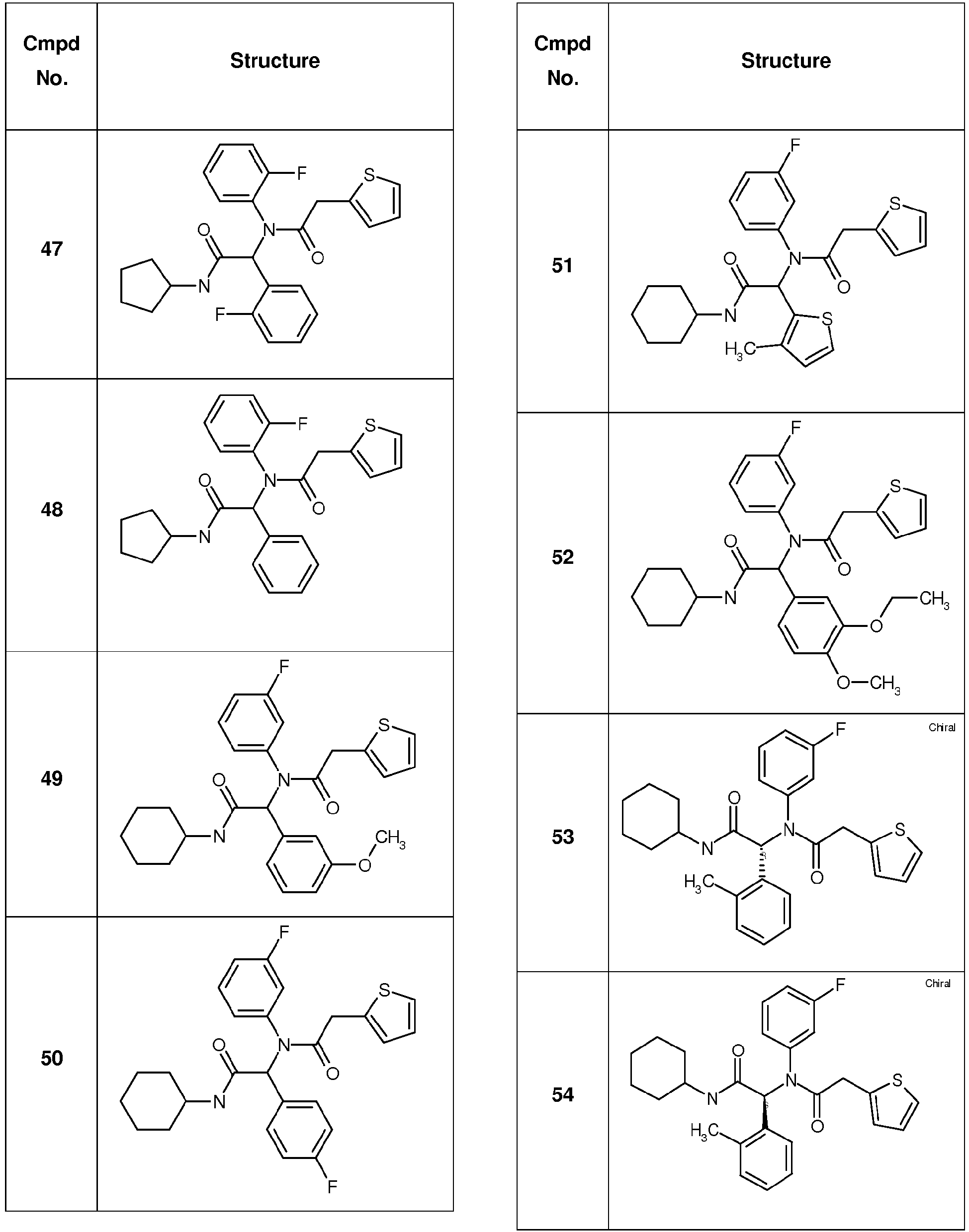

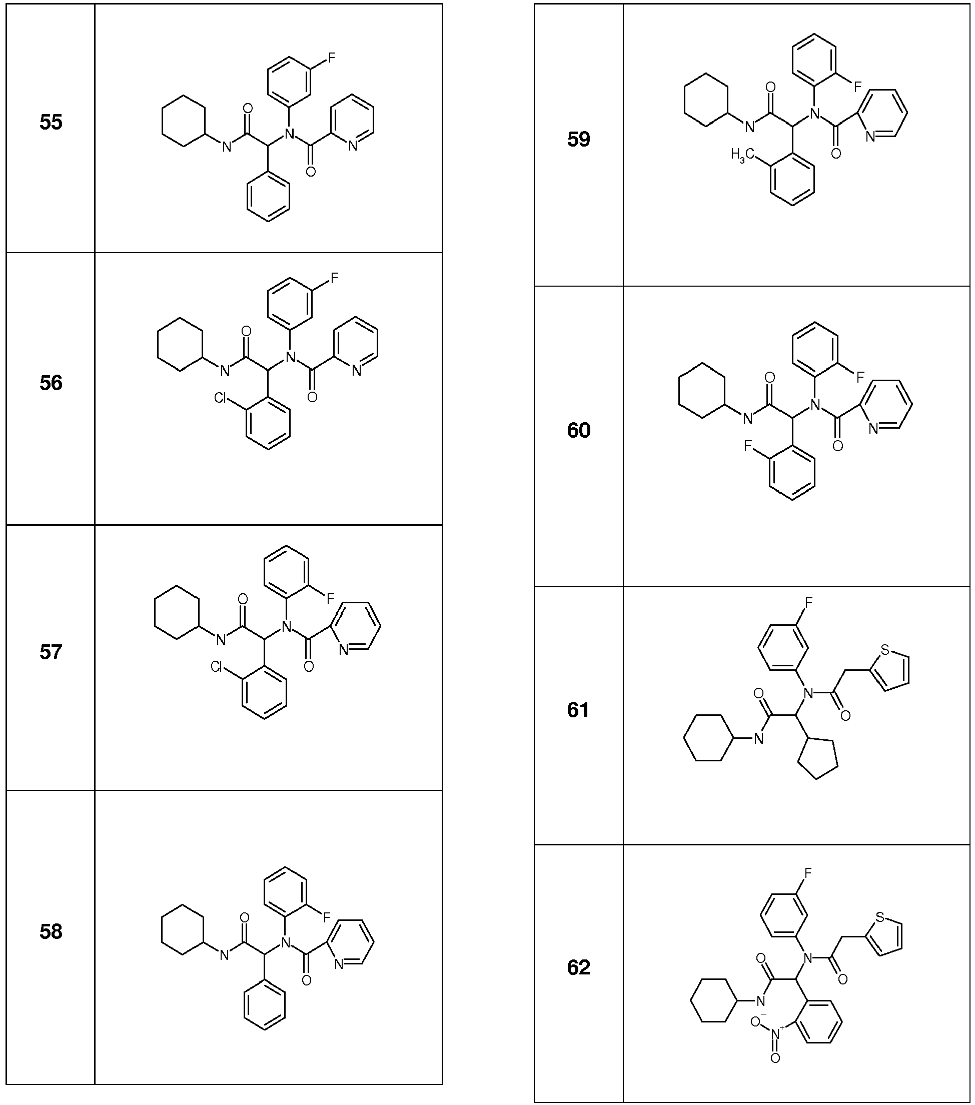

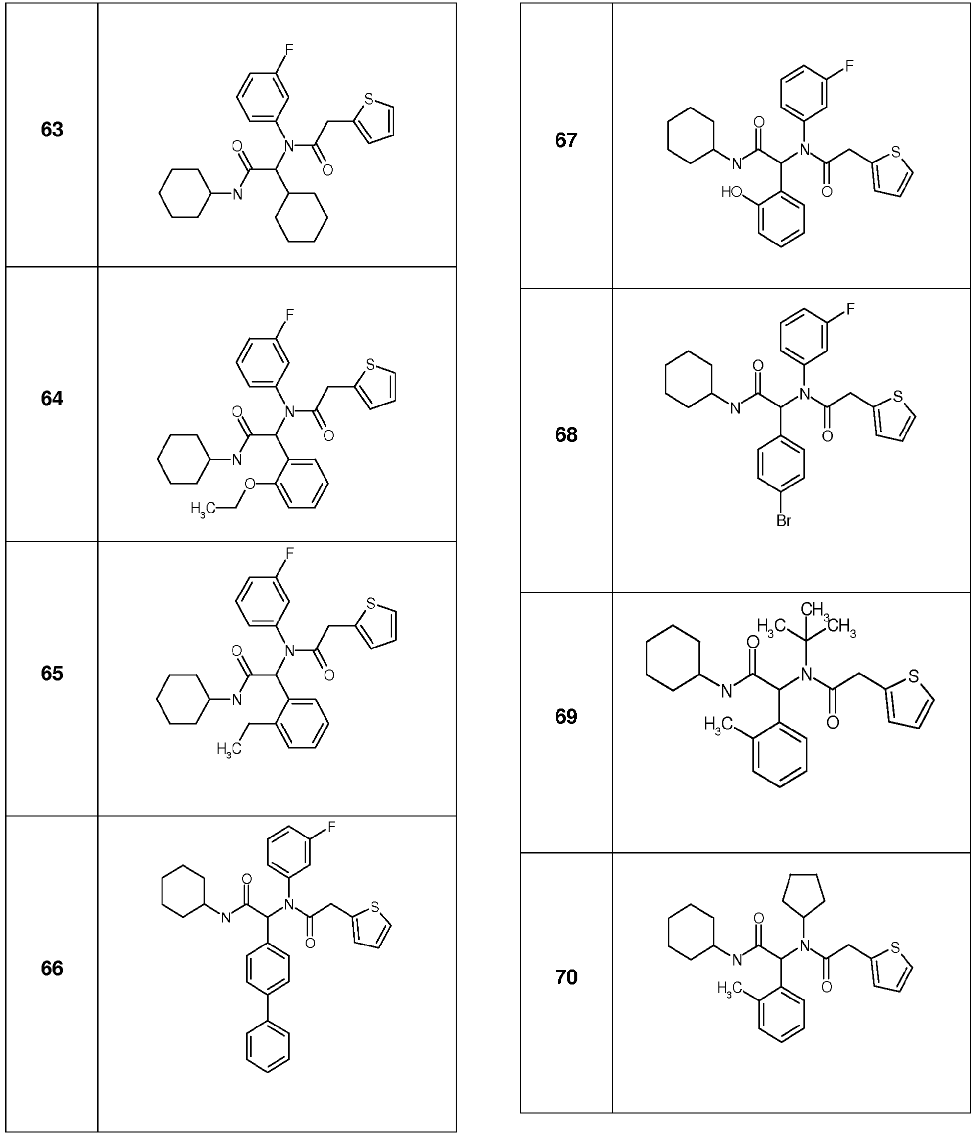

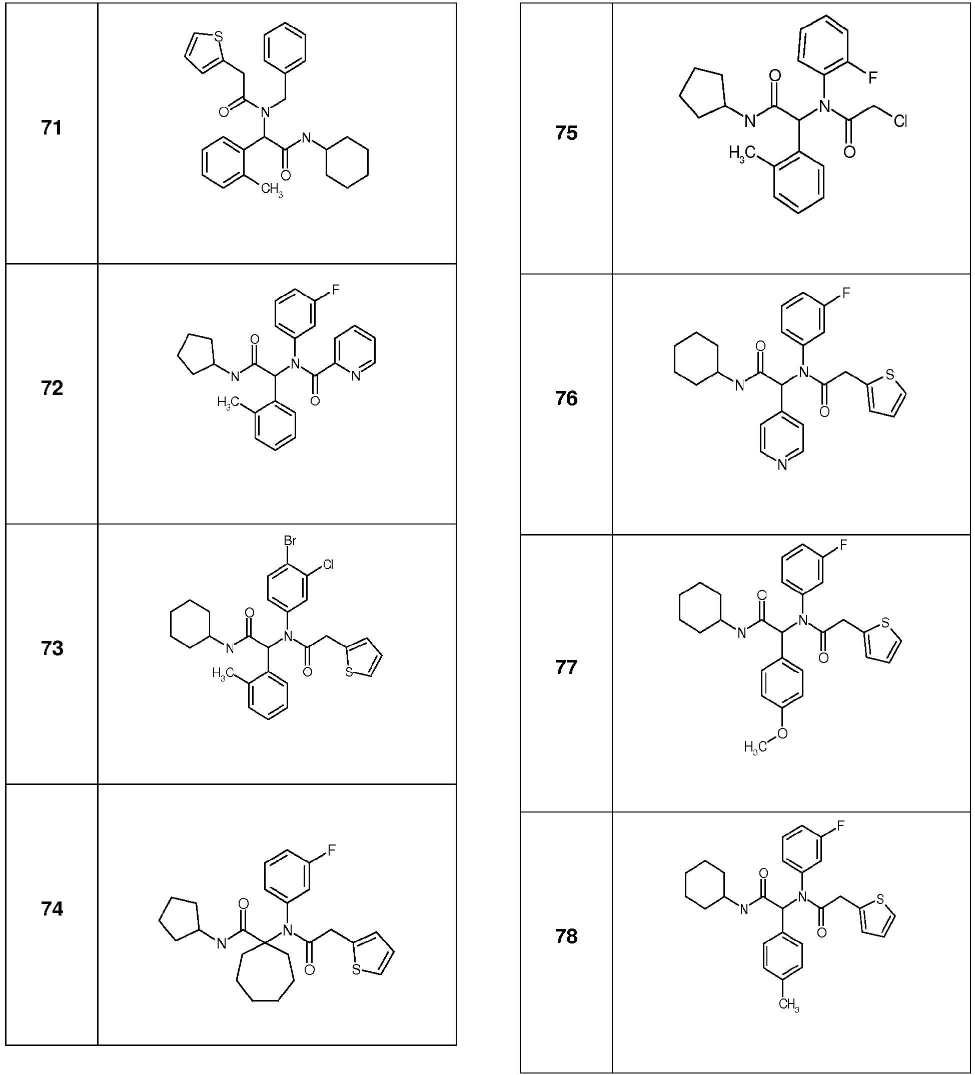

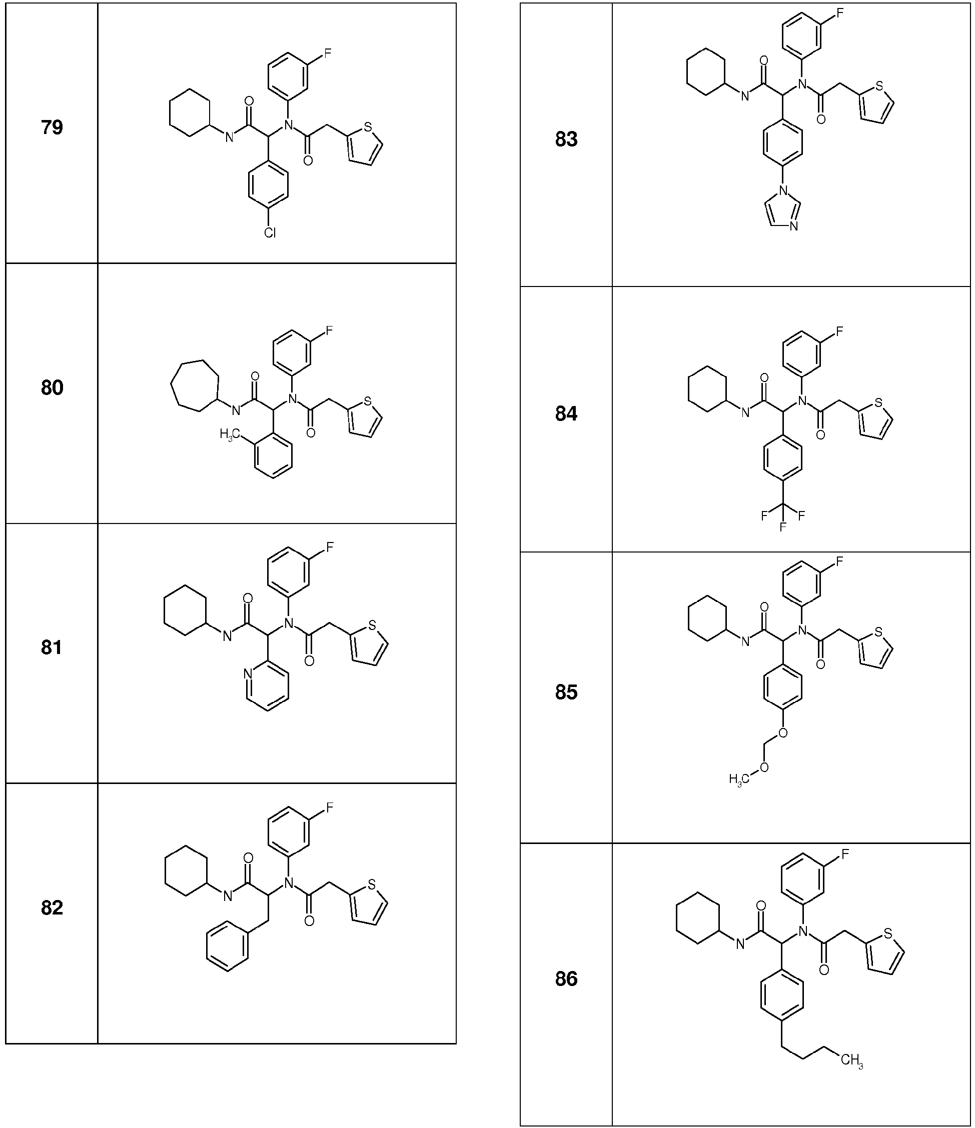

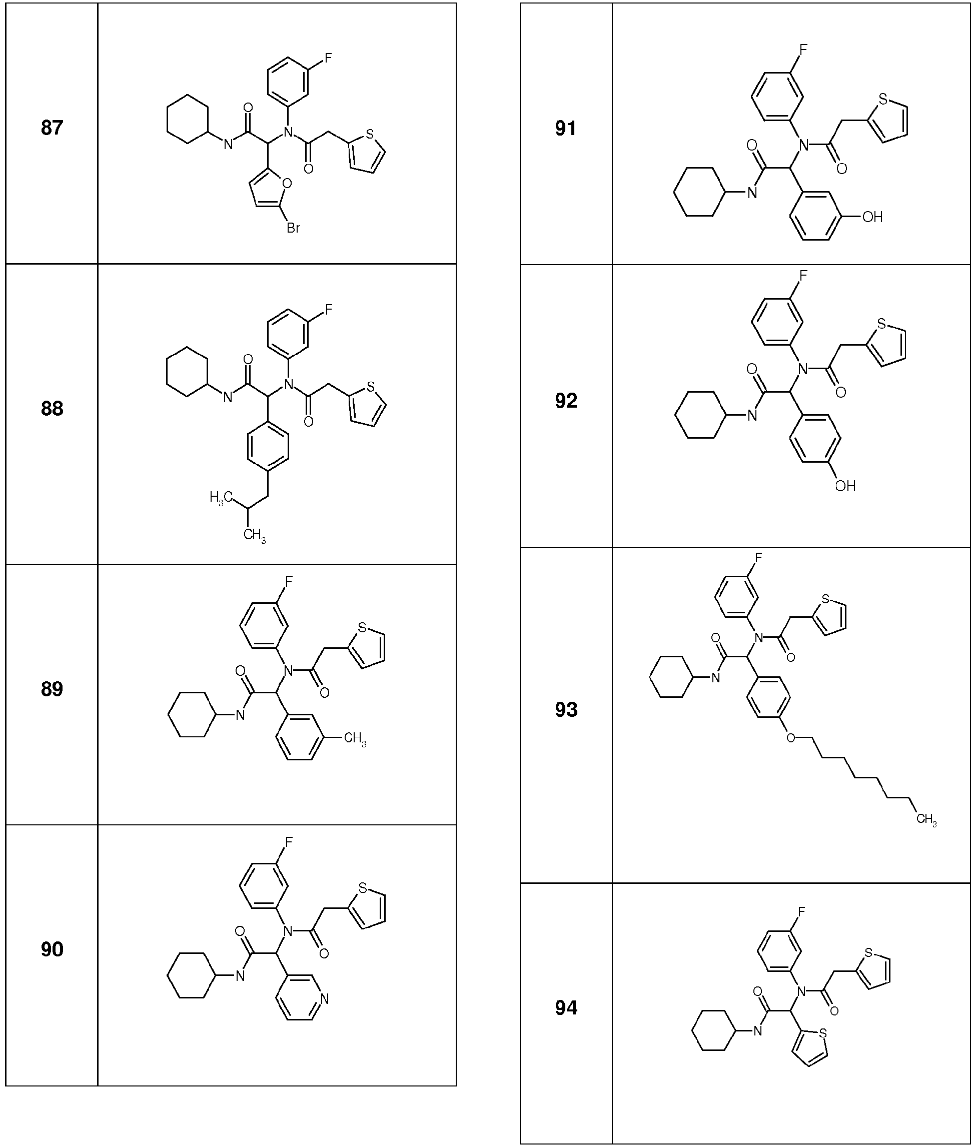

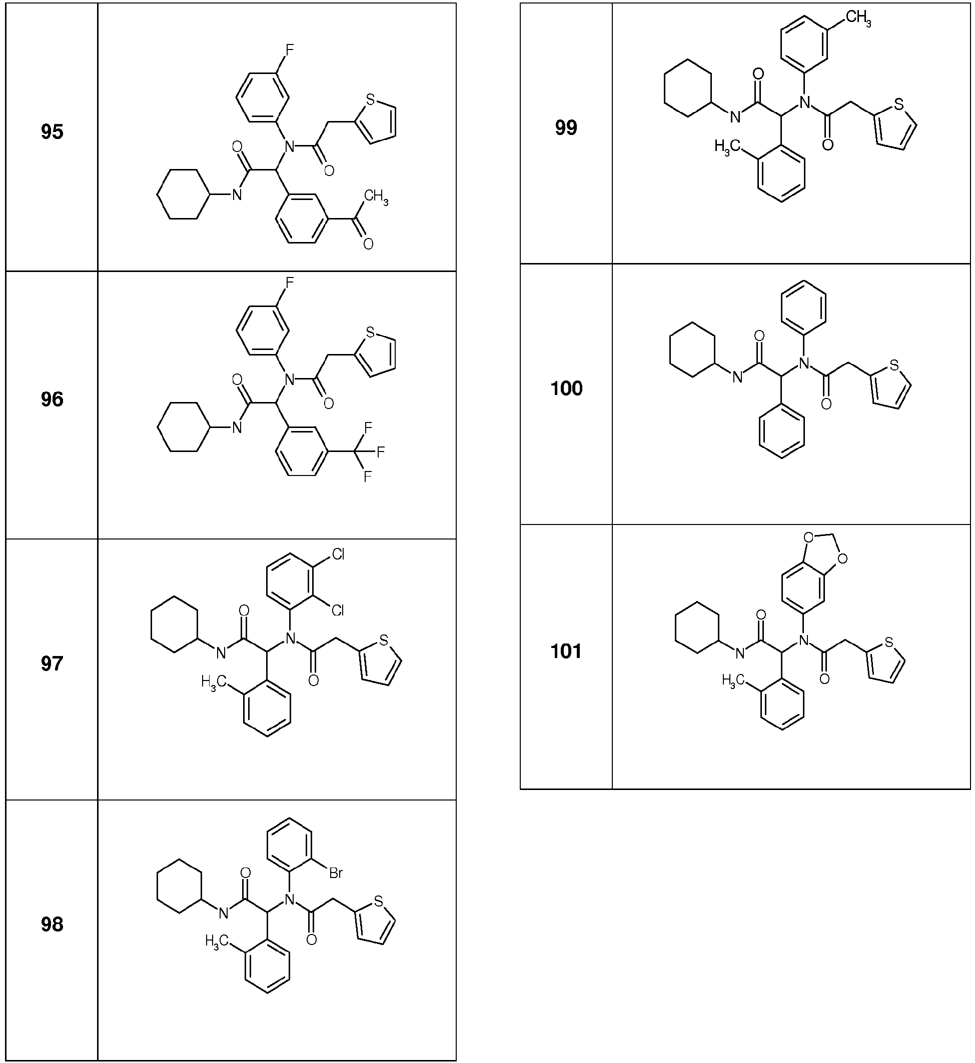

In another embodiment, the compound is selected from any one of Compound numbers 8, 15, 30, 31, 34, 44, 54, 80, 99 from Table 1.

Further described is a compound of Formula II: 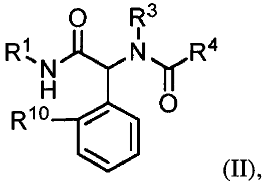 or a pharmaceutically acceptable salt thereof, wherein:
R¹ is a C₄-C₇ monocyclic or bicyclic cycloalkyl optionally substituted on a single carbon atom with 1 to 2 fluoro;
R³ is selected from 3-fluorophenyl, 3-methylphenyl, 3-chlorophenyl, and thien-2-ylmethyl;
R⁴ is selected from saturated heterocyclyl, -CH₂-heterocyclyl, -CH₂-heteroaryl, benzyl, -CH(R¹¹⁾-N(R¹¹)-heteroaryl, -CH(R¹¹)-N(R¹¹)-phenyl, -CH(R¹¹)-N(R¹¹)-heterocyclyl, -CH(R¹¹)-N(R¹¹)- C(O)CH₃, and -CH₂-O-heteroaryl, wherein each R¹¹ is independently selected from hydrogen and methyl; and each saturated heterocyclyl, heterocyclyl, phenyl, benzyl and heteroaryl is optionally substituted; and
R¹⁰ is selected from methyl, hydrogen, fluoro, chloro, and bromo.

In certain embodiments of a compound of Formula II, when R¹ is cyclopentyl or cyclohexyl, and R³ is thien-2-ylmethyl, then R⁴ is other than thien-2-ylmethyl, 1H-benizimidazol-1-ylmethyl, 1H-indol-3-ylmethyl, or 1H-benzotriazol-1-ylmethyl;
when R¹ is cyclopentyl, R¹⁰ is hydrogen, and R³ is 3-fluorophenyl, 3-methylphenyl, or 3-chlorophenyl, then R⁴ is other than thien-2-ylmethyl;
when R¹ is cyclopentyl, R¹⁰ is methyl and R³ is 3-fluorophenyl, then R⁴ is other than thien-2-ylmethyl or 1H-benzotriazol-1-ylmethyl;
when R¹ is cyclopentyl, R¹⁰ is fluoro and R³ is 3-methylphenyl, then R⁴ is other than thien-2-ylmethyl or 1H-benzotriazol-1-ylmethyl;
when R¹ is cyclopentyl, R¹⁰ is fluoro and R³ is 3-fluorophenyl, then R⁴ is other than thien-2-ylmethyl;
when R¹ is cyclohexyl, R¹⁰ is hydrogen, and R³ is 3-methylphenyl, or 3-chlorophenyl, then R⁴ is other than thien-3-ylmethyl; and
when R¹ is cyclohexyl, R¹⁰ is hydrogen, and R³ is 3-fluorophenyl, then R⁴ is other than 1H-benzotriazol-1-ylmethyl.

In certain aspects for Formula II, R³ is 3-fluorophenyl.

In certain aspects of the above embodiments of Formula II:
R¹ is selected from cyclohexyl, cyclopentyl, cycloheptyl, 3,3-difluorocyclobutyl, 4,4,-difluorocyclohexyl, and bicyclo[2.2.1]heptanyl; and
R⁴ is selected from 1-(methylmethoxycarbonylamino)ethyl, 1,2,3,4-tetrahydroquinolin-1-yl, 1-ethoxycarbonylpiperidin-2-yl, 1-ethoxycarbonylpyrrolidin-2-yl, 1H-benzimidazol-1-ylmethyl, 1H-indazol-3-ylmethyl, indolin-1-ylmethyl, 1H-indol-3-ylmethyl, 1H-indol-5-ylmethyl, 1H-pyrrolo[2,3-b]pyridine-3-ylmethyl, 1H-pyrrolo[3,2-b]pyridin-3-ylmethyl, 1-methoxycarbonylpiperidin-2-yl, 1-methoxycarbonylpyrrolidin-2-yl, 2-fluoropyridin-3-ylaminomethyl, 2-imino-4-fluoropyridin-1-ylmethyl, 2-methoxyphenylaminomethyl, 2-methyl-1H-benzimidazol-1-ylmethyl, 2-methylimidazol-1-ylmethyl, 2-trifluoromethyl-1H-imidazol-1-yl, 3-cyanophenylaminomethyl, 3-fluoropyridin-2-ylaminomethyl, 3-methoxyphenylaminomethyl, 4-(1,3,4-oxadiazole-2-yl)phenylaminomethyl, 4-(dimethylaminocarbonyloxy)phenylmethyl, 4,5-dichloroimidazol-1-ylmethyl, 4-cyanophenylaminomethyl, 4-fluorophenylaminomethyl, 4-fluoropyridin-2-ylaminomethyl, 4-hydroxyphenylmethyl, 4-methoxycarbonylmorpholin-3-yl, 4-methoxycarbonylpiperazin-1-ylmethyl, 4-methoxyphenylaminomethyl, 4-methylcarbonyloxyphenylmethyl, 5-fluoropyridin-2-aminomethyl, 5-fluoropyridin-2-oxymethyl, 6-fluoropyridin-3-ylaminomethyl, benzomorpholin-4-ylmethyl, methoxycarbonylaminomethyl, methylmethoxycarbonylaminomethyl, methylphenylaminomethyl, phenylaminomethyl, pyridin-2-oxymethyl, pyridin-2-ylaminomethyl, pyridin-2-yloxymethyl, pyridin-3-oxymethyl, pyridin-3-ylmethyl, pyridin-4-ylmethyl, thiazol-4-ylmethyl, and thien-2-ylmethyl.

In another embodiment, a compound is selected from any one of the compounds set forth in Table 2, below.

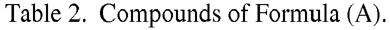

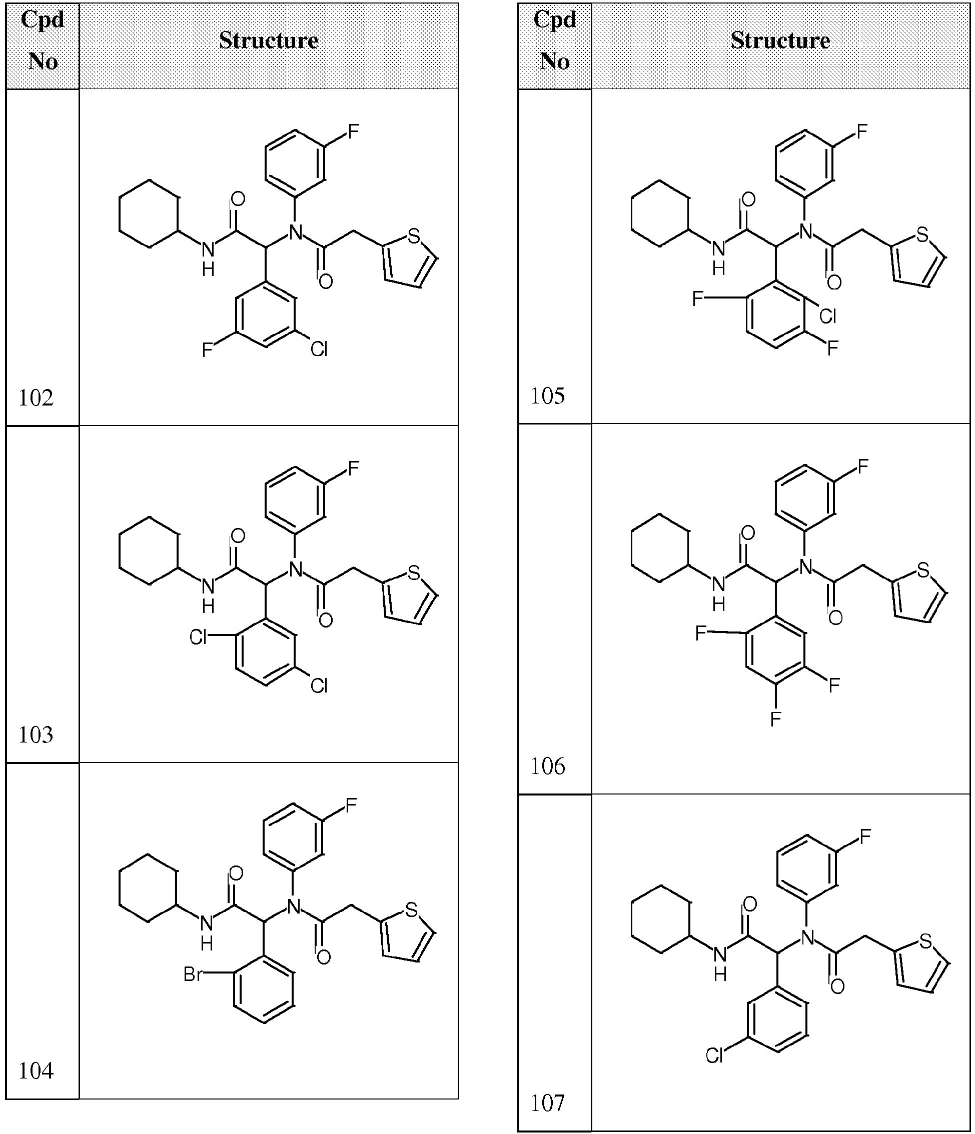

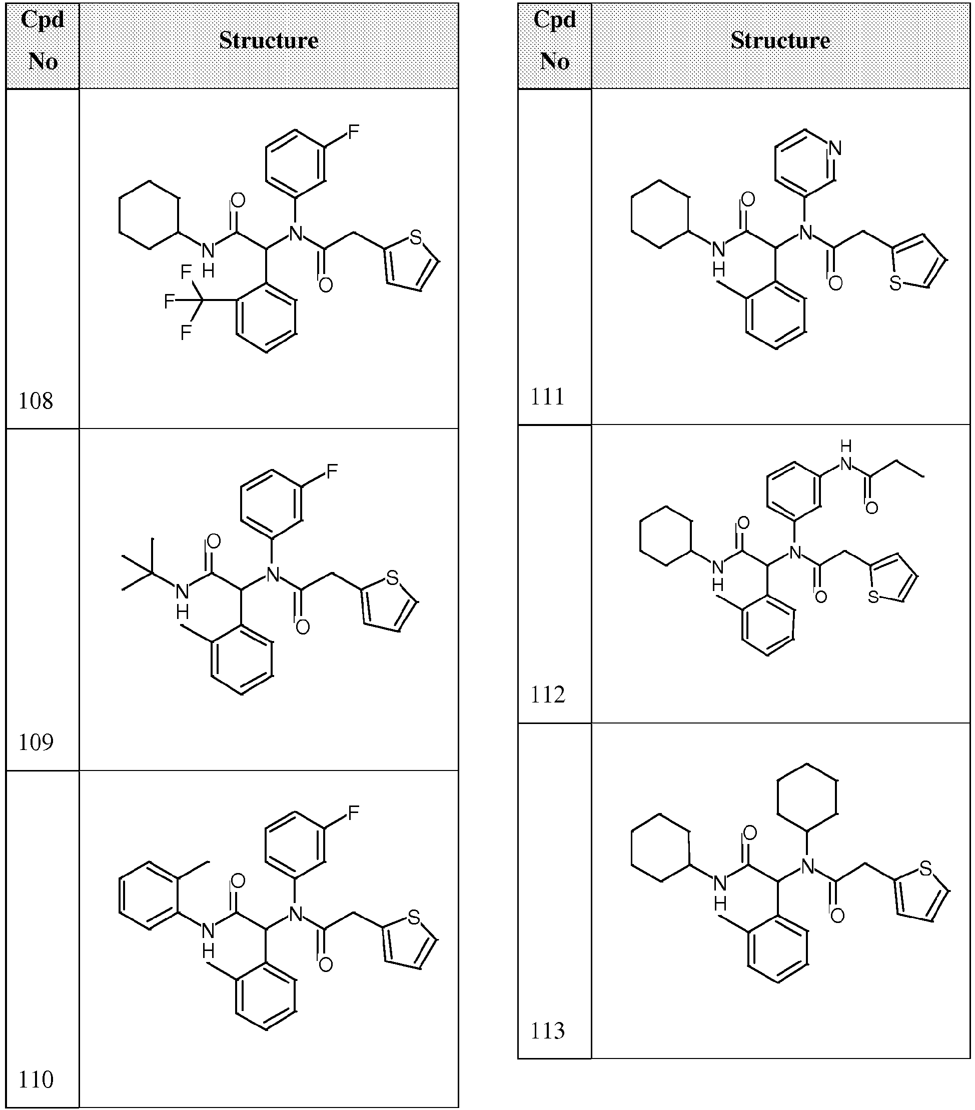

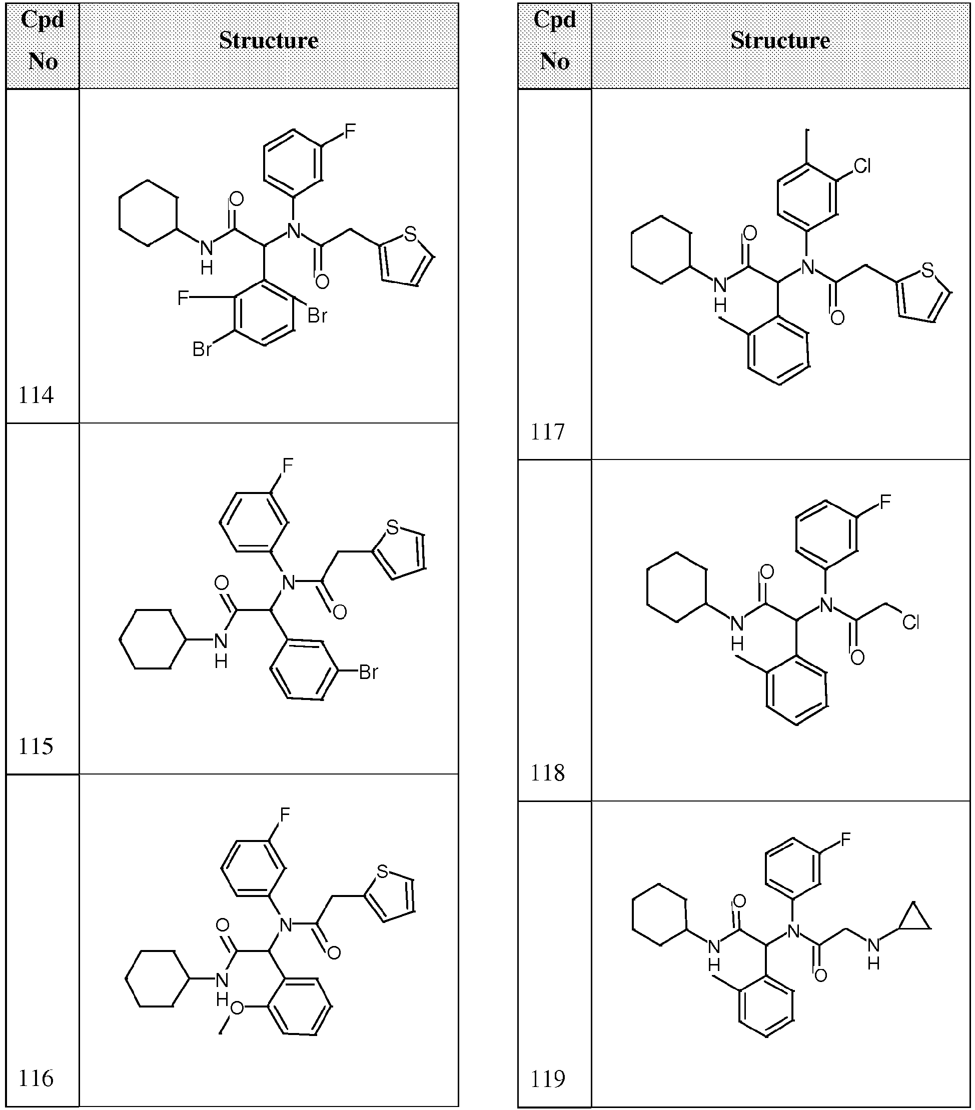

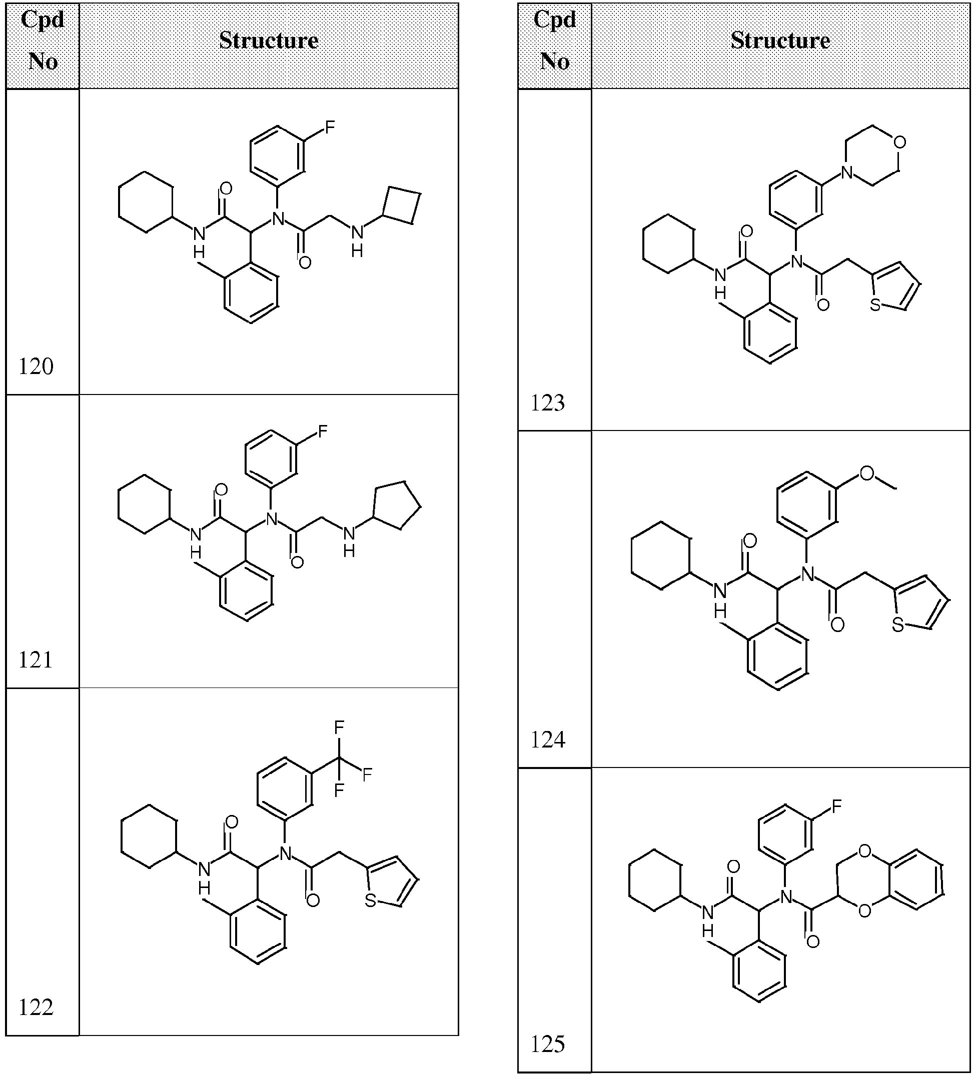

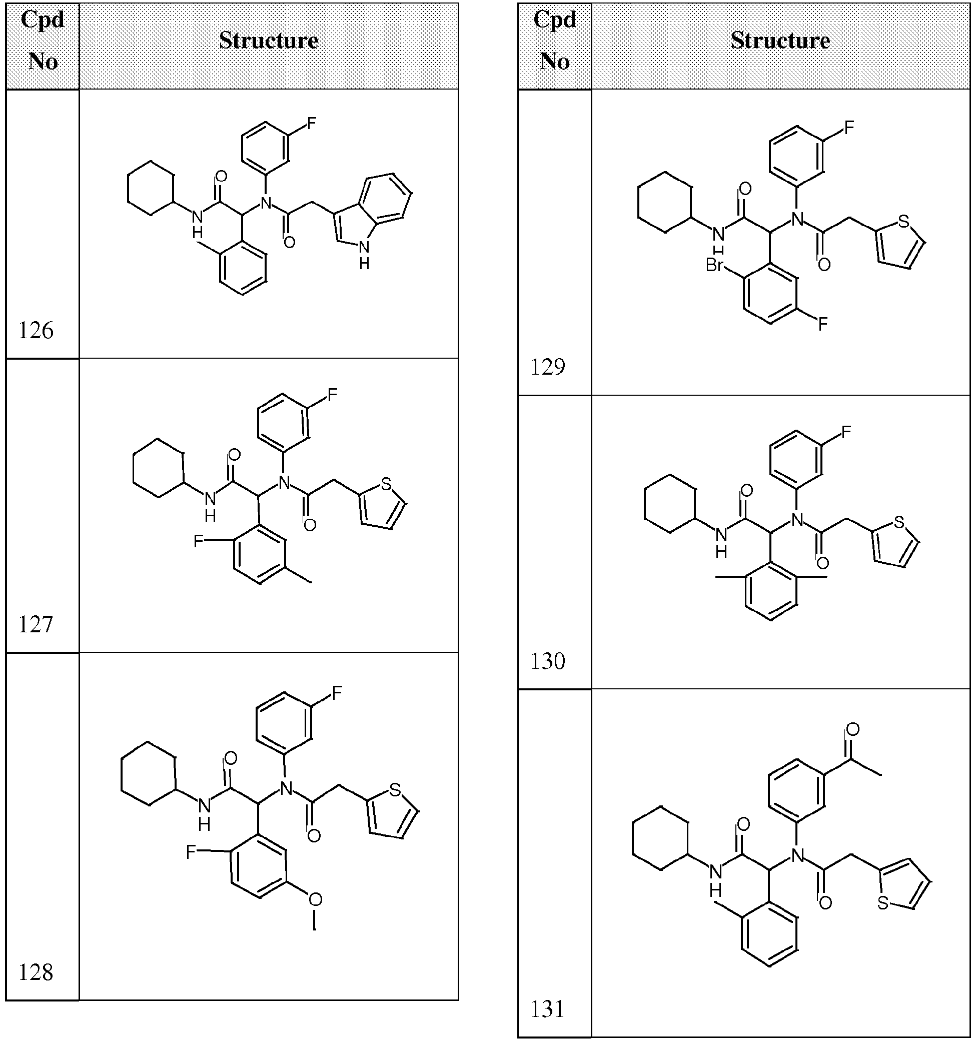

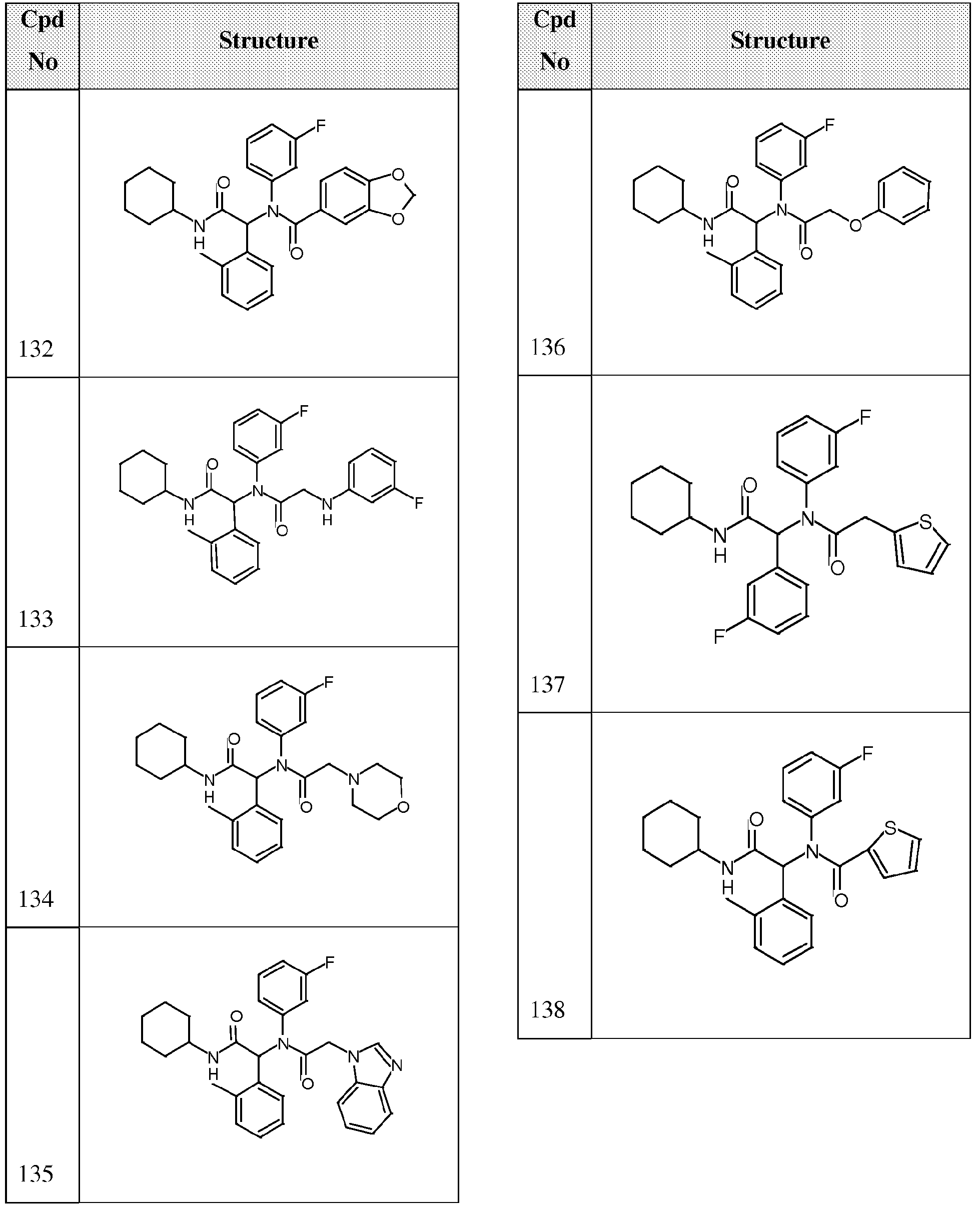

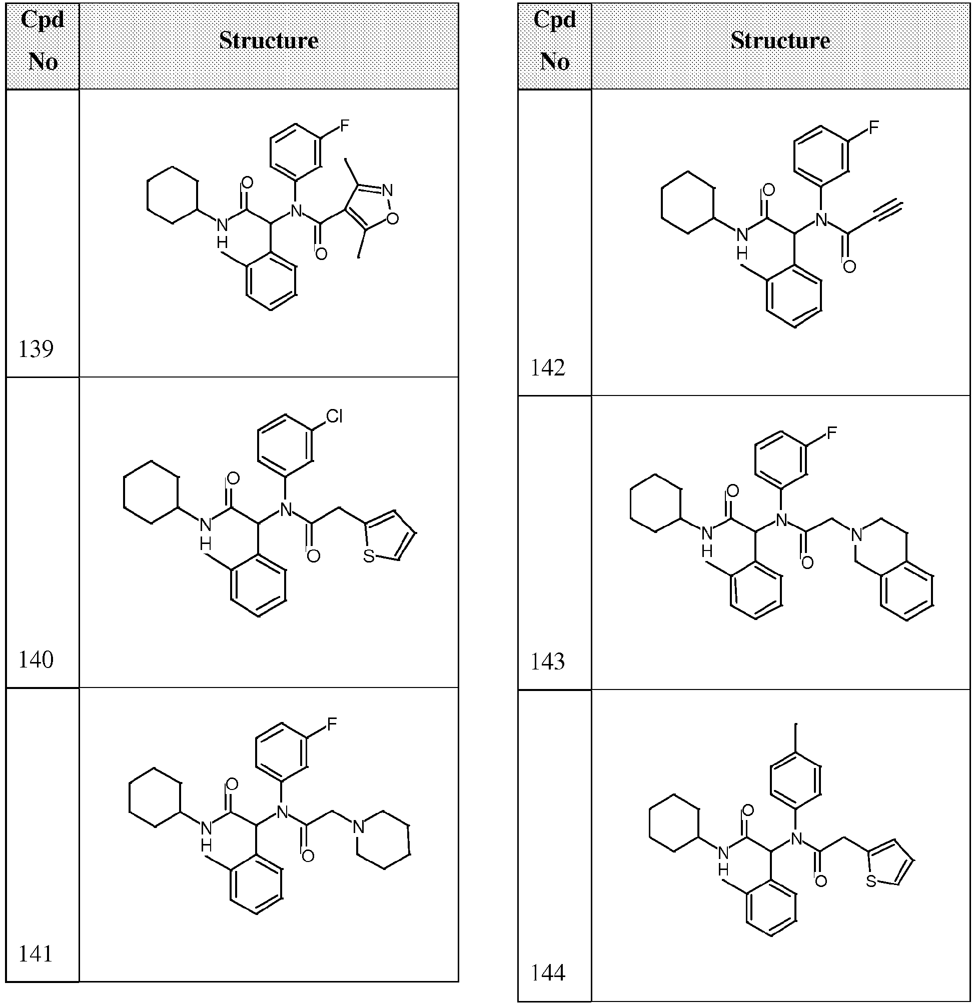

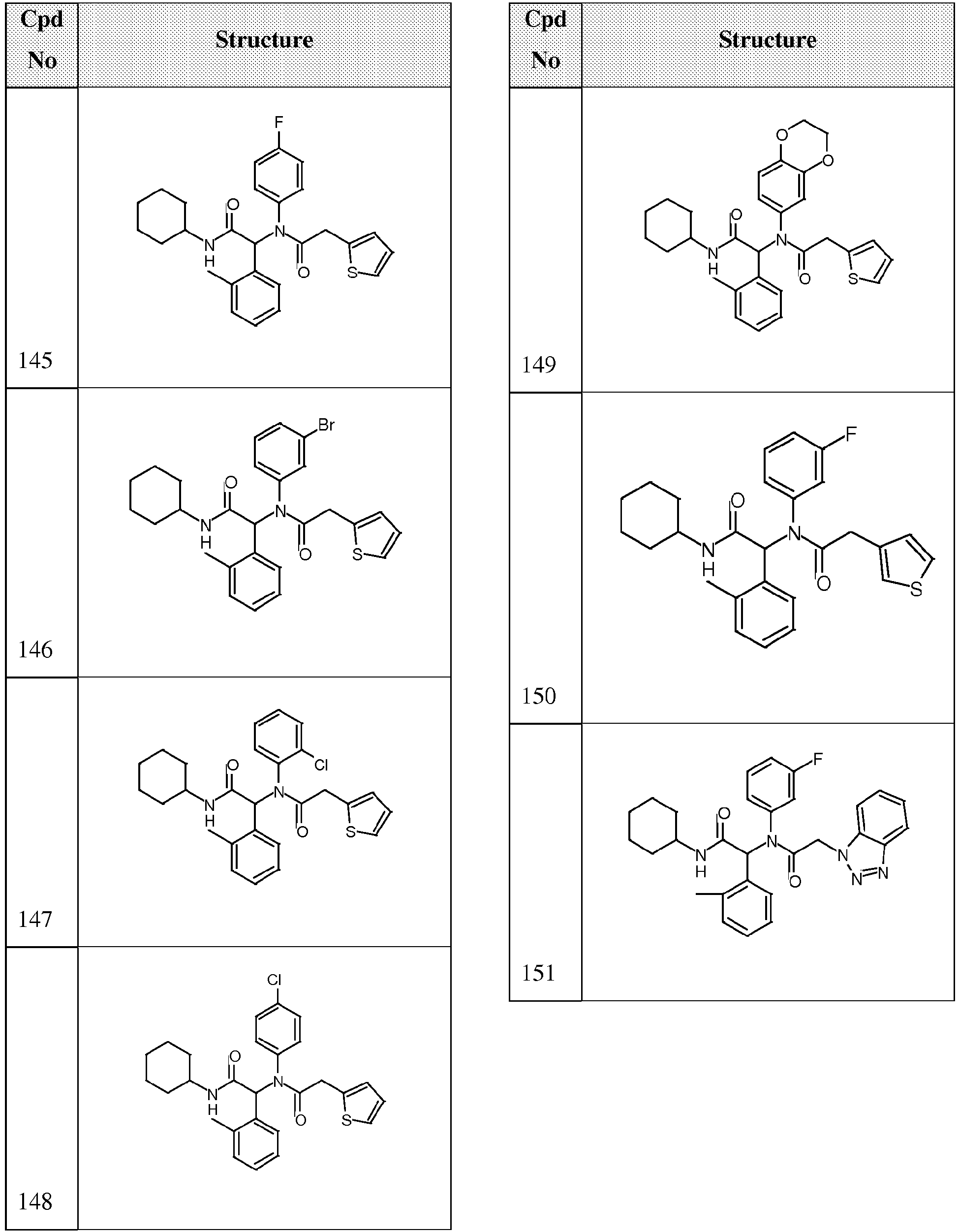

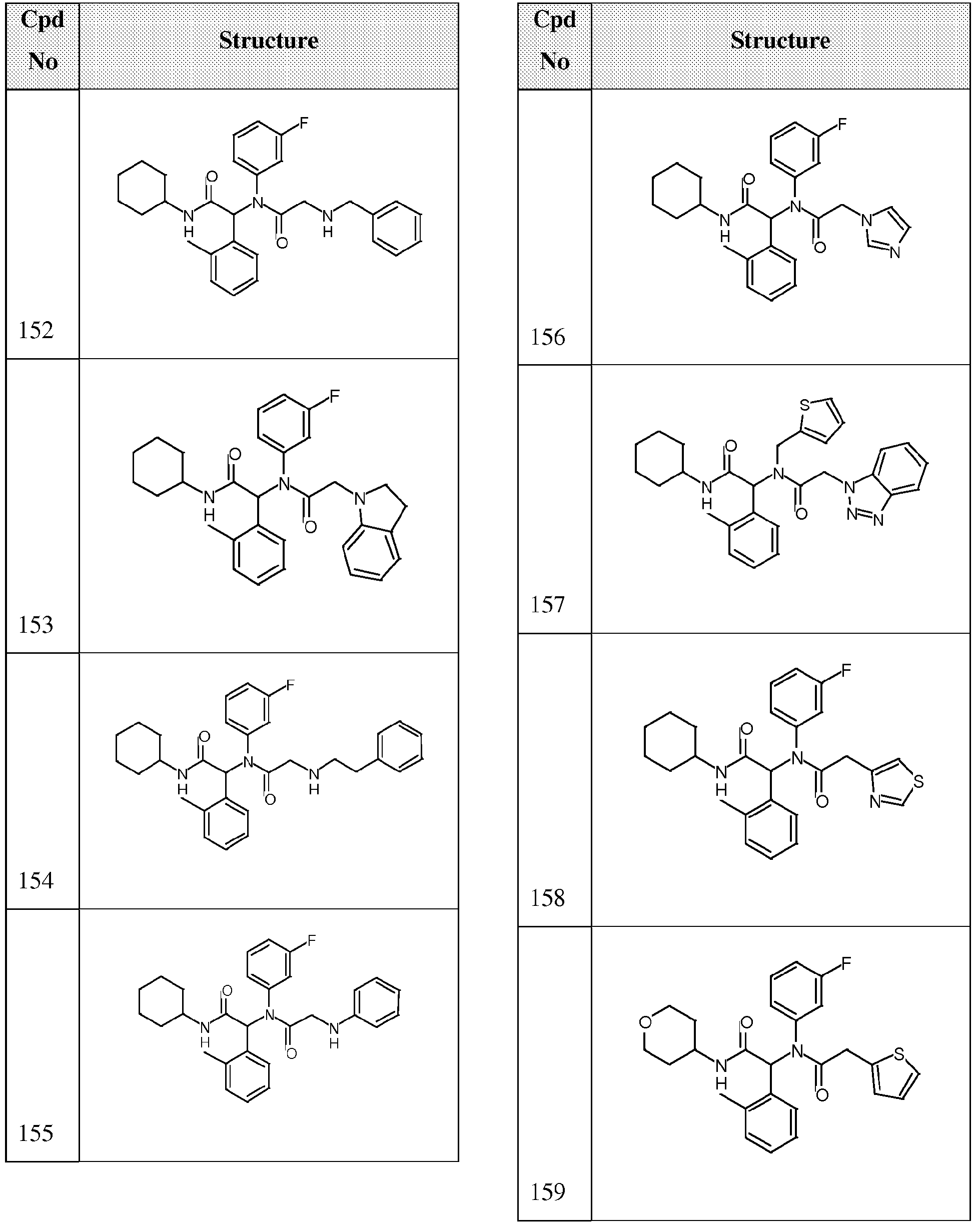

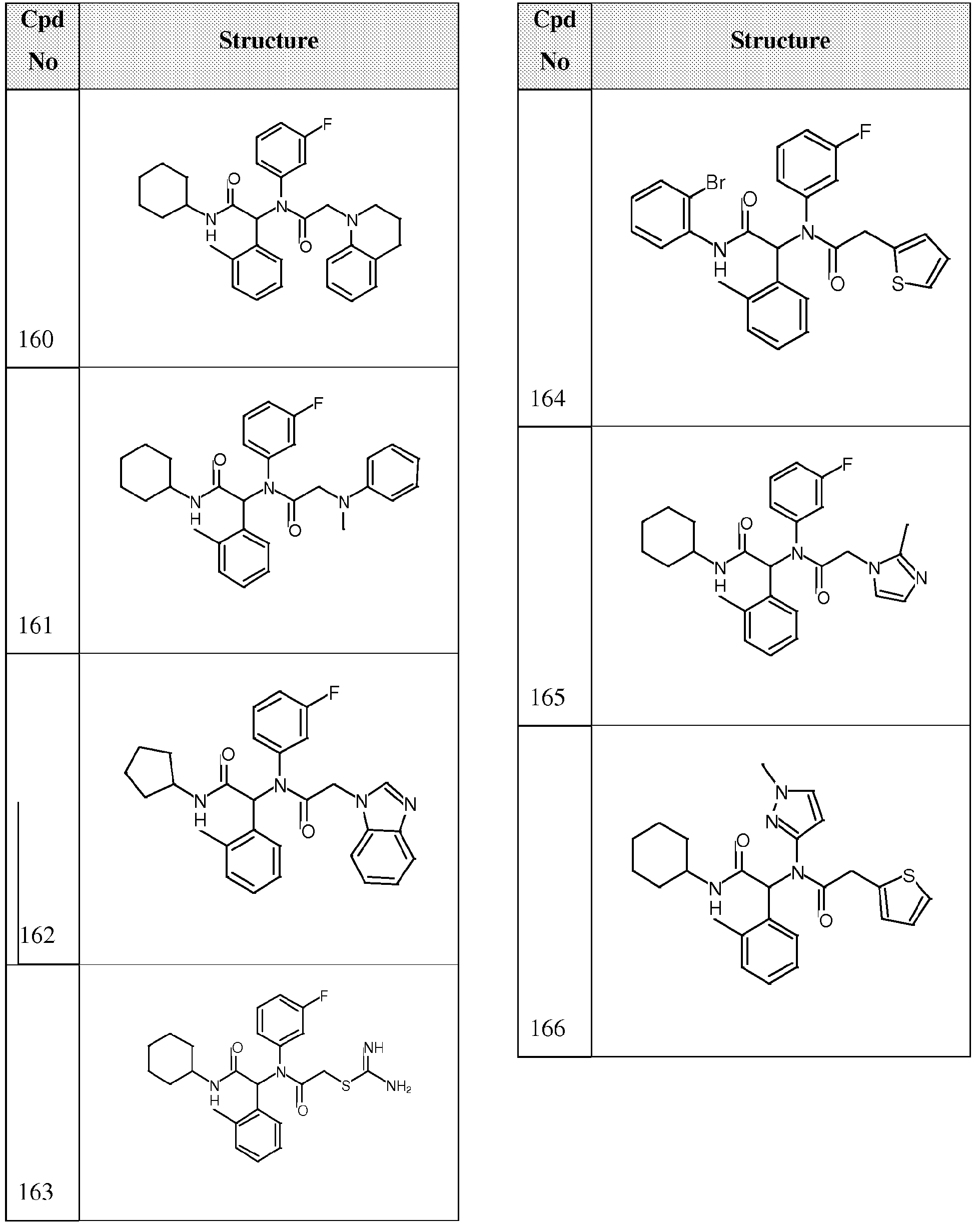

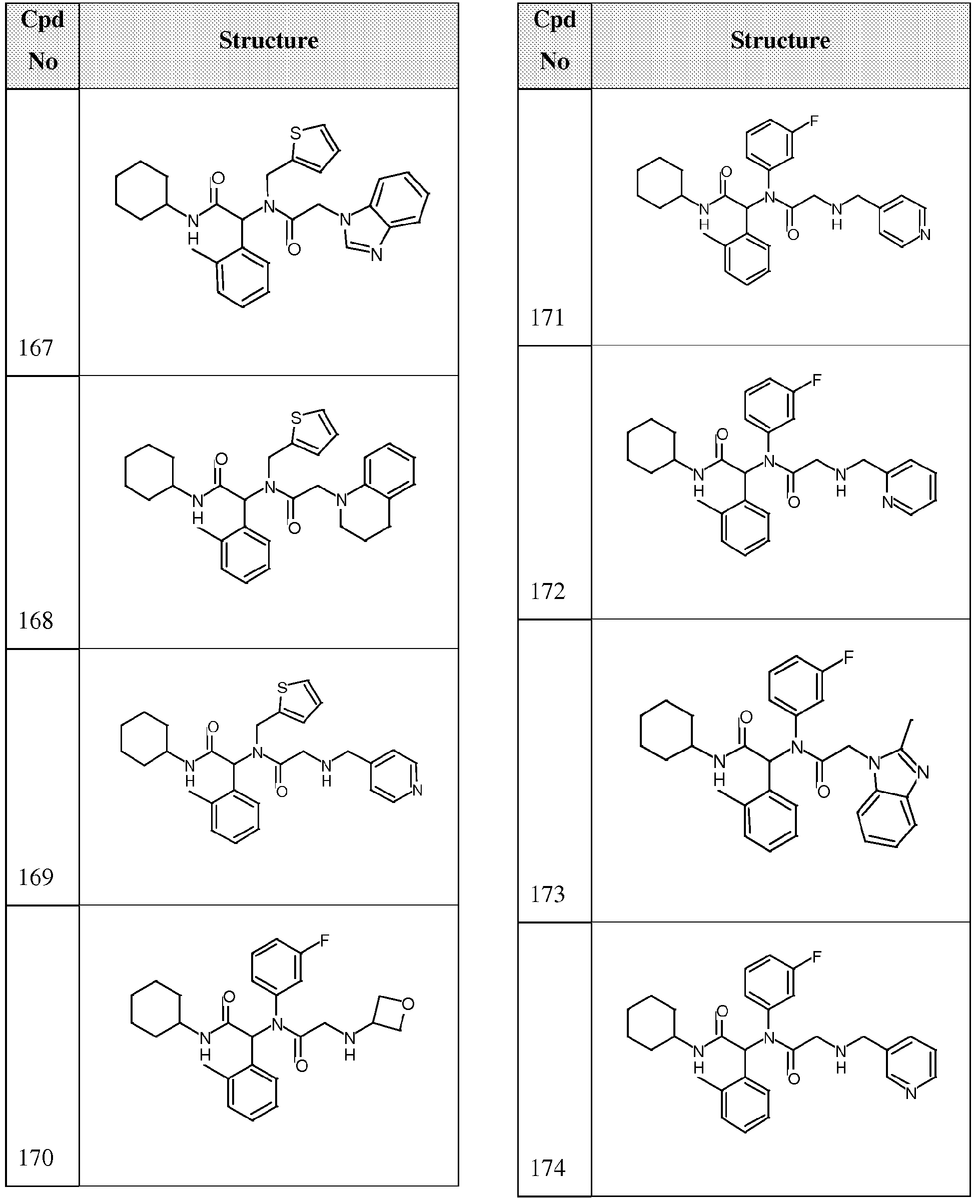

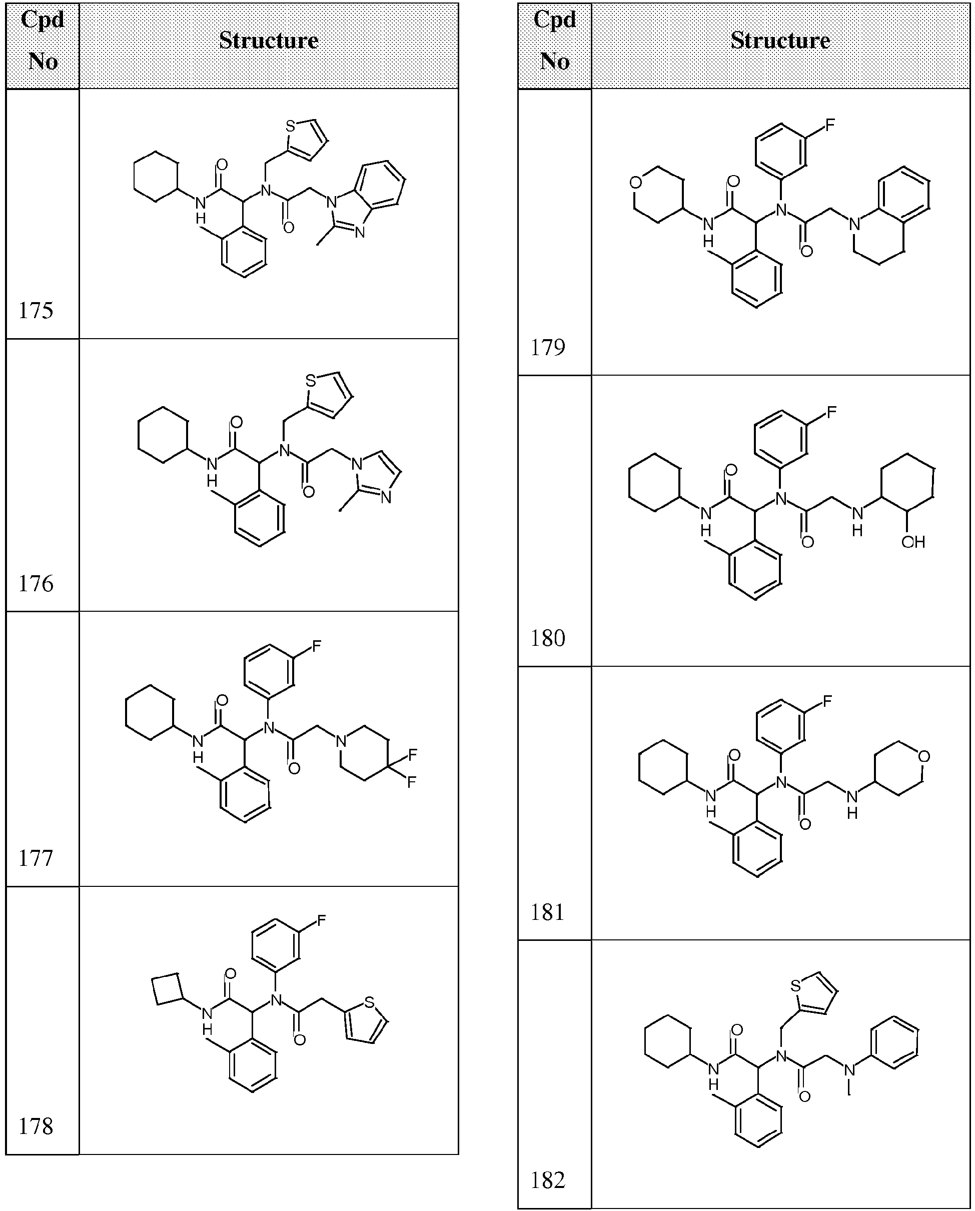

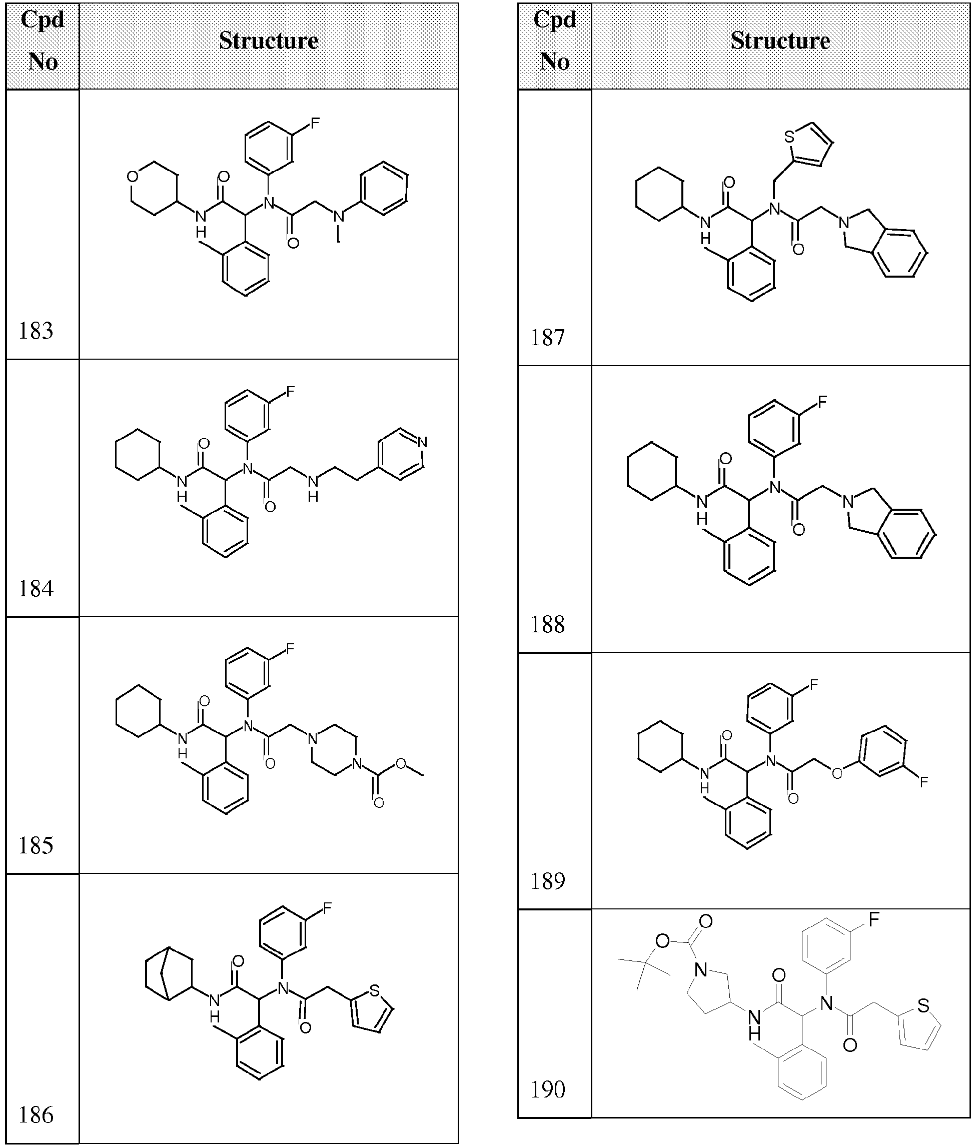

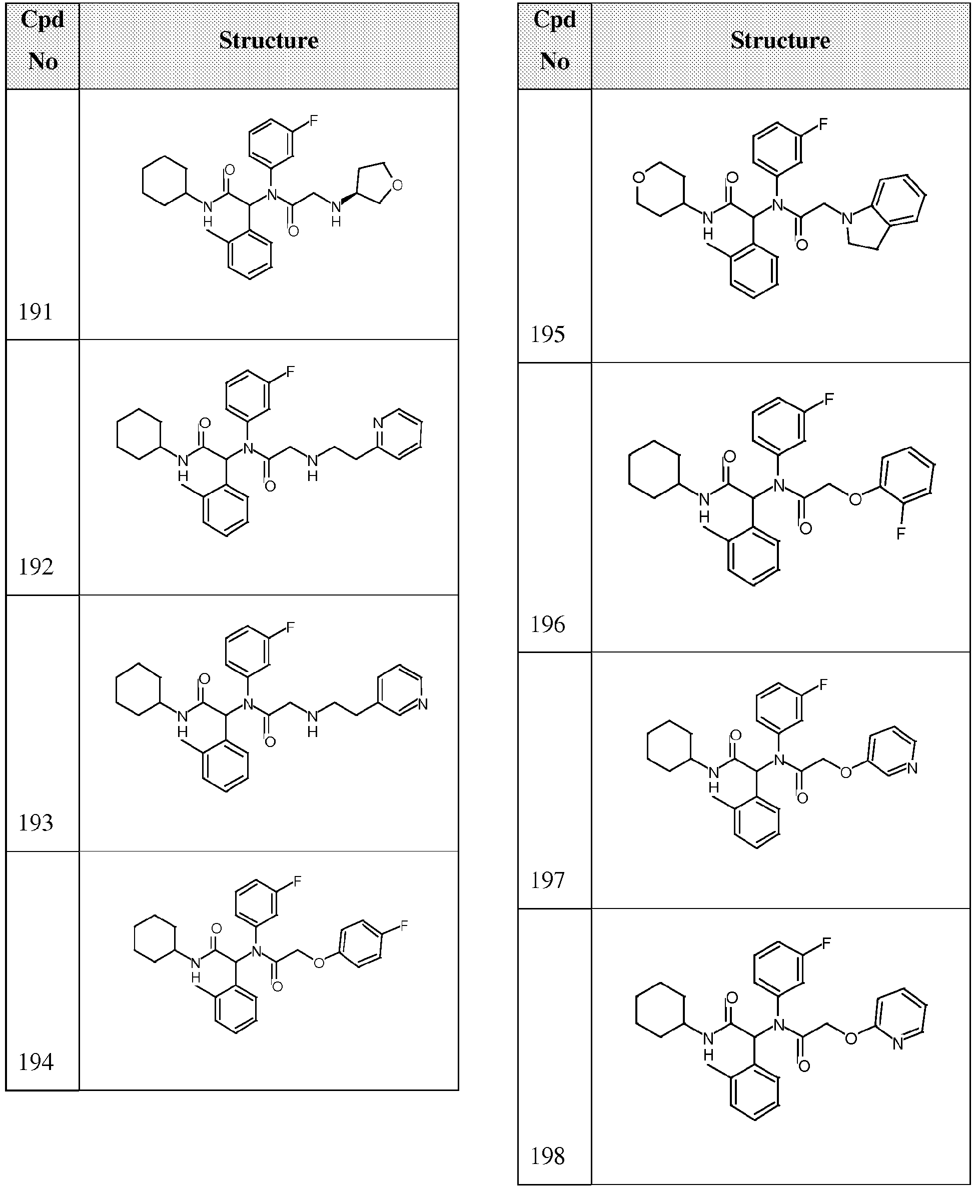

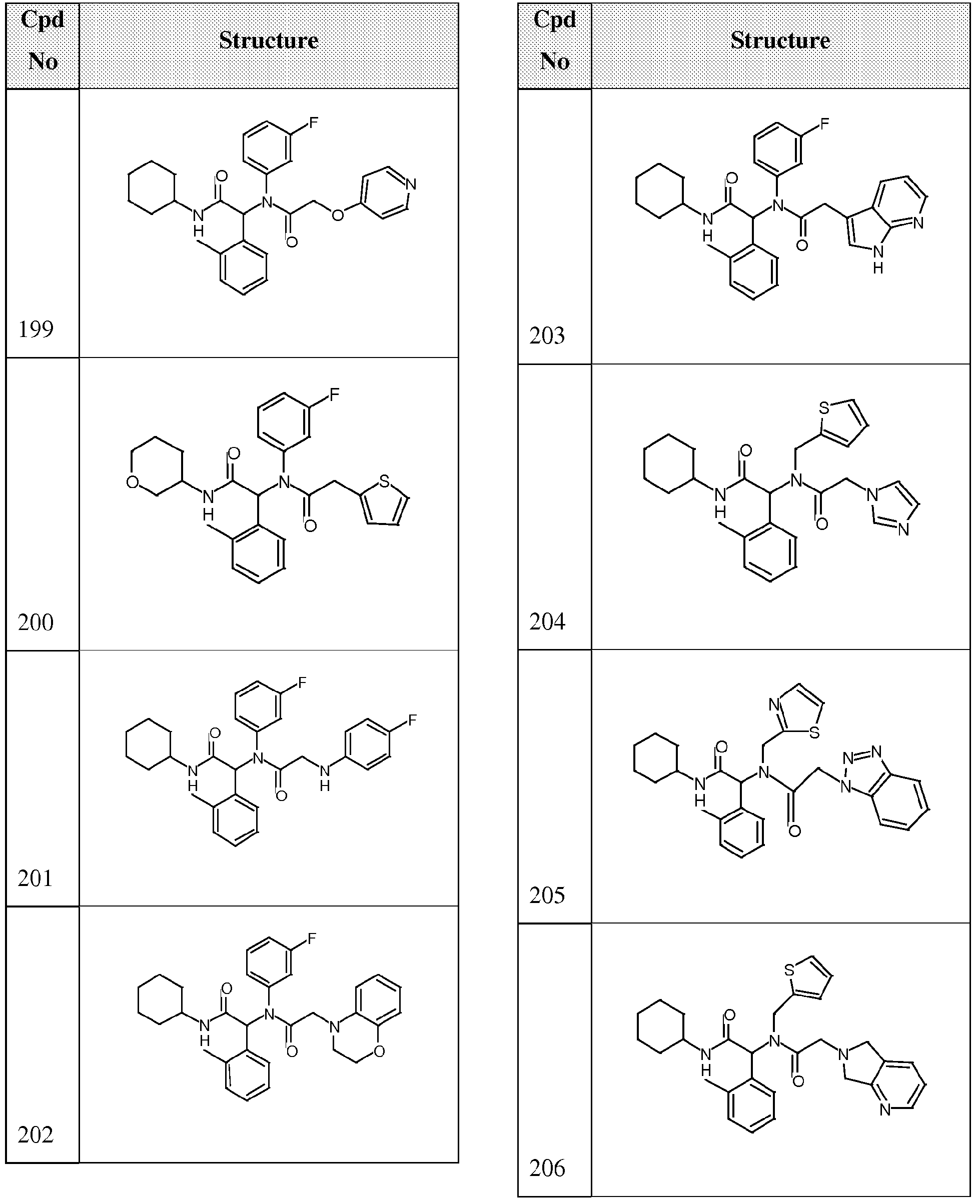

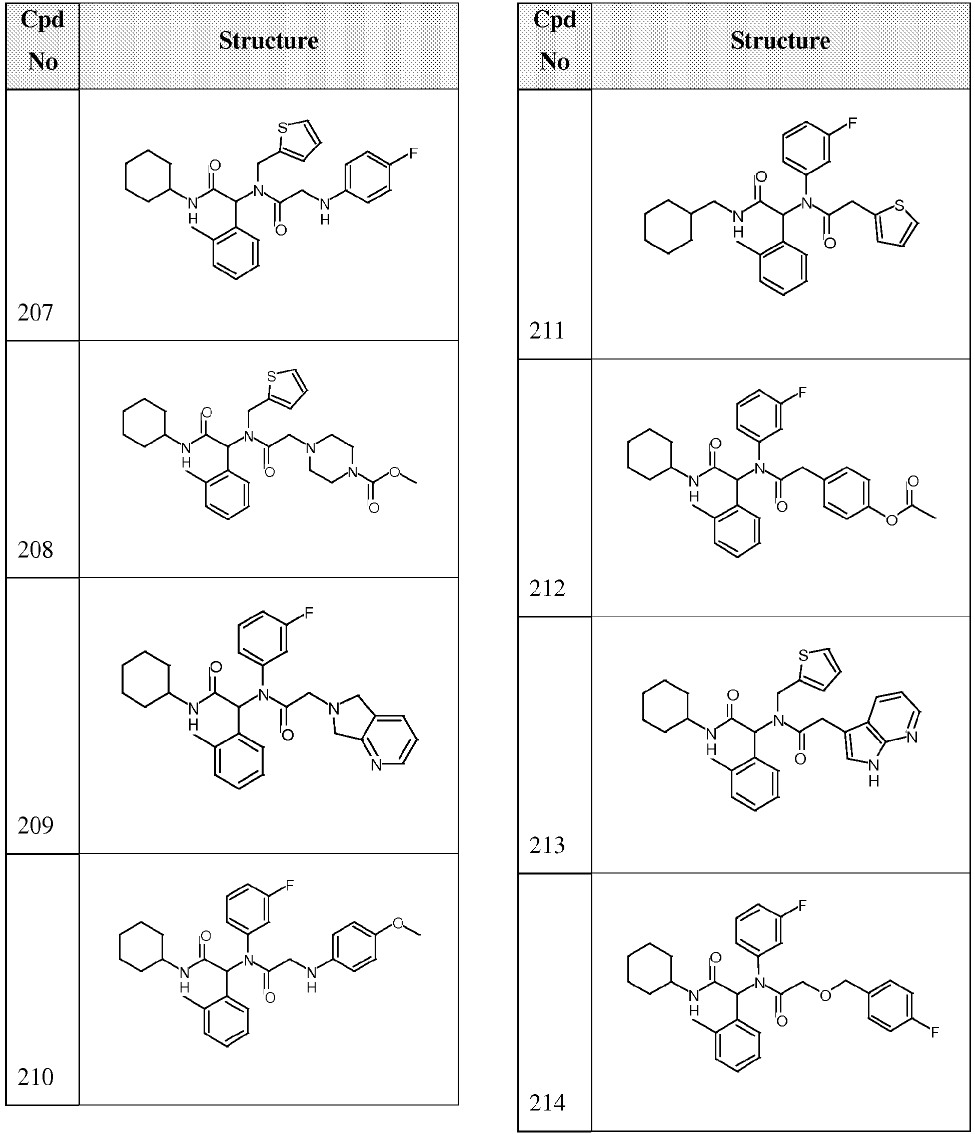

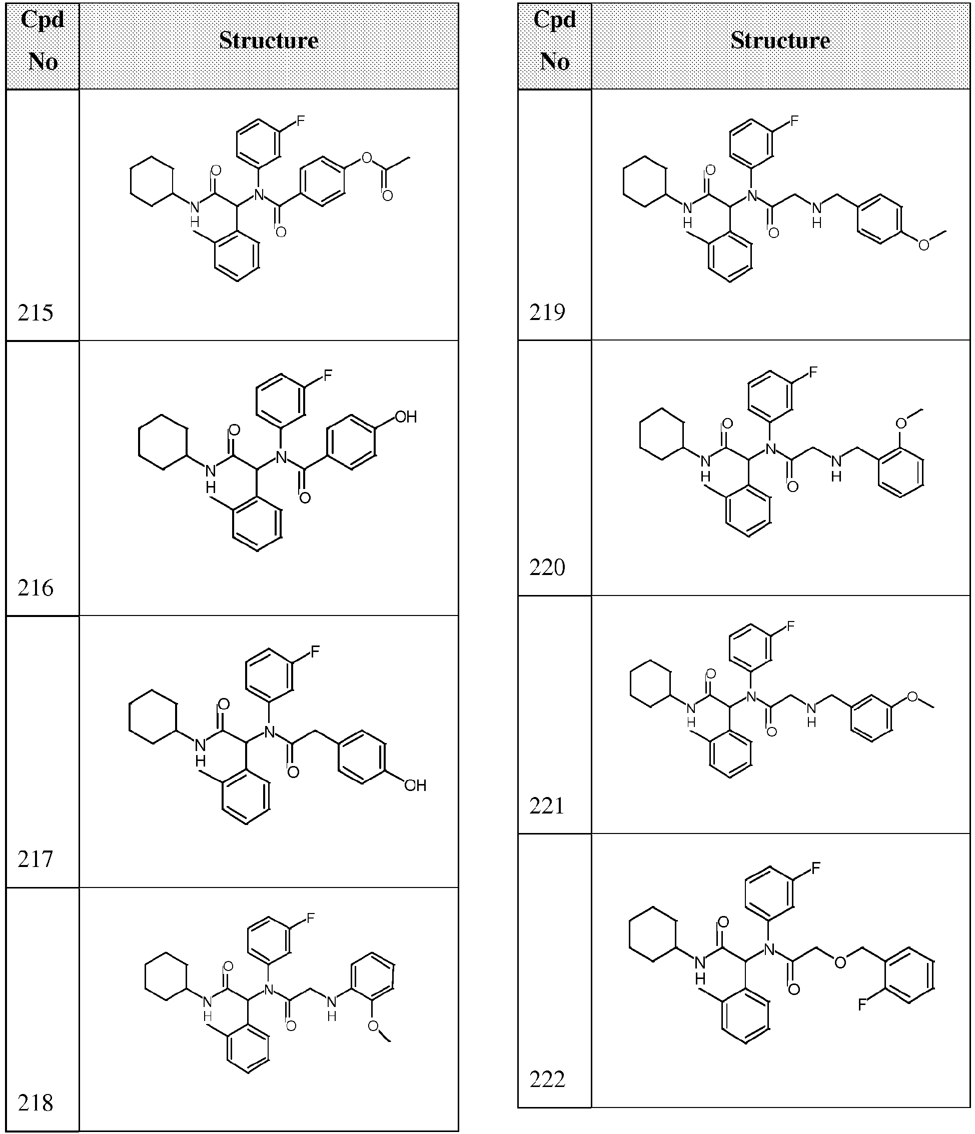

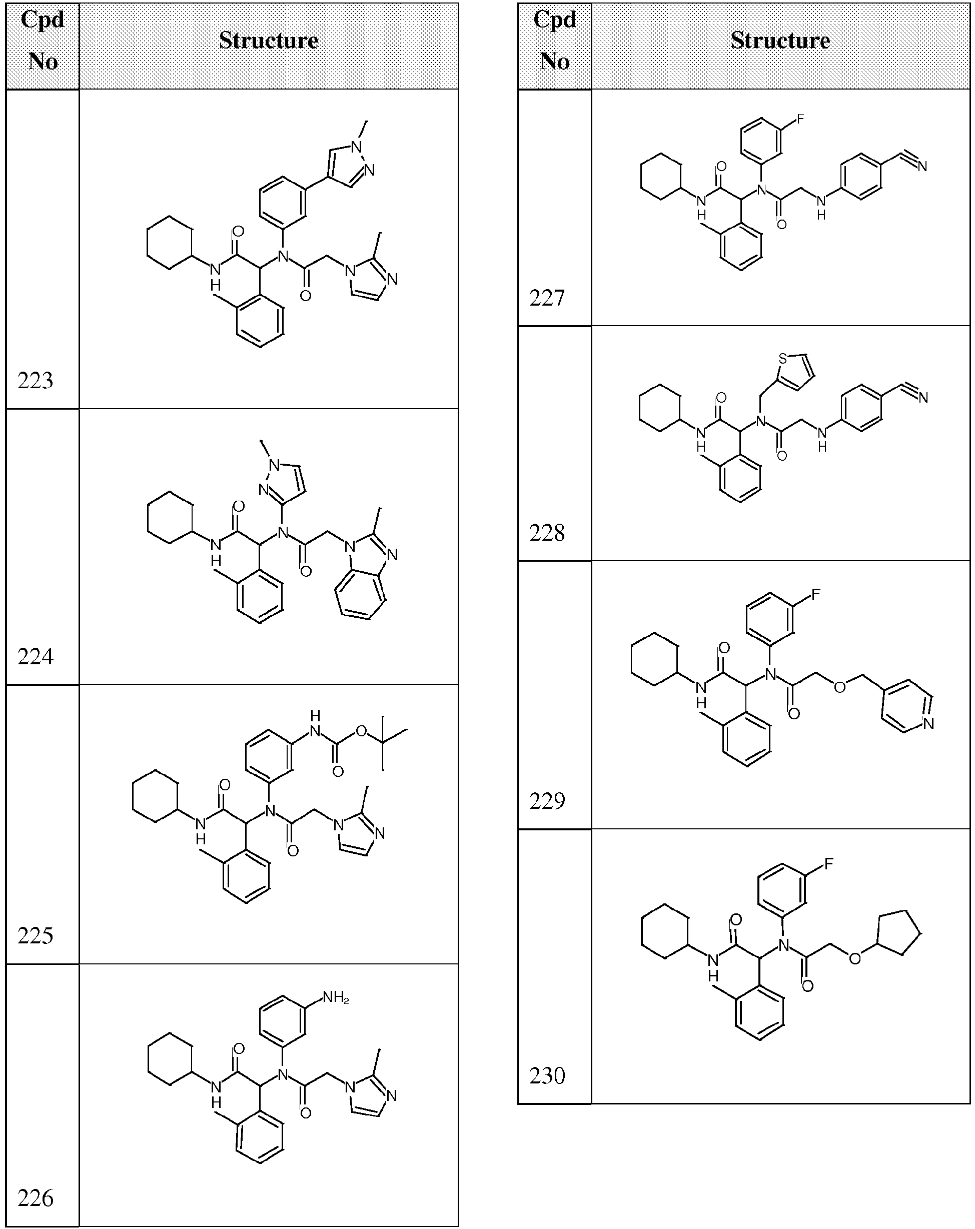

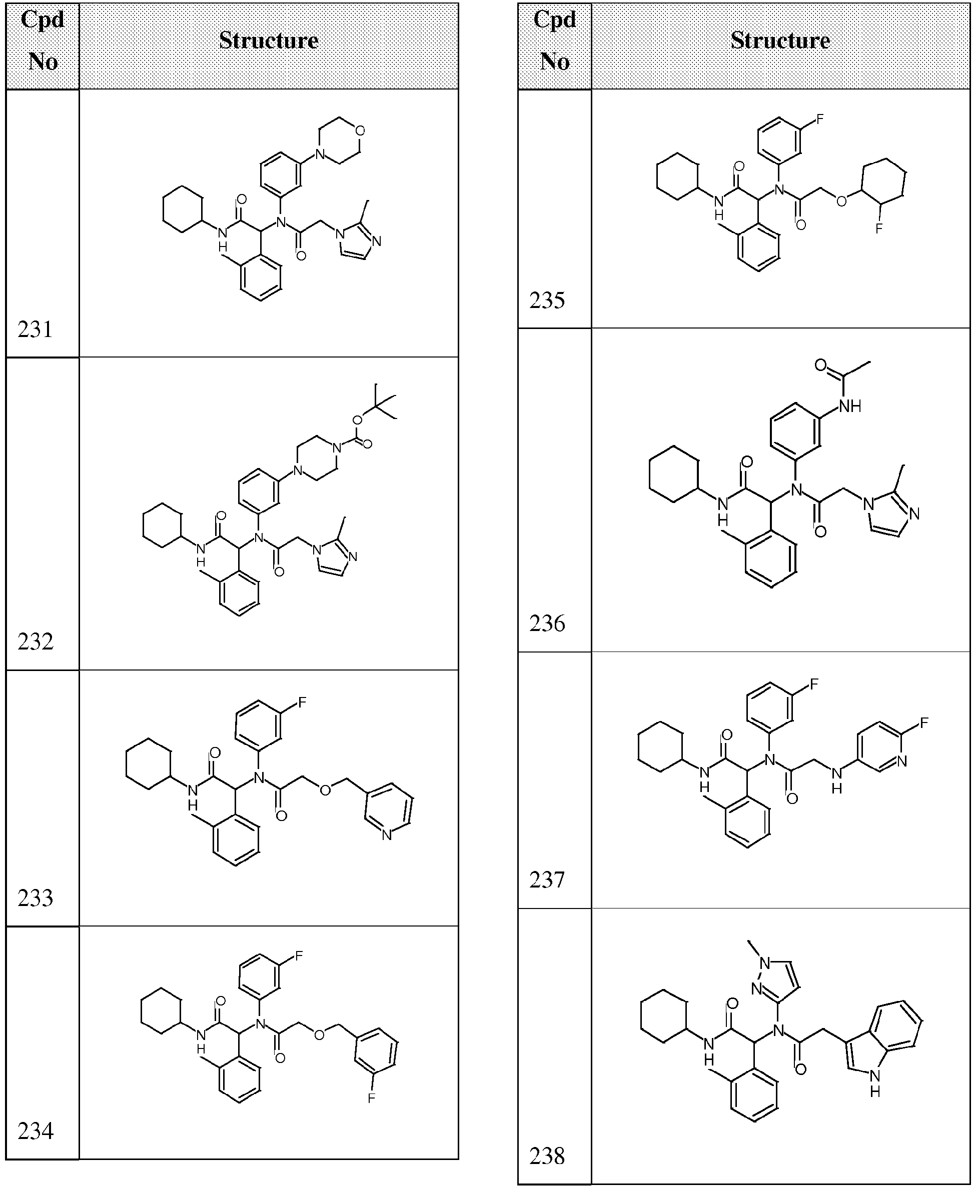

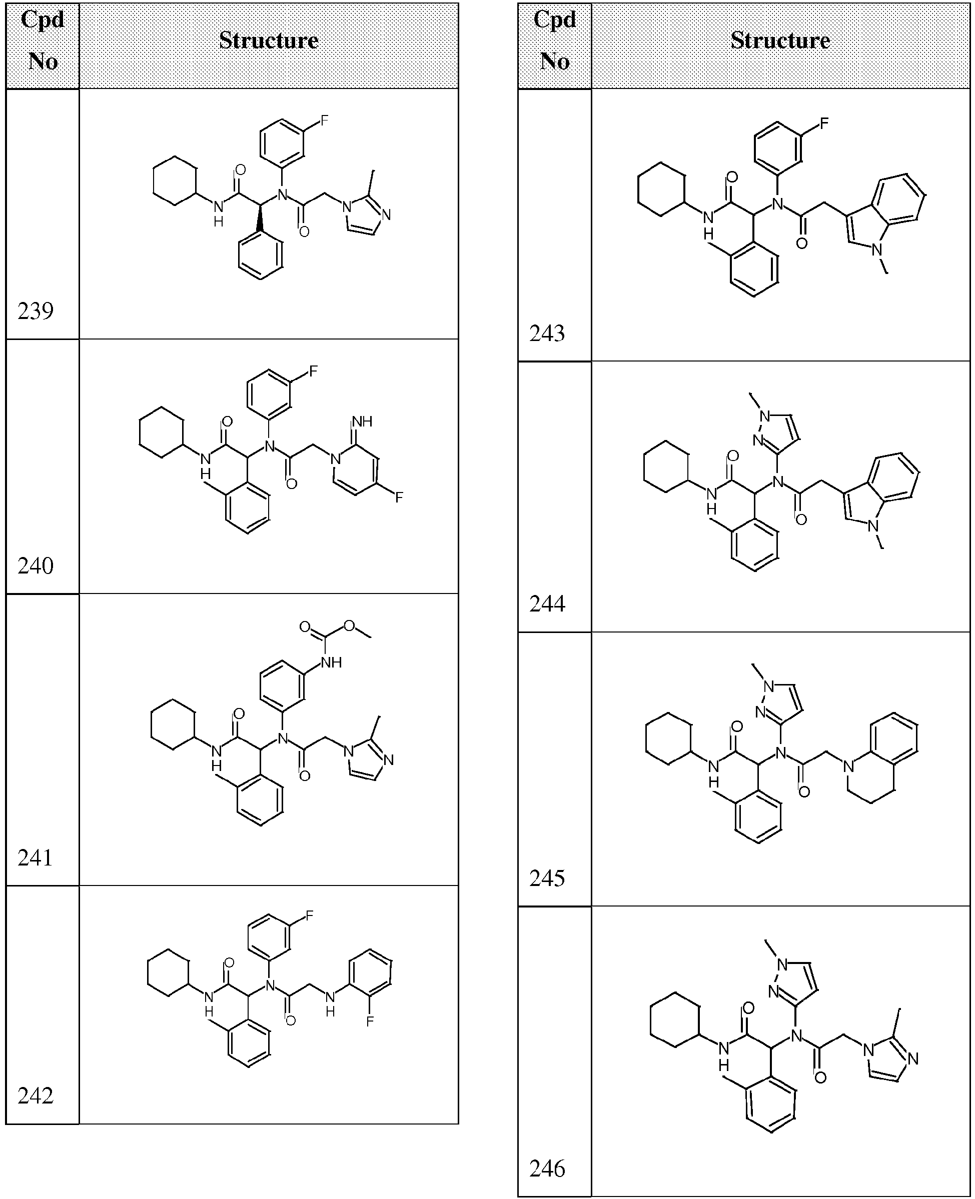

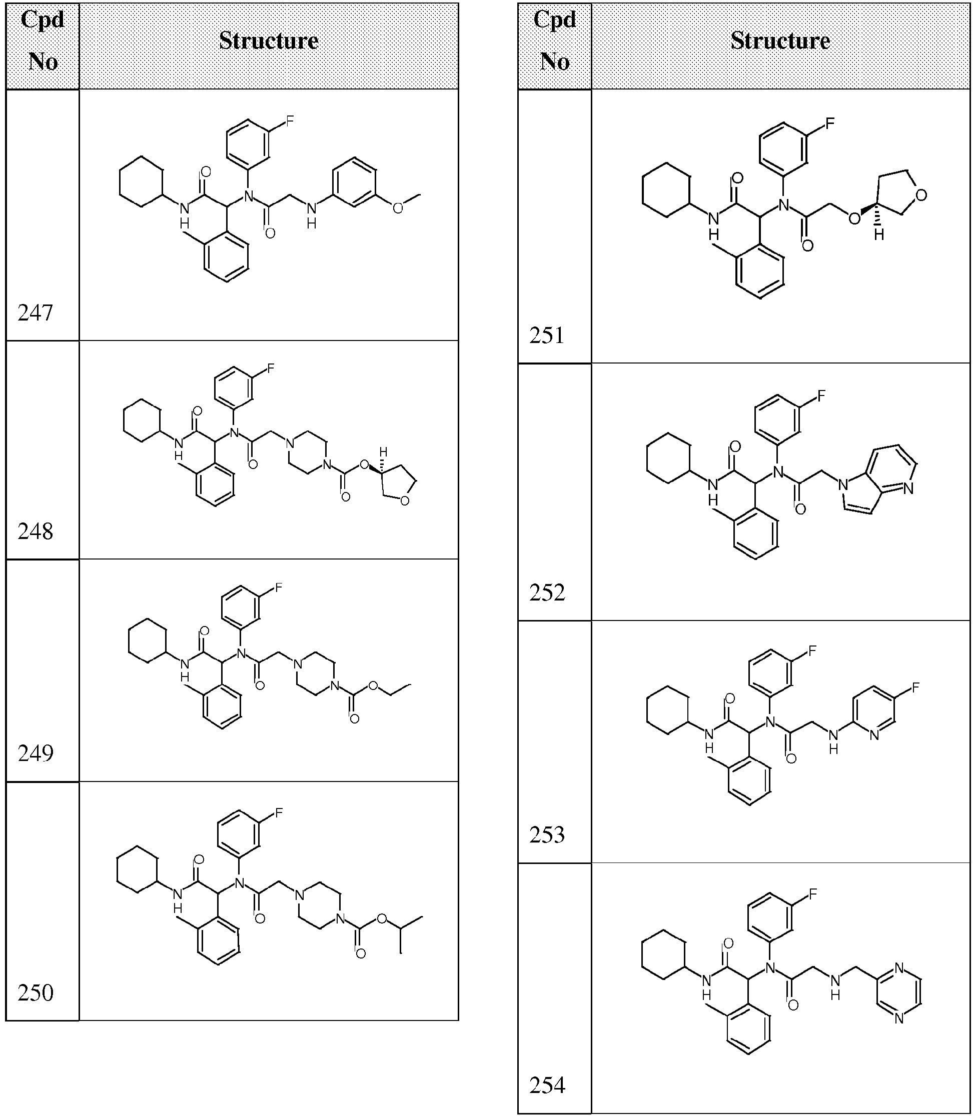

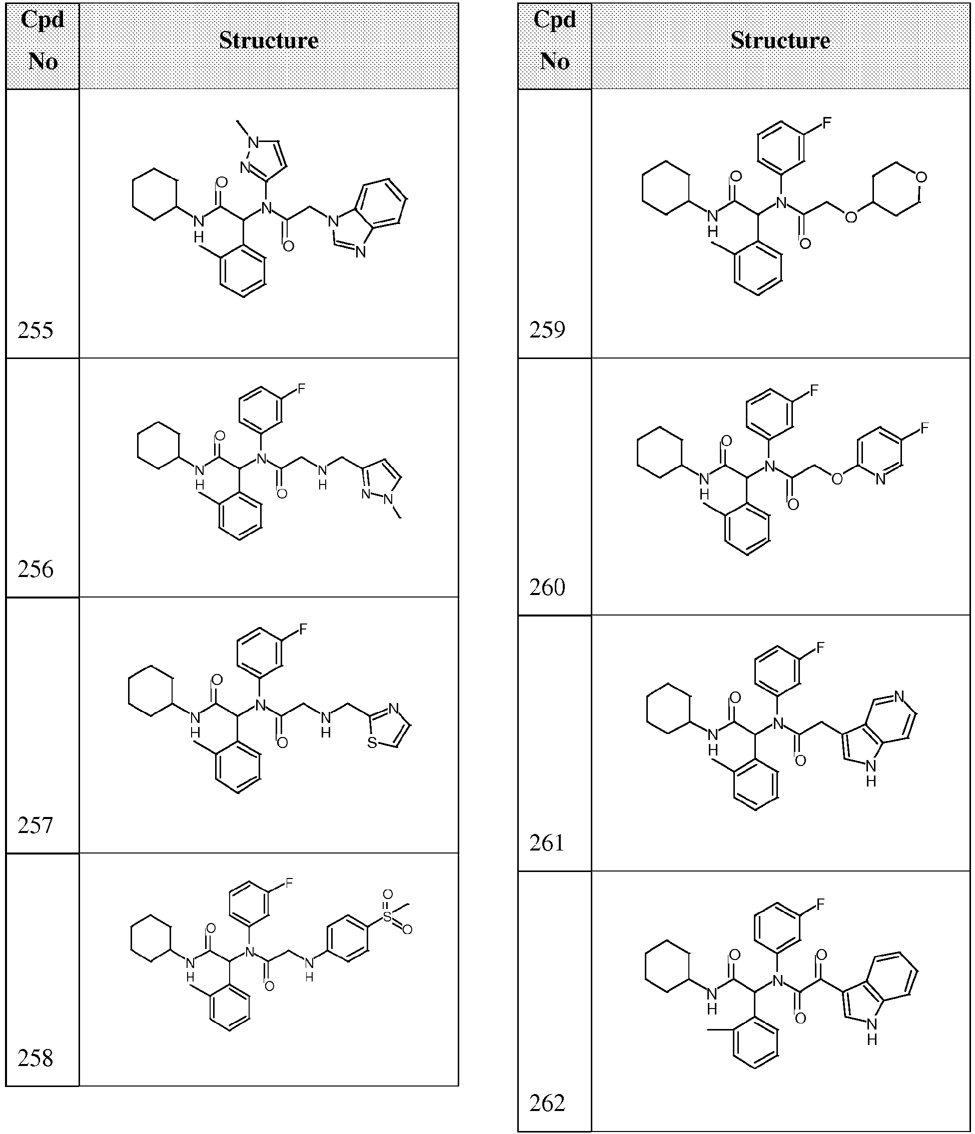

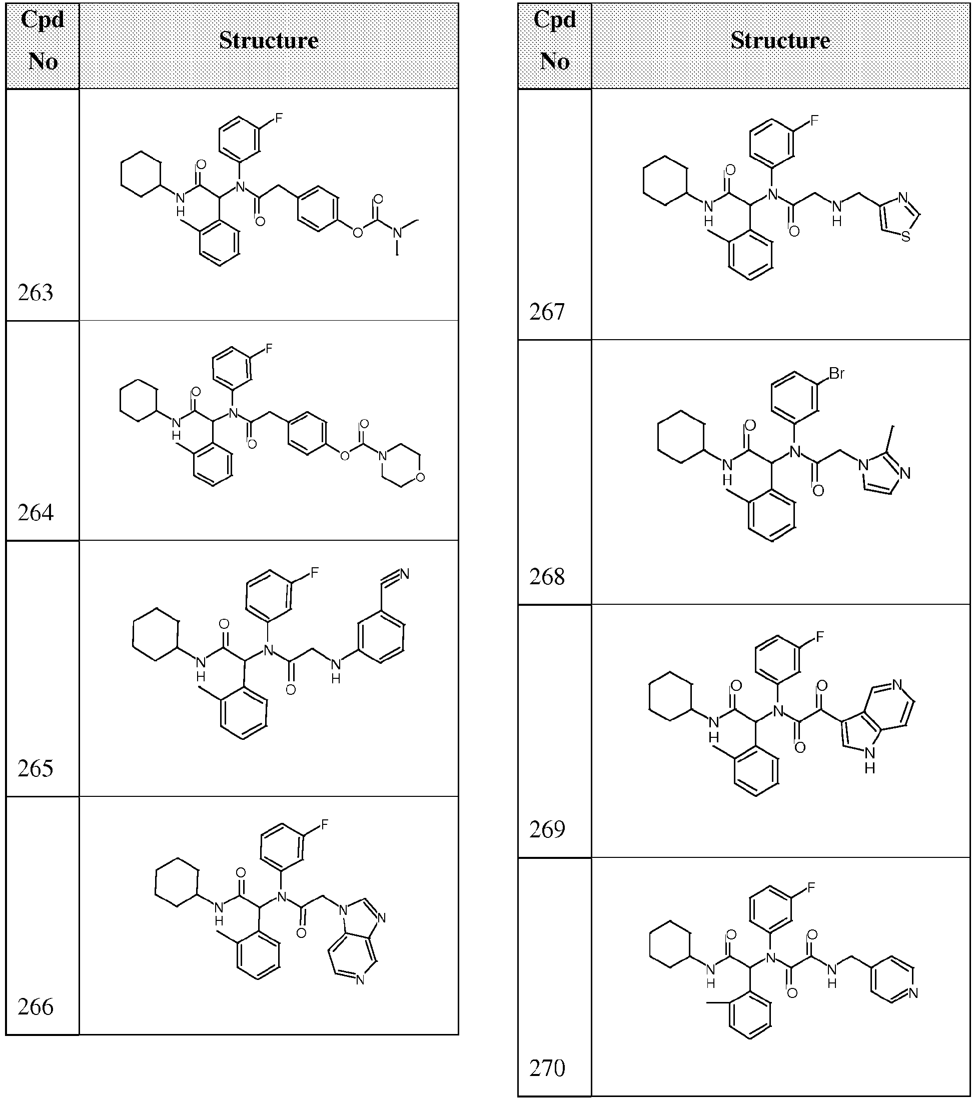

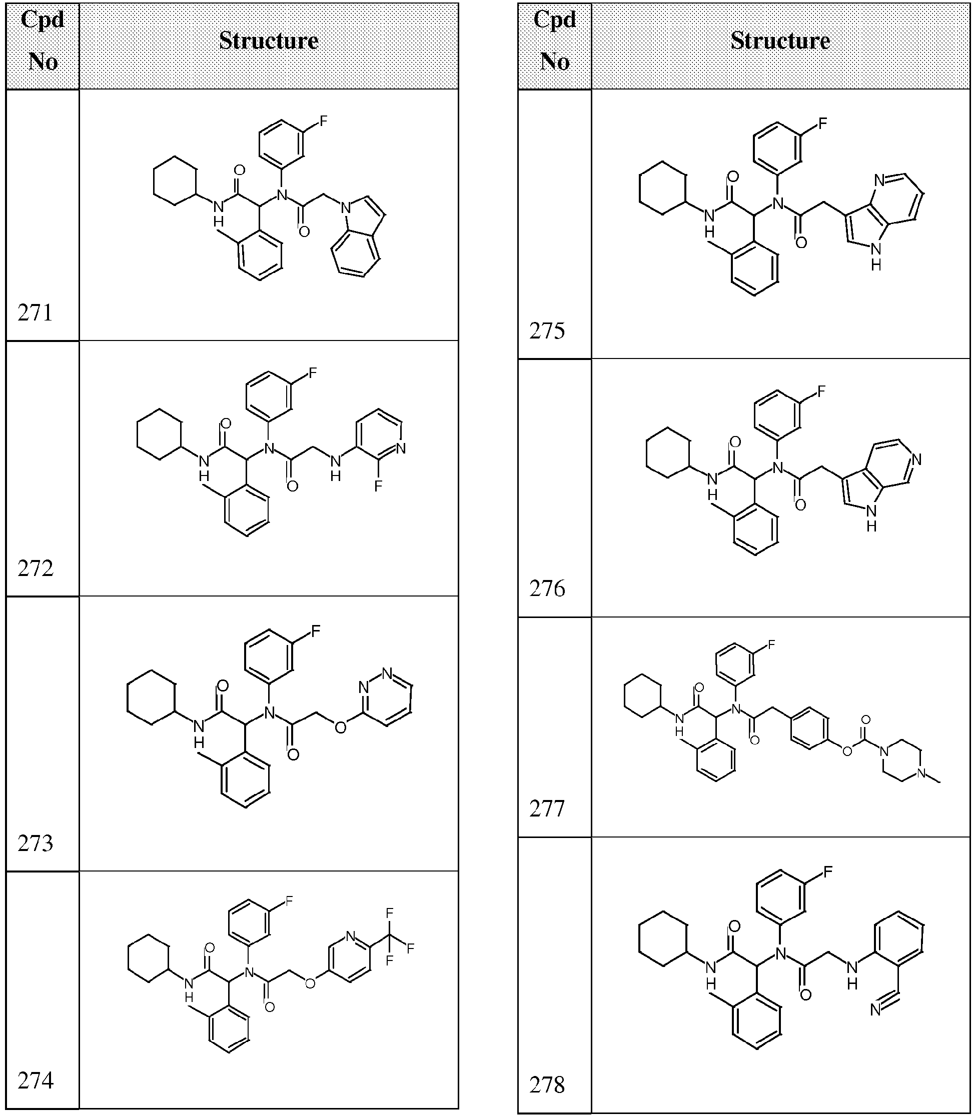

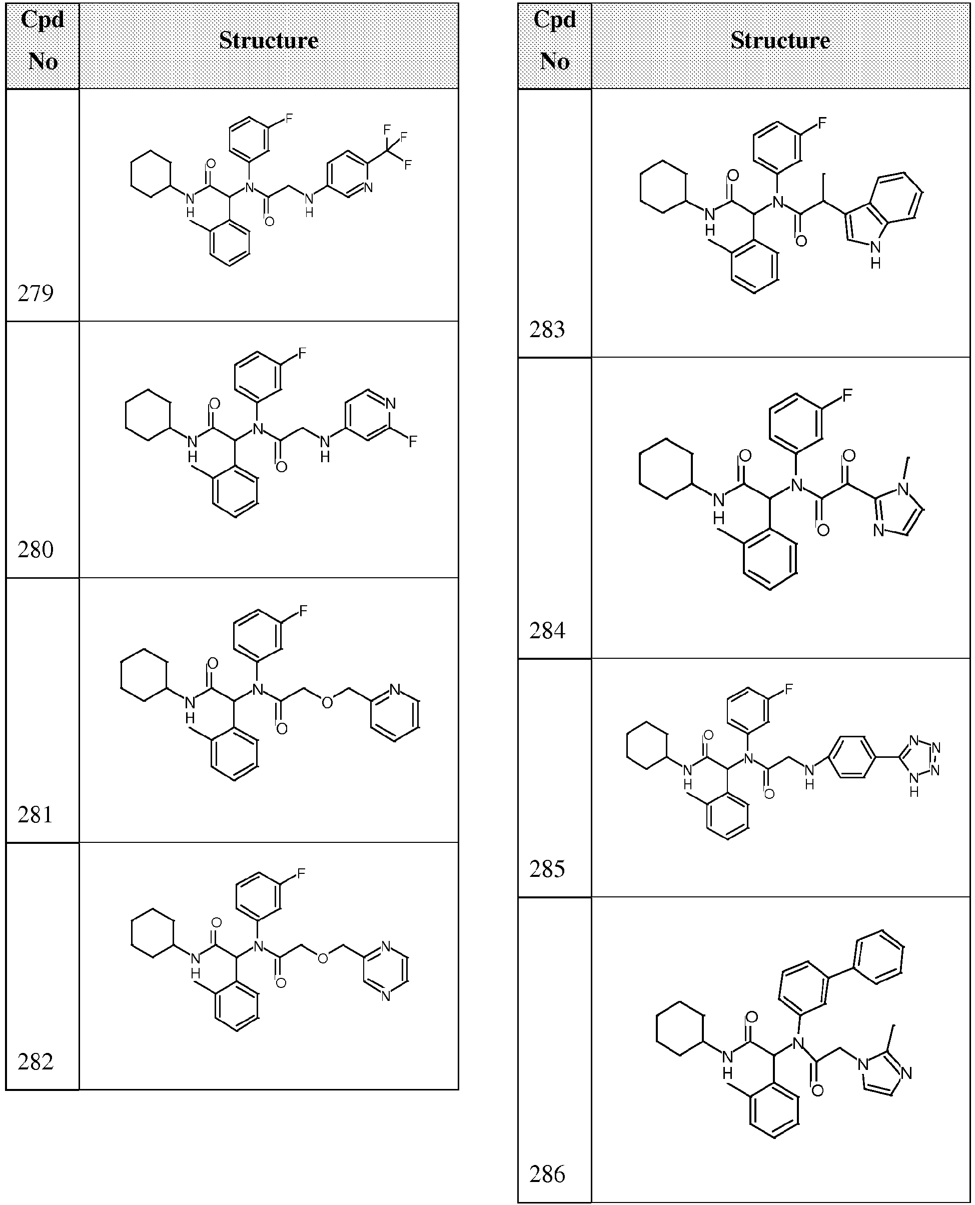

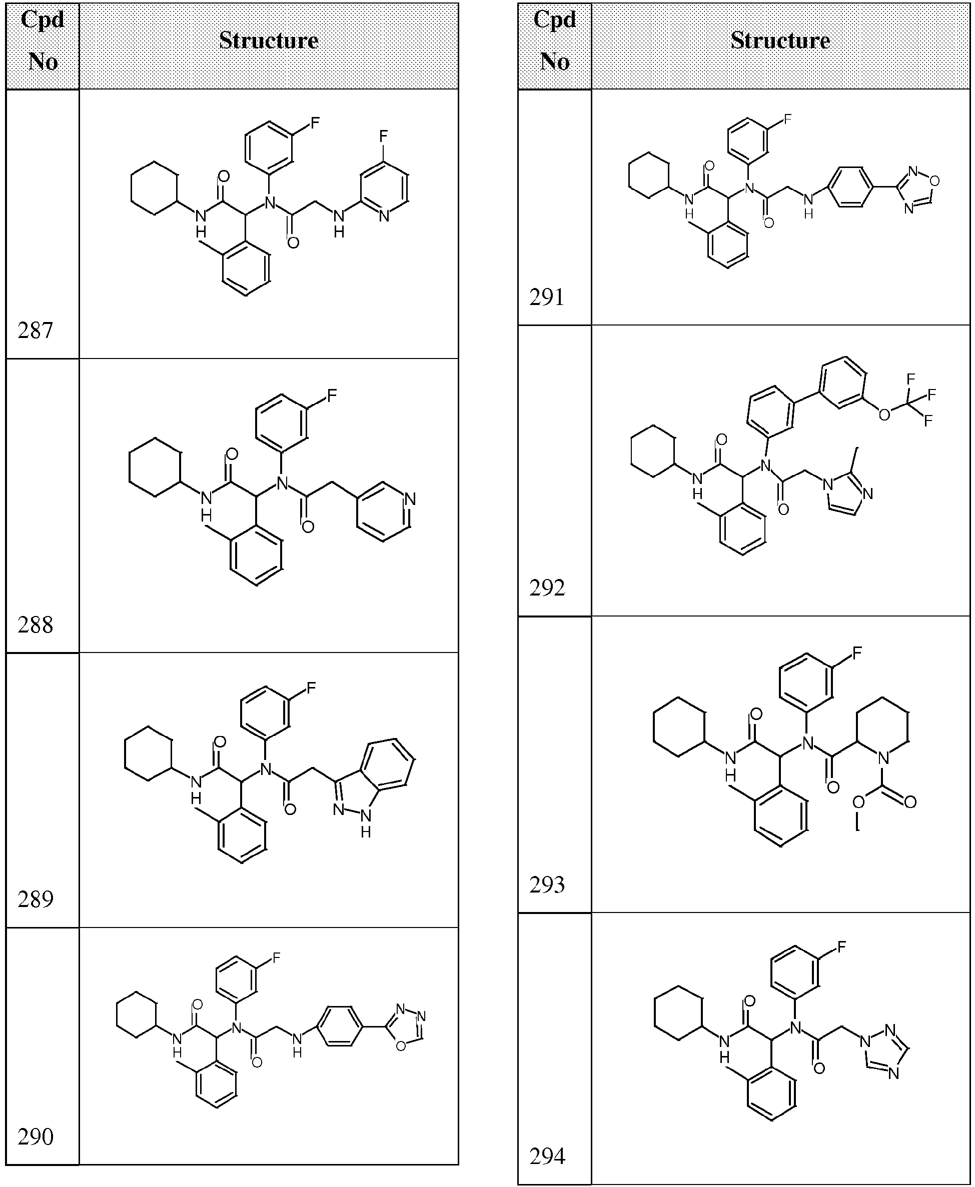

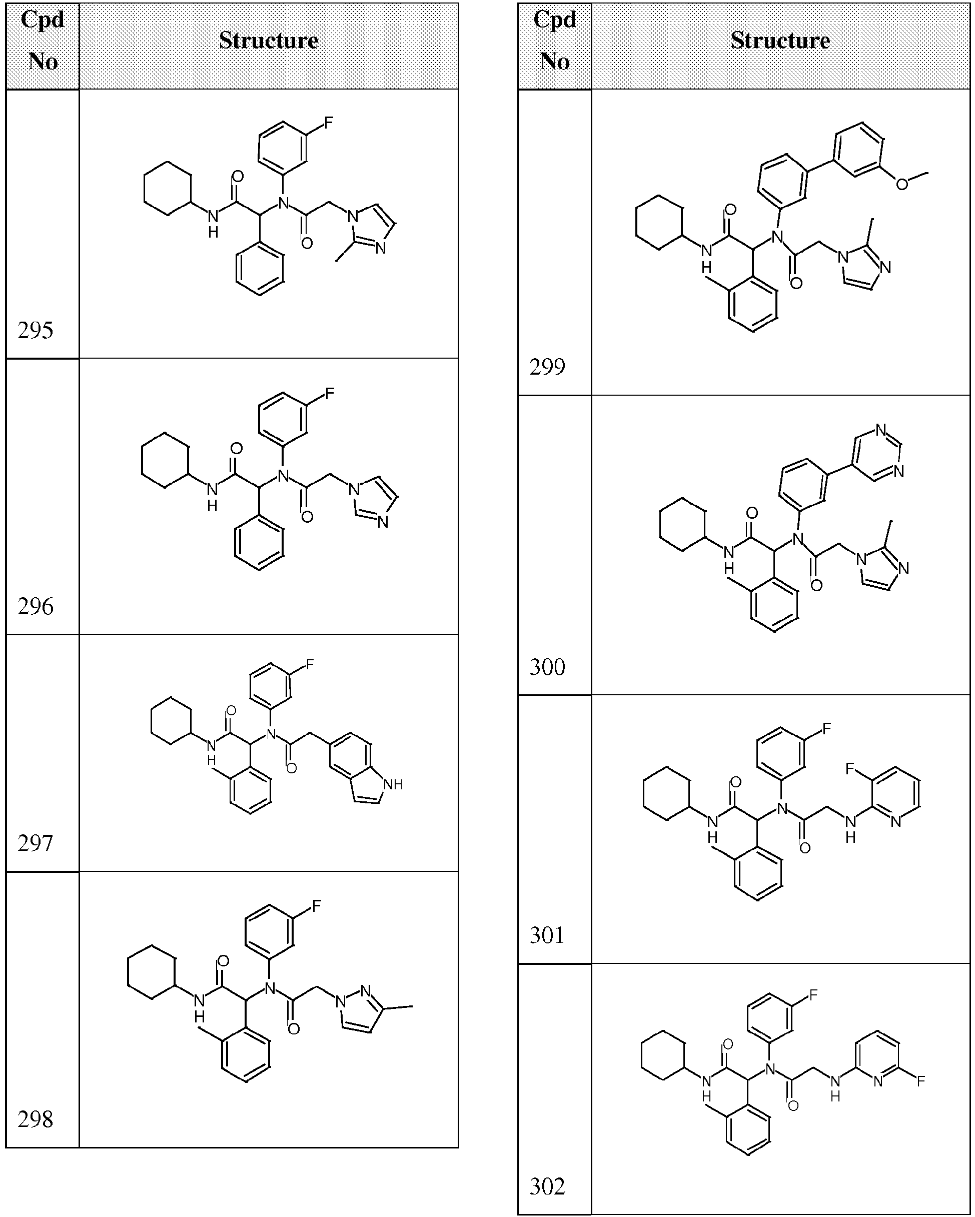

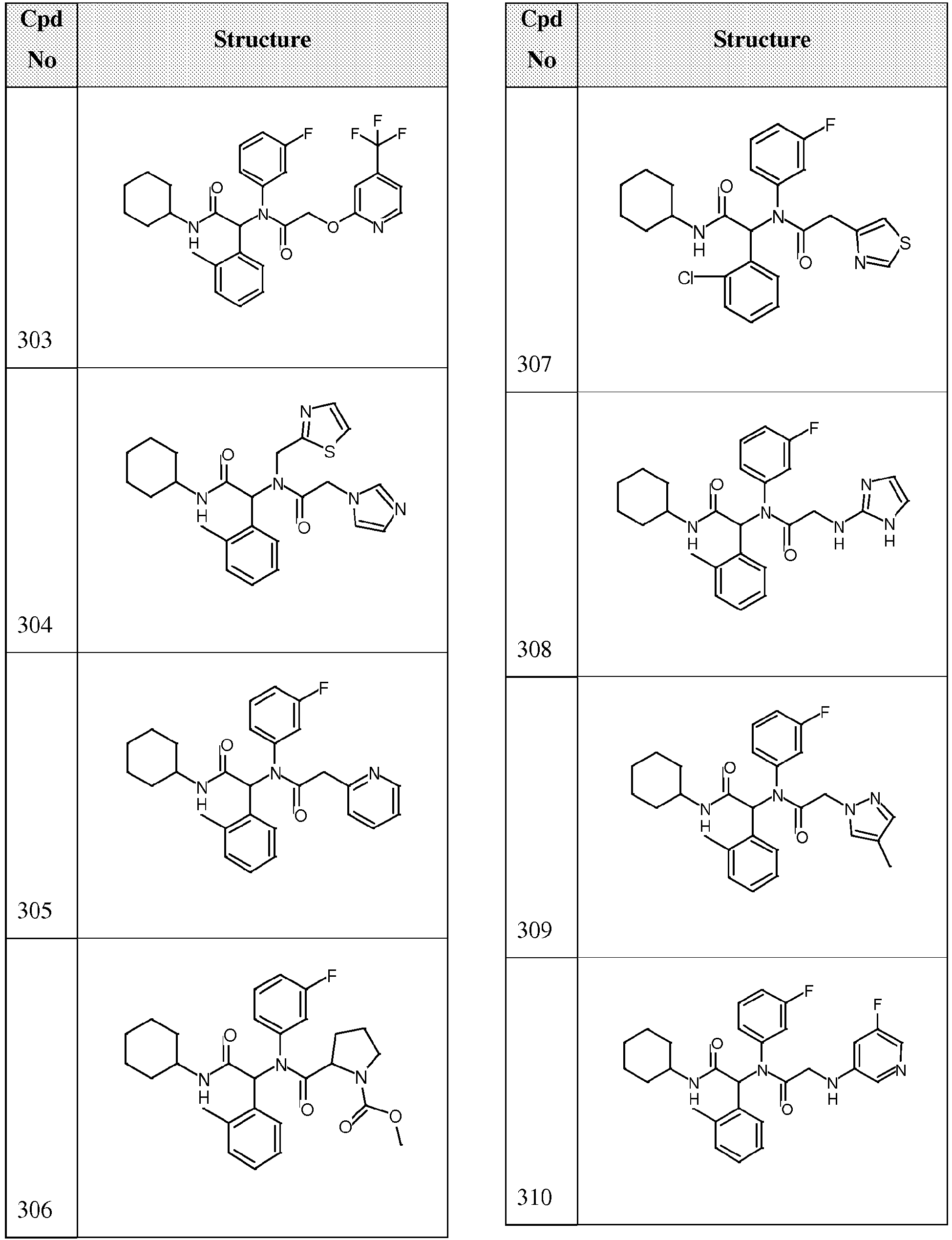

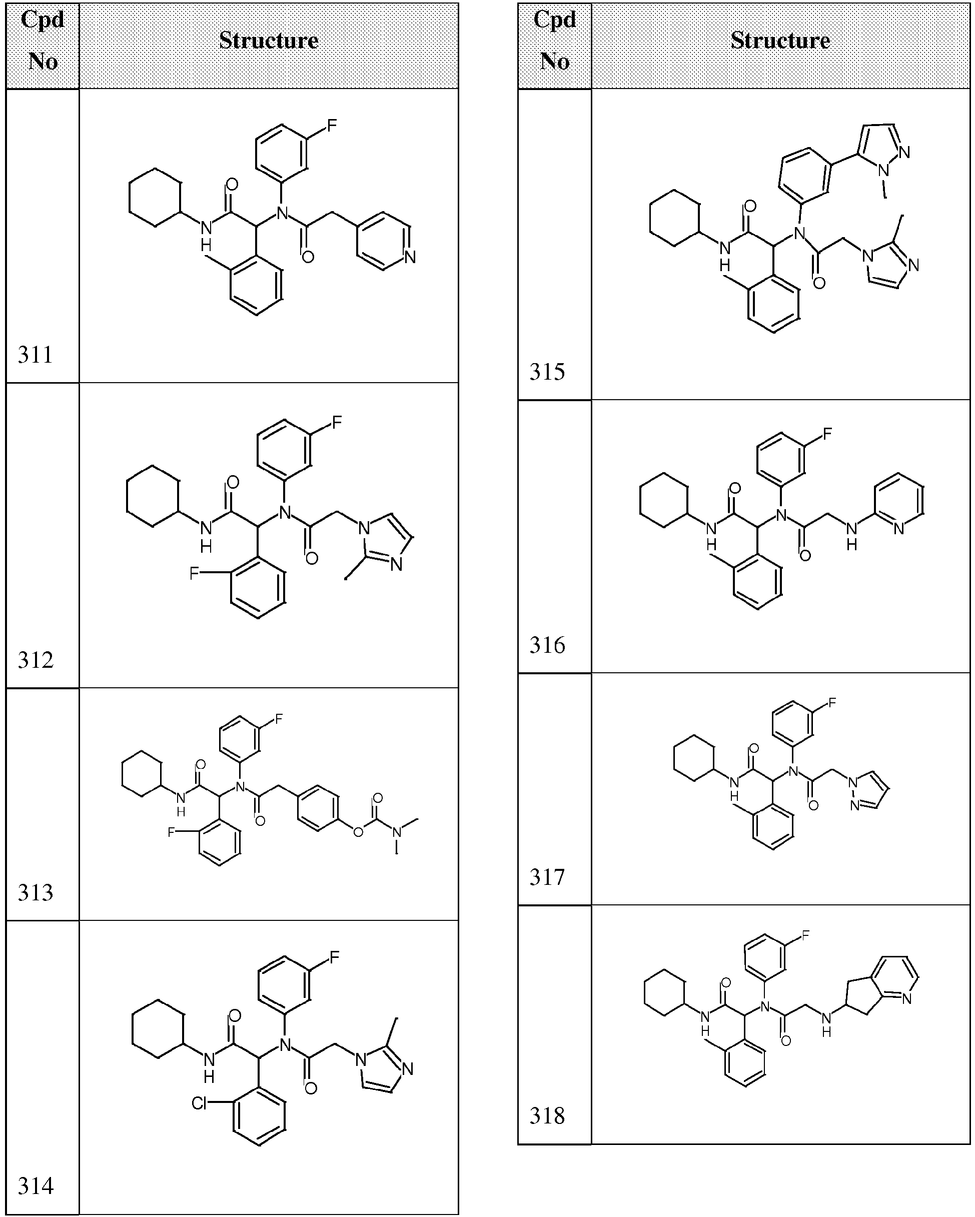

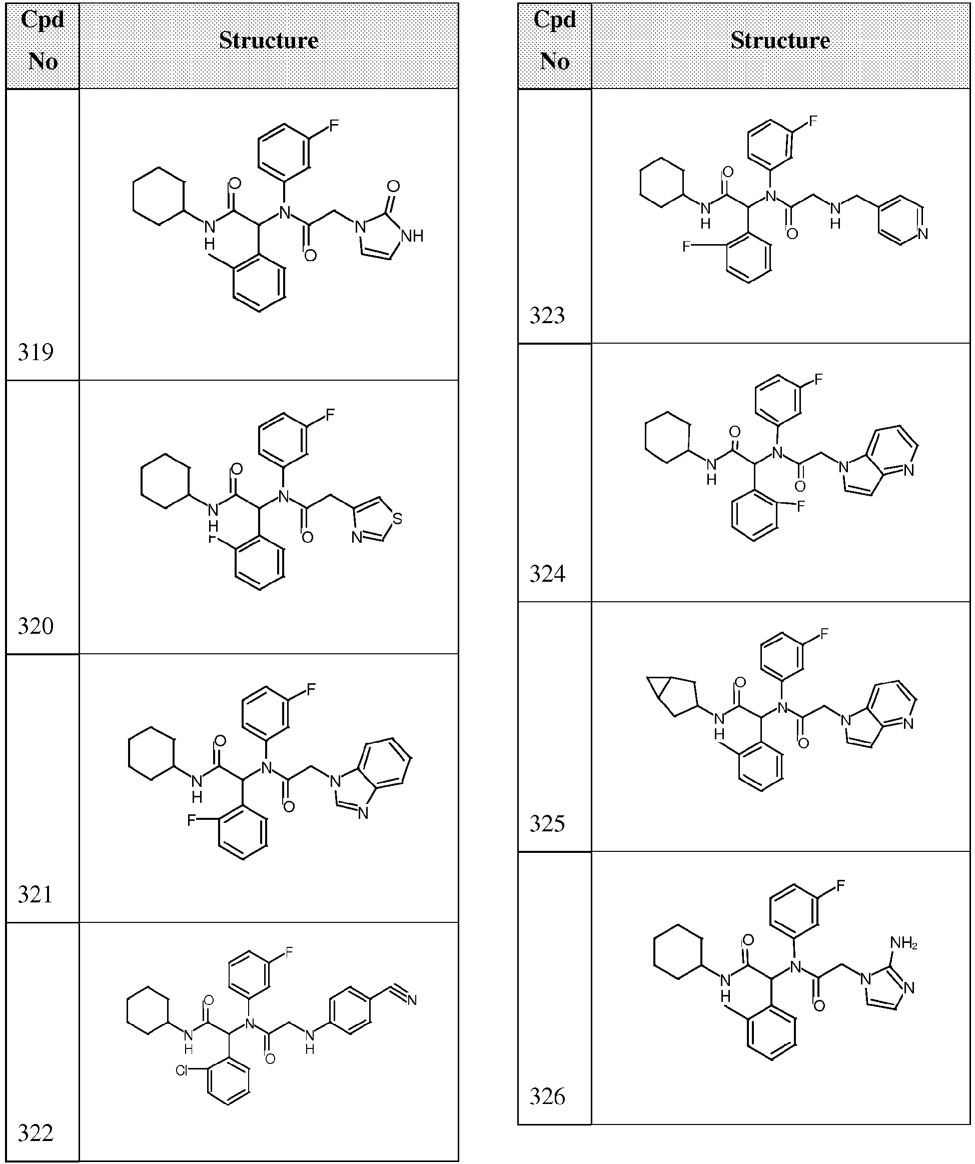

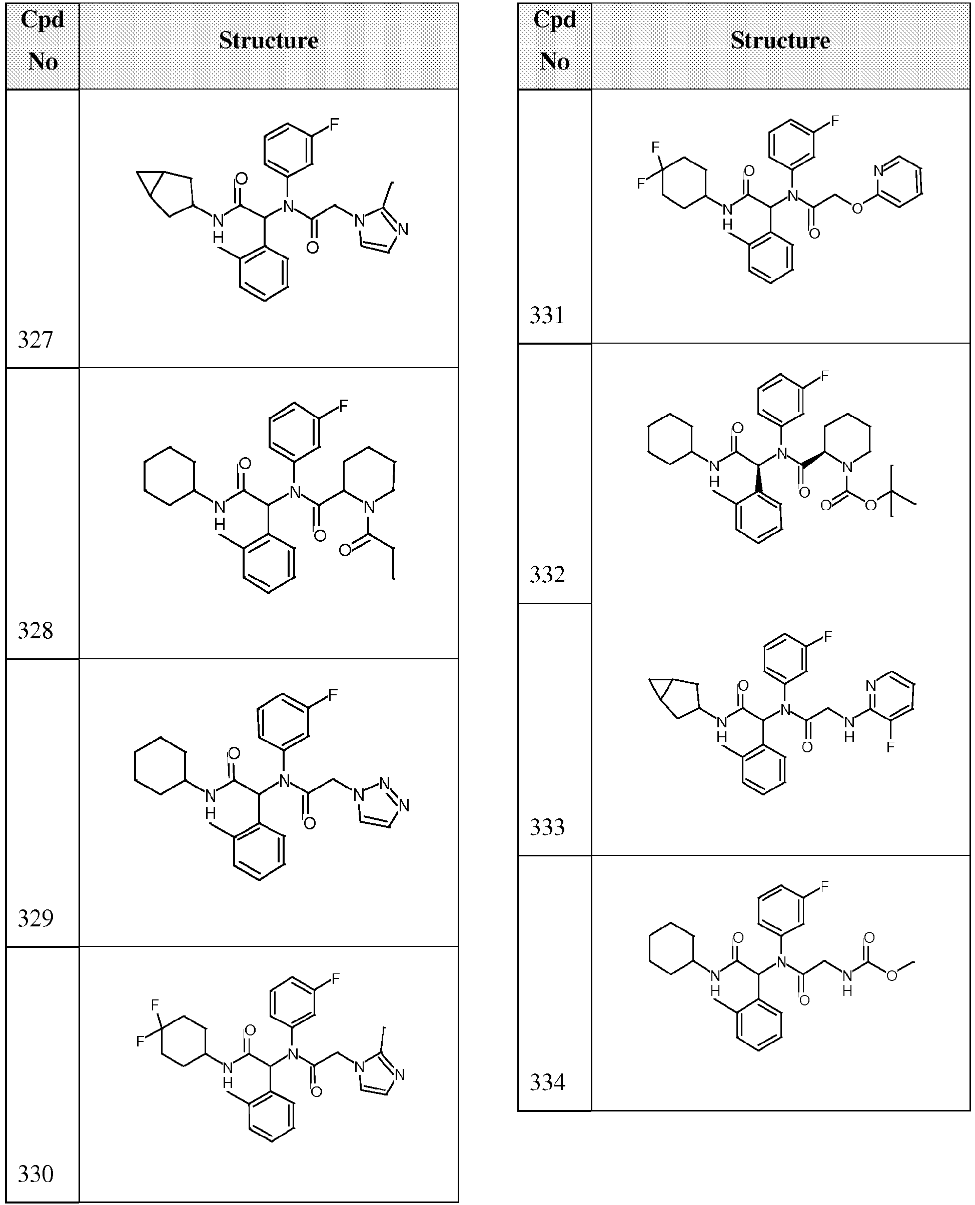

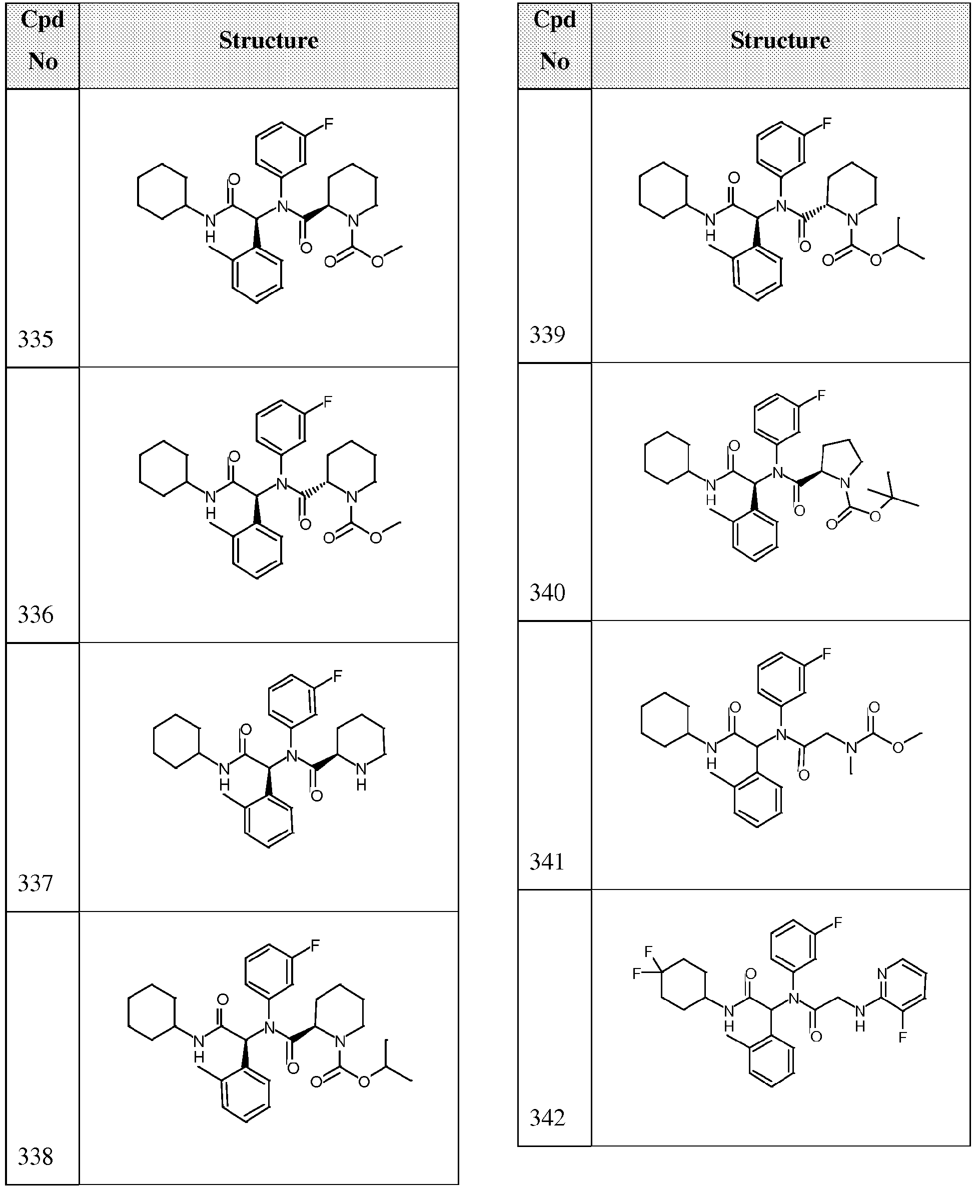

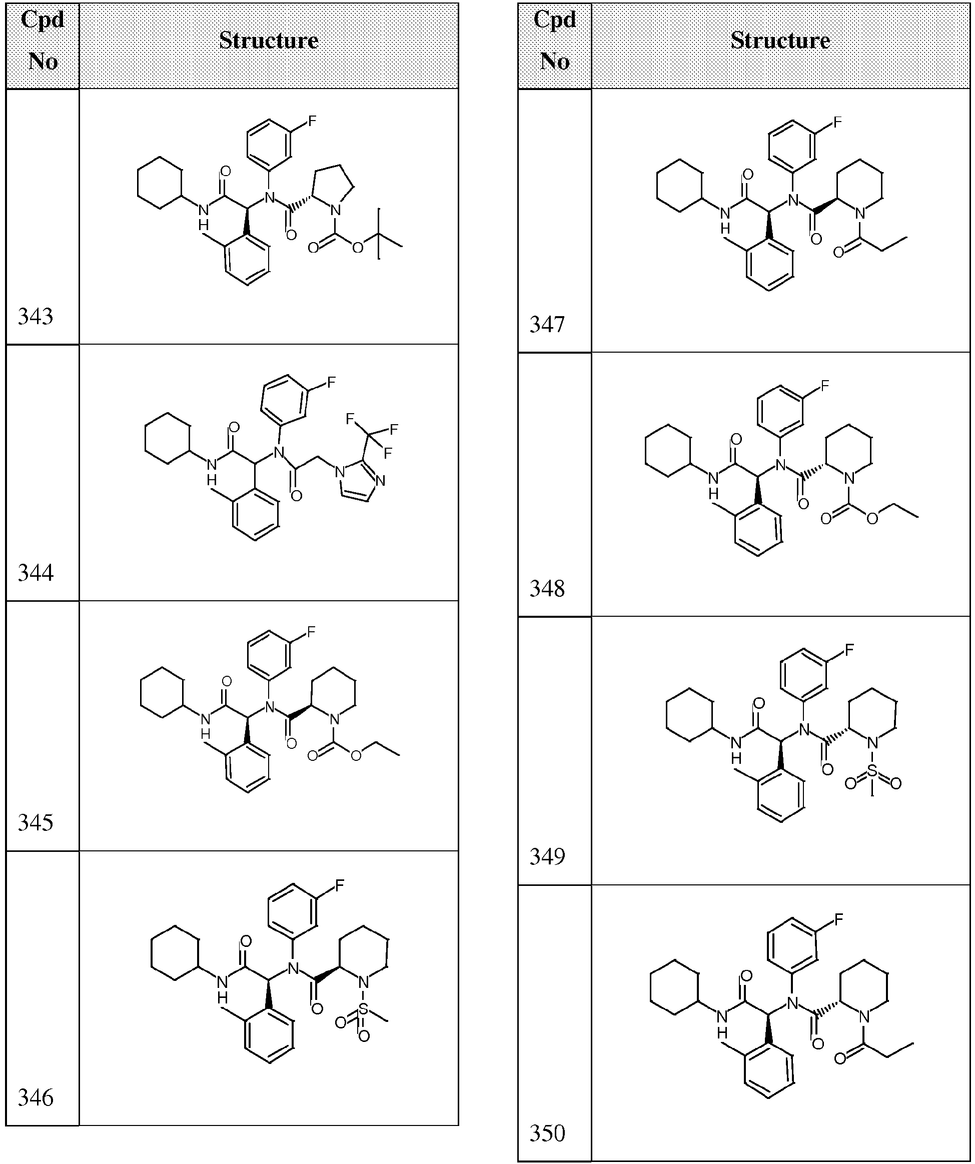

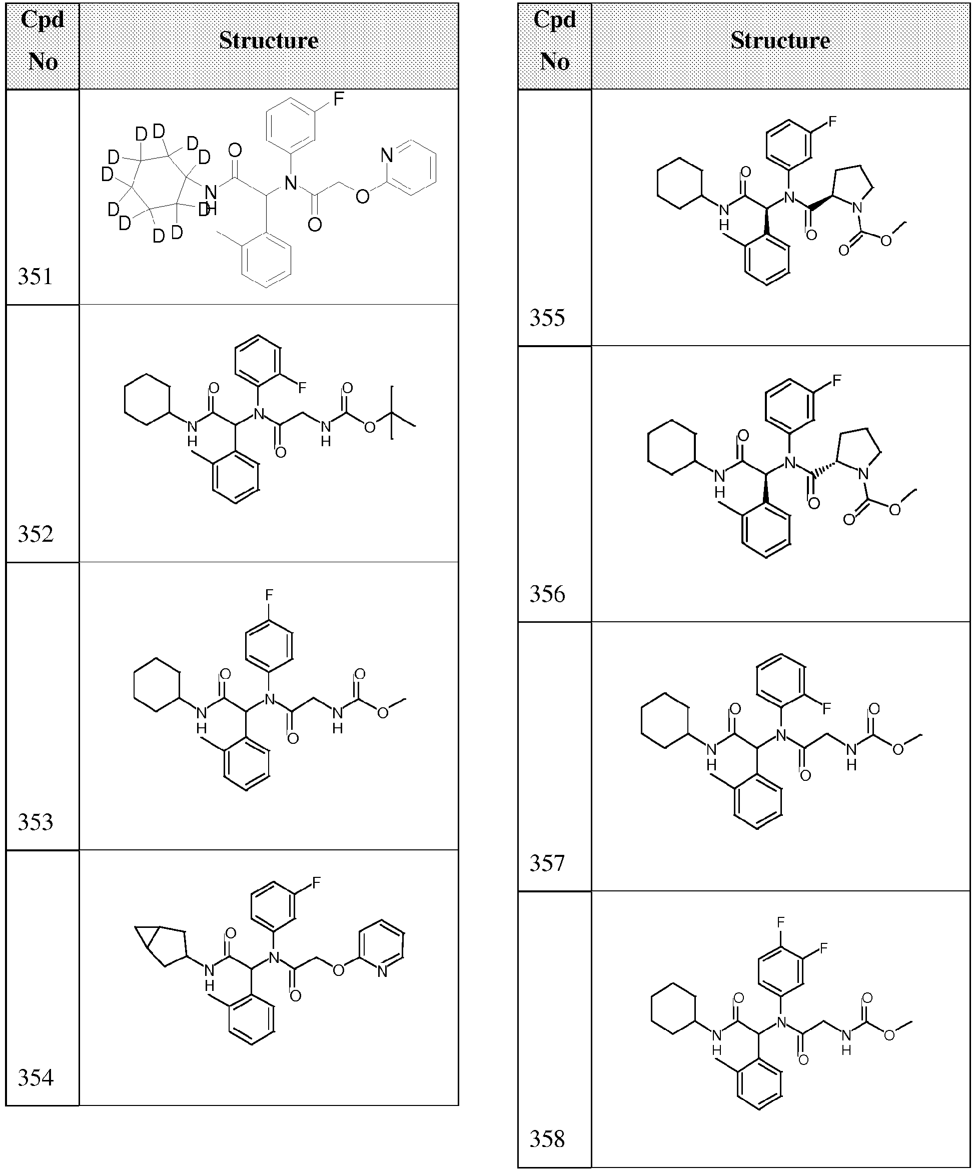

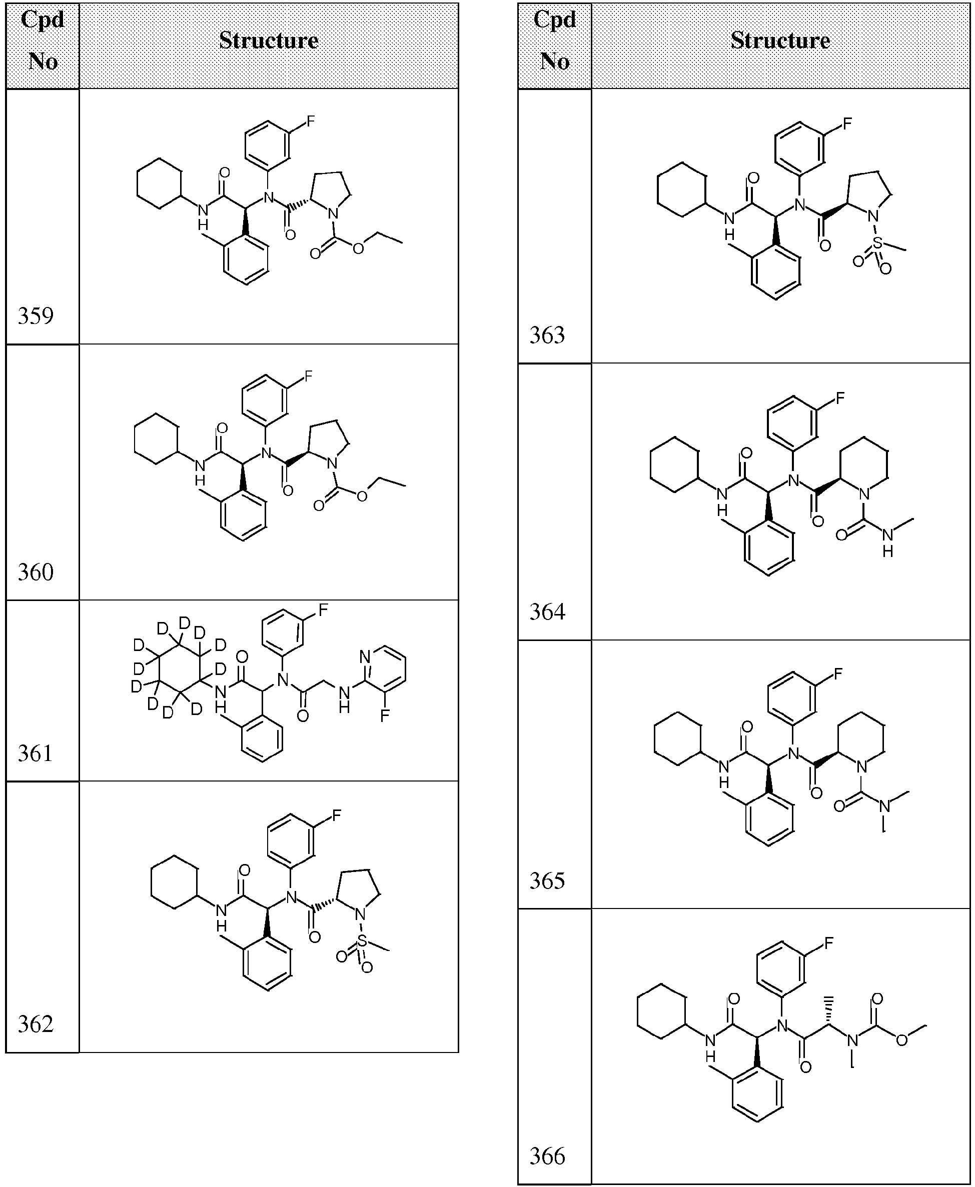

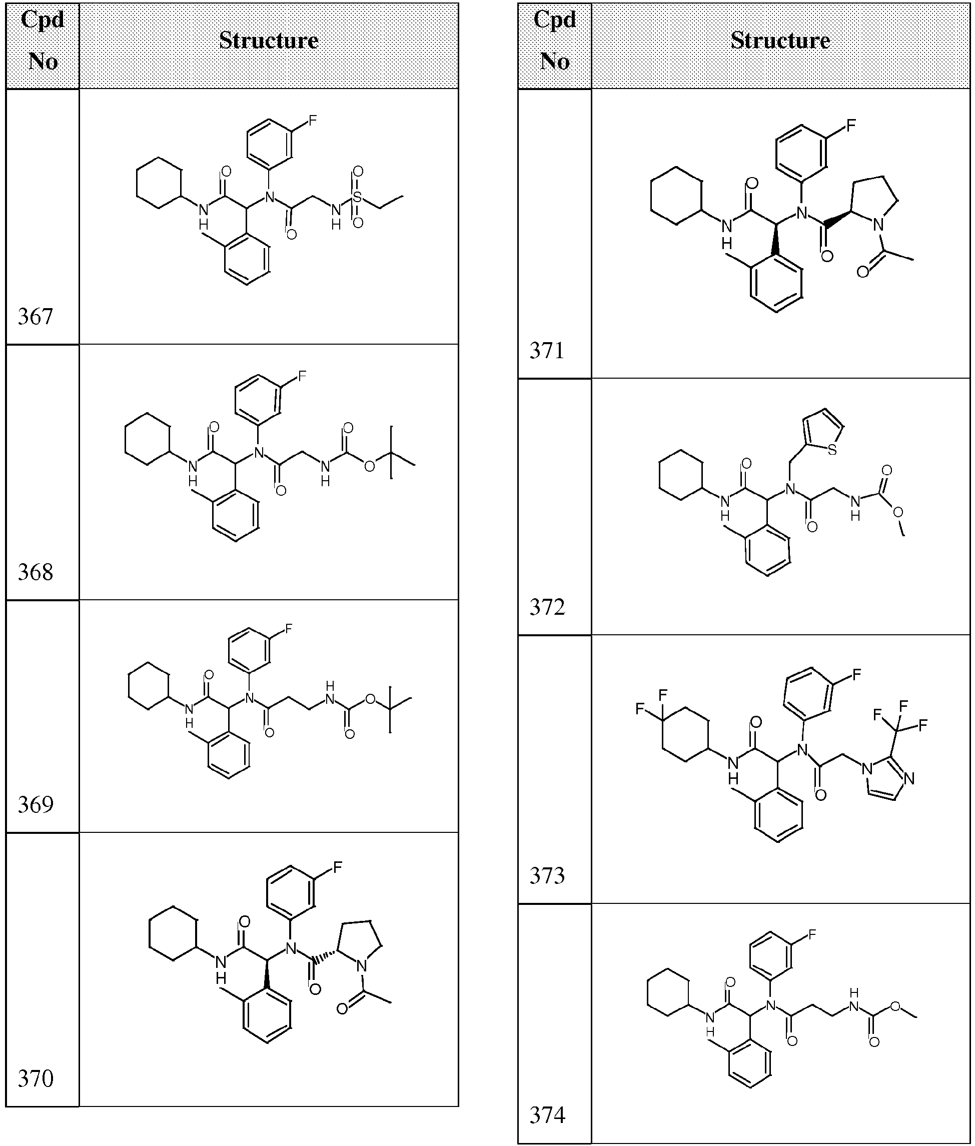

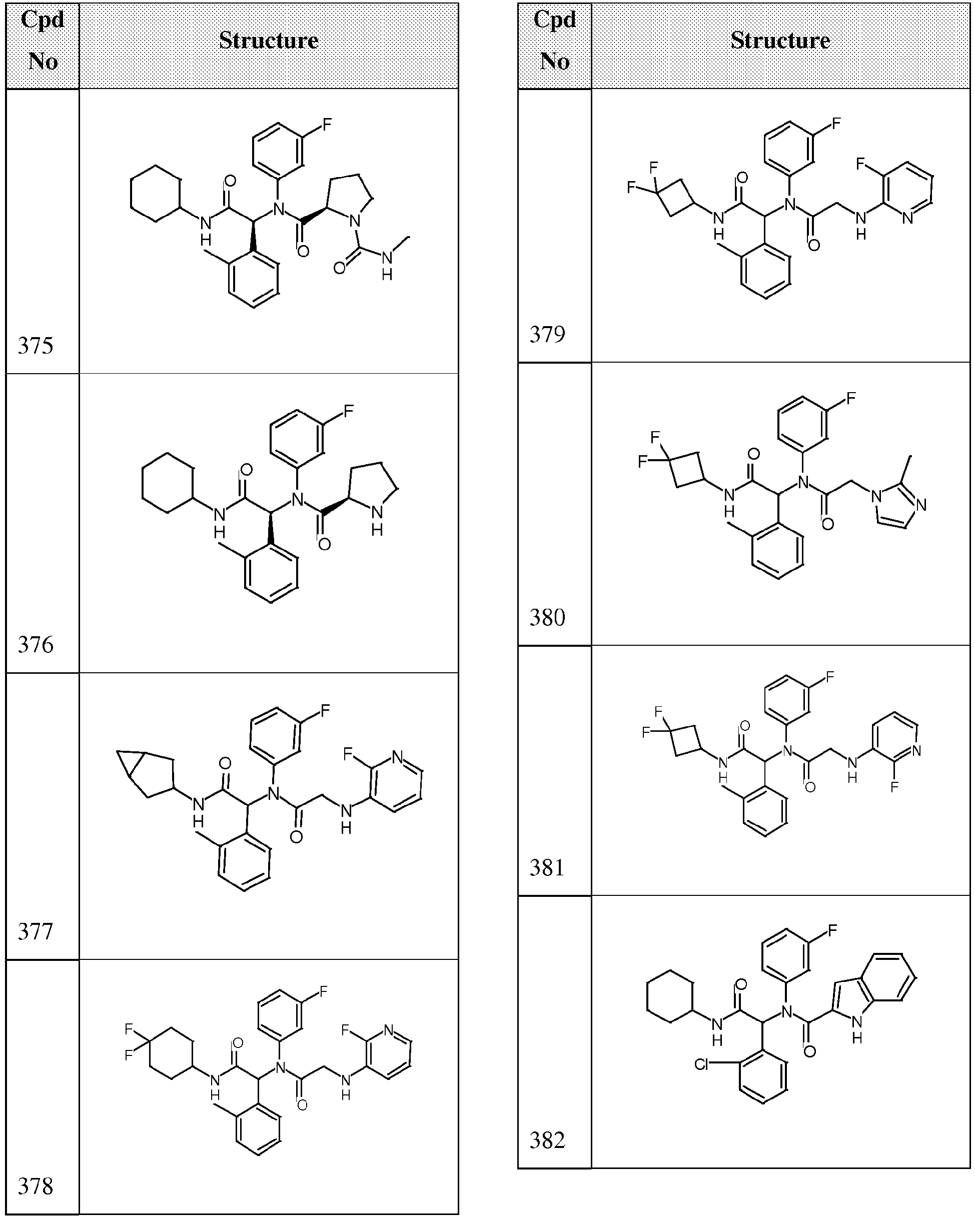

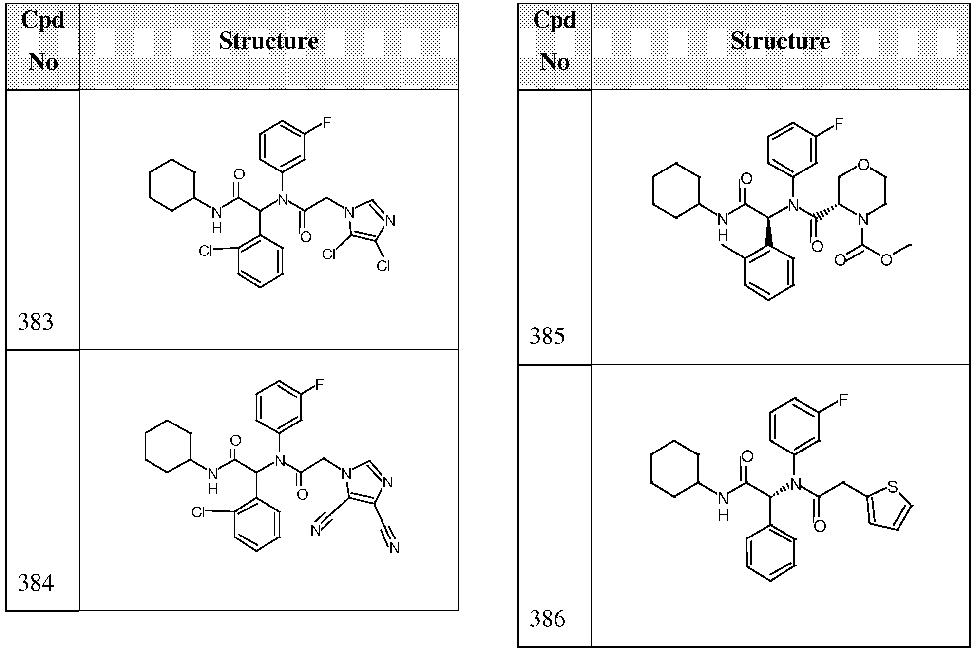

In another embodiment, the compound is selected from any one of Compound numbers 104, 126, 135, 140, 150, 155, 160, 161, 165, 173, 185, 186, 197, 198, 201, 202, 203, 210, 212, 213, 217, 218, 227, 228, 237, 240, 247, 253, 260, 265, 271, 272, 275, 276, 287, 288, 289, 290, 291, 293, 297, 301, 306, 307, 311, 313, 314, 316, 320, 321, 322, 331, 334, 341, 344, 348, 351, 356, 359, 361, 366, 378, 381, and 385 from Table 2.

The compounds of this invention may contain one or more asymmetric centers and thus occur as racemates, racemic mixtures, scalemic mixtures, and diastereomeric mixtures, as well as single enantiomers or individual stereoisomers that are substantially free from another possible enantiomer or stereoisomer. The term "substantially free of other stereoisomers" as used herein means a preparation enriched in a compound having a selected stereochemistry at one or more selected stereocenters by at least about 60%, 65%, 70%, 75%, 80%, 85%, 90%, 95%, 96%, 97%, 98%, or 99%. The term "enriched" means that at least the designated percentage of a preparation is the compound having a selected stereochemistry at one or more selected stereocenters. Methods of obtaining or synthesizing an individual enantiomer or stereoisomer for a given compound are known in the art and may be applied as practicable to final compounds or to starting material or intermediates.

In one embodiment, when R² and R⁹ are different, the compound of Formula I is enriched for a structure or structures having a selected stereochemistry at the carbon atom that is bound to R² and R⁹. In one embodiment, the selected stereochemistry at that carbon atom is R. In another embodiment the selected stereochemistry at that carbon atom is S. For example, the compound is enriched in the specific stereoisomer by at least about 60%, 65%, 70%, 75%, 80%, 85%, 90%, 95%, 96%, 97%, 98%, or 99%.

The compounds of formula I may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; and the like.

Unless otherwise indicated when a disclosed compound is named or depicted by a structure without specifying the stereochemistry and has one or more chiral centers, it is understood to represent all possible stereoisomers of the compound.

The compounds of this invention may also be represented in multiple tautomeric forms, in such instances, the invention expressly includes all tautomeric forms of the compounds described herein, even though only a single tautomeric form may be represented (e.g., alkylation of a ring system may result in alkylation at multiple sites, the invention expressly includes all such reaction products). All such isomeric forms of such compounds are expressly included in the present invention. All crystal forms of the compounds described herein are expressly included in the present invention.

Certain compounds of the invention are available from commercial and/or public compound libraries, such as those sold by Evotec AG (Hamburg, Germany) and its affiliates, Asinex Ltd (Moscow, Russia) and its affiliates, and thorough the National Institute of Health. Other compounds of the invention can be synthesized by the ordinary skilled artisan using methods well known in the art, such as through Ugi chemistry.

For example, compounds may be prepared according to one or more of the following general schemes.

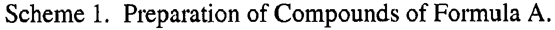

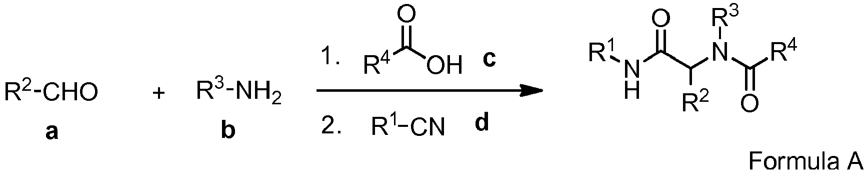

Compounds of Formula A were prepared by reacting the aldehyde of R²(**a**) with an amine of R³(**b**) in methanol. The carboxylic acid of R⁴(**c**) and the cyano of R¹(**d**) are then added to the mixture to produce a compound of the invention (more particularly a compound of Formula I-b or I-c). The HCl salt form of the resulting compound was prepared by mixing the compound with HCl/Et₂O.

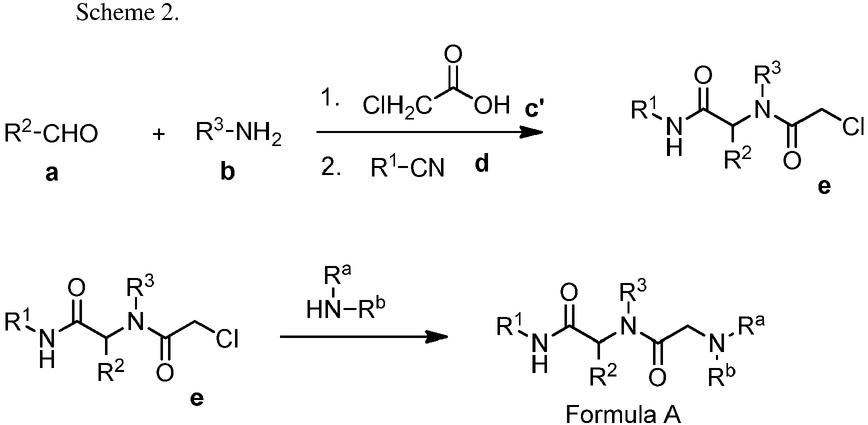

Certain compounds of Formula A comprising an amine in R⁴ were also prepared from chloroacetyl **e** according to Scheme 2. Chloroacetyl **e** was synthesized according to Scheme 1, using 2-chloroacetic acid (**c'**) in place of the carboxylic acid of R⁴. Chloroacetyl **e** was then used to produce compounds of the invention containing secondary and tertiary amines in R⁴. In Scheme 2, R^{a} represents hydrogen or C₁-C₃ alkyl; and R^{b} represents -R⁶-R⁷, as those variables are defined for Formula A; or R^{a} and R^{b} are taken together to form an optionally substituted heterocyclyl or heteroaryl.

The reaction between chloroacetyl **e** and the amine may be achieved under several different conditions: a) in the presence of Et₃N in DCM and TBAI; b) by refluxing in the presence of Et₃N in toluene under an N₂ atmosphere; c) in the presence of NaI in acetone and moderate heat (e.g., 70°C); or d) in the presence of Et₃N in DMF.

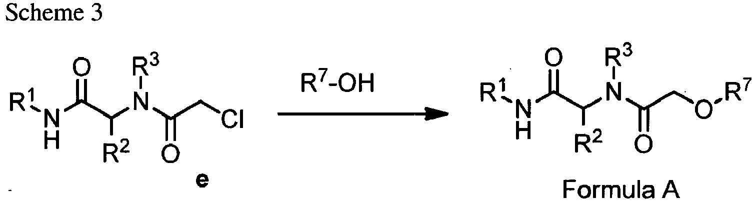

Certain compounds of Formula A wherein R¹ is -CH₂-O-R⁷ were prepared from chloroacetyl e and the appropriate R⁷ hydroxyl. This reaction can be carried out in the presence of KOH and DMSO, or in the presence of K₂CO₃ in MeCN, heated to 40°C.

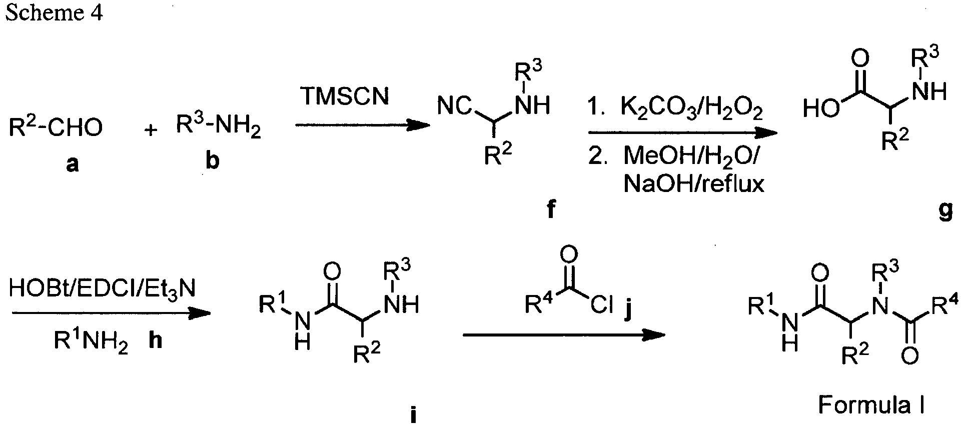

Certain compounds are produced according to Scheme 4. The aldehyde of R²(**a**) is combined with the amine of R³(**b**) in the presence of TMSCN to produce cyanomethylamine **f.** The cyano moiety is coverted to the corresponding carboxylic acid **g** by reaction with K₂CO₃ and H₂O₂, followed by reflux in aqueous MeOH and NaOH. The R¹ amine (**h**) is then reacted with **g** in the presence of HOBt/EDCI/Et₃N in DCM to produce **i,** which is then reacted with a chlorocarbonyl derivative of R⁴ to produce a compound of the invention.

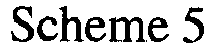

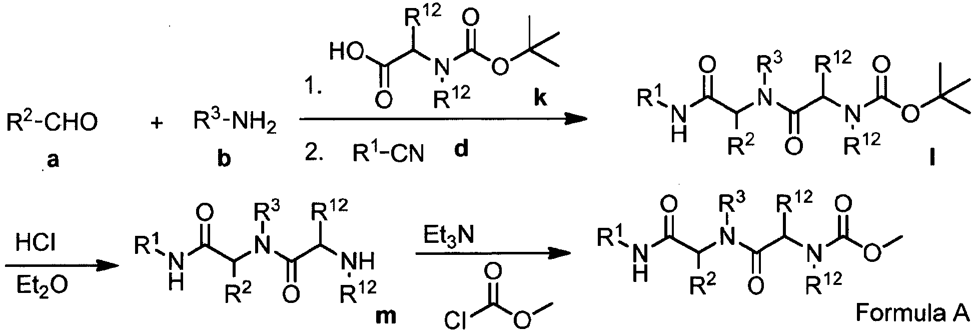

Certain compounds where R⁴ is -C(R¹¹)-N(R¹¹)-O-CH₃, or 1-methyloxycarbonylpyrrolidin-2-yl are produced according to Scheme 5. In Scheme 5, each R¹² is independently hydrogen or methyl, or two adjacent R¹² are taken together with the carbon and nitrogen atoms to which they are respectively bound to form a pyrrolidine or piperidine ring. In Scheme 5, t-butyl derivative **I** is formed according to Scheme 1, using carboxylic acid **k** in place of carboxylic acid of R⁴(**c**). Treatment of **I** with acid produces amine **m,** which is converted to the compound of Formula A by treatment with methyl chloroformate.

Compounds produced by any of the general schemes set forth above may be further modified (e.g., through the addition of substituents to rings, etc.) to produce additional compounds of the invention. The specific approaches and compounds shown above are not intended to be limiting. The chemical structures in the schemes herein depict variables that are hereby defined commensurately with chemical group definitions (moieties, atoms, etc.) of the corresponding position in the compound formulae herein, whether identified by the same variable name (i.e., R¹, R², R³, etc.) or not. The suitability of a chemical group in a compound structure for use in the synthesis of another compound is within the knowledge of one of ordinary skill in the art.

Additional methods of synthesizing compounds of Formula A and their synthetic precursors, including those within routes not explicitly shown in schemes herein, are within the means of chemists of ordinary skill in the art, as well as set forth in the specific examples. Synthetic chemistry transformations and protecting group methodologies (protection and deprotection) useful in synthesizing the applicable compounds are known in the art and include, for example, those described in Larock R, Comprehensive Organic Transformations, VCH Publishers (1989); Greene, TW et al., Protective Groups in Organic Synthesis, 3rd Ed., John Wiley and Sons (1999); Fieser, L et al., Fieser and Fieser's Reagents for Organic Synthesis, John Wiley and Sons (1994); and Paquette, L, ed., Encyclopedia of Reagents for Organic Synthesis, John Wiley and Sons (1995) and subsequent editions thereof.

Combinations of substituents and variables envisioned by this invention are only those that result in the formation of stable compounds.

It may be convenient or desirable to prepare, purify, and/or handle a corresponding salt of the active compound, for example, a pharmaceutically-acceptable salt. Examples of pharmaceutically acceptable salts are discussed in Berge et al., 1977, "Pharmaceutically Acceptable Salts." J. Pharm. Sci. Vol. 66, pp. 1-19.

For example, if the compound is anionic, or has a functional group which may be anionic (*e.g.,* -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of suitable organic cations include, but are not limited to, ammonium ion (*i*.*e*., NH₄⁺) and substituted ammonium ions (*e.g*., NH₃R⁺, NH₂R²⁺, NHR³⁺, NR⁴⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group that may be cationic (*e.g*., -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucoheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

Unless otherwise specified, a reference to a particular compound also includes salt forms thereof.

### Compositions and routes of administration

The compounds utilized in the methods described herein may be formulated together with a pharmaceutically acceptable carrier or adjuvant into pharmaceutically acceptable compositions prior to be administered to a subject. In another embodiment, such pharmaceutically acceptable compositions further comprise additional therapeutic agents in amounts effective for achieving a modulation of disease or disease symptoms, including those described herein.

The term "pharmaceutically acceptable carrier or adjuvant" refers to a carrier or adjuvant that may be administered to a subject, together with a compound of this invention, and which does not destroy the pharmacological activity thereof and is nontoxic when administered in doses sufficient to deliver a therapeutic amount of the compound.

Pharmaceutically acceptable carriers, adjuvants and vehicles that may be used in the pharmaceutical compositions of this invention include, but are not limited to, ion exchangers, alumina, aluminum stearate, lecithin, self-emulsifying drug delivery systems (SEDDS) such as d-α-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α-, β-, and γ-cyclodextrin, or chemically modified derivatives such as hydroxyalkylcyclodextrins, including 2- and 3-hydroxypropyl-β-cyclodextrins, or other solubilized derivatives may also be advantageously used to enhance delivery of compounds of the formulae described herein.

The pharmaceutical compositions of this invention may be administered orally, parenterally, by inhalation spray, topically, rectally, nasally, buccally, vaginally or via an implanted reservoir, preferably by oral administration or administration by injection. The pharmaceutical compositions of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intraarticular, intraarterial, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as Tweens or Spans and/or other similar emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

The pharmaceutical compositions of this invention may also be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

Topical administration of the pharmaceutical compositions of this invention is useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active compound suspended or dissolved in a carrier with suitable emulsifying agents. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. When the compositions of this invention comprise a combination of a compound of the formulae described herein and one or more additional therapeutic or prophylactic agents, both the compound and the additional agent should be present at dosage levels of between about 1 to 100%, and more preferably between about 5 to 95% of the dosage normally administered in a monotherapy regimen. The additional agents may be administered separately, as part of a multiple dose regimen, from the compounds of this invention. Alternatively, those agents may be part of a single dosage form, mixed together with the compounds of this invention in a single composition.

The compounds described herein can, for example, be administered by injection, intravenously, intraarterially, subdermally, intraperitoneally, intramuscularly, or subcutaneously; or orally, buccally, nasally, transmucosally, topically, in an ophthalmic preparation, or by inhalation, with a dosage ranging from about 0.5 to about 100 mg/kg of body weight, alternatively dosages between 1 mg and 1000 mg/dose, every 4 to 120 hours, or according to the requirements of the particular drug. The methods herein contemplate administration of an effective amount of compound or compound composition to achieve the desired or stated effect. Typically, the pharmaceutical compositions of this invention will be administered from about 1 to about 6 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Alternatively, such preparations contain from about 20% to about 80% active compound.

Lower or higher doses than those recited above may be required. Specific dosage and treatment regimens for any particular subject will depend upon a variety of factors, including the activity of the specific compound employed, the age, body weight, general health status, sex, diet, time of administration, rate of excretion, drug combination, the severity and course of the disease, condition or symptoms, the subject's disposition to the disease, condition or symptoms, and the judgment of the treating physician.

Upon improvement of a subject's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level. Subjects may, however, require intermittent treatment on a long-term basis upon any recurrence of disease symptoms.

The pharmaceutical compositions described above comprising a compound of formula I or a compound described in any one of the embodiments herein, may further comprise another therapeutic agent useful for treating cancer.

### Methods of Use

Provided is a compound for use in a method for inhibiting a mutant IDH1 activity comprising contacting a subject in need thereof a compound of formula I, a compound described in any one of the embodiments herein, or a pharmaceutically acceptable salt thereof. In one embodiment, the mutant IDH1 has an R132X mutation. In one aspect of this embodiment, the R132X mutation is selected from R132H, R132C, R132L, R132V, R132S and R132G. In another aspect, the R132X mutation is R132 H.

Also provided are compounds for use in methods of treating a cancer characterized by the presence of a mutant allele of IDH1 comprising the step of administering to subject in need thereof (a) a compound of formula I, a compound described in any one of the embodiments herein, or a pharmaceutically acceptable salt thereof, or (b) a pharmaceutical composition comprising (a) and a pharmaceutically acceptable carrier.

In one embodiment, the cancer to be treated is characterized by a mutant allele of IDH1 having an R132X mutation. In one aspect of this embodiment, the R132X mutation is selected from R132H, R132C, R132L, R132V, R132S and R132G. In another aspect, the R132X mutation is R132 H. A cancer can be analyzed by sequencing cell samples to determine the presence of a mutation at amino acid 132 of IDH1.

In certain embodiments, the cancer to be treated is further characterized by elevated levels of 2HG. In one aspect of this embodiment, the efficacy of cancer treatment is monitored by measuring the levels of 2HG in the subject. Typically levels of 2HG are measured prior to treatment, wherein an elevated level is indicative of the use of the compound of Formula I to treat the cancer. Once the elevated levels are established, the level of 2HG is determined during the course of and/or following termination of treatment to establish efficacy. In certain embodiments, the level of 2HG is only determined during the course of and/or following termination of treatment. A reduction of 2HG levels during the course of treatment and following treatment is indicative of efficacy. Similarly, a determination that 2HG levels are not elevated during the course of or following treatment is also indicative of efficacy. Typically, the these 2HG measurements will be utilized together with other well-known determinations of efficacy of cancer treatment, such as reduction in number and size of tumors and/or other cancer-associated lesions, improvement in the general health of the subject, and alterations in other biomarkers that are associated with cancer treatment efficacy.

2HG can be detected in a sample by LC/MS. The sample is mixed 80:20 with methanol, and centrifuged at 3,000 rpm for 20 minutes at 4 degrees Celsius. The resulting supernatant can be collected and stored at -80 degrees Celsius prior to LC-MS/MS to assess 2-hydroxyglutarate levels. A variety of different liquid chromatography (LC) separation methods can be used. Each method can be coupled by negative electrospray ionization (ESI, -3.0 kV) to triple-quadrupole mass spectrometers operating in multiple reaction monitoring (MRM) mode, with MS parameters optimized on infused metabolite standard solutions. Metabolites can be separated by reversed phase chromatography using 10 mM tributyl-amine as an ion pairing agent in the aqueous mobile phase, according to a variant of a previously reported method (Luo et al. J Chromatogr A 1147, 153-64, 2007). One method allows resolution of TCA metabolites: t = 0, 50% B; t = 5, 95% B; t= 7, 95% B; t= 8, 0% B, where B refers to an organic mobile phase of 100% methanol. Another method is specific for 2-hydroxyglutarate, running a fast linear gradient from 50% -95% B (buffers as defined above) over 5 minutes. A Synergi Hydro-RP, 100mm × 2 mm, 2.1 µm particle size (Phenomonex) can be used as the column, as described above. Metabolites can be quantified by comparison of peak areas with pure metabolite standards at known concentration. Metabolite flux studies from ¹³C-glutamine can be performed as described, *e.g.,* in Munger et al. Nat Biotechnol 26, 1179-86, 2008.

In one embodiment 2HG is directly evaluated.

In another embodiment a derivative of 2HG formed in process of performing the analytic method is evaluated. By way of example such a derivative can be a derivative formed in MS analysis. Derivatives can include a salt adduct, *e.g.,* a Na adduct, a hydration variant, or a hydration variant which is also a salt adduct, e.g., a Na adduct, e.g., as formed in MS analysis.

In another embodiment a metabolic derivative of 2HG is evaluated. Examples include species that build up or are elevated, or reduced, as a result of the presence of 2HG, such as glutarate or glutamate that will be correlated to 2HG, *e.g.,* R-2HG.

Exemplary 2HG derivatives include dehydrated derivatives such as the compounds provided below or a salt adduct thereof:

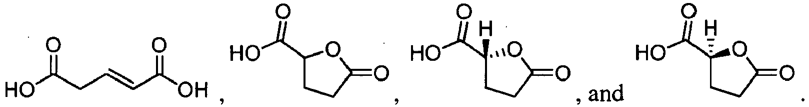

In an embodiment the cancer is a tumor wherein at least 30, 40, 50, 60, 70, 80 or 90% of the tumor cells carry an IDH1 mutation at the time of diagnosis or treatment.

In one embodiment, the cancer to be treated is characterized by a mutant allele of IDH1 wherein the IDH1 mutation result in a new ability of the enzyme to catalyze the NAPH-dependent reduction of α-ketoglutarate to *R*(-)-2-hydroxyglutarate in a patient. In one aspect of this embodiment, the IDH1 mutation is an R132X mutation. In another aspect of this embodiment, the R132X mutation is selected from R132H, R132C, R132L, R132V, R132S and R132G. In another aspect, the R132X mutation is R132 H or R132C. A cancer can be analyzed by sequencing cell samples to determine the presence and specific nature of (e.g., the changed amino acid present at) a mutation at amino acid 132 of IDH1.

Without being bound by theory, applicants believe that mutant alleles of IDH1 wherein the IDH1 mutation result in a new ability of the enzyme to catalyze the NAPH-dependent reduction of α-ketoglutarate to *R*(-)-2-hydroxyglutarate, and in particular R132H mutations of IDH1, characterize a subset of all types of cancers, without regard to their cellular nature or location in the body. Thus, the compounds and compounds for use in methods of this invention are useful to treat
type of cancer that is characterized by the presence of a mutant allele of IDH1 imparting such acitivity and in particular an IDH1 R132H mutation.

The compounds for use in methods described herein can be used to treat a cancer, for example those described by the National Cancer Institute. A cancer can be evaluated to determine whether it contains an IDH mutant using a method described herein. Exemplary cancers described by the National Cancer Institute include: Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood; Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma, Childhood Brain Stem; Glioma, Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS- Related; Lymphoma, Central Nervous System (Primary); Lymphoma, Cutaneous T-Cell; Lymphoma, Hodgkin's, Adult; Lymphoma, Hodgkin's, Childhood; Lymphoma, Hodgkin's During Pregnancy; Lymphoma, Non-Hodgkin's, Adult; Lymphoma, Non- Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Non-Hodgkin's Lymphoma, Adult; Non-Hodgkin's Lymphoma, Childhood; Non- Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood; Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T- Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor. Metastases of the aforementioned cancers can also be treated or prevented in accordance with the methods described herein.
The compounds for use in methods described herein are useful in treating cancer of the nervous system, e.g. brain tumor, *e.g.,* glioma, *e.g.,* glioblastoma multiforme (GBM). Gliomas, a type of brain tumors, can be classified as grade I to grade IV on the basis of histopathological and clinical criteria established by the World Health Organization (WHO). WHO grade I gliomas are often considered benign. Gliomas of WHO grade II or III are invasive, progress to higher-grade lesions. WHO grade IV tumors (glioblastomas) are the most invasive form. Exemplary brain tumors include, *e.g.,* astrocytic tumor (*e.g.,* pilocytic astrocytoma, subependymal giant-cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma, and primary pediatric glioblastoma); oligodendroglial tumor (*e.g.,* oligodendroglioma, and anaplastic oligodendroglioma); oligoastrocytic tumor (*e.g.,* oligoastrocytoma, and anaplastic oligoastrocytoma); ependymoma (*e.g.,* myxopapillary ependymoma, and anaplastic ependymoma); medulloblastoma; primitive neuroectodermal tumor, schwannoma, meningioma, metatypical meningioma, anaplastic meningioma; and pituitary adenoma. Exemplary cancers are described in Acta Neuropathol (2008) 116:597-602 and N Engl J Med. 2009 Feb 19; 360(8):765-73.

In an embodiment, the cancer is glioblastoma.

In an embodiment, the cancer is paragangliomas.

In an embodiment, the cancer is fibrosarcoma.

In an embodiment, the cancer is prostate cancer, *e.g*., stage T1 (*e.g.,* T1a, T1b and T1c), T2 (*e.g.,* T2a, T2b and T2c), T3 (*e.g.,* T3a and T3b) and T4, on the TNM staging system. In embodiments the prostate cancer is grade G1, G2, G3 or G4 (where a higher number indicates greater difference from normal tissue). Types of prostate cancer include, *e.g.,* prostate adenocarcinoma, small cell carcinoma, squamous carcinoma, sarcomas, and transitional cell carcinoma. In one aspect of this embodiment the disorder is localized or metastatic prostate cancer, *e.g.,* prostate adenocarcinoma.

In an embodiment, the disorder is a hematological cancer, *e.g.,* a leukemia, *e.g.,* AML, or acute lymphoblastic leukemia ("ALL"). In one aspect of this embodiment the cancer is ALL (*e.g.,* an adult or pediatric form). In one aspect of this embodiment the cancer is B-ALL or TALL

IDH1 R132X mutations are known to occur in certain types of cancers as indicated in Table 3, below.

**Table 3. IDH mutations associated with certain cancers**

| **Cancer Type** | **IDH1 R132X Mutation** | **Tumor Type** |
|---|---|---|
| brain tumors | R132H | primary tumor |
| | R132C | primary tumor |
| | R132S | primary tumor |
| | R132G | primary tumor |
| | R132L | primary tumor |
| | R132V | primary tumor |
| fibrosarcoma | R132C | HT1080 fibrosarcoma cell line |
| Acute Myeloid Leukemia (AML) | R132H | primary tumor |
| | R132G | primary tumor |
| | R132C | primary tumor |
| Prostate cancer | R132H | primary tumor |
| | R132C | primary tumor |
| Acute lymphoblastic leukemia (ALL) | R132C | primary tumor |
| paragangliomas | R132C | primary tumor |

Accordingly in one embodiment, the cancer is a cancer selected from any one of the cancer types listed in Table 3, and the IDH R132X mutation is one or more of the IDH1 R132X mutations listed in Table 3 for that particular cancer type.

Treatment methods described herein can additionally comprise various evaluation steps prior to and/or following treatment with a compound of formula I or a compound described in any one of the embodiments described herein.

In one embodiment, prior to and/or after treatment with a compound of Formula A, I, I-a, I-b, I-c or II or a compound described in any one of the embodiments described herein, the method further comprises the step of evaluating the growth, size, weight, invasiveness, stage and/or other phenotype of the cancer.

In one embodiment, prior to and/or after treatment with a compound of Formula A, I, I-a, I-b, I-c or II or a compound described in any one of the embodiments described herein, the method further comprises the step of evaluating the IDH1 genotype of the cancer. This may be achieved by ordinary methods in the art, such as DNA sequencing, immuno analysis, and/or evaluation of the presence, distribution or level of 2HG.

In one embodiment, prior to and/or after treatment with a compound of Formula A, I, I-a, I-b, I-c or II or a compound described in any one of the embodiments described herein, the method further comprises the step of determining the 2HG level in the subject. This may be achieved by spectroscopic analysis, *e.g.,* magnetic resonance-based analysis, *e.g*., MRI and/or MRS measurement, sample analysis of bodily fluid, such as serum or spinal cord fluid analysis, or by analysis of surgical material, *e.g*., by mass-spectroscopy.

### Combination therapies

In some embodiments, the methods described herein comprise the additional step of co-administering to a subject in need thereof a second therapy *e.g.,* an additional cancer therapeutic agent or an additional cancer treatment. Exemplary additional cancer therapeutic agents include for example, chemotherapy, targeted therapy, antibody therapies, immunotherapy, and hormonal therapy. Additional cancer treatments include, for example: surgery, and radiation therapy. Examples of each of these treatments are provided below.

The term "co-administering" as used herein with respect to an additional cancer therapeutic agents means that the additional cancer therapeutic agent may be administered together with a compound of this invention as part of a single dosage form (such as a composition of this invention comprising a compound of the invention and an second therapeutic agent as described above) or as separate, multiple dosage forms. Alternatively, the additional cancer therapeutic agent may be administered prior to, consecutively with, or following the administration of a compound of this invention. In such combination therapy treatment, both the compounds of this invention and the second therapeutic agent(s) are administered by conventional methods. The administration of a composition of this invention, comprising both a compound of the invention and a second therapeutic agent, to a subject does not preclude the separate administration of that same therapeutic agent, any other second therapeutic agent or any compound of this invention to said subject at another time during a course of treatment. The term "co-administering" as used herein with respect to an additional cancer treatment means that the additional cancer treatment may occur prior to, consecutively with, concurrently with or following the administration of a compound of this invention.

In some embodiments, the additional cancer therapeutic agent is a chemotherapy agent. Examples of chemotherapeutic agents used in cancer therapy include, for example, antimetabolites (*e.g.,* folic acid, purine, and pyrimidine derivatives) and alkylating agents (*e.g.,* nitrogen mustards, nitrosoureas, platinum, alkyl sulfonates, hydrazines, triazenes, aziridines, spindle poison, cytotoxic agents, topoisomerase inhibitors and others). Exemplary agents include Aclarubicin, Actinomycin, Alitretinoin, Altretamine, Aminopterin, Aminolevulinic acid, Amrubicin, Amsacrine, Anagrelide, Arsenic trioxide, Asparaginase, Atrasentan, Belotecan, Bexarotene, bendamustine, Bleomycin, Bortezomib, Busulfan, Camptothecin, Capecitabine, Carboplatin, Carboquone, Carmofur, Carmustine, Celecoxib, Chlorambucil, Chlormethine, Cisplatin, Cladribine, Clofarabine, Crisantaspase, Cyclophosphamide, Cytarabine, Dacarbazine, Dactinomycin, Daunorubicin, Decitabine, Demecolcine, Docetaxel, Doxorubicin, Efaproxiral, Elesclomol, Elsamitrucin, Enocitabine, Epirubicin, Estramustine, Etoglucid, Etoposide, Floxuridine, Fludarabine, Fluorouracil (5FU), Fotemustine, Gemcitabine, Gliadel implants, Hydroxycarbamide, Hydroxyurea, Idarubicin, Ifosfamide, Irinotecan, Irofulven, Ixabepilone, Larotaxel, Leucovorin, Liposomal doxorubicin, Liposomal daunorubicin, Lonidamine, Lomustine, Lucanthone, Mannosulfan, Masoprocol, Melphalan, Mercaptopurine, Mesna, Methotrexate, Methyl aminolevulinate, Mitobronitol, Mitoguazone, Mitotane, Mitomycin, Mitoxantrone, Nedaplatin, Nimustine, Oblimersen, Omacetaxine, Ortataxel, Oxaliplatin, Paclitaxel, Pegaspargase, Pemetrexed, Pentostatin, Pirarubicin, Pixantrone, Plicamycin, Porfimer sodium, Prednimustine, Procarbazine, Raltitrexed, Ranimustine, Rubitecan, Sapacitabine, Semustine, Sitimagene ceradenovec, Strataplatin, Streptozocin, Talaporfin, Tegafur-uracil, Temoporfin, Temozolomide, Teniposide, Tesetaxel, Testolactone, Tetranitrate, Thiotepa, Tiazofurine, Tioguanine, Tipifarnib, Topotecan, Trabectedin, Triaziquone, Triethylenemelamine, Triplatin, Tretinoin, Treosulfan, Trofosfamide, Uramustine, Valrubicin, Verteporfin, Vinblastine, Vincristine, Vindesine, Vinflunine, Vinorelbine, Vorinostat, Zorubicin, and other cytostatic or cytotoxic agents described herein.

Because some drugs work better together than alone, two or more drugs are often given at the same time. Often, two or more chemotherapy agents are used as combination chemotherapy.

In some embodiments the additional cancer therapeutic agent is a targeted therapy agent. Targeted therapy constitutes the use of agents specific for the deregulated proteins of cancer cells. Small molecule targeted therapy drugs are generally inhibitors of enzymatic domains on mutated, overexpressed, or otherwise critical proteins within the cancer cell. Prominent examples are the tyrosine kinase inhibitors such as Axitinib, Bosutinib, Cediranib, dasatinib, erlotinib, imatinib, gefitinib, lapatinib, Lestaurtinib, Nilotinib, Semaxanib, Sorafenib, Sunitinib, and Vandetanib, and also cyclin-dependent kinase inhibitors such as Alvocidib and Seliciclib. Monoclonal antibody therapy is another strategy in which the therapeutic agent is an antibody which specifically binds to a protein on the surface of the cancer cells. Examples include the anti-HER2/neu antibody trastuzumab (HERCEPTIN®) typically used in breast cancer, and the anti-CD20 antibody rituximab and Tositumomab typically used in a variety of B-cell malignancies. Other exemplary antibodies include Cetuximab, Panitumumab, Trastuzumab, Alemtuzumab, Bevacizumab, Edrecolomab, and Gemtuzumab. Exemplary fusion proteins include Aflibercept and Denileukin diftitox. In some embodiments, the targeted therapy can be used in combination with a compound described herein, *e.g.,* a biguanide such as metformin or phenformin, preferably phenformin.

Targeted therapy can also involve small peptides as "homing devices" which can bind to cell surface receptors or affected extracellular matrix surrounding the tumor. Radionuclides which are attached to these peptides (*e.g.,* RGDs) eventually kill the cancer cell if the nuclide decays in the vicinity of the cell. An example of such therapy includes BEXXAR®.

In some embodiments, the additional cancer therapeutic agent is an immunotherapy agent. Cancer immunotherapy refers to a diverse set of therapeutic strategies designed to induce the subject's own immune system to fight the tumor. Contemporary methods for generating an immune response against tumors include intravesicular BCG immunotherapy for superficial bladder cancer, and use of interferons and other cytokines to induce an immune response in renal cell carcinoma and melanoma subjects.

Allogeneic hematopoietic stem cell transplantation can be considered a form of immunotherapy, since the donor's immune cells will often attack the tumor in a graft-versus-tumor effect. In some embodiments, the immunotherapy agents can be used in combination with a compound or composition described herein.

In some embodiments, the additional cancer therapeutic agent is a hormonal therapy agent. The growth of some cancers can be inhibited by providing or blocking certain hormones. Common examples of hormone-sensitive tumors include certain types of breast and prostate cancers. Removing or blocking estrogen or testosterone is often an important additional treatment. In certain cancers, administration of hormone agonists, such as progestogens may be therapeutically beneficial. In some embodiments, the hormonal therapy agents can be used in combination with a compound or a composition described herein.

Other possible additional therapeutic modalities include imatinib, gene therapy, peptide and dendritic cell vaccines, synthetic chlorotoxins, and radiolabeled drugs and antibodies.

### EXAMPLES

### Abbreviations list

### Genera 46

- anhy.: anhydrous
- conc.: concentrated
- aq.: aqueous
- min: minute(s)
- ml: milliliter
- mmol: millimole(s)
- mol: mole(s)
- MS: mass spectrometry
- NMR: nuclear magnetic resonance
- TLC: thin layer chromatography
- HPLC: high-performance liquid chromatography
- prep-HPLC: preparative high-performance liquid chromatography

### -Spectrum

- Hz: hertz
- δ: chemical shift
- J: coupling constant
- s: singlet
- d: doublet
- t: triplet
- q: quartet
- m: multiplet
- br: broad
- qd: quartet of *doublets*
- dquin: doublet of quintets
- dd: *doublet* of *doublets*
- dt: *doublet* of triplets

### Solvents and Reagents

- CHCl₃: chloroform
- DCM: dichloromethane
- DMF: Dimethylformamide
- DME: 1,2-dimethoxyethane
- CCl₄: carbon tetrachloride
- DMSO: dimethylsulfoxide
- Et₂O: diethyl ether
- EtOH: ethyl alcohol
- EtOAc: ethyl acetate
- MeOH: methyl alcohol
- MeCN: acetonitrile
- PE: petroleum ether
- THF: tetrahydrofuran
- AcOH: acetic acid
- HClO₄: perchloric acid
- HCOOH: formic acid
- t-BuOH: tert-butanol
- SOCl₂: thionyl dichloride
- HCl: hydrochloric acid
- H₂SO₄: sulfuric acid
- NH4Cl: ammonium chloride
- KOH: potassium hydroxide
- NaOH: sodium hydroxide
- LiOH·H₂O: lithium hydroxide monohydrate
- K₂CO₃: potassium carbonate
- Na₂CO₃: sodium carbonate
- TFA: trifluoroacetic acid
- Na₂SO₄: sodium sulfate
- NaBH₄: sodium borohydride
- NaHCO₃: sodium bicarbonate
- LiHMDS: lithium hexamethyldisilylamide
- NaHMDS: sodium hexamethyldisilylamide
- LAH: lithium aluminum hydride
- NaBH₄: sodium borohydride
- LDA: lithium diisopropylamide
- PPh₃: Triphenylphosphine
- ZnEt₂: Diethyl zinc
- Et₃N: triethylamine
- DMAP: 4-(dimethylamino)pyridine
- DIEA: N,N-diisopropylethylamine
- NH₄OH: ammonium hydroxide
- EDCI: 1-ethyl-3-(3-dimethylaminopropyl)carbodiimide
- HOBt: 1-hydroxybenzotriazole
- HATU: O-(7-azabenzotriazol-1-yl)-N,N,N',N'-tetra-methyluronium
- BINAP: 2,2'-bis(diphenylphosphanyl)-1,1'-binaphthyl
- Pd(dppf)Cl₂: [1,1']-Bis(diphenylphosphino)ferrocene]dichloropalladium(II)
- TBAI: Tetrabutylammonium iodide
- TEBA: Benzyltriethylammonium chloride
- TMSCN: Trimethylsilyl cyanide
- NMP: 1-Methyl-pyrrolidin-2-one
- MsCl: Methanesulfonyl chloride
- DPPA: Diphenylphosphoryl azide
- Pd(OH)₂: Palladium (II) hydroxide
- DAST: diethylaminosulfur trifluoride

### General experimental notes

In the following examples, the reagents (chemicals) were purchased from commercial sources (such as Alfa, Acros, Sigma Aldrich, TCI and Shanghai Chemical Reagent Company), and used without further purification. Flash chromatography was performed on an Ez Purifier III via column with silica gel particles of 200-300 mesh. Analytical and preparative thin layer chromatography plates (TLC) were HSGF 254 (0.15-0.2 mm thickness, Shanghai Anbang Company, China). Nuclear magnetic resonance (NMR) spectra were obtained on a Brucker AMX-300 or a AMX-300 NMR (Brucker, Switzerland). Chemical shifts were reported in parts per million (ppm, δ) downfield from tetramethylsilane. Mass spectra were run with electrospray ionization (ESI) from a Waters LCT TOF Mass Spectrometer (Waters, USA). HPLC chromatographs were recorded on an Agilent 1200 Liquid Chromatography (Agilent, USA, column: Ultimate 4.6mmx50mm, 5µM, mobile phase A: 0.1% formic acid in water; mobile phase B: acetonitrile). Microwave reactions were run on an Initiator 2.5 Microwave Synthesizer (Biotage, Sweden).
**Example 1. Preparation of N-cyclohexyl-2-[(2-imidazol-1-yl-acetyl)-thiophen-2-ylmethyl-amino]-2-o-tolyl-acetamide (Compound 204) and its HCl Salt.** Compound 204 was prepared according to Scheme 1, above, using the following protocol. 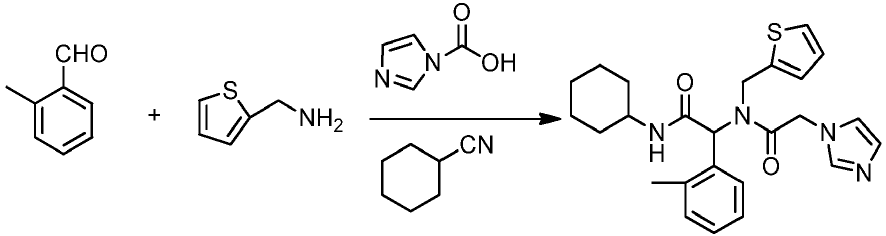
***Step A: Compound 204.*** A mixture of 2-methyl-benzaldehyde (193 mg, 1.61 mmol) and thiophen-2-yl-methylamine (182 mg, 1.61 mmol) in MeOH (4 ml) was stirred at RT for 30 minutes. Imidazol-1-yl-acetic acid (202 mg, 1.61 mmol) was added and the reaction mixture stirred for 10 minutes. Cyclohexyl isocyanide (176 mg, 1.61 mmol) was then added and the reaction mixture was stirred at RT overnight. The precipitate was filtered and washed with MeOH to afford the desired product (463 mg, 64% yield). ¹H NMR (300 MHz, DMSO-d6): δ 8.15-8.01 (m, 1H), 7.62-7.52 (m, 1H), 7.31-6.69 (m, 9H), 6.24 (s, 1H), 5.65-4.66 (m, 4H), 2.60 (m, 1H), 2.20-2.05 (m, 3H), 1.76-1.51 (m, 5H), 1.29-0.83 (m, 5H); MS: 451.2 (M+1)⁺.
***Step B: Compound 204 HCl Salt.*** Compound 204 (460 mg, 1.02 mmol) in HCl/Et₂O (5 M, 20 ml) was stirred at room temperature for 3 hours. The resulting mixture was concentrated and the solid was treated with Et₂O to give the HCl salt (350 mg, 70% yield). ¹H NMR (400 MHz, DMSO-d6): δ 14.43 (s, 1H), 9.15-9.04 (m, 1H), 8.28-8.05 (m, 1H), 7.64-6.23 (m, 10H), 5.95-4.41 (m, 4H), 3.60 (m, 1H), 2.23 (s, 3H), 1.74-1.51 (m, 5H), 1.30-0.71 (m, 5H); MS: 451.1 (M+1)⁺.

The following analogs were synthesized via the procedure set forth in Scheme 1, using the appropriate aldehyde of R²(**a**), amine of R³(**b**), carboxylic acid of R⁴(**c**), and cyano of R¹ (**d**) using the reagents and solvents set forth in step A, above, and purified via various method including TLC, Chromatography, HPLC or chiral HPLC. The corresponding HCl salt was made as set forth in step B, above.

### Compound 361

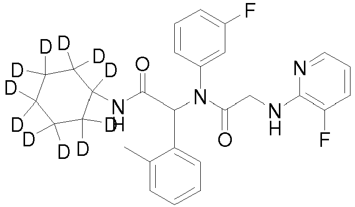

¹H NMR (400 MHz, CDCl₃): δ 7.76 (br, 1H), 7.16-7.09 (m, 4H), 6.93-6.78 (m, 3H), 6.50(m, 1H), 6.37 (d, 1H), 5.60(s, 1H), 4.29(d, 1H), 3.88 (dq, 2H), 2.39 (s, 3H); MS: 504.1 (M+1)⁺.

### Compound 342

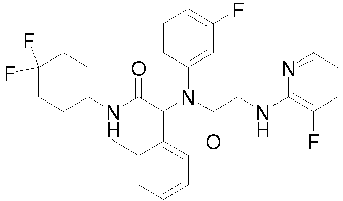

¹H NMR(400 MHz, MeOD-d4): δ 7.66(d, 2H), 7.17-6.95 (m, 4H), 6.86-6.67 (m, 4H), 6.49(m, 1H), 6.28 (S, 1H), 3.84 (d, 1H), 3.80 (m, 1H), 2.36 (s, 3H), 1.95-1.74 (m, 6H), 1.52-1.34 (m, 2H); MS: 529.2 (M+1)⁺.

### Compound 379

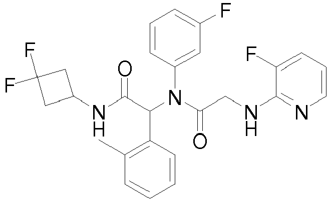

¹H NMR (400 MHz, DMSO-d6): 8.60 (m, 1H), 7.80 (d, 1H, *J* = 4.8), 7.39-7.34 (m, 1H), 7.19-7.05 (s, 4H), 6.90 (t, 1H, *J* = 4.0), 6.67-6.56 (m, 4H), 6.24 (s, 1H), 4.11(br, 1H), 3.96 (dd, 1H, *J* = 15.2, 3.2), 3.62 (dd, 1H, *J* = 15.2, 3.2), 2.95 (br, 1H), 2.40(s, 3H), 1.31-1.18 (m, 4H); MS: 500.7 (M+1)⁺.

### Compound 17

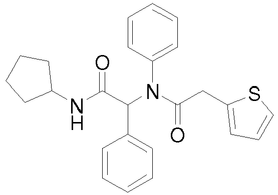

¹H NMR(300 MHz, CDCl₃): δ 7.22-7.09 (m, 9H), 6.89-6.86 (m, 1H), 6.71-6.70 (m, 1H), 6.03(s, 1H), 5.73-5.70 (d, 1H), 4.23(m, 1H), 3.62(s, 2H), 2.36(s, 3H), 1.96 (m, 1H), 1.58-1.54(m, 5H), 1.40-1.35 (m, 2H); 419.1 (M+1)⁺.

### Compound 333 (HCl Salt)

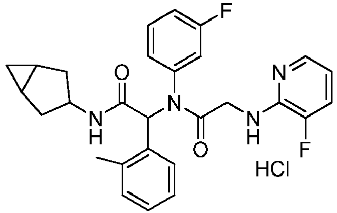

¹H NMR (400 MHz, MeOD-d4): δ 8.12 (br, 1H), 7.82 (br, 1H), 7.46 (s, 2H), 7.16-6.82 (m, 7H), 6.35 (s, 1H), 5.04 (d, 1H), 4.78 (d, 1H), 4.33 (br, 2H), 2.59 (s, 3H), 2.48 (s, 3H), 2.30-2.27 (m, 2H), 1.75-1.68 (m,2H), 1.37-1.29 (m,2H), 0.46 (q, 1H), 0.01 (q, 1H); MS: 491.2 (M+1)⁺.

### Compound 268

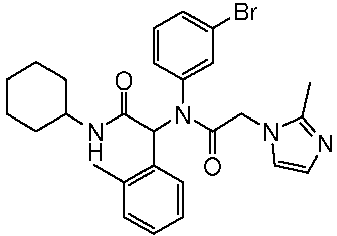

¹H NMR (400 MHz, DMSO-d6): δ 8.22-7.99 (m, 2H), 7.37-7.35 (d, 1H, J=6.8), 7.29-6.62 (m, 8H), 6.18 (s, 1H), 4.66-4.61 (m, 1H), 4.37-4.30 (m, 1H), 3.61 (s, 1H), 2.36 (s, 3H), 2.09-2.01 (m, 3H), 1.73-1.52 (m, 5H), 1.25-0.95 (m, 5H); MS: 523.0 (M+1)⁺.

### Compound 227

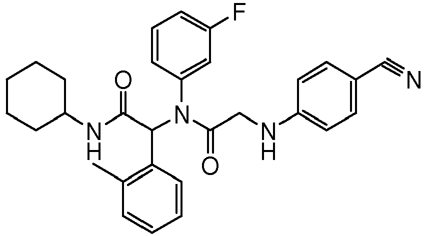

¹H NMR (400 MHz, DMSO-d6): δ 8.03 (s, 1H), 7.85 (dr, 1H), 7.44-7.42 (d, 2H, J=8.8), 7.12-6.99 (m, 4H), 6.89-6.73 (m, 4H), 6.56-6.54 (d, 2H, J=8.8), 6.22(s, 1H), 3.86-3.80 (m, 1H), 3.63-3.61 (m, 1H), 3.44-3.40 (m, 1H), 2.37 (s, 3H), 1.76-1.52 (m, 5H), 1.29-0.96 (m, 5H); MS: 499.2 (M+1)⁺.

### Compound 228

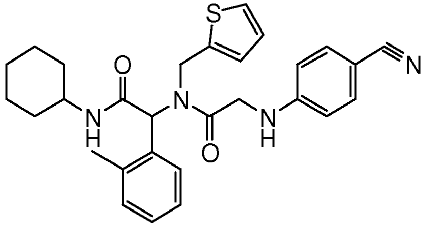

¹H NMR (400 MHz, DMSO-d6): δ 8.16-8.00 (m, 1H), 7.51-7.41 (m, 2H), 7.33-7.17 (m, 4H), 7.08-6.93 (m, 1H), 6.81-6.78 (m, 1H), 6.67-6.54 (m, 3H), 6.29-5.66 (m, 1H), 5.04-4.85 (m, 1H), 4.72-4.42 (m, 1H), 4.27-4.06 (m, 1H), 3.90-3.77 (m, 1H), 3.61 (s, 1H), 2.22-2.01 (m, 3H), 1.75-1.52 (m, 5H), 1.29-1.09 (m, 5H); MS: 501.2 (M+1)⁺.

### Compound 329

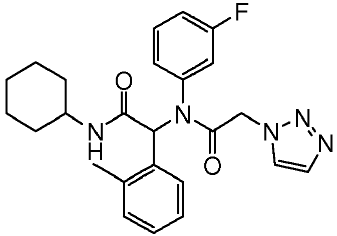

¹H NMR (400 MHz, DMSO-d6): δ 8.05-8.01 (m, 2H), 7.89-7.71 (m, 2H), 7.28-7.03 (m, 4H), 6.89-6.86 (m, 1H), 6.74-6.72 (d, 1H, J=7.2), 6.19 (s, 1H), 5.20-5.16 (d, 1H, J=15.6), 4.92-4.89 (m, 1H), 3.63-3.61 (m, 1H), 2.39 (s, 3H), 1.70-1.51 (m, 5H), 1.27-0.94 (m, 5H); MS: 450.2 (M+1)⁺.

### Compound 42

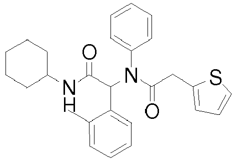

¹H NMR (300 MHz, DMSO-d6): δ 7.92 (d, 2H, *J*=7.8 Hz), 7.35-7.33 (m, 1H), 7.29-7.25 (m, 1H), 7.14-7.06 (m, 2H), 6.98 (t, 2H, *J*=7.5 Hz), 6.91-6.82 (m,1H), 6.79 (t, 1H, *J*=7.5 Hz), 6.69-6.66 (m,2H), 6.55-6.50 (m, 1H), 6.24 (s, 1H), 3.65-3.45 (m, 3H), 2.30 (s, 3H), 1.77-1.51 (m, 5H), 1.25-0.93 (m, 5H); MS: 447.2 (M+1)⁺.

### Compound 113

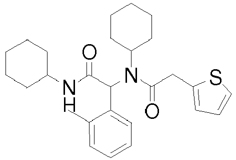

¹H NMR (300 MHz, DMSO-d6): δ 7.99-7.40 (m, 1H), 7.37 (d, 1H, *J*=6.6 Hz), 7.23 (br, 4H), 6.94-6.89 (m, 2H), 5.66 (s,1H), 4.00-3.90 (m,2H), 3.57 (s, 1H), 3.00 (s,1H), 2.27-1.91 (m, 5H), 1.71-1.31 (m, 6H), 1.26-0.63 (m, 12H); MS: 451.64 (M-1)⁻.

### Compound 166

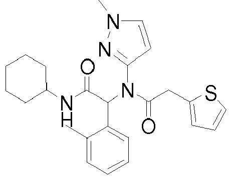

¹H NMR (300 MHz, DMSO-d6): δ 8.09 (d, 2H, *J*=7.5 Hz), 7.43 (d, 1H, *J*=1.8 Hz), 7.36-7.34 (m, 1H), 7.11-7.04 (m, 2H), 6.95-6.90 (m, 2H), 6.80-6.78 (m,2H), 6.11(s, 1H), 5.89 (d,1H, *J*=2.1 Hz), 3.73-3.35 (m, 6H), 2.26 (s, 3H), 1.74-1.50 (m, 5H), 1.34-1.08 (m, 5H); MS: 451.2 (M+1)⁺.

### Compound 205

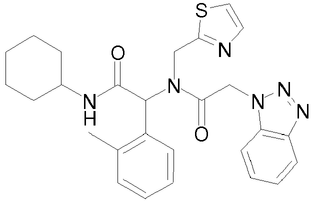

¹H NMR (300 MHz, DMSO-d6): δ 8.11-8.08 (m, 2H), 7.76-7.74 (m, 2H), 7.62-7.59 (m, 1H), 7.50-7.49 (m, 1H), 7.46-7.42 (m, 1H), 7.27 (d, 1H, *J*=5.7 Hz), 7.24-7.22 (m, 1H), 7.17-7.14 (m, 1H), 6.88 (d, 1H, *J*=5.7 Hz), 6.26-6.24 (m, 2H), 6.11(s, 1H), 5.28-4.90 (m, 2H), 3.63-3.60 (m, 1H), 1.99 (s, 3H), 1.76-1.49 (m, 5H), 1.27-1.06 (m, 5H); MS: 503.2 (M+1)⁺.

### Compound 15

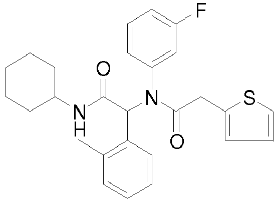

¹H NMR (300 MHz, DMSO-d6): δ 7.15-6.72 (m, 10H), 6.40 (s, 1H), 5.38-5.36 (m, 1H), 3.85-3.81 (m, 1H), 3.65 (s, 1H), 2.35 (s, 3H), 1.97-1.56 (m, 5H), 1.36-0.96 (m, 5H); MS: 465.2 (M+1)⁺.

### Compound 230

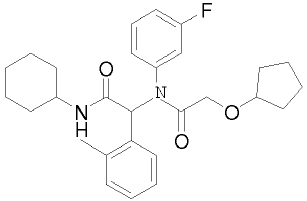

¹H NMR (300 MHz, DMSO-d6): δ 8.02-7.99 (d, 2H), 7.09-6.69 (m, 7H), 6.20 (s, 1H), 3.83-3.57 (m, 4H), 2.34 (s, 3H), 1.73-1.19 (m, 18H); MS: 467.3 (M+1)⁺.

### Compound 214

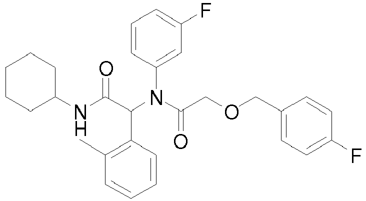

¹H NMR (300 MHz, DMSO-d6): δ 8.05-8.03 (d, 2H), 7.33-6.72 (m, 11H), 6.25 (s, 1H), 4.40 (s, 2H), 3.99-3.95 (d, 1H), 3.73-3.69 (d, 1H), 3.67-3.62 (m, 1H), 2.35 (s, 3H), 1.79-1.53 (m, 5H), 1.30-0.97 (m, 5H); MS: 507.2 (M+1)⁺.

### Compound 176

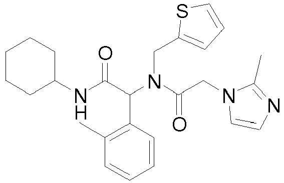

¹H NMR (300 MHz, DMSO-d6): δ 8.22-7.99 (m, 1H), 7.31-6.71 (m, 9H), 6.25 (s, 1H), 5.68-4.71 (m, 4H), 3.61-3.57 (m, 1H), 2.22-2.01 (m, 6H), 1.76-1.51 (m, 5H), 1.30-0.95 (m, 5H); MS: 465.2 (M+1)⁺.

### Compound 204

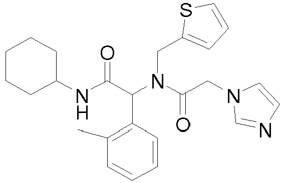

¹H NMR (300 MHz, DMSO-d6): δ 8.15-8.01 (m, 1H), 7.62-7.52 (m, 1H), 7.31-6.69 (m, 9H), 6.24 (s, 1H), 5.65-4.66 (m, 4H), 2.60 (m, 1H), 2.20-2.05 (m, 3H), 1.76-1.51 (m, 5H), 1.29-0.83 (m, 5H); MS: 451.2 (M+1)⁺.

### Compound 13

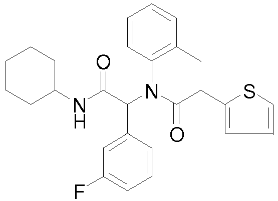

¹H NMR (300 MHz, DMSO-d6): δ 7.49-6.80 (m, 9H), 6.65 (s, 1H), 6.11-5.95 (m, 1H), 5.94-5.39 (m, 1H), 3.80-3.74 (m, 1H), 3.56 (s, 1H), 2.10 (s, 1.5H), 1.84 (s, 1.5H), 1.93-1.52 (m, 5H), 1.39-1.01 (m, 5H); MS: 465.2 (M+1)+.

### Compound 243

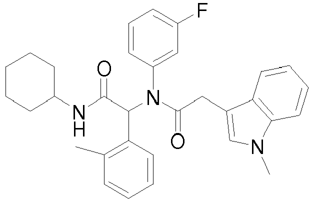

¹H NMR (400 MHz, DMSO-d6): δ7.97-7.80 (m, 2H), 7.37-6.26 (m, 13H), 3.71 (s, 3H), 3.62-3.50 (m, 3H), 2.33 (s, 3H), 1.75-1.51 (m, 5H), 1.28-0.94 (m, 5H); MS: 512.2 (M+1)⁺.

### Compound 305

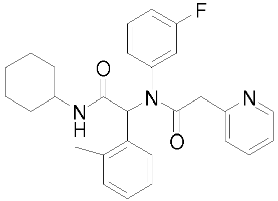

¹H NMR (400 MHz, DMSO-d6): δ 8.42-8.41 (d, 1H, J=4.0 MHz), 8.01 (s, 1H), 7.67-7.66 (m, 2H), 7.23-6.25 (m, 10H), 3.67-3.54 (m, 2H), 3.17 (d, 1H, J=4.8MHz), 2.38 (s, 3H), 1.77-1.52 (m, 5H), 1.29-0.87 (m, 5H); MS: 460.1 (M+1)⁺.

### Compound 311

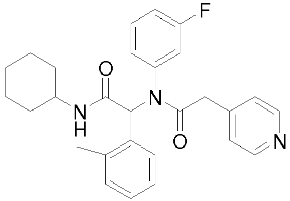

¹H NMR (400 MHz, DMSO-d6): δ 8.44-8.43 (m, 2H), 8.02 (s, 1H), 7.73 (s, 1H), 7.25-6.53 (m, 9H), 6.24 (s, 1H), 3.62-3.35 (m, 3H), 2.36 (s, 3H), 1.72-1.52 (m,5H), 1.23-0.93 (m, 5H); MS: 460.1 (M+1)⁺.

### Compound 294

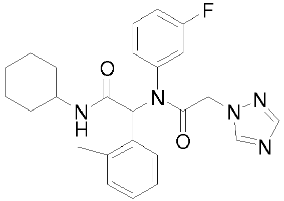

¹H NMR (400 MHz, DMSO-d6): δ 8.39 (s, 1H), 8.05-7.87 (m, 3H), 7.36-6.58 (m, 7H), 6.19 (s, 1H), 4.96-4.70 (m, 2H), 3.61 (m, 1H), 2.39 (s, 3H), 1.74-1.52 (m, 5H), 1.28-0.93 (m, 5H); MS: 450.1 (M+1)⁺.

### Compound 320

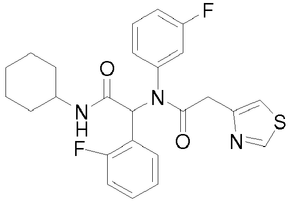

¹H NMR (400 MHz, DMSO-d6): δ 8.98 (d, 1H, J=1.6 MHz), 8.19-8.17 (d, 1H, J=7.2 MHz), 7.62-6.69 (m, 9H), 6.31 (s, 1H), 3.67-3.52 (m, 3H), 1.74-1.55 (m, 5H), 1.29-0.99 (m, 5H); MS: 470.0 (M+1)⁺.

### Compound 312

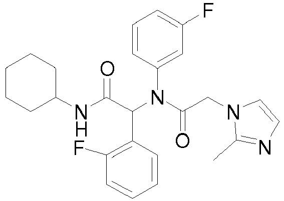

¹H NMR (400 MHz, DMSO-d6): δ 8.22 (d, 1H, J=4.8 MHz), 7.75 (s, 1H), 7.26-6.71 (m, 9H), 6.27 (s, 1H), 4.75-4.39 (m, 2H), 3.62 (m, 1H), 2.13 (s, 3H), 1.75-1.54 (m, 5H), 1.27-0.99 (m, 5H); MS: 467.1 (M+1)⁺.

### Compound 46

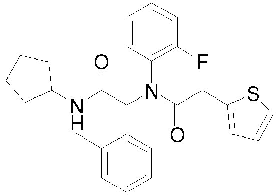

¹H NMR (300 MHz, DMSO-d6): δ 8.12-8.07 (m, 1H), 8.02 (d, 1H, J=6.9 Hz), 7.36-6.66 (m, 10H), 6.31 (s, 1H), 4.09-4.02 (m, 1H), 3.54 (s, 2H), 2.36 (s, 3H), 1.86-1.14 (m, 8H); MS: 451.1 (M+1)⁺.

### Compound 47

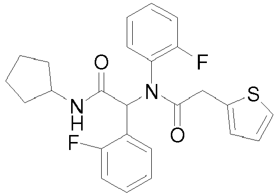

¹H NMR (300 MHz, DMSO-d6): δ 8.19 (d, 1H, J=7.2 Hz), 7.95-7.94 (m, 1H), 7.36-6.73 (m, 10H), 6.35(s, 1H), 4.08-4.02 (m, 1H), 3.55 (s, 2H), 1.85-1.15 (m, 8H); MS: 455.1 (M+1)⁺.

### Compound 2

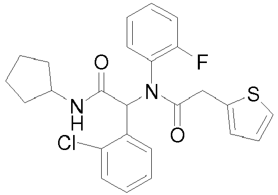

¹H NMR (300 MHz, DMSO-d6): δ 8.22 (d, 1H, J=7.2 Hz), 8.05-7.99 (m, 1H), 7.37-6.75 (m, 10H), 6.49 (s, 1H), 4.08-4.02 (m, 1H), 3.56 (s, 2H), 1.84-1.16 (m, 8H); MS: 471.1 (M+1)⁺.

### Compound 48

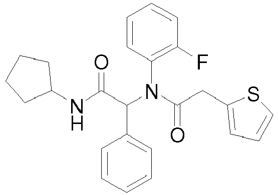

¹H NMR (300 MHz, DMSO-d6): δ 8.09 (d, 1H, J=7.2 Hz), 7.90-7.89 (m, 1H), 7.36-6.72 (m, 11H), 5.99 (s, 1H), 4.05-4.02 (m, 1H), 3.52 (s, 2H), 1.77-1.21 (m, 8H); MS: 437.1 (M+1)⁺.

### Compound 49

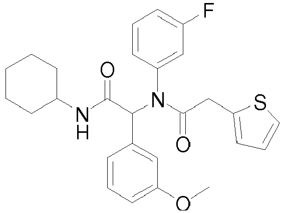

¹H NMR (300 MHz, DMSO-d6): δ 8.00 (d, 1H, J=7.5 Hz), 7.36-6.60 (m, 11H), 6.03 (s, 1H), 3.60-3.56 (m, 6H), 1.71-1.56 (m, 5H), 1.24-0.93 (m, 5H); MS: 481.1 (M+1)⁺.

### Compound 50

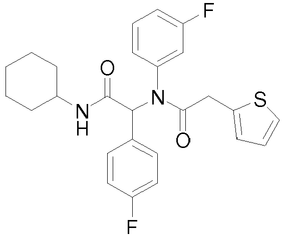

¹H NMR (300 MHz, DMSO-d6): δ 8.05 (d, 1H, J=7.5 Hz), 7.36-6.73 (m, 11H), 6.05 (s, 1H), 3.58-3.56 (m, 3H), 1.72-1.50 (m, 5H), 1.20-0.91 (m, 5H); MS: 469.1 (M+1)⁺.

### Compound 51

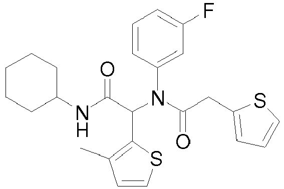

¹H NMR (300 MHz, DMSO-d6): δ 8.08 (d, 1H, J=7.5 Hz), 7.36-6.71 (m, 9H), 6.32 (s, 1H), 3.59-3.56 (m, 3H), 2.14 (s, 3H), 1.73-1.48 (m, 5H), 1.25-1.02 (m, 5H); MS: 471.1 (M+1)⁺.

### Compound 115

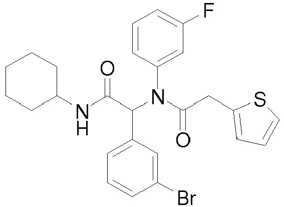

¹H NMR (300 MHz, DMSO-d6): δ 8.08 (d, 1H, J=7.5 Hz), 7.36-6.73 (m, 11H), 6.04 (s, 1H), 3.60-3.57 (m, 3H), 1.71-1.55 (m, 5H), 1.25-1.01 (m, 5H); MS: 529.1 (M+1)⁺.

### Compound 89

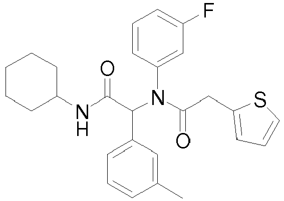

¹H NMR (300 MHz, DMSO-d6): δ 7.98 (d, 1H, J=7.5 Hz), 7.36-6.73 (m, 11H), 6.03 (s, 1H), 3.58-3.56 (m, 3H), 2.14 (s, 3H), 1.71-1.46 (m, 5H), 1.25-0.94 (m, 5H); MS: 465.2 (M+1)⁺.

### Compound 91

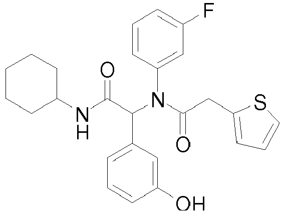

¹H NMR (300 MHz, DMSO-d6): δ 9.24 (s, 1H), 8.01-7.98 (m, 1H), 7.36-6.47 (m, 11H), 5.98 (s, 1H), 3.58-3.54 (m, 3H), 1.71-1.50 (m, 5H), 1.24-0.97 (m, 5H); MS: 467.1 (M+1)⁺.

### Compound 62

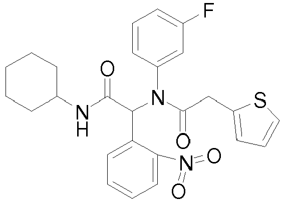

¹H NMR (300 MHz, DMSO-d6): δ 8.15 (d, 1H, J=7.5 Hz), 7.98-7.95 (m, 1H), 7.46-6.76 (m, 10H), 6.47 (s, 1H), 3.65-3.51 (m, 3H), 1.66-1.52 (m, 5H), 1.23-0.91 (m, 5H); MS: 496.1 (M+1)⁺.

### Compound 92

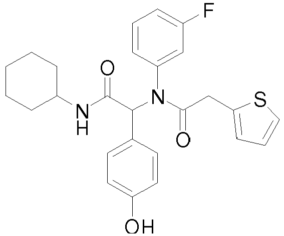

¹H NMR (300 MHz, DMSO-d6): δ 9.32 (s, 1H), 7.91 (d, 1H, J=7.5 Hz), 7.35-6.47 (m, 11H), 5.95 (s, 1H), 3.55-3.53 (m, 3H), 1.77-1.55 (m, 5H), 1.24-0.96 (m, 5H); MS: 467.1 (M+1)⁺.

### Compound 65

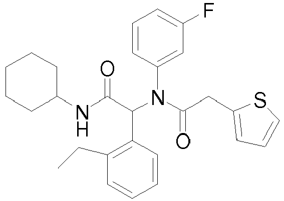

¹H NMR (300 MHz, DMSO-d6): δ 7.98 (d, 1H, J=7.5 Hz), 7.36-6.27 (m, 11H), 5.75 (s, 1H), 3.62-3.57 (m, 3H), 2.74-2.64 (m, 2H), 1.74-1.48 (m, 5H), 1.28-0.95 (m, 8H); MS: 479.2 (M+1)⁺.

### Compound 116

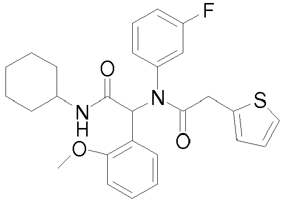

¹H NMR (300 MHz, DMSO-d6): δ 8.04 (d, 1H, J=7.5 Hz), 7.37-6.63 (m, 11H), 6.24 (s, 1H), 3.79(s, 1H), 3.64-3.54 (m, 3H), 1.74-1.50 (m, 5H), 1.26-0.97 (m, 5H); MS: 481.2 (M+1)⁺.

### Compound 94

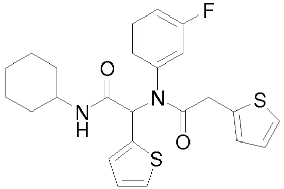

¹H NMR (300 MHz, DMSO-d6): δ 8.10 (d, 1H, J=7.8 Hz), 7.37-6.71 (m, 10H), 6.29 (s, 1H), 3.59-3.55 (m, 3H), 1.75-1.56 (m, 5H), 1.24-1.03 (m, 5H); MS: 457.1 (M+1)⁺.

### Compound 127

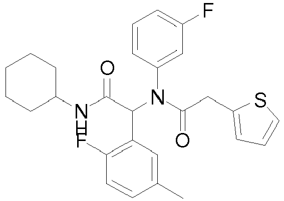

¹H NMR (300 MHz, DMSO-d6): δ 8.13 (d, 1H, J=7.5 Hz), 7.36-6.64 (m, 10H), 6.26 (s, 1H), 3.69-3.57 (m, 3H), 2.04 (s, 3H), 1.74-1.50 (m, 5H), 1.23-1.00 (m, 5H); MS: 483.1 (M+1)⁺.

### Compound 128

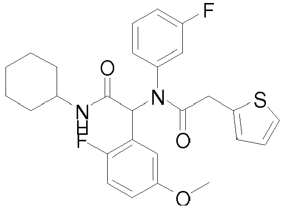

¹H NMR (300 MHz, DMSO-d6): δ 8.19 (d, 1H, *J*=7.5 Hz), 7.36-6.34 (m, 10H), 6.25 (s, 1H), 3.64-3.58 (m, 3H), 3.51 (s, 3H), 1.75-1.50 (m, 5H), 1.26-0.99 (m, 5H); MS: 499.1 (M+1)⁺.

### Compound 203

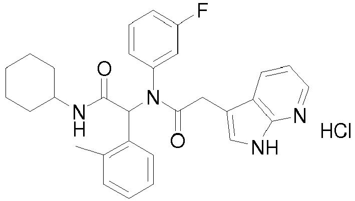

¹H NMR (300 MHz, DMSO-d6): δ 12.05 (s, 1H), 8.33-8.31 (m, 1H), 8.13-7.76 (m, 2H), 7.31-6.61 (m, 10H), 6.26 (s, 1H), 3.66-3.37 (m, 3H), 2.34 (s, 3H), 1.73-1.50 (m, 5H), 1.23-0.95 (m, 5H); MS: 499.2 (M+1)⁺.

### Compound 213

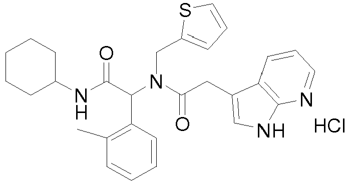

¹H NMR (300 MHz, DMSO-d6): δ 12.03 (s, 1H), 8.33-7.96 (m, 3H), 7.50-5.66 (m, 10H), 5.00-3.87 (m, 4H), 3.79 (m, 1H), 2.19 (s, 1.5H), 1.78-1.51 (m, 6.5H), 1.29-1.04 (m, 5H); MS: 501.2 (M+1)⁺.

### Compound 261

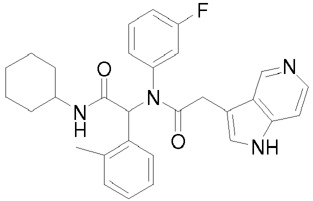

¹H NMR (400 MHz, DMSO-d6): δ 12.02 (s, 1H), 8.84 (s, 1H), 8.27-6.63 (m, 12H), 6.26 (s, 1H), 3.73-3.51 (m, 3H), 2.36 (s, 3H), 1.74-1.52 (m, 5H), 1.27-0.93 (m, 5H); MS: 499.1 (M+1)⁺.

### Compound 269

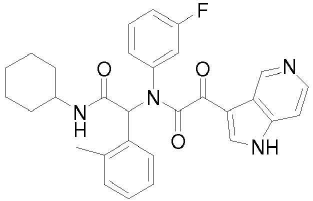

¹H NMR (400 MHz, DMSO-d6): δ 12.52 (s, 1H), 9.08 (s, 1H), 8.47-6.75 (m, 12H), 6.41 (s, 1H), 3.78-3.76 (m, 1H), 2.38 (s, 3H), 1.91-1.56 (m, 5H), 1.35-0.85 (m, 5H); MS: 513.1 (M+1)⁺.

### Compound 223

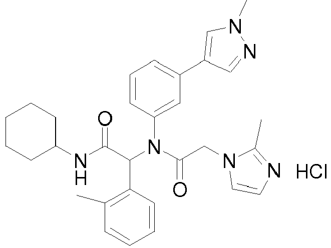

¹H NMR (400 MHz, DMSO-d6): δ 14.30-14.24 (m, 1H), 8.03-6.83 (m, 13H), 6.17 (s, 1H), 5.03-4.66 (m, 2H), 3.89-3.52 (m, 4H), 2.49-2.37 (m, 6H), 1.75-1.71 (m, 5H), 1.25-1.06 (m, 5H); MS: 525.3 (M+1)⁺.

### Compound 275

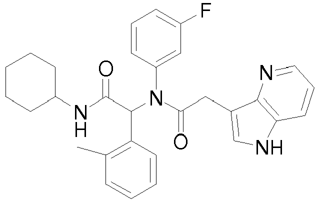

¹H NMR (400 MHz, DMSO-d6): δ 11.40 (s, 1H), 8.30 (s, 1H), 8.01-6.72 (m, 12H), 6.27 (s, 1H), 3.66-3.17 (m, 3H), 2.37 (s, 3H), 1.73-1.52 (m, 5H), 1.28-0.95 (m, 5H); MS: 499.1 (M+1)⁺.

### Compound 276

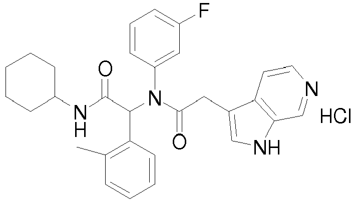

¹H NMR (400 MHz, DMSO-d6): δ 14.92 (d, 1H, J=1.6 MHz), 12.74 (s, 1H), 9.09 (s, 1H), 8.25-6.72 (m, 12H), 6.25 (s, 1H), 3.77-3.53 (m, 3H), 2.37 (s, 3H), 1.73-1.52 (m, 5H), 1.27-0.97 (m, 5H); MS: 499.1 (M+1)⁺.

### Compound 283

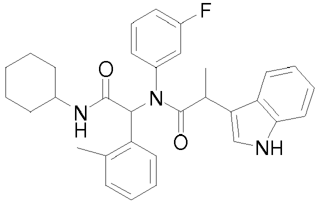

¹H NMR (400 MHz, DMSO-d6): δ 10.87 (s, 1H), 7.95-6.23 (m, 15H), 3.75-3.50 (m, 2H), 2.41 (s, 1.43 H), 2.13 (s, 1.59H), 1.77-1.54 (m, 5H), 1.39-1.09 (m, 8H); MS: 512.2 (M+1)⁺.

### Compound 304

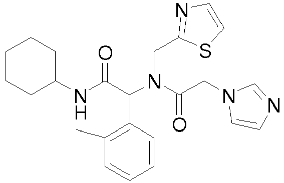

¹H NMR (400 MHz, DMSO-d6): δ 8.12 (d, 1H, J=7.6 MHz), 7.87 (s, 1H), 7.64 (d, 1H, J=3.2 MHz), 7.45 (d, 1H, J=3.2 MHz), 7.25-7.12 (m, 4H), 6.99 (s, 1H), 6.89 (d, 1H, J=7.6 MHz), 6.17 (s, 1H), 5.38 (d, 2H, J=4.0 MHz), 5.10 (d, 1H, J=18.4 MHz), 4.83 (d, 1H, J=18.4 MHz), 3.61 (m, 1H), 1.99 (s, 3H), 1.76-1.51 (m, 5H), 1.29-0.99 (m, 5H); MS: 452.1 (M+1)⁺.

### Compound 26

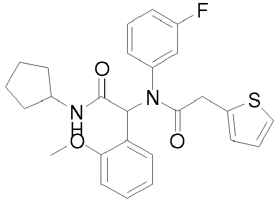

¹H NMR (300 MHz, DMSO-d6): δ 8.12 (d, 1H, J = 8), 7.36-6.62 (m, 9H), 6.23 (s, 1H), 4.05 (m, 1H), 3.78 (s, 3H), 3.61-3.50 (m, 2H), 1.78 (m, 2H), 1.58-1.43 (m, 5H), 1.24 (m, 1H); MS: 467.1 (M+1)⁺.

### Compound 43

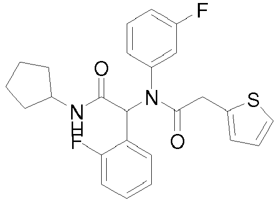

¹H NMR (300 MHz, DMSO-d6): δ 8.25 (d, 1H, J = 8), 7.36-6.74 (m, 9H), 6.29 (s, 1H), 4.08 (m, 1H), 3.63 (m, 2H), 1.78 (m, 2H), 1.58-1.43 (m, 5H), 1.25 (m, 1H); MS: 455.1 (M+1)⁺.

### Compound 44

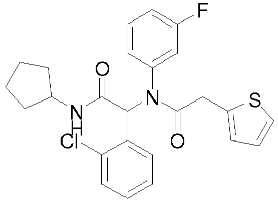

¹H NMR (300 MHz, DMSO-d6): δ 8.29 (d, 1H, J = 8), 7.35-6.72 (m, 9H), 6.34 (s, 1H), 4.09 (m, 1H), 3.68-3.53 (m, 2H), 1.78 (m, 2H), 1.63-1.48 (m, 5H), 1.26 (m, 1H); MS: 471.1 (M+1)⁺.

### Compound 45

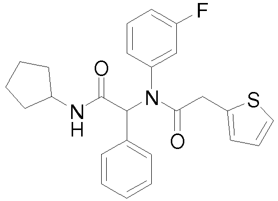

¹H NMR (300 MHz, DMSO-d6): δ 8.12 (d, 1H, J = 8), 7.35-6.72 (m, 10H), 6.06 (s, 1H), 4.05 (m, 1H), 3.61-3.51 (m, 2H), 1.87-1.64 (m, 2H), 1.60-1.38 (m, 5H), 1.28(m, 1H); MS: 437.1 (M+1)⁺.

### Compound 129

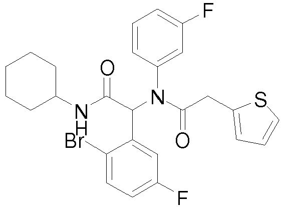

¹H NMR (300 MHz, DMSO-d6): δ 8.35 (d, 1H, J = 7.5), 7.62-6.64 (m, 8H), 6.24 (s, 1H), 3.69-3.04 (m, 3H), 1.75-1.50 (m, 5H), 1.36-0.96 (m, 5H); MS: 547.0, 549.0 (M+1)⁺.

### Compound 102

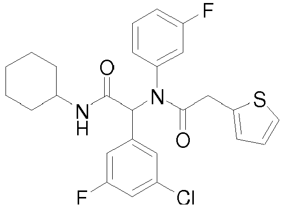

¹H NMR (300 MHz, DMSO-d6): δ 8.14 (d, 1H, J = 7.5), 7.37-6.74 (m, 9H), 6.02 (s, 1H), 3.60 (m, 3H), 1.73-1.50 (m, 5H), 1.32-0.96 (m, 5H); MS: 503.1, 505.1 (M+1)⁺.

### Compound 103

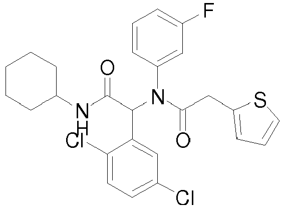

¹H NMR (300 MHz, DMSO-d6): δ 8.35 (d, 1H, J = 7.5), 7.41-6.74 (m, 8H), 6.30 (s, 1H), 3.66-3.52 (m, 3H), 1.73-1.50 (m, 5H), 1.32-1.02 (m, 5H); MS: 519.1, 521.1 (M+1)⁺.

### Compound 78

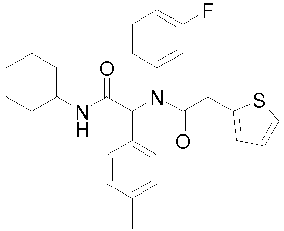

¹H NMR (300 MHz, DMSO-d6): δ 7.97 (d, 1H, J = 7.5), 7.34-6.74 (m, 10H), 6.02 (s, 1H), 3.57 (m, 3H), 2.15 (s, 3H), 1.73-1.50 (m, 5H), 1.32-0.95 (m, 5H); MS: 465.2 (M+1)⁺.

### Compound 80

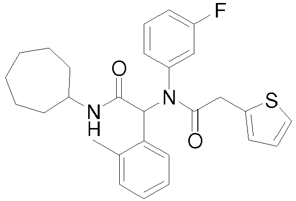

¹H NMR (300 MHz, DMSO-d6): δ 8.05 (d, 1H, J = 7), 7.77-6.72 (m, 10H), 6.23 (s, 1H), 3.80 (m, 1H), 3.60 (m, 2H), 2.34 (s, 3H), 1.80-1.25 (m, 12H); MS: 479.2 (M+1)⁺.

### Compound 67

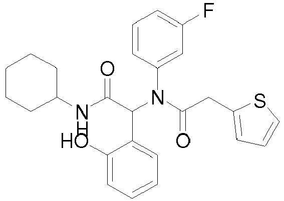

¹H NMR (300 MHz, DMSO-d6): δ9.70 (s, 1H), 8.02 (d, 1H, J = 7.5), 7.35-6.44 (m, 11H), 6.20 (s, 1H), 3.60 (m, 3H), 1.70-1.50 (m, 5H), 1.24-1.00 (m, 5H); MS: 467.1 (M+1)⁺.

### Compound 106

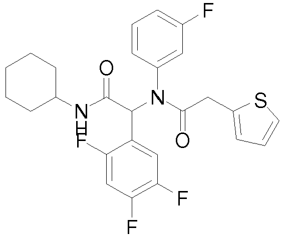

¹H NMR (300 MHz, DMSO-d6): δ 8.22 (d, 1H, J = 7.5), 7.52-6.75 (m, 8H), 6.23 (s, 1H), 3.62 (m, 3H), 1.70-1.50 (m, 5H), 1.35-1.00 (m, 5H); MS: 505.1 (M+1)⁺.

### Compound 114

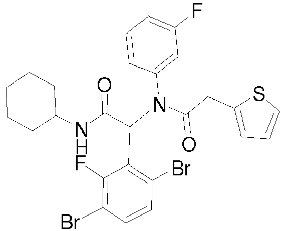

¹H NMR (300 MHz, DMSO-d6): δ 7.99 (d, 1H, J = 7.5), 7.73-6.78 (m, 8H), 6.39 (s, 1H), 3.64-3.50 (m, 3H), 1.70-1.50 (m, 5H), 1.35-1.00 (m, 5H); MS: 624.9 (M+1)⁺.

### Compound 87

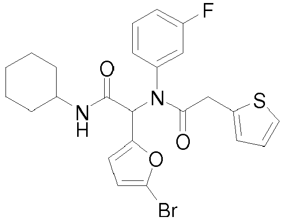

¹H NMR (300 MHz, DMSO-d6): δ 8.13 (d, 1H, J = 7.5), 7.35-6.73 (m, 5H), 6.30 (d, 1H, J = 3.3), 6.12 (s, 1H), 6.07 (d, 1H, J = 3.3), 3.60 (m, 3H), 1.72-1.50 (m, 5H), 1.30-1.00 (m, 5H); MS: 519.0 (M+1)⁺.

### Compound 108

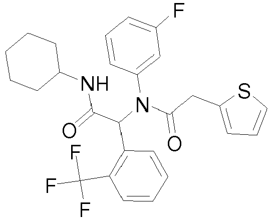

1H NMR (300 MHz, DMSO-d6): δ 8.02 (d, 1H, J = 7.5), 7.65-6.73 (m, 8H), 6.40 (s, 1H), 3.59(m, 3H), 1.72-1.50 (m, 5H), 1.39-1.00 (m, 5H); MS: 519.1 (M+1)⁺.

### Compound 130

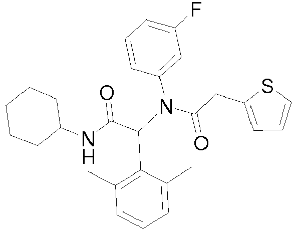

¹H NMR (300 MHz, DMSO-d6): δ 7.79 (m, 2H), 7.45-6.67 (m, 7H), 6.40 (s, 1H), 5.85-5.64 (m, 1H), 3.56 (m, 3H), 2.15-1.50 (m, 11H), 1.25-1.07 (m, 5H); MS: 479.2 (M+1)⁺.

### Compound 394

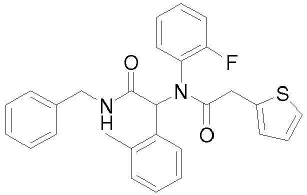

¹H NMR (300 MHz, DMSO-d6): δ 8.66 (m, 1H), 7.36-6.74 (m, 14H), 6.34 (s, 1H), 4.35 (m, 2H), 3.64 (m, 2H), 2.35 (s, 3H); MS: 473.1 (M+1)⁺.

### Compound 109

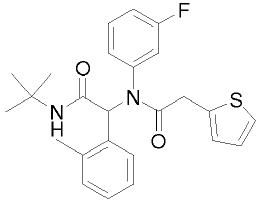

¹H NMR (300 MHz, DMSO-d6): δ 7.76 (br, 2H), 7.34-6.74 (m, 9H), 6.22 (s, 1H), 3.60 (m, 2H), 2.33 (s, 3H), 1.25 (s, 9H); MS: 439.1 (M+1)⁺.

### Compound 110

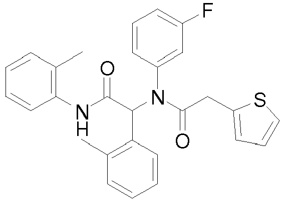

¹H NMR (300 MHz, DMSO-d6): δ9.63 (s, 1H), 7.36-6.76 (m, 13H), 6.50 (s, 1H), 3.64 (m, 2H), 2.42 (s, 3H), 2.10(s, 3H); MS: 471.1 (M -1)⁻.

### Compound 125

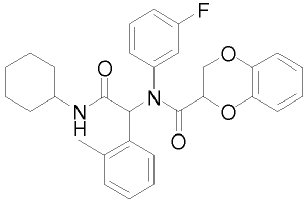

¹H NMR (300 MHz, DMSO-d6): δ 8.10 (d, 1H, J = 7.5), 7.09-6.65 (m, 12H), 6.45 (s, 1H), 4.15 (m, 3H), 3.64 (m, 1H), 2.34 (s, 3H), 1.75-1.50 (m, 5H), 1.38-1.03 (m, 5H); MS: 503.2 (M+1)⁺.

### Compound 126

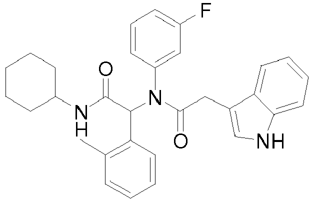

¹H NMR (300 MHz, DMSO-d6): δ 10.80 (s, 1H), 7.96-6.70 (m, 13H), 6.28 (s, 1H), 3.64-3.42 (m, 3H), 2.32 (s, 3H), 1.75-1.50 (m, 5H), 1.34-1.03 (m, 5H); MS: 498.2 (M+1)⁺.

### Compound 150

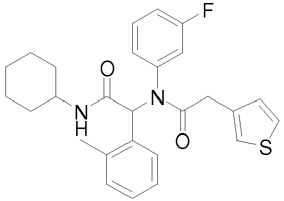

¹H NMR (300 MHz, DMSO-d6): δ 7.97 (d, 1H, J = 7.5), 7.74-6.74 (m, 10H), 6.26 (s, 1H), 3.64 (m, 1H), 3.38 (m, 2H), 2.33 (s, 3H), 1.78-1.52 (m, 5H), 1.34-0.95 (m, 5H); MS: 465.2 (M+1)⁺.

### Compound 132

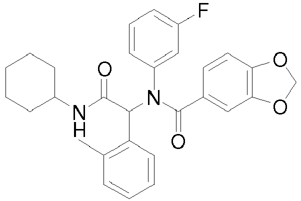

¹H NMR (300 MHz, DMSO-d6): δ 8.09 (d, 1H, J = 7.5), 7.15-6.73 (m, 11H), 6.45 (s, 1H), 5.96 (s, 2H), 3.64 (m, 1H), 2.35 (s, 3H), 1.70-1.52 (m, 5H), 1.34-1.03 (m, 5H); MS: 489.2 (M+1)⁺.

### Compound 137

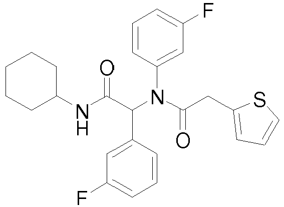

¹H NMR (300 MHz, DMSO-d6): δ 8.07 (d, 1H, J = 7.5), 7.30-6.73 (m, 11H), 6.06 (s, 1H), 3.58 (m, 3H), 1.78-1.52 (m, 5H), 1.34-1.03 (m, 5H); MS: 469.1 (M+1)⁺.

### Compound 138

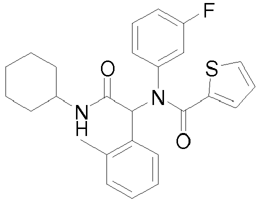

¹H NMR (300 MHz, DMSO-d6): δ 8.06 (d, 1H, J = 7.5), 7.65 (d, 1H, J = 3.2), 7.15-6.55 (m, 8H), 6.41 (s, 1H), 3.63 (m, 1H), 2.39 (s, 3H), 1.78-1.52 (m, 5H), 1.34-1.03 (m, 5H); MS: 451.1 (M+1) ⁺.

### Compound 139

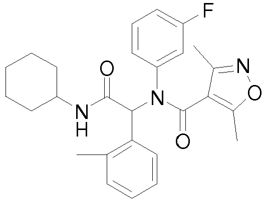

¹H NMR (300 MHz, DMSO-d6): δ 8.19 (d, 1H, J = 7.5), 7.29-6.83 (m, 8H), 6.51(s, 1H), 3.69 (m, 1H), 2.32 (s, 3H), 2.14 (s, 6H), 1.80-1.52 (m, 5H), 1.34-1.03 (m, 5H); MS: 464.2 (M+1)⁺.

### Compound 107

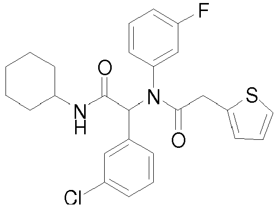

¹H NMR (300 MHz, DMSO-d6): δ 8.10 (d, 1H, J = 7.5), 7.30-6.87 (m, 10H), 6.74 (s, 1H), 6.05 (s, 1H), 3.60 (m, 3H), 1.70-1.52 (m, 5H), 1.34-0.95 (m, 5H); MS: 485.1, 487.1 (M+1)⁺.

### Compound 142

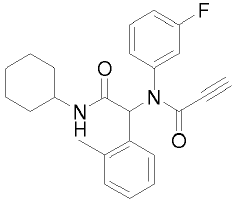

¹H NMR (300 MHz, DMSO-d6): δ 8.07 (d, 1H, J = 7.5), 7.21-6.76 (m, 8H), 619 (s, 1H), 4.24(s, 1H), 3.63 (m, 1H), 2.33 (s, 3H), 1.78-1.52 (m, 5H), 1.32-0.98 (m, 5H); MS: 393.2 (M+1)⁺.

### Compound 158

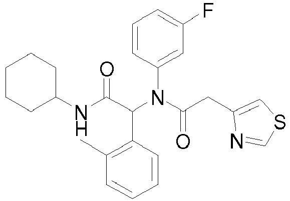

¹H NMR (300 MHz, DMSO-d6): δ 8.96 (s, 1H), 7.99-6.54 (m, 10H), 6.25 (s, 1H), 3.65 (m, 3H), 2.36 (s, 3H), 1.70-1.50 (m, 5H), 1.32-0.96 (m, 5H); MS: 466.2 (M+1)⁺.

### Compound 104

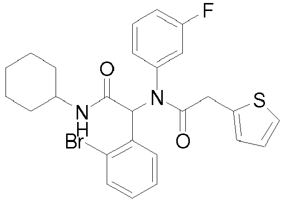

¹H NMR (300 MHz, DMSO-d6): δ 8.25 (d, 1H, J = 7.5), 7.70-6.74 (m, 9H), 6.28 (s, 1H), 3.63 (m, 3H), 1.70-1.50 (m, 5H), 1.30-0.95 (m, 5H); MS: 529.1, 531.1 (M+1)⁺.

### Compound 105

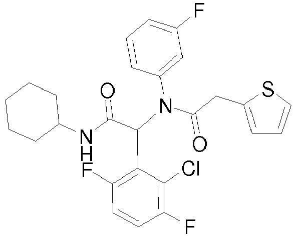

¹H NMR (300 MHz, DMSO-d6): δ 7.98 (d, 1H, J = 7.5), 7.76-6.77 (m, 8H), 6.46 (m, 2H), 3.62 (m, 3H), 1.74-1.50 (m, 5H), 1.32-0.96 (m, 5H); MS: 521.1 (M+1)⁺.

### Compound 5

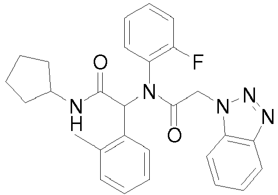

¹H NMR (300 MHz, DMSO-d6): δ 8.28-6.74 (m, 13H), 6.26 (s, 1H), 5.28 (m, 2H), 4.05 (m, 1H), 2.40 (s, 3H), 1.78 (m, 2H), 1.57-1.23 (m, 6H); MS: 486.2 (M+1)⁺.

### Compound 151

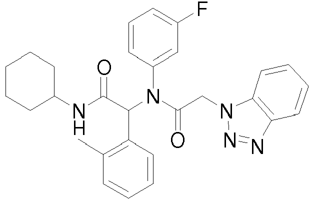

¹H NMR (300 MHz, DMSO-d6): δ 8.05-6.78 (m, 12H), 6.19 (s, 1H), 5.58-5.15 (m, 2H), 3.59 (m, 1H), 2.41 (s, 3H), 1.69-1.53 (m, 5H), 1.32-0.96 (m, 5H); MS: 500.2 (M+1)⁺.

### Compound 157

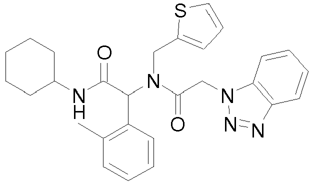

¹H NMR (300 MHz, DMSO-d6): δ 8.26-6.64 (m, 12H), 6.21 (s, 1H), 6.06-4.47 (m, 4H), 3.59 (m, 1H), 2.22 (s, 3H), 1.69-1.47 (m, 5H), 1.32-0.96 (m, 5H); MS: 502.2 (M+1)⁺.

### Compound 262

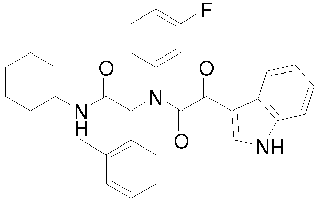

¹H NMR (400 MHz, DMSO-d6): δ 12.22 (s, 1H), 8.35 (m, 2H), 7.90 (d, 1H, J = 5.7), 7.46-6.74 (m, 11H), 6.40 (s, 1H), 3.78 (m, 1H), 2.37 (s, 3H), 1.87-1.60 (m, 5H), 1.34-1.07 (m, 5H); MS: 512.1 (M+1)⁺.

### Compound 270

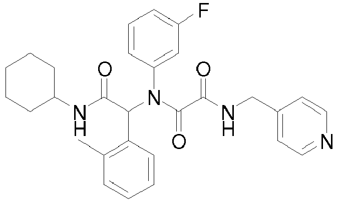

¹H NMR (400 MHz, DMSO-d6): δ 9.50 (m, 1H), 8.68 (d, 2H, J = 4.5), 8.15 (d, 1H, J = 5.7), 7.38-6.74 (m, 9H), 6.22 (s, 1H), 4.35 (m, 2H), 3.64 (m, 1H), 2.40 (s, 3H), 1.72-1.50 (m, 5H), 1.34-1.07 (m, 5H); MS: 503.1 (M+1)⁺.

### Compound 284

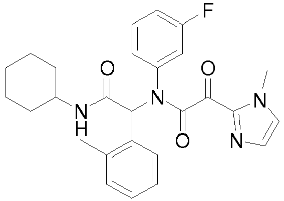

¹H NMR (400 MHz, DMSO-d6): δ 8.28 (d, 1H, J = 5.7), 7.55 (s, 1H), 7.29-6.79 (m, 9H), 6.37 (s, 1H), 3.69 (m, 4H), 2.48 (s, 3H), 1.79-1.50 (m, 5H), 1.34-1.07 (m, 5H); MS: 477.1 (M+1)⁺.

### Compound 301

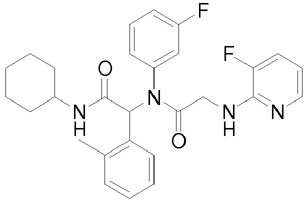

¹H NMR (400 MHz, DMSO-d6): δ 8.01-7.78 (m, 3H), 7.38-6.55 (m, 10H), 6.22(s, 1H), 3.94 (m, 1H), 3.61 (m, 2H), 2.38 (s, 3H), 1.70-1.50 (m, 5H), 1.34-1.00 (m, 5H); MS: 493.1 (M+1)⁺.

### Compound 316

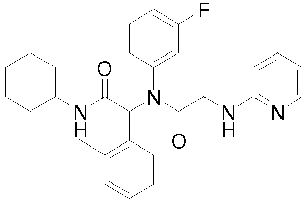

¹H NMR (400 MHz, DMSO-d6): δ 8.01-7.88 (m, 3H), 7.35-6.46 (m, 11H), 6.22 (s, 1H), 3.81 (m, 1H), 3.61 (m, 2H), 2.38 (s, 3H), 1.70-1.50 (m, 5H), 1.34-1.07 (m, 5H); MS: 475.1 (M+1)⁺.

### Compound 310

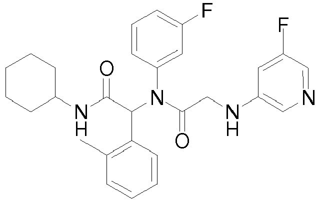

¹H NMR (400 MHz, DMSO-d6): δ 8.04-7.68 (m, 4H), 7.23-6.46 (m, 8H), 6.22 (s, 1H), 3.84-3.35 (m, 3H), 2.38 (s, 3H), 1.70-1.50 (m, 5H), 1.34-1.07 (m, 5H); MS: 493.1 (M+1)⁺.

### Compound 30

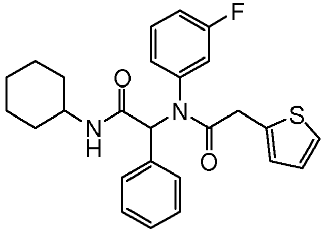

¹H NMR (300 MHz, DMSO-d6): δ 8.04-8.02 (d, 1H, J=5.7), 7.35-6.72 (m, 11H), 6.07 (s, 1H), 3.61-3.58 (m, 3H), 1.72-1.63 (m, 5H), 1.24-1.14 (m, 5H); MS: 451.1 (M+1)⁺.

### Compound 31

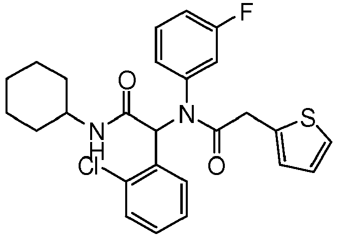

¹H NMR (300 MHz, DMSO-d6): δ 8.25-8.23 (d, 1H, J=6), 7.74-6.73 (m, 10H), 6.35 (s, 1H), 3.69-3.52 (m, 3H), 1.75-1.51 (m, 5H), 1.30-0.97 (m, 5H); MS: 485.1 (M+1)⁺.

### Compound 56

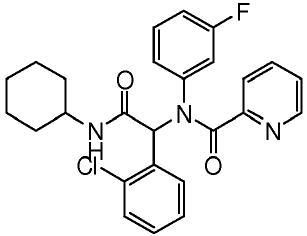

¹H NMR (300 MHz, DMSO-d6): δ 8.38-8.32 (m, 2H), 7.73-6.68 (t, 1H), 7.39-6.73 (m, 10H), 5.75 (s, 1H), 3.70-3.66 (m, 1H), 1.75-1.52 (m, 5H), 1.30-1.02 (m, 5H); MS: 466.1 (M+1)⁺.

### Compound 32

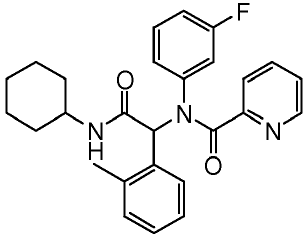

¹H NMR (300 MHz, DMSO-d6): δ 8.32 (s, 1H), 8.18(br, 1H),7.73-7.67 (t, 1H), 7.36-6.73 (m, 10H), 6.45 (s, 1H), 3.69-3.66 (m 1H), 2.41 (s, 3H), 1.76-1.57 (m, 5H), 1.28-1.03 (m, 5H); MS: 446.2 (M+1)⁺.

### Compound 33

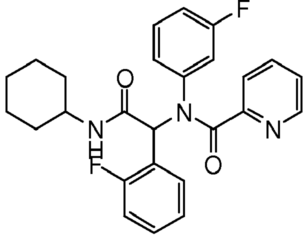

¹H NMR (300 MHz, DMSO-d6): δ 8.32-8.28 (m, 2H), 7.71-7.69 (t, 1H), 7.39-7.36 (d, 1H,J = 7.8), 7.26-6.77 (m, 9H), 6.53 (s, 1H), 3.69-3.65 (m, 1H), 1.77-1.60 (m, 5H), 1.29-1.07 (m, 5H); MS: 450.1 (M+1)⁺.

### Compound 34

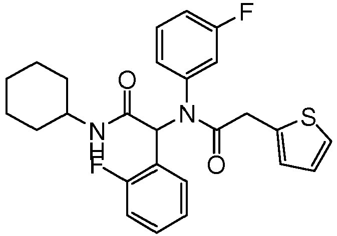

¹H NMR (300 MHz, DMSO-d6): δ 8.18-8.15 (d, 1H,J=6.9), 7.36-7.34 (d, 1H,J=8.1), 7.24-6.84 (m, 7H), 6.74-6.73 (d,1H,J=2.7), 6.30 (s, 1H), 3.69-3.52 (m, 3H), 1.74-1.51 (m, 5H), 1.29-0.97 (m, 5H); MS: 469.1 (M+1)⁺.

### Compound 98

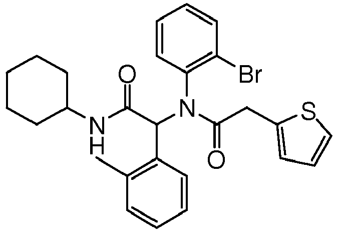

¹H NMR (300 MHz, DMSO-d6): δ 8.38-8.35 (d, 1H,J=8.1), 7.79-7.77 (d, 1H,J=7.5), 7.42-6.63 (m, 10H), 6.35 (s, 1H), 3.58-3.49 (m, 3H), 2.54 (s,3H), 1.77-1.51 (m, 5H), 1.27-0.88 (m, 5H); MS: 525.1 (M+1)⁺.

### Compound 117

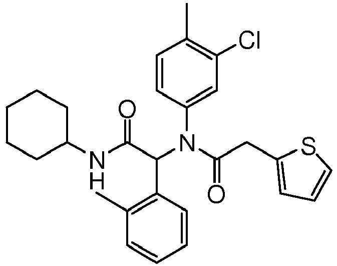

¹H NMR (300 MHz, DMSO-d6): δ 7.99-7.95 (br, 1H), 7.36-6.47 (m, 9H), 6.23 (s, 1H), 3.66-3.48 (m, 3H), 2.32 (s,3H), 2.18 (s,3H),1.77-1.51 (m, 5H), 1.29-0.98 (m, 5H); MS: 495.1 (M+1)⁺.

### Compound 99

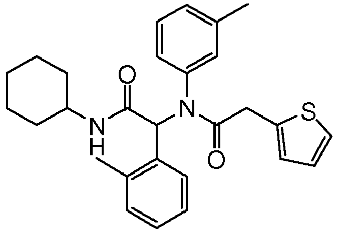

¹H NMR (300 MHz, DMSO-d6): δ 7.92-7.89 (d, 1H, J=7.5), 7.71 (br, 1H), 7.35-7.33 (b, 1H, J=6.3), 7.09-6.31 (m, 8H), 6.22 (s, 1H), 3.61-3.45 (m, 3H), 2.33 (s, 3H), 2.22-1.96 (m, 3H), 1.77-1.51 (m, 5H), 1.29-0.92 (m, 5H); MS: 461.2 (M+1)⁺.

### Compound 118

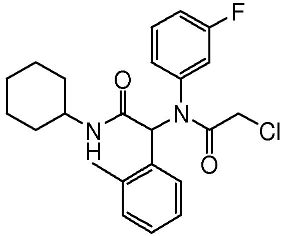

¹H NMR (300 MHz, DMSO-d6): δ 8.05-8.02 (d, 1H, J=8.1), 7.90-7.61 (br, 1H), 7.11-6.97 (m, 4H), 6.87-6.82 (t, 1H), 6.72-6.70 (d, 1H, J=7.5), 6.21 (s, 1H), 4.17-3.88 (q, 2H), 3.65-3.61 (m, 1H), 2.36 (s, 3H), 1.79-1.52 (m, 5H), 1.30-0.96 (m, 5H); MS: 417.1 (M+1)⁺.

### Compound 101

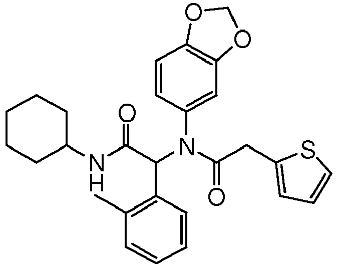

¹H NMR (300 MHz, DMSO-d6): δ 7.97-5.98 (m, 11H), 5.89 (s, 1H), 3.69-3.53 (m, 3H), 2.36-2.33 (m, 3H), 1.77-1.53 (m, 5H), 1.29-0.95 (m, 5H); MS: 491.2 (M+1)⁺.

### Compound 100

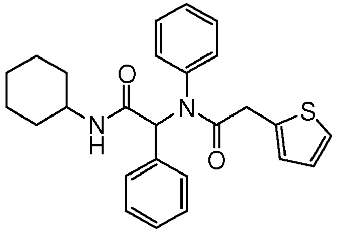

¹H NMR (300 MHz, DMSO-d6): δ 7.96-7.94 (d, 1H, J=7.5), 7.35-7.33 (m, 1H), 7.14-7.01 (m, 8H), 6.91-6.88 (m, 1H), 6.71 (s, 1H), 3.59-3.50 (m, 3H), 1.76-1.51 (m, 5H), 1.28-0.95 (m, 5H); MS: 433.2 (M+1)⁺.

### Compound 251

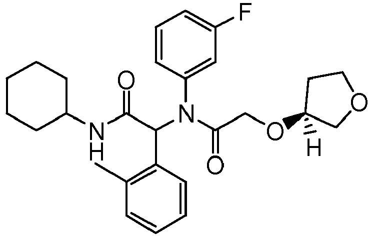

¹H NMR (300 MHz, DMSO-d6): δ 8.03-8.02 (d, 1H, J=4.2), 7.10-6.70 (m, 6H), 6.21 (s, 1H), 4.04 (s, 1H), 3.94-3.89 (m, 1H), 3.70-3.54 (m, 6H), 2.35 (s, 3H), 1.82-1.53 (m, 7H), 1.29-0.96 (m, 5H); MS: 469.2 (M+1)⁺.

### Compound 222

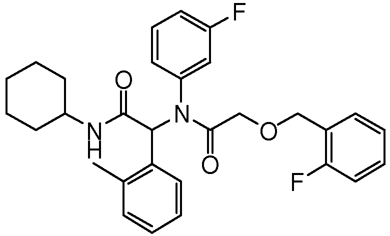

¹H NMR (300 MHz, DMSO-d6): δ 8.04-8.01 (d, 1H, J=7.5H), 7.40-6.70 (m, 10H), 6.24 (s, 1H), 4.50-4.49 (m, 2H), 4.01-4.62 (m, 3H), 2.36 (s, 3H), 1.80-1.52 (m, 5H), 1.31-0.96 (m, 5H); MS: 507.2 (M+1)⁺.

### Compound 229

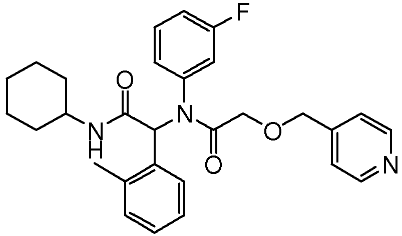

¹H NMR (300 MHz, DMSO-d6): δ 8.51-8.48 (m, 2H), 8.04-8.02 (d, 1H, J=7.2H), 7.27-7.25 (d, 2H, J = 6), 7.10-6.70 (m, 6H), 6.24 (s, 1H), 4.50 (s, 2H), 4.08-3.77 (m, 2H), 3.63-3.62 (m, 1H), 2.35 (s, 3H), 1.80-1.52 (m, 5H), 1.30-0.96 (m, 5H); MS: 490.2 (M+1)⁺.

### Compound 233

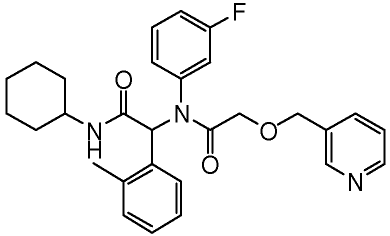

¹H NMR (300 MHz, DMSO-d6): δ 8.49-8.48 (m, 2H), 8.06-8.04 (m, 1H), 7.70-7.68 (d, 1H, J=5.7), 7.37-6.72 (m, 6H), 6.25 (s, 1H), 4.47 (s, 2H), 4.04-3.74 (m, 2H), 3.65-3.63 (m, 1H), 2.36 (s, 3H), 1.80-1.52 (m, 5H), 1.30-0.96 (m, 5H); MS: 490.2 (M+1)⁺.

### Compound 234

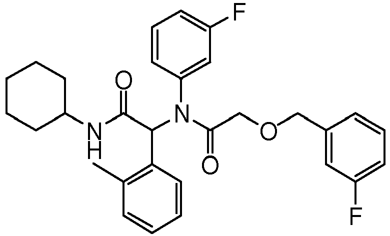

¹H NMR (300 MHz, DMSO-d6): δ 8.05-8.03 (d, 1H, J=7.2), 7.37-6.71 (m, 10H), 6.24 (s, 1H), 4.45 (s, 2H), 4.03-3.72 (m, 2H), 3.63-3.62 (m, 1H), 2.35 (s, 3H), 1.80-1.52 (m, 5H), 1.31-0.96 (m, 5H); MS: 507.2 (M+1)⁺.

### Compound 235

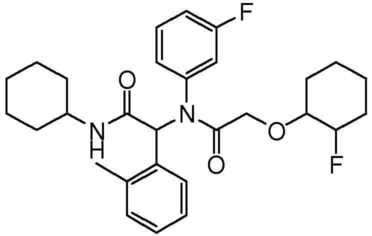

¹H NMR (300 MHz, DMSO-d6): δ 8.03-8.00 (d, 1H, J=7.8), 7.10-6.69 (m, 7H), 6.20 (s, 1H), 4.42 (s, 1H), 4.24(s, 1H), 4.06-3.76 (m, 2H), 3.63-3.60 (m, 1H), 2.35 (s, 3H), 1.89-1.49 (m, 9H), 1.30-0.95 (m, 9H); MS: 499.2 (M+1)⁺.

### Compound 259

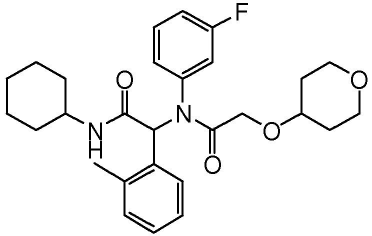

¹H NMR (300 MHz, DMSO-d6): δ 8.02-8.01 (d, 1H, J=4.8), 7.10-6.70 (m, 7H), 6.21 (s, 1H), 3.96-3.61 (m, 5H), 3.42-3.38 (m, 1H), 3.27-3.22 (m, 2H), 2.35 (s, 3H), 1.79-1.53 (m, 7H), 1.30-0.96 (m, 7H); MS: 483.1 (M+1)⁺.

### Compound 273

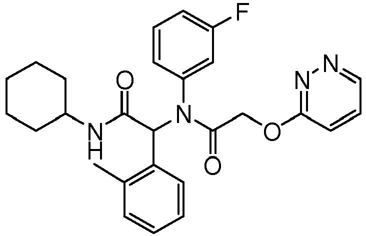

¹H NMR (300 MHz, DMSO-d6): δ 8.01 (s, 1H), 7.90-7.86 (m, 2H), 7.44-7.41 (m, 1H), 7.13-6.48 (m, 8H), 6.21 (s, 1H), 4.67-4.35 (m, 2H), 3.62-3.60 (m, 1H), 2.39 (s, 3H), 1.72-1.52 (m, 5H), 1.28-0.96 (m, 5H); MS: 477.1 (M+1)⁺.

### Compound 274

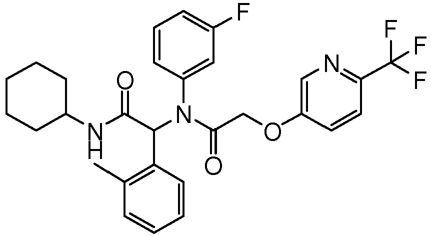

¹H NMR (300 MHz, DMSO-d6): δ 8.35 (s, 1H), 8.05-8.03 (br, 1H), 7.83-7.81 (d, 1H, J=6.6), 7.45-6.75 (m, 8H), 6.20(s, 1H), 4.91-4.46 (m, 2H), 3.63-3.61 (m, 1H), 2.37 (s, 3H), 2.28 (s, 4H), 1.74-1.52 (m, 5H), 1.29-0.95 (m, 5H); MS: 539.3 (M+1)⁺.

### Compound 281

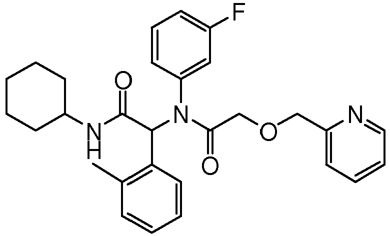

¹H NMR (300 MHz, DMSO-d6): δ 8.47 (s, 1H), 8.05 (s, 1H), 7.79-7.75 (m, 1H), 7.37-6.71 (m, 9H), 6.24 (s, 1H), 4.52 (s, 2H), 4.09-3.80 (m, 2H), 3.64-3.63 (m, 1H), 2.35 (s, 3H), 1.79-1.52 (m, 5H), 1.29-0.96 (m, 5H); MS: 490.1 (M+1)⁺.

### Compound 282

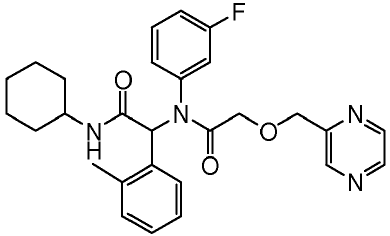

¹H NMR (300 MHz, DMSO-d6): δ 8.64 (s, 1H), 8.57 (s, 2H), 8.05-8.03 (m, 1H), 7.79 (br, 1H), 7.10-6.71 (m, 6H), 6.24 (s, 1H), 4.61 (s, 2H), 4.14-3.85 (m, 2H), 3.64-3.63 (m, 1H), 2.33 (s, 3H), 1.79-1.52 (m, 5H), 1.29-0.96 (m, 5H); MS: 491.1 (M+1)⁺.

### Compound 303

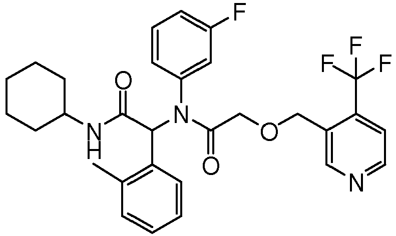

¹H NMR (300 MHz, DMSO-d6): δ 8.38 (s, 1H), 8.01-8.00 (m, 1H), 7.88 (br, 1H), 7.35-6.70 (m, 8H), 6.20 (s, 1H), 4.82-4.56 (m, 2H), 3.61-3.59 (m, 1H), 2.39(s, 3H), 2.28 (s, 4H), 1.70-1.51 (m, 5H), 1.27-0.95 (m, 5H); MS: 544.1 (M+1)⁺..

### Compound 35

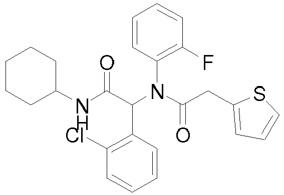

¹H NMR (300 MHz, DMSO-d6): δ 8.15-8.13 (d, 1H, J = 8.4), 8.02-7.99 (d, 1H, J = 1.2), 7.99-6.74 (m, 10H), 6.49 (s, 1H), 3.61-3.56 (m, 3H), 1.75-1.51 (m, 5H), 1.32-1.0.85 (m, 5H); MS: 485.1 (M+1)⁺.

### Compound 36

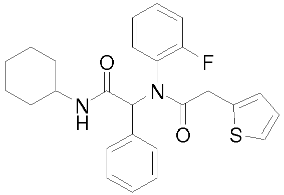

¹H NMR (300 MHz, DMSO-d6): δ 8.02-7.99 (d, 1H, J = 7.5), 7.92-7.89 (t, 1H), 7.35-6.88 (m, 9H), 6.72 (s, 1H), 6.01 (s, 1H), 3.61-3.52 (m, 3H), 1.77-1.50 (m, 5H), 1.28-0.88 (m, 5H); MS: 451.1 (M+1)⁺.

### Compound 73

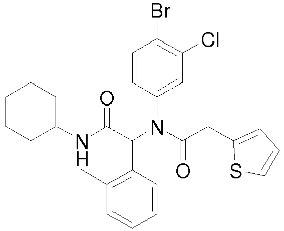

¹H NMR (300 MHz, DMSO-d6): δ 8.05-7.35 (m, 2H), 7.11-6.88 (m, 9H), 6.24 (s, 2H), 3.67-3.58 (m, 3H), 2.33(s, 3H), 1.78-1.51 (m, 5H), 1.29-0.85 (m, 5H); MS: 561.0 (M+1)⁺.

### Compound 60

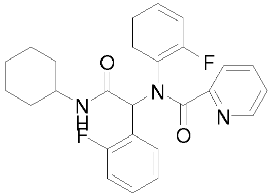

¹H NMR (300 MHz, DMSO-d6): δ 8.25-6.60 (m, 13H), 6.53 (s, 1H), 3.75-3.35 (s, 1H), 2.49-1.52 (m, 5H), 1.31-0.89 (m, 5H); MS: 450.1 (M+1)⁺.

### Compound 39

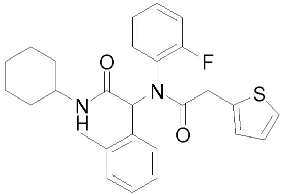

¹H NMR (300 MHz, DMSO-d6): δ 8.10-8.09 (d, 1H, J = 2.1), 7.96-7.93 (d, 1H, J = 7.5), 7.36-7.35 (d, 1H, J = 1.2), 7.33-6.67 (m, 8H), 6.32 (s, 1H), 3.75-3.54 (m, 3H), 2.37 (m, 3H), 1.89-1.56 (m, 5H), 1.24-1.19 (m, 5H); MS: 465.2 (M+1)⁺.

### Compound 111

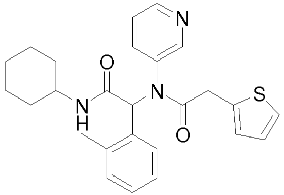

¹H NMR (300 MHz, DMSO-d6): δ 8.28-8.26 (d, 1H, J = 10.2), 8.04-8.02 (d, 1H J = 7.5), 7.36-6.63 (m, 9H), 6.29 (s, 1H), 3.67-3.5 5(m, 3H), 2.36 (s, 3H), 1.714-1.56 (m, 5H), 1.25-1.15 (m, 5H); MS: 448.2 (M+1)⁺.

### Compound 112

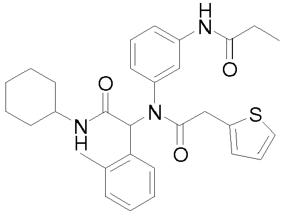

¹H NMR (300 MHz, DMSO-d6): δ 10.1-9.65 (m, 1H), 7.05-6.74 (m, 11H), 6.20 (s, 1H), 3.59-3.52 (m, 3H), 2.50-2.26 (m, 5H), 1.25 (m, 12H); MS: 518.2 (M+1)⁺.

### Compound 122

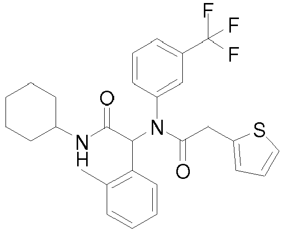

¹H NMR (300 MHz, DMSO-d6): δ 8.05-8.03 (m, 2H), 7.48-6.67 (m, 10H), 6.28 (s, 1H), 3.69-3.52 (m, 3H), 2.35 (s, 3H), 1.79-1.52 (m, 5H), 1.30-0.95 (m, 5H); MS: 515.2 (M+1)⁺.

### Compound 123

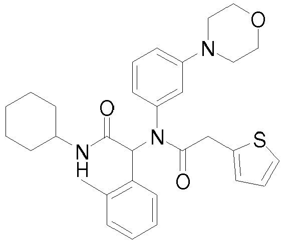

¹H NMR (300 MHz, DMSO-d6): δ7.91-7.82 (m, 1H), 7.66-7.33 (m, 2H), 7.07-6.68 (m, 10H), 6.22 (s, 1H), 6.15-5.85 (m, 1H), 3.72-3.50 (m, 7H), 3.00-2.68 (m, 4H), 2.33 (s, 3H), 1.78-1.52 (m, 5H), 1.29-0.94 (m, 5H); MS: 532.2 (M+1)⁺.

### Compound 131

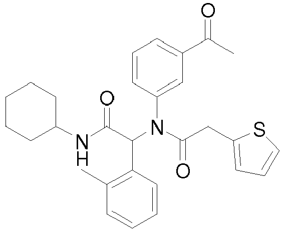

¹H NMR (300 MHz, DMSO-d6): δ 8.00-7.98 (m, 2H), 7.69-7.67 (d, 1H, J = 7.5), 7.35-6.77 (m, 9H), 6.29 (s, 1H), 3.66-3.31 (m, 3H), 2.50-2.24 (m, 6H), 1.79-1.52 (m, 5H), 1.30-0.97 (m, 5H); MS: 489.2 (M+1)⁺.

### Compound 140

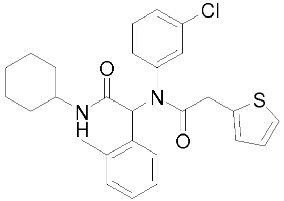

¹H NMR (300 MHz, DMSO-d6): δ 8.01-7.85 (m, 2H), 7.36-6.68 (m, 10H), 6.25 (s, 1H), 3.66-3.32 (m, 3H), 2.35(s, 3H), 1.78-1.52 (m, 5H), 1.30-0.97 (m, 5H); MS: 481.1 (M+1)⁺.

### Compound 124

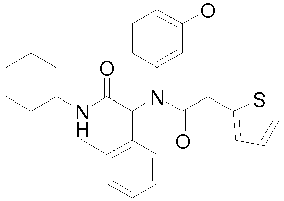

¹H NMR (300 MHz, DMSO-d6): δ 7.91 (s, 1H), 7.35-7.33 (m, 2H), 7.07-6.67 (m,8H), 6.23-6.01 (m, 2H), 3.67-3.42 (m, 6H), 2.35 (s, 3H), 1.78-1.52 (m, 5H), 1.29-0.94 (m, 5H); MS: 477.2 (M+1)⁺.

### Compound 149

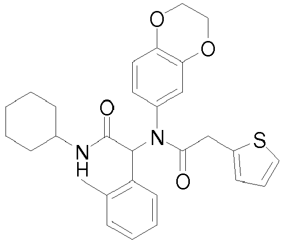

¹H NMR (300 MHz, DMSO-d6): δ 7.86 (s, 1H), 7.35-7.3 (m, 2H), 7.08-6.70 (m, 7H), 6.19 (s, 1H), 6.05-5.95 (m, 1H), 4.15-4.06 (m, 4H), 3.65-3.48 (m, 3H), 2.32 (s, 3H), 1.71-1.51 (m, 5H), 1.29-0.93 (m, 5H); MS: 505.2 (M+1)⁺.

### Compound 144

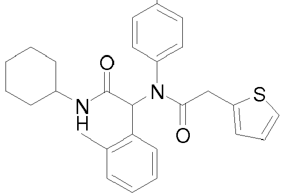

¹H NMR (300 MHz, DMSO-d6): δ 7.91-7.88 (d, 1H, J = 7.8), 7.34-7.33 (s, 1H), 7.09-6.68 (m, 10H), 6.24 (s, 1H), 3.60-3.31 (m, 3H), 2.33 (s, 3H), 2.16 (s, 3H), 1.77-1.51 (m, 5H), 1.29-0.93 (m, 5H); MS: 462.2 (M+1)⁺.

### Compound 145

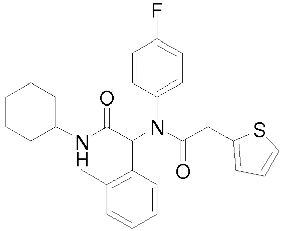

¹H NMR (300 MHz, DMSO-d6): δ 7.96-7.93 (d, 2H, J = 6.9), 7.35-7.33 (m, 1H), 7.11-6.65 (m, 8H), 6.24 (s, 1H), 3.65-3.47 (m, 3H), 2.34 (s, 3H), 1.78-1.51 (m, 5H), 1.29-0.98 (m, 5H); MS: 465.2 (M+1)⁺.

### Compound 146

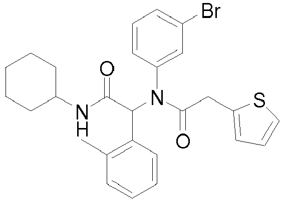

¹H NMR (300 MHz, DMSO-d6): δ 8.21-7.99 (m, 2H), 7.36-7.31 (m, 2H), 7.09-6.71 (m, 8H), 6.25 (s, 1H), 3.68-3.62 (m, 3H), 2.33 (s, 3H), 1.78-1.52 (m, 5H), 1.30-0.94 (m, 5H); MS: 527.1 (M+1)⁺.

### Compound 147

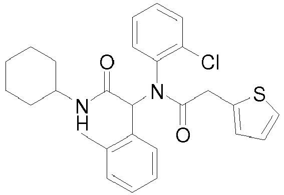

¹H NMR (300 MHz, DMSO-d6): δ 8.34-8.31 (m, 1H), 7.84-7.82 (d, 1H, J = 7.5), 7.36-6.64 (m, 10H), 6.37 (s, 1H), 3.33-3.49 (m, 1H), 3.31 (s, 2H), 3.31 (s, 3H), 1.75-1.49 (m, 5H), 1.35-0.78 (m, 5H); MS: 481.1 (M+1)⁺.

### Compound 148

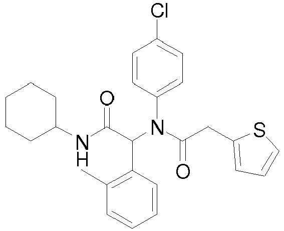

¹H NMR (300 MHz, DMSO-d6): δ 7.98-7.96 (d, 2H, J = 7.5), 7.36-7.34 (m, 2H), 7.11-6.67 (m, 8H), 6.24 (s, 1H), 3.66-3.31 (m, 3H), 2.33 (s, 3H), 1.78-1.51 (m, 5H), 1.29-0.97 (m, 5H); MS: 481.1 (M+1)⁺.

### Compound 238

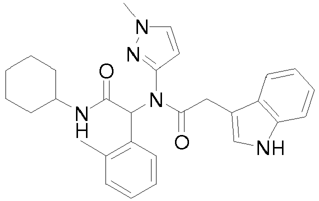

¹H NMR (300 MHz, DMSO-d6): δ 10.82 (s, 1H), 8.10-8.07 (d, 1H, J = 7.8), 7.52-6.58 (m, 10H), 6.14 (s, 1H), 5.87-5.86 (d, 1H, J = 2.1), 3.61-3.37 (m, 6H), 2.28 (s, 3H), 1.70-1.64 (m, 5H), 1.29-1.06 (m, 5H); MS:484.3 (M+1)⁺.

### Compound 244

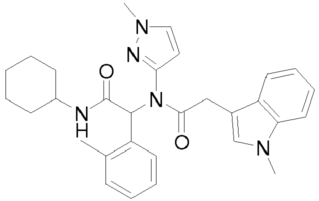

¹H NMR (400 MHz, DMSO-d6): δ 8.11-8.09 (d, 1H, J = 7.6), 7.44 (s, 1H), 7.37-7.34 (m, 2H), 7.11-6.75 (m, 7H), 6.12 (s, 1H), 5.89 (s, 1H), 3.72 (s, 3H), 3.62-3.51 (m, 6H), 2.28 (s, 3H), 1.63-1.50 (m, 5H), 1.47-1.09 (m, 5H); MS:498.3 (M+1)⁺.

### Compound 307

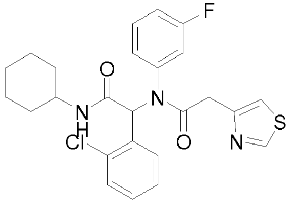

¹H NMR (300 MHz, DMSO-d6): δ 8.98-8.97 (d, 1H, J = 1.6), 8.27-8.25 (d, 1H, J = 6.0), 7.69 (s, 1H), 7.39-6.76 (m, 8H), 6.35(s, 1H), 3.67-3.53 (m, 3H), 1.76-1.52 (m, 5H), 1.29-0.98 (m, 5H); MS:486.0 (M+1)⁺.

### Compound 8

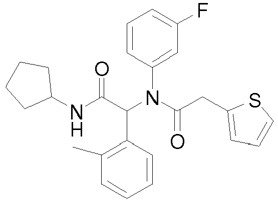

¹H NMR (300 MHz, CDCl₃): δ 7.26-6.72 (m, 10H), 6.39 (s, 1H), 5.45 (m, 1H), 4.28-4.25 (m, 1H), 3.65 (s, 2H), 2.35 (s, 3H), 1.97-1.93 (m, 2H), 1.58-1.51 (m, 4H), 1.27-1.25 (m, 2H); MS: 451.1 (M+1)⁺.

### Compound 4

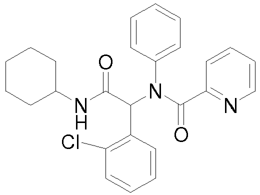

¹H NMR (300 MHz, CDCl3): δ 8.41-8.35 (m, 1H), 7.56-6.92 (m, 13H), 6.65 (s, 1H), 3.94 (m, 1H), 2.15-1.85 (m, 2H), 1.68-1.58 (m, 2H), 1.42-1.11 (m, 6H); MS: 448.1 (M+1)⁺.

### Compound 10

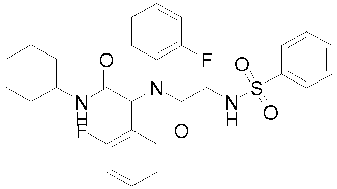

¹H NMR (300 MHz, CDCl₃): δ 7.84- 7.75 (m, 3H), 7.62-7.48 (m, 3H), 7.23-6.73 (m, 6H), 6.25 (s, 1H), 5.53-5.50 (m, 1H), 5.43-5.39 (m, 1H), 3.77-3.74 (m, 1H), 3.50-3.49 (m, 2H), 1.92-1.59 (m, 4H), 1.54-0.95 (m, 6H); MS: 542.2 (M+1)⁺.

### Compound 28

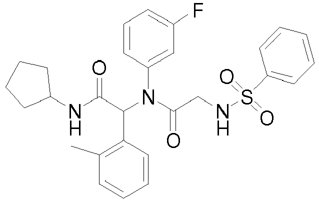

¹H NMR (300 MHz, CDCl₃): δ 7.81-7.78 (m, 2H), 7.69-7.49 (m, 4H), 7.11-7.09 (m, 2H), 6.91-6.83 (m, 2H), 6.66-6.63 (m, 1H), 6.23 (s, 1H), 5.74-5.72 (m, 1H), 5.35 (d, 1H, J=8.1MHz), 4.20-4.18 (m, 1H), 3.51-3.46 (m, 2H), 2.24 (s, 3H), 1.94-1.89 (m, 2H), 1.55-1.50 (m, 4H), 1.27-1.22 (m, 2H); MS: 524.1 (M+1)⁺.

### Compound 29

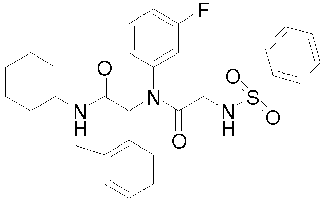

¹H NMR (300 MHz, CDCl₃): δ 7.81-7.78 (m, 2H), 7.59-7.48 (m, 4H), 7.11-6.81 (m, 5H), 6.66-6.63 (m, 1H), 6.23 (s, 1H), 5.73-5.69 (m, 1H), 5.26 (d, 1H, J=8.1), 3.75 (m, 1H), 3.50-3.45 (m, 2H), 2.24 (s, 3H), 1.85-1.84 (m, 2H), 1.65-1.55 (m, 4H), 1.34-1.25 (m, 2H), 1.08-0.98 (m, 2H); MS: 538.2 (M+1)⁺.

### Compound 54

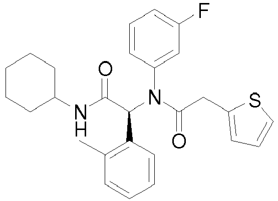

The single isomer was isolated via chiral HPLC.¹H NMR (300 MHz, DMSO-d6): δ 7.15-6.72 (m, 10H), 6.40 (s, 1H), 5.38-5.36 (m, 1H), 3.85-3.81 (m, 1H), 3.65 (s, 1H), 2.35 (s, 3H), 1.97-1.56 (m, 5H), 1.36-0.96 (m, 5H); MS: 465.2 (M+1)⁺.

### Compound 164

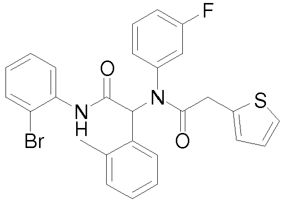

¹H NMR (CDCl₃, 300MHz), δ 8.34 (d, 1H, J=12.3 MHz), 7.65 (s, 1H), 7.47-7.43 (m, 1H), 7.40-7.35 (m, 1H), 7.18-7.16 (m, 4H), 6.94-6.70 (m, 7H), 6.65 (s, 1H), 3.72 (s, 2H), 2.41 (s, 3H); MS: 366.1 (M+1)⁺.

### Compound 231

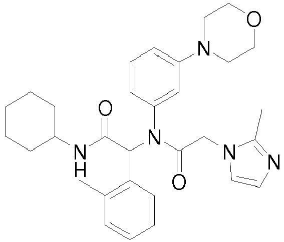

¹H NMR (CDCl3, 300MHz), δ 7.54 (s, 1H), 7.27 (s, 1H), 7.12 (m, 2H), 6.90-6.83 (m, 5H), 6.34 (s, 1H), 5.83 (m, 1H), 5.33 (m, 1H), 4.42 (m, 2H), 3.86-3.74 (m, 5H), 3.14 (m, 2H), 2.76 (m, 2H), 2.38 (s, 3H), 2.29 (s, 3H), 1.66-1.26 (m, 4H), 1.10-0.95 (m, 6H); MS: 530.3 (M+1)⁺.

### Compound 271

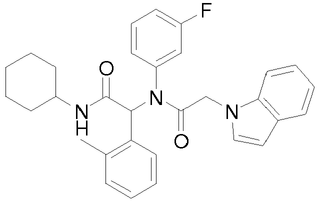

¹H NMR (CDCl₃, 300MHz), δ 7.56 (d, 1H, J=6.0 MHz), 7.15-7.07 (m, 6H), 6.92-6.87 (m, 4H), 6.76 (m, 1H), 6.45 (d, 1H, J=1.8 MHz), 6.36 (s, 1H), 5.24 (m, 1H), 4.66 (s, 2H), 3.80 (m, 1H), 2.34 (s, 3H), 1.89-1.56 (m, 4H), 1.30-1.04 (m, 6H); MS: 498.1 (M+1)⁺.

### Compound 297

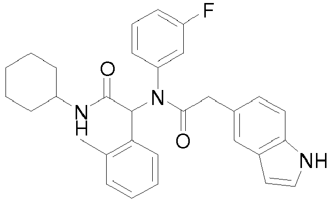

¹H NMR (CDCl₃, 400MHz), δ 8.11 (s, 1H), 7.27-6.79 (m, 11H), 6.44 (s, 1H), 6.41 (s, 1H), 5.35 (d, 1H, J = 7.2), 3.84 (m, 1H), 3.64-3.52 (m, 2H), 2.32 (s, 3H), 1.65-1.57 (m, 4H), 1.34-0.89 (m, 6H); MS: 498.1 (M+1)⁺.

### Compound 288 and its HCL salt

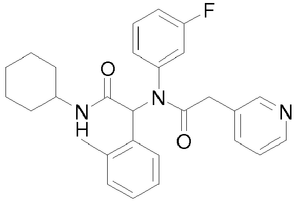

¹H NMR (CDCl₃, 400MHz), δ 8.45 (d, 1H, J=3.6), 8.19 (s, 1H), 7.60 (d, 1H, J=7.6), 7.24-6.75 (m, 8H), 6.38 (s, 1H), 5.33 (m, 1H), 3.83 (m, 1H), 3.49-3.46 (m, 2H), 2.35 (s, 3H), 1.98-1.61 (m, 4H), 1.33-1.07 (m, 6H); MS: 460.1 (M+1)⁺.
HCl Salt:
¹H NMR (DMSO-d6, 400MHz), δ ppm: 8.74-8.73 (m, 1H), 8.62 (s, 1H), 8.23-8.21 (m, 1H), 8.01-7.87 (m, 3H), 7.12-6.71 (m, 6H), 6.23 (s, 1H), 3.79-3.56 (m, 3H), 2.33 (s, 3H), 1.73-1.52 (m, 5H), 1.28-0.98 (m, 5H); MS: 460.1 (M+1)⁺.

### Compound 289

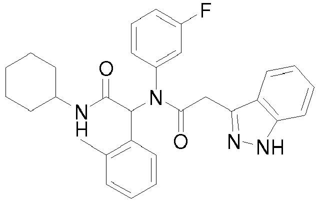

¹H NMR (CDCl₃, 400MHz), δ 9.91 (s, 1H), 7.68-6.79 (m, 12H), 6.47 (s, 1H), 5.66 (m, 1H), 3.86 (m, 3H), 2.35 (s, 3H), 1.93-1.89 (m, 2H), 1.67-1.62 (m, 3H), 1.33-1.10 (m, 5H); MS: 499.1 (M+1)⁺.

### Compound 295

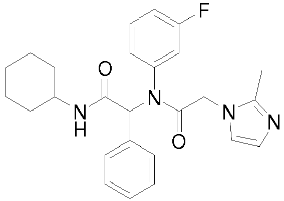

¹H NMR (CDCl3, 400MHz), δ 7.23-6.77 (m, 9H), 6.05 (s, 1H), 5.38 (m, 1H), 4.35-4.33 (m, 2H), 3.82 (m, 1H), 2.24 (s, 3H), 1.93-1.52 (m, 5H), 1.33-1.10 (m, 5H); MS: 449.1 (M+1)⁺.

### Compound 296

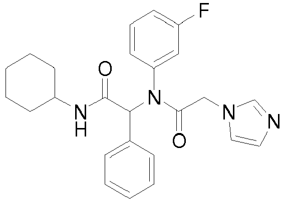

¹H NMR (CDCl₃, 400MHz), δ 7.52-6.94 (m, 10H), 6.05 (s, 1H), 5.42 (, 1H), 4.47 (s, 2H), 3.81 (m, 1H), 1.93-1.07 (m, 10H); MS: 435.1 (M+1)⁺.

### Compound 232

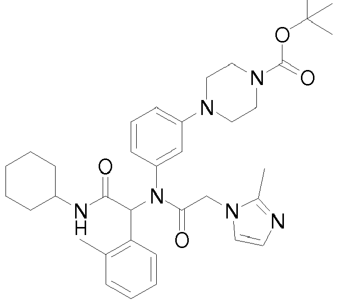

¹H-NMR (CDCl₃, 300MHz), δ 7.55 (s, 1H), 7.31 (s, 1H), 7.13-7.01 (m, 2H), 6.90-9.65 (m, 5H), 6.33 (s, 1H), 6.21-5.80 (m, 1H), 5.40-5.21 (m, 1H), 4.45-4.23 (m, 2H), 3.80 (m, 1H), 3.58-3.46 (m, 4H), 3.13 (m, 2H), 2.77 (m, 2H), 2.38 (s, 3H), 2.27 (s, 3H), 1.88-1.61 (m, 5H), 1.49 (s, 9H), 1.33-0.91 (m, 5H); MS: 629.4 (M+1)⁺.

### Compound 287

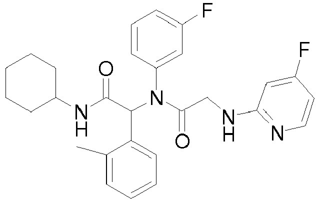

¹H NMR (400 MHz, DMSO-d6): δ 8.02-6.71 (m, 11H), 6.44-6.38 (m, 2H), 6.23 (s, 1H), 3.92-3.90 (m, 1H), 3.61-3.57 (m, 2H), 2.33 (s, 3H), 1.77-1.52 (m, 5H), 1.29-0.96 (m, 5H); MS: 493.1 (M+1)⁺.

**Example 2: Preparation of Compound 160 and its HCl Salt.** Compound 160 was synthesized following Scheme 2, above using the following protocol.

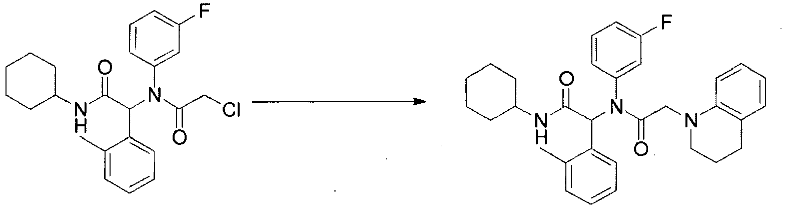

To a mixture of Compound 118 (300 mg, 0.72 mmol), 1,2,3,4-Tetrahydro-quinoline (200 mg, 1.5 mmol) and Et₃N (300 mg, 3 mmol) in DCM (10 ml) was added TBAI (266 mg, 0.72 mmol) at room temperature. The reaction mixture was stirred for 24 hours at the same temperature. The resulting mixture was washed with water, saturated NaHCO₃ solution, brine, dried over Na₂SO₄ and filtered. The solvent was evaporated in vacuo and the crude mixture was purified by TLC to give the desired product (120 mg, 32% yield). ¹H NMR (300 MHz, DMSO-d6): δ 7.94-7.93(m, 2H), 7.14-6.19 (m, 10H), 3.86-3.57 (m, 3H), 3.25 (s, 2H), 2.63-2.66 (t, 2H), 2.37 (s, 3H), 1.81-1.51 (m, 5H), 1.27-0.92 (m, 5H); MS: 514.3 (M+1)⁺.
HCl Salt:
¹H NMR (300 MHz, DMSO-d6): δ7.93(br, 2H), 7.13-6.18(m, 11H), 4.09(m, 1H), 3.86-3.55(m, 3H), 3.22(m, 2H), 2.63(m, 2H), 2.36(s, 3H), 1.72-1.50(m, 7H), 1.32-0.89(m, 5H); MS: 514.3 (M+1)⁺.

The following compounds were also synthesized via Scheme 2 following the general procedure set forth above for Compound 118. The corresponding HCl salt was synthesized following the general procedure set forth in Example 1, step B.

### Compound 179

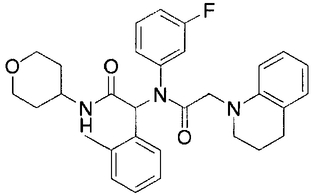

¹H NMR (300 MHz, DMSO-d6), δ 8.08 (d, 1H, J=6.3), 7.86 (br, 0.5H), 7.15-7.00 (m, 7H), 6.70 (d, 2H, J=7.5), 6.47 (t, 1H,), 6.21 (s, 1H, J=7.2), 6.24 (s, 1H), 6.21 (d, 1H, J=5.4), 3.87-3.72 (m, 4H), 3.63 (br, 0.5H), 3.57 (br, 0.4H), 3.38-3.23 (m, 3H), 2.64 (t, 2H, J=5.7), 2.37 (s, 3H), 1.81-1.78 (m, 2H), 1.71-1.66 (br, 2H), 1.40-1.35 (m, 1H), 1.23-1.15 (m, 1H); MS: 516.2 (M+1)⁺.

### Compound 330

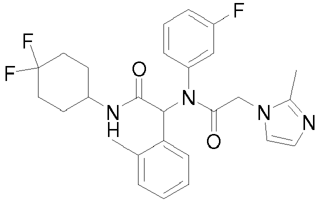

¹H NMR (400 MHz, MeOD-d4), δ 7.69 (br, 1H), 7.27 (br, 0.4H), 7.03-6.70 (br, 8H), 6.57-6.47 (br, 1H), 6.24 (ds, 1H), 4.58 (d, 1H, J=17), 4.39 (d, 1H, J=17), 3.81 (br, 1H), 2.35 (s, 1H), 2.14 (s, 1H), 1.96-1.75 (m, 6H), 1.50-1.34 (m, 2H); MS: 499.2 (M+1)⁺.

### Compound 187

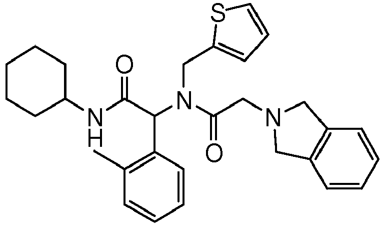

¹H NMR (300 MHz, DMSO-d6): δ 7.98-7.82 (m, 1H), 7.24-6.28 (m, 10H), 5.84-5.64 (m, 1H), 5.10-4.62 (m, 1H), 4.27-4.22 (m, 1H), 4.05-3.99 (m, 4H), 3.68-3.51 (m, 2H), 3.32 (s, 1H), 2.20-1.93 (m, 3H), 1.73-1.44 (m, 4H), 1.25-0.95 (m, 6H); MS: 502.2 (M+1)⁺.

### Compound 191

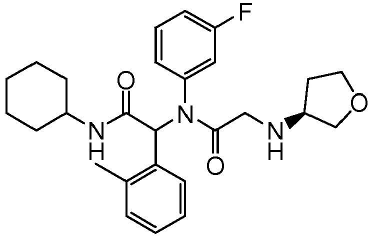

¹H NMR (300 MHz, DMSO-d6): δ 8.03-8.00 (d, 1H,J=8.1), 7.10-7.08 (m, 2H), 7.03-6.97 (m, 2H), 6.87-6.82 (m, 1H), 6.72-6.70 (d, 1H, J=7.5), 6.23 (s, 1H), 3.70-3.54 (m, 4H),3.21-3.12 (m, 2H), 2.91-2.85 (d, 1H, J=16.5), 2.35 (s, 3H), 1.84-1.50 (m, 7H), 1.30-0.95 (m, 6H); MS: 468.2 (M+1)⁺.

### Compound 188

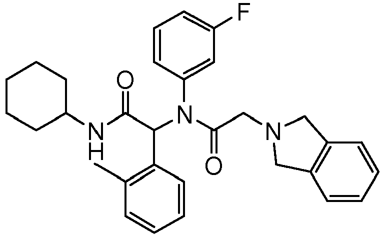

¹H NMR (300 MHz, DMSO-d6): δ 8.01-7.99 (d, 1H,J=7.8), 7.76 (dr, 1H), 7.24-6.94 (m, 8H), 6.88-6.83 (m, 1H), 6.73-6.70 (d, 1H, J=7.5), 6.26 (s, 1H), 3.99 (s, 4H),3.64-3.62 (d, 1H, J=7.5), 3.46 (s, 1H), 3.32 (s, 1H), 2.37 (s, 3H), 1.78-1.51 (m, 5H), 1.30-0.95 (m, 5H); MS: 500.2 (M+1)⁺.

### Compound 192

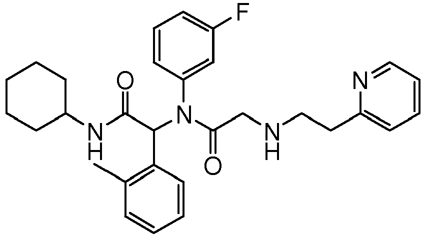

¹H NMR (300 MHz, DMSO-d6): δ 8.49-8.47 (d, 1H,J=4.2), 8.10-8.09 (m, 1H), 7.84-7.79 (m, 2H), 7.37-7.32 (m, 2H), 7.11-6.98 (m, 4H), 6.87-6.82 (m, 1H), 6.72-6.70 (d, 1H, J=7.8),6.26 (s, 1H), 3.63-3.60 (m, 1H), 3.42 (s, 1H), 3.08-2.82 (m, 5H), 2.39 (s, 3H), 1.78-1.51 (m, 5H), 1.29-0.95 (m, 5H); MS: 503.3 (M+1)⁺.

### Compound 184 and its HCl Salt

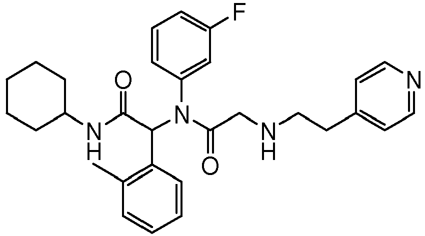

¹H NMR (300 MHz, DMSO-d6): δ 8.42-8.41 (d, 2H,J=4.2), 8.01-7.99 (d, 1H,J=5.1), 7.73 (dr, 1H), 7.21-6.94 (m, 6H), 6.86-6.83 (m, 1H), 6.72-6.70 (d, 1H, J=6), 6.52 (dr, 1H), 6.23 (s, 1H), 3.63-3.61 (m, 1H), 3.13-3.08 (d, 1H, J=12.3), 2.88-2.84 (d, 1H, J=12.3), 2.69-2.64 (m, 4H), 2.33 (s, 3H), 1.78-1.52 (m, 5H), 1.29-0.96 (m, 5H); MS: 503.3 (M+1)⁺.
HCl Salt:
¹H NMR (400 MHz, DMSO-d6): δ9.25(m, 2H), 8.77(d, 2H, J = 4.5), 8.18(m, 1H), 7.80(m, 3H), 7.38-6.59(m, 8H), 6.24(s, 1H), 3.82-3.63 (m, 5H), 3.24-3.16(m, 4H), 2.38(s, 3H), 1.72-1.50(m, 7H), 1.32-1.07(m, 5H); MS: 503.3 (M+1)⁺.

### Compound 201 and its HCl Salt

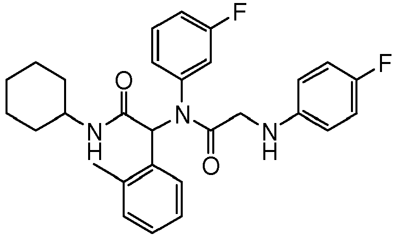

¹H NMR (400 MHz, DMSO-d6): δ 8.02 (s, 1H), 7.86 (dr, 1H), 7.12-7.00 (m, 4H), 6.90-6.85 (m, 3H), 6.74-6.72 (d, 1H, J=7.2), 6.44-6.41 (m, 2H), 6.23 (s, 1H), 5.67-5.64 (m, 1H), 3.70-3.61 (m, 2H), 2.36 (s, 3H), 1.76-1.52 (m, 5H), 1.29-0.96 (m, 5H); MS: 492.2 (M+1)⁺.
HCl Salt:
¹H NMR (400MHz, DMSO-d6): δ8.05(s, 1H), 7.85(dr, 1H), 7.12-6.86(m, 7H), 6.74-6.53(m, 3H), 6.23(s, 1H), 5.67-5.64(m, 1H), 3.70-3.61(m, 2H), 3.38-3.33(d, 1H, J=20), 2.37(s, 3H), 1.73-1.51(m, 5H), 1.28-0.98(m, 5H); MS: 492.2 (M+1)⁺.

### Compound 193

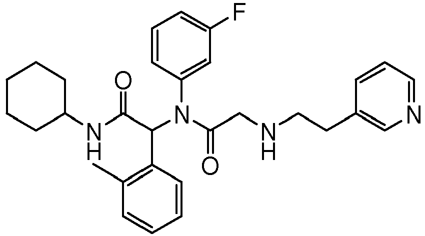

¹H NMR (300 MHz, DMSO-d6): δ 8.39-8.37 (m, 2H), 8.01-7.99 (d, 1H,J=7.5), 7.60-7.58 (d, 1H, J=8.1), 7.29-7.25 (m, 1H), 7.10-6.96 (m, 4H), 6.86-6.82 (m, 1H), 6.72-6.70 (d, 1H, J=7.2), 6.23 (s, 1H), 3.63-3.60 (d, 1H, J=8.1), 3.17-3.12 (d, 1H, J=16.2), 2.93-2.87 (m, 1H), 2.71-2.62 (m, 4H), 2.33 (s, 3H), 1.78-1.51 (m, 5H), 1.29-0.95 (m, 5H); MS: 503.3 (M+1)⁺.

### Compound 206

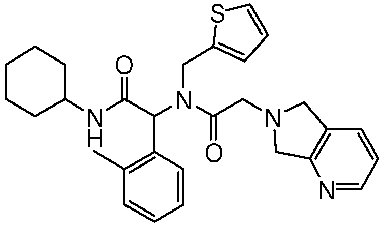

¹H NMR (400 MHz, DMSO-d6): δ 8.35-8.33 (m, 1H), 7.96-7.65 (m, 2H), 7.26-7.11 (m, 5H), 6.98-6.72 (m, 1H), 6.54-6.26 (m, 1H), 5.80-5.62 (m, 1H), 5.05-4.63 (m, 1H), 4.26-4.22 (d, 1H, J=15.2), 4.05-3.94 (m, 4H), 3.78-3.74 (m, 1H), 3.53-3.48 (m, 1H), 3.26-3.23 (m, 1H), 2.20-1.94 (m, 3H), 1.72-1.43 (m, 4H), 1.23-0.76 (m, 6H); MS: 503.2 (M+1)⁺.

### Compound 209 and its HCl Salt

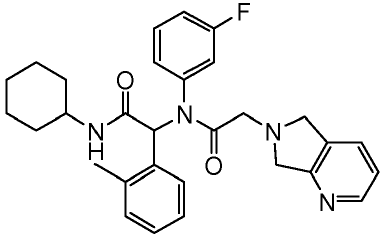

¹H NMR (400 MHz, DMSO-d6): δ 8.33-8.32 (d, 1H, J=4.4), 8.01-7.99 (d, 1H, J=7.2), 7.63-7.61 (d, 2H, J=7.6), 7.19-6.95 (m, 5H), 6.87-6.84 (m, 1H), 6.73-6.61 (d, 1H, J=7.6), 6.25 (s, 1H), 3.99-3.94 (m, 4H), 3.63-3.62 (m, 1H), 3.44-3.40 (d, 1H, J=15.6), 3.23-3.19 (d, 1H, J=16), 2.36 (s, 3H), 1.74-1.52 (m, 5H), 1.29-0.95 (m, 5H); MS: 501.3 (M+1)⁺.
HCl Salt:
¹H NMR (400MHz, DMSO-d6): δ8.33-8.32(d, 1H, J=4.8), 8.01-7.95(m, 1H), 7.63-7.61(d, 1H, J=7.6), 7.19-6.95(m, 5H), 6.87-6.84(m, 1H), 6.73-6.71(d, 1H, J=7.6), 6.25(s, 1H), 3.99-3.90(m, 4H), 3.62(s, 1H), 3.45-3.41(d, 1H, J=16), 3.23-3.18(d, 1H, J=18), 2.37(s, 3H), 1.74-1.52(m, 5H), 1.29-0.85(m, 5H); MS: 501.3 (M+1)⁺.

### Compound 218

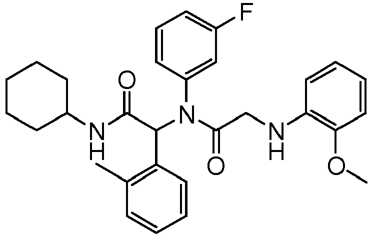

¹H NMR (400 MHz, DMSO-d6): δ 8.04 (s, 1H), 7.84 (dr, 1H), 7.12-7.01 (m, 3H), 6.88-6.68 (m, 4H), 6.57-6.54 (m, 1H), 6.24 (s, 1H), 6.12-6.10 (d, 1H, J=7.6), 5.16-5.13 (m, 1H), 3.79 (s, 3H), 3.73-3.61 (m, 2H), 3.37 (s, 1H), 2.37 (s, 3H), 1.78-1.52 (m, 5H), 1.29-0.96 (m, 5H); MS: 504.2 (M+1)⁺.

### Compound 210

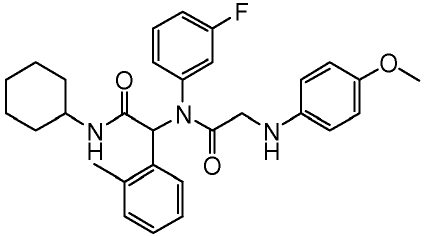

¹H NMR (400 MHz, DMSO-d6): δ 8.01 (s, 1H), 7.84 (dr, 1H), 7.10-7.01 (m, 3H), 6.88-6.84 (m, 1H), 6.74-6.66 (m, 4H), 6.40-6.38 (d, 2H, J=8.8), 6.24 (s, 1H), 6.27 (s, 1H), 3.61 (s, 5H), 3.26 (s,1H), 2.36 (s, 3H), 1.77-1.52 (m, 5H), 1.28-0.99 (m, 5H); MS: 504.3 (M+1)⁺.

### Compound 219

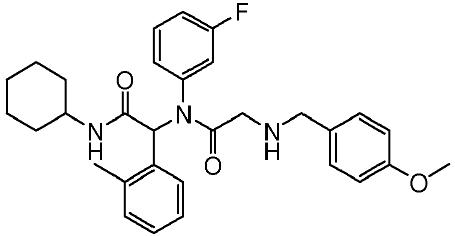

¹H NMR (400 MHz, DMSO-d6): δ 8.08 (s, 1H), 7.75 (dr, 1H), 7.21-7.19 (d, 2H, J=8.4), 7.12-6.96 (m, 4H), 6.87-6.84 (m, 3H), 6.73-6.71 (m, 1H), 6.24 (s, 1H), 3.72-3.61 (m, 6H), 3.28-3.24 (d, 1H, J=16.8), 2.99-2.95 (d, 1H, J=16.4), 2.35 (s, 3H), 1.76-1.52 (m, 5H), 1.30-0.96 (m, 5H); MS: 518.3 (M+1)⁺.

### Compound 220

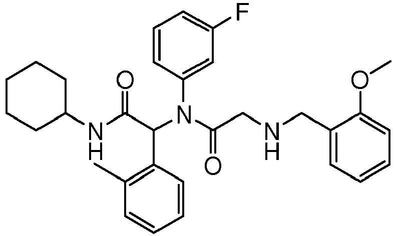

¹H NMR (400 MHz, DMSO-d6): δ 8.07-8.06 (d, 1H, J=5.2), 7.77 (dr, 1H), 7.27-7.21 (m, 2H), 7.11-6.83 (m, 7H), 6.72-6.70 (d, 1H, J=7.6), 6.48 (dr, 1H), 6.23 (s, 1H), 3.75-3.62 (m, 6H), 3.30-3.26 (m, 2H), 3.01-2.97 (d, 1H, J=16), 2.35 (s, 3H), 1.78-1.52 (m, 5H), 1.29-0.95 (m, 5H); MS: 518.3 (M+1)⁺.

### Compound 221 (HCl Salt)

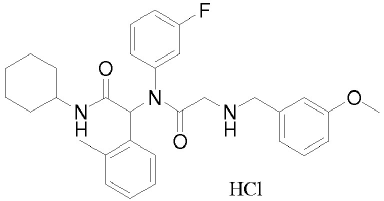

¹H NMR (400MHz, DMSO-d6): δ9.29(s, 1H), 8.16(s, 1H), 7.76(s, 1H), 7.30-7.26(m, 1H), 7.11-6.87(m, 8H), 6.73-6.71(d, 1H, J=6.4), 6.52(s, 1H), 6.23(s, 1H), 4.06-3.94(m, 2H), 3.74-3.65(m, 6H), 2.36(s, 3H), 1.78-1.52(m, 5H), 1.30-0.98(m, 5H); MS: 518.3 (M+1)⁺.

### Compound 247

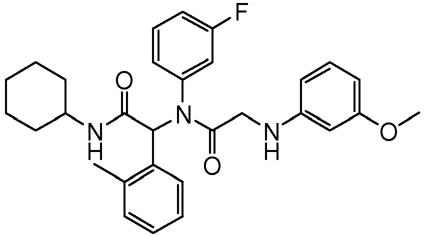

¹H NMR (400 MHz, DMSO-d6): δ 8.03 (s, 1H), 7.86 (dr, 1H), 7.12-6.72 (m, 6H), 6.23 (s, 1H), 6.13-6.11 (m, 1H), 6.03-5.99 (m, 2H), 5.74-5.71 (m, 1H), 3.70-3.61 (m, 5H), 3.33 (s, 1H), 2.33 (s, 3H), 1.77-1.52 (m, 5H), 1.28-0.85 (m, 5H); MS: 504.2 (M+1)⁺.

### Compound 256

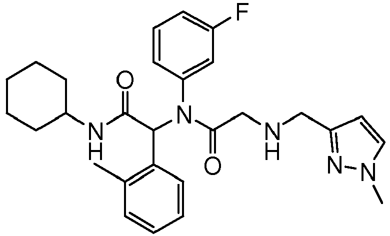

¹H NMR (400 MHz, DMSO-d6): δ 8.03-8.01 (d, 1H, J=6.8), 7.75 (dr, 1H), 7.51 (s, 1H), 7.11-6.93 (m, 3H), 6.86-6.82 (m, 1H), 6.71-6.69 (d, 1H, J=7.2), 6.46 (dr, 1H), 6.25 (s, 1H), 6.01 (s, 1H), 3.73 (s, 3H), 3.64-3.61 (m, 1H), 3.51 (s, 1H), 3.10-3.06 (d, 1H, J=16.8), 2.87-2.83 (d, 1H, J=16), 2.36 (s, 3H), 2.15 (s, 1H), 1.79-1.52 (m, 5H), 1.29-0.95 (m, 5H); MS: 492.3 (M+1)⁺.

### Compound 267

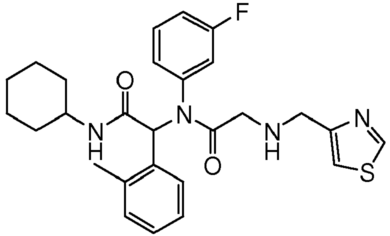

¹H NMR (400 MHz, DMSO-d6): δ 9.01-9.00 (d, 1H, J=1.6), 8.04-8.02 (d, 1H, J=5.2), 7.73 (dr, 1H), 7.38 (s, 1H), 7.10-6.93 (m, 4H), 6.86-6.83 (m, 1H), 6.72-6.70 (d, 1H, J=7.2), 6.49 (dr, 1H), 6.25 (s, 1H), 3.75 (s, 2H), 3.64-3.62 (m, 1H), 3.18-3.14 (d, 1H, J=16.4), 2.91-2.87 (d, 1H, J=16.4), 2.44 (s, 1H), 2.35 (s, 3H), 1.78-1.52 (m, 5H), 1.29-0.95 (m, 5H); MS: 495.1 (M+1)⁺.

### Compound 257

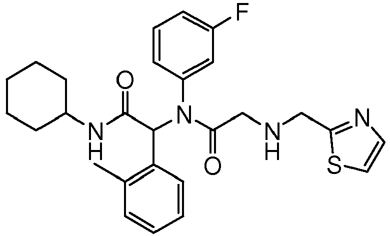

¹H NMR (400 MHz, DMSO-d6): δ 8.04-8.02 (d, 1H, J=6.4), 7.67-7.66 (d, 1H, J=3.2), 7.55-7.54 (d, 1H, J=3.2), 7.11-6.82 (m, 5H), 6.71-6.69 (d, 1H, J=7.6), 6.25 (s, 1H), 3.95 (s, 2H), 3.64-3.62 (m, 1H), 3.22-3.18 (d, 1H, J=16.8), 2.98-2.94 (d, 2H, J=16.4), 2.37 (s, 3H), 1.76-1.52 (m, 5H), 1.30-0.95 (m, 5H); MS: 495.1 (M+1)⁺.

### Compound 318

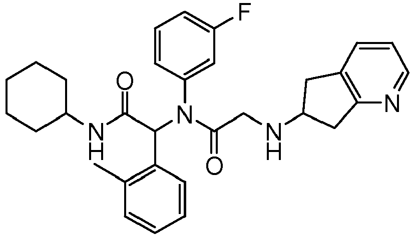

¹H NMR (400 MHz, DMSO-d6): δ 8.25-8.24 (d, 1H, J=4.4), 8.05 (s, 1H), 7.79 (dr, 1H), 7.54-7.52 (d, 1H, J=7.2), 7.26-6.99 (m, 5H), 6.88-6.84 (m, 1H), 6.73-6.59 (m, 2H), 6.24 (s, 1H), 3.63-3.52 (m, 2H), 3.33-3.15 (m, 1H), 3.04-2.95 (m, 2H), 2.70-2.63 (m, 2H), 2.36-2.31 (m, 3H), 1.78-1.52 (m, 5H), 1.29-0.84 (m, 5H); MS: 515.1 (M+1)⁺.

### Compound 119

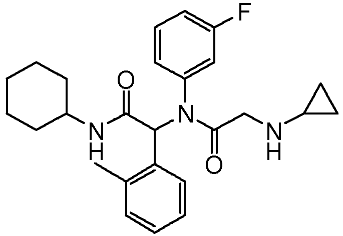

¹H NMR (300 MHz, DMSO-d6): δ 8.01-7.99 (d, 1H, J=6.9), 7.88-7.61 (br, 1H), 7.11-6.98 (m, 4H), 6.84-6.82 (t, 1H), 6.72-6.70 (d, 1H, J=7.5), 6.26 (s, 1H), 3.64-3.62 (m, 1H), 3.16-2.86 (q, 2H), 2.35 (s, 3H), 2.06-2.04 (m, 1H), 1.79-1.51 (m, 5H), 1.30-1.16 (m, 5H), 0.26-0.09 (m, 4H); MS: 438.2(M+1)⁺.

### Compound 120

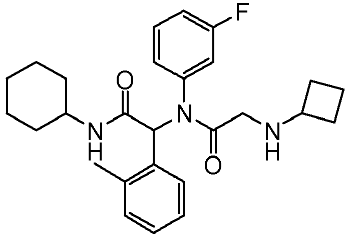

¹H NMR (300 MHz, DMSO-d6): δ 8.00-7.97 (d, 1H, J=6.9), 7.88-7.61 (br, 1H), 7.10-6.94 (m, 4H), 6.88-6.81 (t, 1H), 6.71-6.69 (d, 1H, J=7.2), 6.24 (s, 1H), 3.63-3.60 (m, 1H), 3.02-2.74 (m, 3H), 2.35 (s, 3H), 1.99-0.96 (m, 16H); MS: 452.2 (M+1)⁺.

### Compound 121

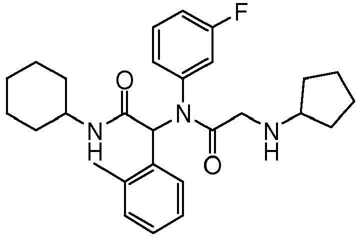

¹H NMR (300 MHz, DMSO-d6): δ 8.00-7.97 (d, 1H, J=7.2), 7.88-7.61 (br, 1H), 7.10-6.94 (m, 4H), 6.87-6.82 (t, 1H), 6.72-6.69 (d, 1H, J=7.5), 6.24 (s, 1H), 3.64-3.61 (m, 1H), 3.09-2.85 (m, 3H), 2.35 (s, 3H), 1.80-1.00 (m, 18H); MS: 466.2 (M+1)⁺.

### Compound 133

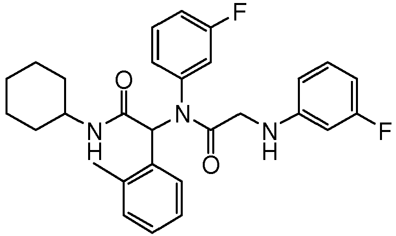

¹H NMR (300 MHz, DMSO-d6): δ 8.01-7.99 (d, 1H, J=6.9), 7.30-6.99 (m, 5H), 6.89-6.84 (t, 1H), 6.75-6.72 (d, 1H, J=7.8), 6.31-6.21 (m, 4H), 6.08-6.05 (m, 1H), 3.69-3.61 (m, 2H), 3.39-3.34 (m, 1H), 2.37 (s, 3H), 1.77-1.52 (m, 5H), 1.29-0.96 (m, 5H); MS: 492.2(M+1)⁺.

### Compound 141

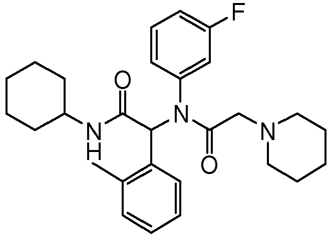

¹H NMR (300 MHz, DMSO-d6): δ 7.96-7.94 (d, 1H, J=6.6), 7.10-6.82 (m, 5H), 6.72-6.69 (d, 1H, J=7.8), 6.23 (s, 1H), 3.64-3.61 (m, 1H), 2.88-2.78 (m, 2H), 2.34 (s, 3H), 2.21 (s, 4H), 1.78-1.52 (m, 5H), 1.36-0.96 (m, 5H); MS: 466.2 (M+1)⁺.

### Compound 152

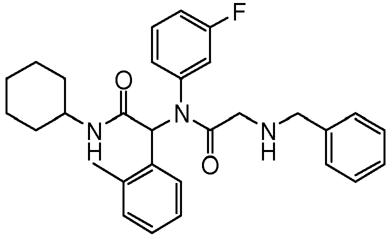

¹H NMR (300 MHz, DMSO-d6): δ 8.02-8.00 (d, 1H, J=7.8), 7.29-6.84 (m, 11H), 6.73-6.70 (d, 1H, J=7.2), 6.26 (s, 1H), 3.63-3.60 (m, 1H), 3.59 (s, 2H), 3.10-2.81 (m, 2H), 2.35 (s, 3H), 1.74-1.52 (m, 5H), 1.27-0.99 (m, 5H); MS: 488.3 (M+1)⁺.

### Compound 154

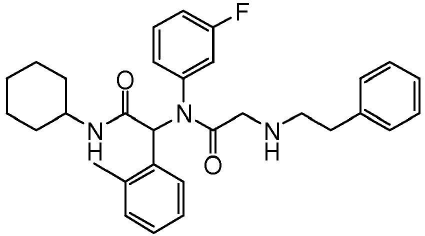

¹H NMR (300 MHz, DMSO-d6): δ 8.00-7.97 (d, 1H, J=7.5), 7.28-6.96 (m, 9H), 6.86-6.82 (t, 1H), 6.72-6.69 (d, 1H, J=7.8), 6.23 (s, 1H), 3.61-3.59 (m, 1H), 3.13-2.84 (m, 2H), 2.63 (s, 4H), 2.34 (s, 3H), 1.79-1.52 (m, 5H), 1.30-1.00 (m, 5H); MS: 502.3 (M+1)⁺.

### Compound 135 and its HCl Salt

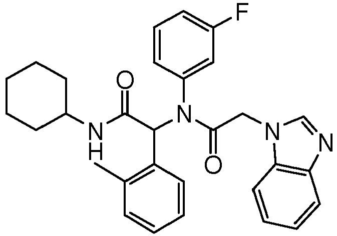

¹H NMR (300 MHz, DMSO-d6): δ 8.05 (s, 1H), 8.00-7.98 (d, 1H, J=8.1), 7.65-7.61 (d, 1H, J=9.3), 7.39-7.36 (d, 1H, J=8.7), 7.27-6.87 (m, 7H), 6.78-6.75 (d, 1H, J=7.5), 6.20 (s, 1H), 5.04-4.69 (m, 2H), 3.61-3.59 (m, 1H), 2.40 (s, 3H), 1.77-1.50 (m, 5H), 1.26-0.94 (m, 5H); MS: 499.2 (M+1)⁺.
HCl Salt:
¹H NMR (300 MHz, DMSO-d6): δ9.51(s, 1H), 8.05(s, 1H), 8.00-7.98(d, 1H, J=8.1), 7.89-7.76(m, 12H), 6.19(s, 1H), 5.38-5.05(m, 2H), 3.57-3.54(m, 1H), 2.43(s, 3H), 1.77-1.50 (m, 5H), 1.26-0.95 (m, 5H); MS: 499.2(M+1)⁺.

### Compound 153

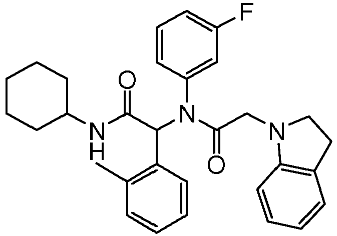

¹H NMR (300 MHz, DMSO-d6): δ 7.98-7.96 (d, 1H, J=7.2), 7.12-6.83 (m, 7H), 6.74-6.72 (d, 1H, J=7.5), 6.54-6.49 (t, 1H), 6.24-6.22 (m, 2H), 3.79-3.49 (m, 3H), 3.38-3.35 (m, 2H), 2.87-2.81 (t, 2H), 2.37 (s, 3H), 1.74-1.51 (m, 5H), 1.29-0.95 (m, 5H); MS: 500.2 (M+1)⁺.

### Compound 143

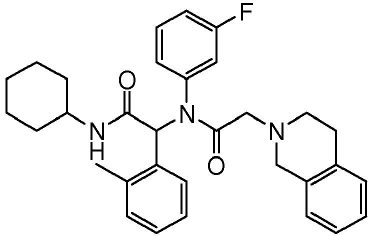

¹H NMR (300 MHz, DMSO-d6): δ 8.00-7.97 (d, 1H, J=7.8), 7.10-6.85 (m, 9H), 6.73-6.71 (d, 1H, J=7.5), 6.27 (s, 1H), 3.56-3.54 (m, 1H), 3.53 (s, 2H), 3.14-2.92 (m, 2H), 2.71-2.61 (m, 4H), 2.35 (s, 3H), 1.75-1.57 (m, 5H), 1.26-0.95 (m, 5H); MS: 514.3 (M+1)⁺.

### Compound 156

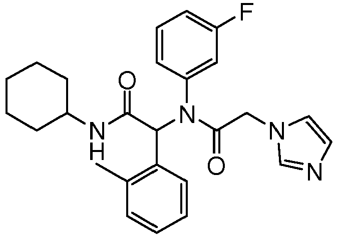

¹H NMR (300 MHz, DMSO-d6): δ 8.02-8.00 (d, 1H, J=7.2), 7.59 (s, 1H), 7.39 (s, 1H), 7.14-7.01 (m, 4H), 6.89-6.84 (t, 1H), 6.73-6.71 (d, 1H, J=7.2), 6.22-6.20 (m, 2H), 4.87-4.56 (m, 2H), 3.62-3.60 (m, 1H), 2.38 (s, 3H), 1.76-1.51 (m, 5H), 1.29-0.94 (m, 5H); MS: 449.2(M+1)⁺.

### Compound 155

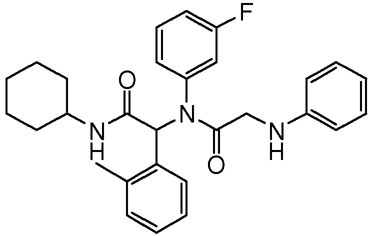

¹H NMR (300 MHz, DMSO-d6): δ 8.02-8.00 (d, 2H, J=7.5), 7.86 (br, 1H), 7.12-6.42 (m, 11H), 5.66 (br, 1H), 3.71-3.37 (m, 3H), 2.37 (s, 3H), 1.77-1.51 (m, 5H), 1.29-1.00 (m, 5H); MS: 474.2 (M+1)⁺.

### Compound 134 and its HCl Salt

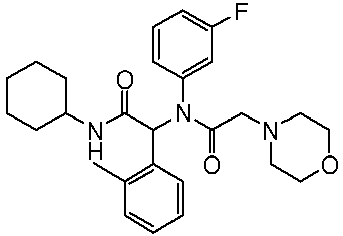

¹H NMR (300 MHz, DMSO-d6): δ 7.98 (s, 1H), 7.76 (br, 1H), 7.09-6.83 (m, 5H), 6.72-6.71 (d, 1H, J=5.7), 6.23 (s, 1H), 3.63-3.62 (m, 1H), 3.46 (s, 4H), 2.85-2.93 (m, 2H), 2.34 (s, 3H), 2.28 (s, 4H), 1.78-1.52 (m, 5H), 1.29-0.95 (m, 5H); MS: 468.2 (M+1)⁺.
HCl Salt:
¹H NMR (300 MHz, DMSO-d6): δ10.65 (br, 1H), 8.18(s, 1H), 7.77(s, 1H), 7.35-6.66 (m, 6H), 6.23(s, 1H), 4.03(br, 1H), 3.82(s, 4H), 3.64-3.62(m, 1H), 3.36-3.17(m, 5H), 2.38(s, 3H), 1.77-1.52 (m, 5H), 1.29-0.95 (m, 5H); MS: 468.3(M+1)⁺.

### Compound 165 and its HCl Salt

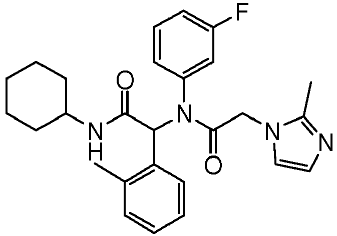

¹H NMR (300 MHz, DMSO-d6): δ 8.00-7.98 (d, 1H, J=7.5), 7.13-6.73 (m, 6H), 6.65 (s, 1H), 4.66-4.31 (m, 2H), 3.64-3.60 (m, 1H), 2.37 (s, 3H), 2.09 (s, 3H),1.74-1.51 (m, 5H), 1.30-0.95 (m, 5H); MS: 463.2 (M+1)⁺.
HCl Salt:
¹H NMR (300 MHz, DMSO-d6): δ14.82(br, 1H), 8.09-8.07(d, 1H, J=6.3), 7.85(br,1H), 7.52(s, 2H), 7.13-6.74(m, 6H), 6.18 (s, 1H), 5.08-4.67(m, 2H), 3.64-3.61 (m, 1H), 2.48(s, 3H), 2.38(s, 3H), 1.74-1.51(m, 5H), 1.30-0.95(m, 5H); MS: 463.2 (M+1)⁺.

### Compound 380

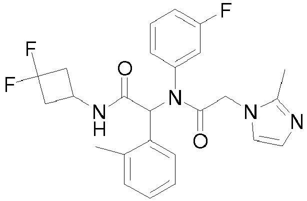

¹H NMR (400 MHz, CDCl3): δ 7.54 (br, 1H), 7.07 (m, 2H), 6.89 (m, 3H), 6.65 (s, 1H), 6.40 (s, 1H), 6.30 (m, 3H), 4.31 (s, 2H), 4.17 (m, 1), 2.85 (m, 2H), 2.30-2.17 (m, 7H), 1.92(m, 2H); MS: 470.9 (M+1)⁺.

### Compound 170

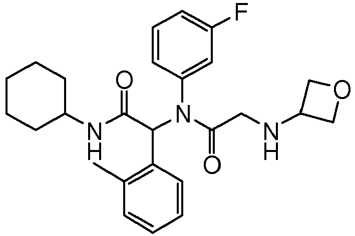

¹H NMR (300 MHz, DMSO-d6): δ 8.01-7.99 (d, 1H, J=7.5), 7.10-6.70 (m, 6H), 6.23 (s, 1H), 4.52-4.47 (m, 2H), 4.28-4.22 (m, 2H), 3.76-3.71 (m, 1H), 3.63-3.61 (m, 1H), 3.12-2.81 (m, 2H), 2.59 (s, 1H), 2.34 (s, 3H), 1.79-1.52 (m, 5H), 1.30-0.96 (m, 5H); MS: 454.3 (M+1)⁺.

### Compound 173 and its HCl Salt

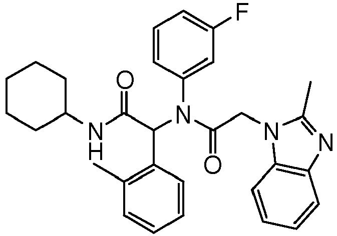

¹H NMR (300 MHz, DMSO-d6): δ 8.00-7.97 (m, 2H), 7.51-6.76 (m, 11H), 6.18 (s, 1H), 4.99-4.51 (m, 2H), 3.60-3.59 (m, 1H), 2.39-2.38 (m, 6H), 1.74-1.50 (m, 5H), 1.28-0.93 (m, 5H); MS: 513.3 (M+1)⁺.
HCl Salt:
¹H NMR (400 MHz, DMSO-d6): δ8.10-6.80(m, 13H), 6.17(s, 1H), 5.40(m, 1H), 4.77(m, 1H), 3.59(m, 1H), 2.74(s, 3H), 2.39(s, 3H), 1.78-1.50(m, 5H), 1.23-0.96(m, 5H); MS: 513.2 (M+1)⁺.

### Compound 180

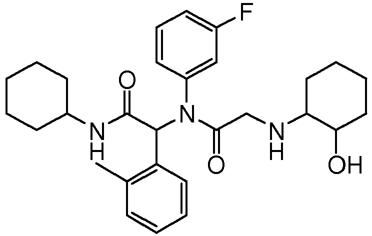

¹H NMR (300 MHz, DMSO-d6): δ 8.01-7.99 (m, 1H), 7.11-6.70 (m, 6H), 6.24 (s, 1H), 4.27 (s, 1H), 3.61-3.51 (m, 2H), 3.12-2.81 (m, 2H), 2.35 (s, 3H), 2.03 (s,1H), 1.80-1.42 (m, 7H), 1.30-0.96 (m, 9H); MS: 496.0 (M+1)⁺.

### Compound 181

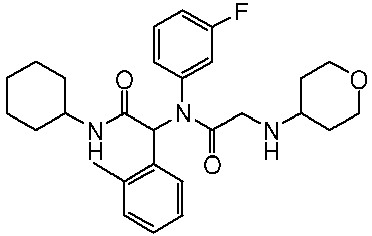

¹H NMR (300 MHz, DMSO-d6): δ 8.07 (s, 1H), 7.79 (br, 1H), 7.12-6.59 (m, 7H), 6.24 (s, 1H), 3.79-3.77 (d, 2H, J=8.1), 3.64-3.62 (m, 1H), 3.40-3.36 (m,1H), 3.22-3.17 (m, 2H), 3.11-3.07 (m, 1H), 2.74 (s, 1H), 2.36 (s,3H), 1.78-1.52 (m, 7H), 1.32-0.97 (m, 8H); MS: 482.3(M+1)⁺.

### Compound 171 and its HCl Salt

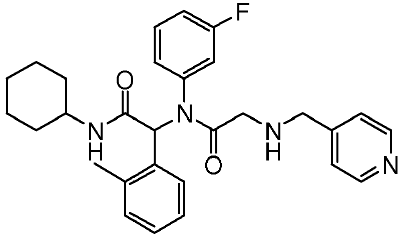

¹H NMR (300 MHz, DMSO-d6): δ 8.44-8.42 (d, 1H, J=5.4), 8.02-8.00 (d, 1H, J=7.2), 7.25-6.70 (m, 8H), 6.25 (s, 1H), 3.65-3.60 (m, 3H), 3.13-2.82 (m, 2H), 2.35 (s, 3H), 1.80-1.52 (m, 5H), 1.31-0.96 (m, 5H); MS: 489.3 (M+1)⁺.
HCl Salt:
¹H NMR (300 MHz, DMSO-d6): δ9.89(br, 1H), 8.79(s, 2H), 8.14(s, 1H), 7.78(s, 3H), 7.14-6.59(m, 7H), 6.22(s, 1H), 4.25(s, 2H), 3.87(m, 1H), 3.63(m, 2H), 2.37(s, 3H), 1.72-1.54(m, 5H), 1.32-0.96(m, 5H); MS: 489.2 (M+1)⁺.

### Compound 174

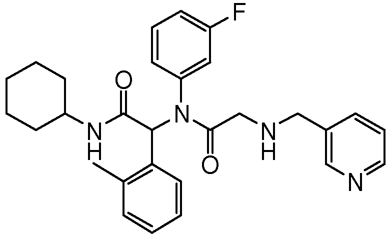

¹H NMR (300 MHz, DMSO-d6): δ 8.44-8.40 (m, 2H), 8.04-8.02 (d, 1H, J=7.5), 7.68-6.70 (m, 8H), 6.25 (s, 1H), 3.67-3.61 (m, 3H), 3.16-2.90 (m, 2H), 2.35 (s, 3H), 1.80-1.52 (m, 5H), 1.31-0.96 (m, 5H); MS: 489.0 (M+1)⁺.

### Compound 172 and its HCl Salt

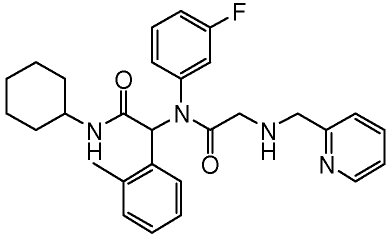

¹H NMR (300 MHz, DMSO-d6): δ 8.48-8.46 (m, 2H), 8.07-8.05 (d, 1H, J=7.2), 7.87-6.70 (m, 10H), 6.26 (s, 1H), 3.95-3.83 (m, 2H), 3.62-3.60 (m, 1H), 3.27-3.05 (s, 2H), 2.37 (s, 3H), 1.72-1.51 (m, 5H), 1.31-0.96 (m, 5H); MS: 489.0 (M+1)⁺.
HCl Salt:
¹H NMR (300 MHz, DMSO-d6): δ9.46(s, 2H), 8.60(d, 1H, J = 3.3), 8.16(s, 1H), 7.87(m, 2H), 7.46-7.07(m, 6H), 6.87(m, 1H), 6.72(s, 1H), 6.54(s, 1H), 6.25(s, 1H), 5.92(br, 2H), 4.27(s, 2H), 3.84(m, 1H), 3.63(m, 2H), 2.39(s, 3H), 1.70-1.50(m, 5H), 1.34-1.00(m, 5H); MS: 489.2 (M+1)⁺.

### Compound 177 and its HCl Salt

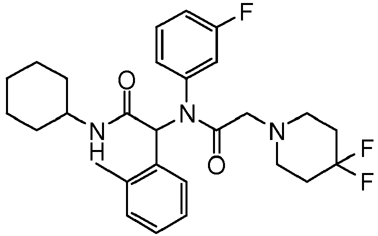

¹H NMR (300 MHz, DMSO-d6): δ 7.97 (s, 1H), 7.72 (br, 1H), 7.09-6.56 (m, 7H), 6.23 (s, 1H), 3.64-3.62 (m, 1H), 3.05-2.92 (m, 2H), 2.45 (s, 4H), 2.34 (s, 3H), 1.83-1.52 (m, 9H), 1.29-0.96 (m, 5H); MS: 502.3 (M+1)⁺.
HCl Salt:
¹H NMR (300 MHz, DMSO-d6): δ 10.29 (br, 1H), 8.13-6.61 (m, 9H), 6.21 (s, 1H), 4.09-3.19 (m, 7H), 2.37-2.29(m, 6H), 1.70-1.50(m, 5H), 1.34-1.00(m, 5H); MS: 502.2 (M+1)⁺.

### Compound 239 (HCl Salt)

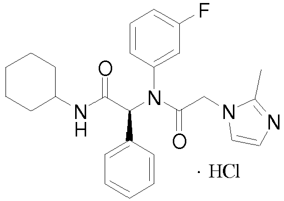

¹H NMR (300 MHz, DMSO-d6): δ14.39(s, 1H), 8.09-8.07(d, 1H, J=7.2), 7.53-7.07(m, 10H), 6.02(s, 1H), 4.94-4.74 (m, 2H), 3.61-3.58(m, 1H), 2.47(s, 3H), 1.72-1.49(m, 5H), 1.23-1.07(m, 5H); MS: 448.2(M+1)⁺.

### Compound 327 (HCl Salt)

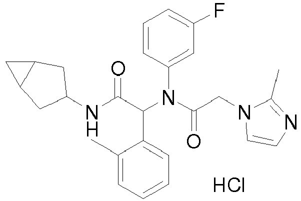

¹H NMR(400 MHz, MeOD-d4): δ 8.12 (br, 1H), 7.82 (br, 1H), 7.46 s, 2H), 7.16-6.82 (m, 7H), 5.04 (d, 1H), 4.78 (d, 1H), 4.33 (m, 1H), 2.58 (s, 3H), 2.48 (s, 3H), 2.29 (m, 2.5H), 1.71 (t, 2H), 1.30 (t, 2H), 0.46 (q, 1H), 0 (q, 1H); MS: 461.2 (M+1)⁺.

### Compound 169

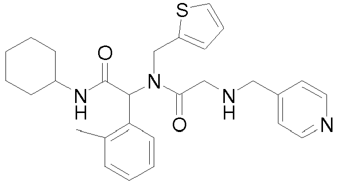

¹H NMR (300 MHz, DMSO-d6): δ 8.49-8.44 (m, 2H), 7.98-7.97 (m, 1H), 7.33-6.30 (m, 9H), 5.73-5.48 (m, 1H), 4.91-4.25 (m, 2H), 3.77-3.23 (m, 5H), 2.19-1.88 (m, 3H), 1.69-1.49 (m, 5H), 1.29-0.98 (m, 6H); MS: 491.2 (M+1)⁺.

### Compound 224

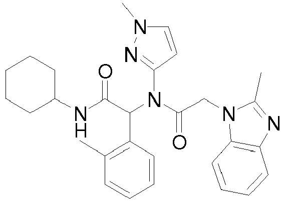

¹H NMR (300 MHz, DMSO-d6): δ 8.11-8.08 (d, 1H, J = 6.9), 7.52-6.88 (m, 9H), 6.17-6.17 (d, 1H, J = 2.1), 6.07 (s, 1H), 5.00-4.94 (d, 1H, J = 17.4), 4.65-4.59 (d, 1H, J = 18.0), 3.65-3.51 (m, 4H), 2.41 (s, 3H), 2.31 (s, 3H), 1.75-1.68 (m, 5H), 1.27-1.16 (m, 5H); MS:499.3 (M+1)⁺.

### Compound 245

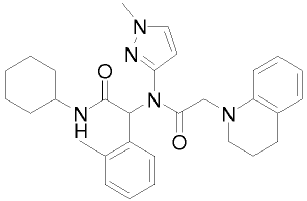

¹H NMR (400 MHz, DMSO-d6): δ 8.15-8.13 (d, 1H, J = 8.0), 7.47-7.46 (d, 1H, J = 2), 7.13-6.80 (m, 6H), 6.49-6.29 (t, 1H), 6.29-6.27 (d, 1H, J = 8.4), 5.99 (s, 1H), 5.98 (s, 1H), 3.98-3.93 (d, 1H, J = 18), 3.67-3.64 (m, 4H), 3.34-3.30 (m, 2H), 2.66-2.65 (m, 1H), 2.30 (s, 3H), 1.84-1.52 (m, 7H), 1.34-1.14 (m, 7H); MS:500.3 (M+1)⁺.

### Compound 250

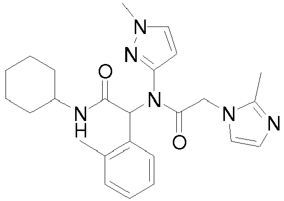

¹H NMR (400 MHz, DMSO-d6): δ 8.10-8.08 (d, 1H, J = 7.6), 7.47 (s, 1H), 7.11-6.85 (m, 5H), 6.66 (s, 1H), 6.09 (s, 2H), 4.73-4.69 (d, 1H, J = 17.6), 4.40-4.36 (d, 1H, J = 16.8), 3.66-3.61 (m, 4H), 2.30 (s, 3H), 2.11 (s, 3H), 1.76-1.52 (m, 5H), 1.52-1.06 (m, 5H); MS:449.2 (M+1)⁺.

### Compound 255

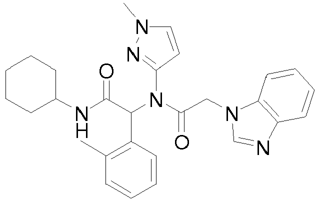

¹H NMR (400 MHz, DMSO-d6): δ 8.14 (s, 1H), 8.10-8.06 (d, 1H, J = 7.6), 7.66-7.60 (d, 1H, J = 7.6), 7.52-7.51 (d, 1H, J = 2.0), 7.33-6.88 (m, 7H), 6.18-6.17 (d, 1H, J = 1.6), 6.09 (s, 1H), 5.12-5.08 (d, 1H, J = 16.8), 4.76-4.72 (d, 1H, J = 17.2), 3.65-3.55 (m, 4H), 2.32 (s, 3H), 1.74-1.63 (m, 5H), 1.30-1.09 (m, 5H); MS:485.2 (M+1)⁺.

### Compound 314

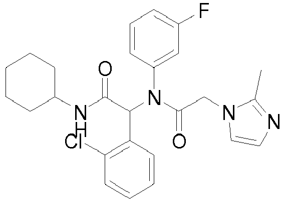

¹H NMR (400 MHz, DMSO-d6): δ 8.30-8.28 (d, 1H, J = 5.2), 7.80-7.79 (d, 1H, J = 6.0), 7.40-6.79 (m, 9H), 6.30 (s, 1H), 4.78 (s, 1H), 4.51-4.47 (d, 1H, J = 16.4), 3.63-3.59 (m, 1H), 2.21 (s, 3H), 1.74-1.51 (m, 5H), 1.28-0.89 (m, 5H); MS:483.1 (M+1)⁺.

### Compound 322

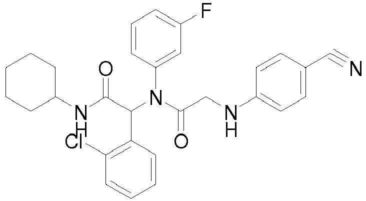

¹H NMR (400 MHz, DMSO-d6): δ 8.28-8.27 (d, 1H J = 6.4), 7.79-6.82 (m, 10H), 6.56-6.53 (d, 2H, J = 8.4), 6.33 (s, 1H), 3.81-3.42 (m, 3H), 1.74-1.52 (m, 5H), 1.28-0.99 (m, 5H); MS: 519.0 (M+1)⁺.

### Compound 285

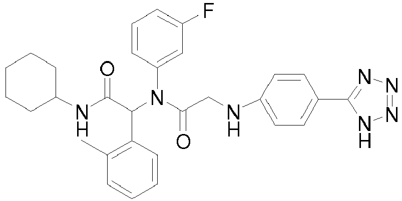

¹H NMR (300 MHz, DMSO-d6): δ 8.21-7.69 (m, 4H), 7.14-6.65 (m, 9H), 6.18-6.16 (d, 1H, J = 7.2), 5.63-5.68 (t, 1H), 5.17-5.12 (t, 1H), 3.64-3.58 (m, 1H), 2.32 (s, 3H), 1.73-1.51 (m, 5H), 1.27-0.87 (m, 5H); MS:542.2 (M+1)⁺.

### Compound 290

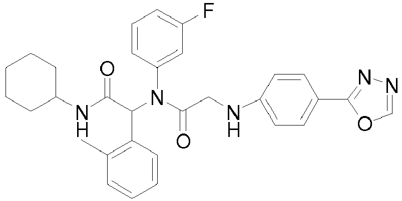

¹H NMR (400 MHz, DMSO-d6): δ 9.14 (s, 1H), 8.05 (s, 1H), 7.90 (s, 1H), 7.71-7.69 (d, 2H J = 8.4), 7.12-6.60 (m, 10H), 6.24 (s, 1H), 3.85-3.82 (d, 1H, J = 14.0), 3.63-3.42 (m, 2H), 2.38 (s, 3H), 1.73-1.51 (m, 5H), 1.28-0.86 (m, 5H); MS: 542.1 (M+1)⁺.

### Compound 291

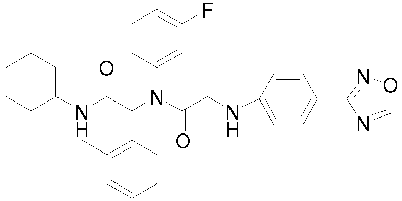

¹H NMR (400 MHz, DMSO-d6): δ 9.52 (s, 1H), 8.05 (s, 1H), 7.89 (s, 1H), 7.73-7.71 (d, 2H, J = 8.8), 7.12-6.47 (m, 9H), 6.24 (s, 1H), 3.80-3.60 (m, 3H), 2.38 (s, 3H), 1.76-1.52 (m, 5H), 1.28-1.05 (m, 5H); MS: 542.1 (M+1)⁺.

### Compound 195

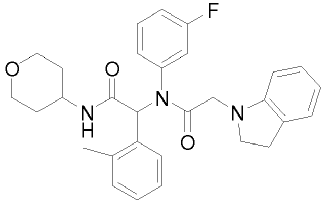

¹H NMR (300 MHz, DMSO-d6): δ 8.12-8.10 (m, 1H), 7.18-6.22 (m, 13H), 3.86-3.74 (m, 3H), 3.54-3.49 (m, 2H), 2.87-2.81 (m, 2H), 2.36 (s, 3H), 2.01-1.67 (m, 2H), 1.29-1.17 (m, 6H); MS: 502.2 (M+1)⁺.

### Compound 207

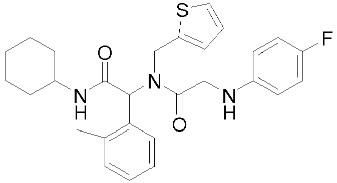

¹H NMR (400 MHz, CDCl3): δ 7.37-6.38 (m, 11H), 5.61-5.55 (m, 1H), 4.87-4.65 (m, 3H), 4.07-3.84 (m, 3H), 2.27 (s, 3H), 2.18-1.92 (m, 2H), 1.67-1.55 (m, 2H), 1.32-1.07 (m, 6H); MS: 494.2 (M+1)⁺.

### Compound 254

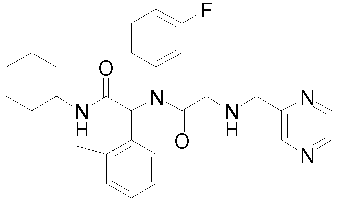

¹H NMR (400 MHz, DMSO-d6): δ 8.60-8.48 (m, 3H), 8.03-7.74 (m, 2H), 7.11-6.24 (m, 8H), 3.79 (s, 2H), 3.62 (m, 1H), 3.21-2.89 (m, 2H), 2.33 (s, 3H), 2.01 (m, 1H), 1.72-1.52 (m, 5H), 1.29-0.81 (m, 5H); MS: 490.2 (M+1)⁺.

### Compound 323

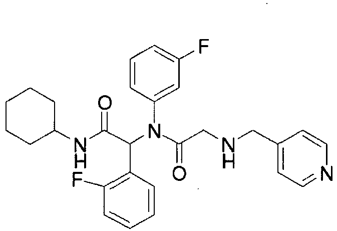

¹H NMR (400 MHz, DMSO-d6): δ 8.47 (d, 2H, J=5.6 MHz), 8.24 (d, 1H, J=7.2 MHz), 7.28-6.87 (m, 10H), 6.32 (s, 1H), 3.74-3.65 (m, 3H), 3.21-3.17 (m, 2H), 3.01-2.95 (m, 1H), 1.74-1.53 (m, 5H), 1.21-0.86 (m, 5H); MS: 493.2 (M+1)⁺.
**Example 3. Preparation of Compound 302.** Compound 302 was also synthesized via Scheme 2 using the following protocol.

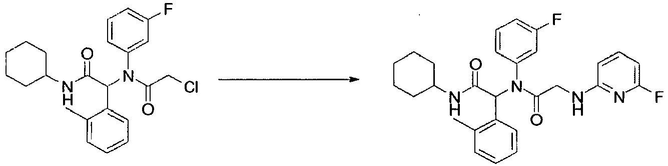

To a solution of Compound 118 (400mg, 0.96 mmol) in acetone (10 ml) was added 6-Fluoro-pyridin-2-ylamine (269 mg, 2.4mmol) and NaI (288 mg, 1.92mmol). The reaction mixture was stirred at 70°C overnight. The resulting mixture was concentrated in vacuo and DCM (20 ml) was added. The organic solution was washed with water, brine, dried over Na₂SO₄ and filtered. The solvent was evaporated in vacuo. The residue was purified by prep-TLC to give the desired product as a white solid (196 mg, 41.52% yield). ¹H NMR (400 MHz, DMSO-d6): δ 8.02 (s, 1H), 7.83 (br, 1H), 7.52-7.46 (m, 1H), 7.33-7.02 (m, 5H), 6.86 (s, 1H), 6.72-6.65 (m, 2H), 6.48-6.47 (d, 1H, J=7.2), 6.23 (s, 1H), 6.12-6.10 (d, 1H, J=6.8), 3.86-3.83 (d, 1H, J=13.6), 3.62-3.61 (d, 1H, J=6), 3.49-3.41 (m, 1H), 2.38 (s, 3H), 1.70-1.52 (m, 5H), 1.25-0.96 (m, 5H); MS: 493.1 (M+1)⁺.

The following compounds were also synthesized via Scheme 2 following the general procedure set forth above for Compound 302. The corresponding HCl salt was synthesized following the general procedure set forth in Example 1, step B.

### Compound 237

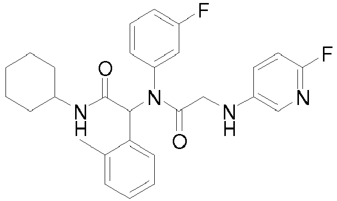

¹H NMR (400MHz, MeOD-d4), δ 8.27-8.017 (br, 2H), 7.04 (s, 1H), 7.11-6.99 (m, 4H), 6.87-6.84 (m, 2H), 6.74-6.72 (m, 2H), 6.21 (s, 1H), 6.01 (t, 1H, J=6), 3.78-3.73 (m, 1H), 3.61 (br, 1H), 3.37-3.33 (m, 1H), 2.36 (s, 3H), 1.72-1.52 (m, 5H), 1.28-0.96 (m, 5H); MS: 493.2 (M+1)⁺.

### Compound 325

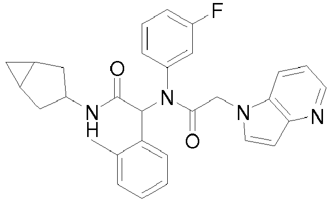

¹H NMR (400MHz, MeOD-d4), δ 8.65 (d, 1H, J=8), 8.62 (d, 1H, J=6), 8.26 (d, 1H, J=4), 8.02 (br, 1H), 7.75 (dd, 1H, J=6), 7.24-7.12 (m, 4H), 7.01-6.90 (m, 4H), 6.41(s, 1H), 5.64 (d, 0.59H, J=16), 3, 5.41 (d, 1H, J=16), 4.35 (t, 1H, J=8), 2.55 (s, 3H), 2.33-2.28 (m, 2H), 1.75-1.69 (m, 2H), 1.38-1.26 (m, 3H), 0.46 (m, 1H), 0 (m, 1H); MS: 497.2 (M+1)⁺.

### Compound 272

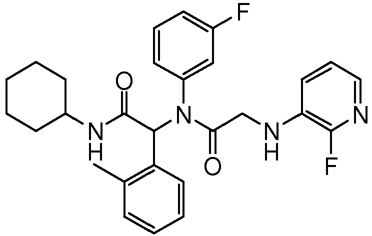

¹H NMR (400 MHz, DMSO-d6): δ 8.02 (s, 1H), 7.85 (dr, 1H), 7.35-7.34 (d, 1H, J=4.8), 7.12-7.03 (m, 4H), 6.87-6.85 (d, 2H, J=6.8), 6.74-6.66 (m, 2H), 6.21 (s, 1H), 5.82 (s, 1H), 3.81-3.79 (m, 1H), 3.62-3.60 (m, 1H), 3.47-3.42 (m, 1H), 3.31-3.26 (m, 1H), 2.43-2.34 (s, 3H), 1.72-1.52 (m, 5H), 1.28-0.95 (m, 5H); MS: 493.1 (M+1)⁺.

### Compound 258

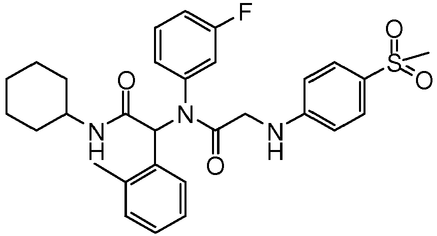

¹H NMR (400 MHz, DMSO-d6): δ 8.04-8.01 (m, 1H), 7.87 (dr, 1H), 7.55-7.53 (d, 2H, J=8.4), 7.12-6.97 (m, 4H), 6.89-6.73 (m, 3H), 6.60-6.58 (d, 2H, J=8.8), 6.22 (s, 1H), 3.85-3.81 (m, 1H), 3.62-3.61 (d, 1H, J=6.4), 3.45-3.41 (m, 1H), 3.03 (s, 3H), 2.38 (s, 3H), 1.76-1.52 (m, 5H), 1.28-0.96 (m, 5H); MS: 552.1 (M+1)⁺.

### Compound 280

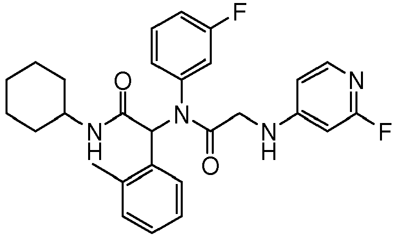

¹H NMR (400 MHz, DMSO-d6): δ 8.59-8.54 (d, 2H, J=19.2), 8.07-7.88 (m, 3H), 7.42-7.05 (m, 4H), 6.90-6.87 (m, 1H), 6.78-6.63 (m, 4H), 6.17 (s, 1H), 4.95-4.74 (m, 2H), 3.62-3.60 (d, 1H, J=6), 2.38 (s, 3H), 1.70-1.52 (m, 5H), 1.28-0.93 (m, 5H); MS: 493.1 (M+1)⁺.

### Compound 308

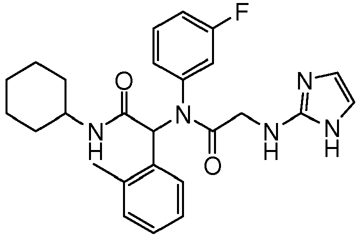

¹H NMR (400 MHz, DMSO-d6): δ 8.04-8.03 (d, 1H, J=4.4), 7.79 (dr, 1H), 7.30 (dr, 1H), 7.12-6.99 (m, 3H), 6.89-6.85 (m, 1H), 6.74-6.72 (d, 2H, J=7.2), 6.43 (s, 1H), 6.31 (s, 1H), 6.19 (s, 1H), 5.07 (s, 2H), 4.40-4.35 (d, 1H, J=16.8), 4.13-4.08 (d, 1H, J=17.6), 3.63-3.61 (m, 1H), 2.38 (s, 3H), 1.76-1.52 (m, 5H), 1.28-0.83 (m, 5H); MS: 464.1 (M+1)⁺.

### Compound 317

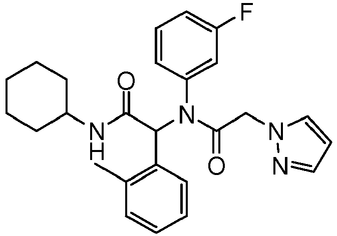

¹H NMR (400 MHz, DMSO-d6): δ 8.03 (s, 1H), 7.84-7.83 (m, 1H), 7.59 (s, 1H), 7.40-7.00 (m, 5H), 6.88-6.85 (m, 1H), 6.73-6.54 (m, 2H), 6.22-6.20 (m, 2H), 4.87-4.83 (d, 1H, J=15.6), 4.60-4.57 (d, 1H, J=15.2), 3.63-3.61 (m, 1H), 2.38-2.34 (s, 3H), 1.72-1.52 (m, 5H), 1.28-0.94 (m, 5H); MS: 449.1 (M+1)⁺.

### Compound 309

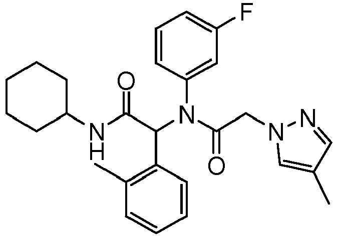

¹H NMR (400 MHz, DMSO-d6): δ 8.03 (s, 1H), 7.84 (dr, 1H), 7.32-7.00 (m, 6H), 6.88-6.84 (m, 1H), 6.72-6.51 (m, 2H), 6.18 (s, 1H), 4.77-4.73 (m, 1H), 4.51-4.47 (m, 1H), 3.62-3.59 (m, 1H), 2.37-2.34 (m, 3H), 1.98-1.96 (m, 3H), 1.76-1.52 (m, 5H), 1.28-0.94 (m, 5H); MS: 463.1 (M+1)⁺.

### Compound 279

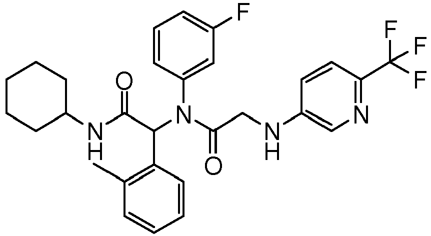

¹H NMR (400 MHz, DMSO-d6): δ 8.03 (s, 2H), 7.86 (dr, 1H), 7.51-7.49 (d, 1H, J=9.2), 7.12-7.01 (m, 3H), 6.87-6.74 (m, 4H), 6.22 (s, 1H), 3.90-3.85 (d, 1H, J=20.4), 3.61 (s, 1H), 3.48-3.44 (d, 1H, J=16), 2.37 (s, 3H), 1.75-1.52 (m, 4H), 1.28-0.99 (m, 6H); MS: 543.1 (M+1)⁺.

### Compound 298

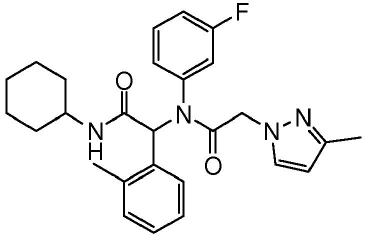

¹H NMR (400 MHz, DMSO-d6): δ 8.03 (s, 1H), 7.84 (s, 1H), 7.45-7.00 (m, 5H), 6.86-6.54 (m, 3H), 6.18 (s, 1H), 5.98 (s, 1H), 4.77-4.73 (m, 1H), 4.47-4.43 (d, 1H, J=16), 3.62-3.61 (m, 1H), 2.38-2.34 (m, 3H), 2.11 (s, 3H), 1.72-1.51 (m, 5H), 1.28-0.94 (m, 5H); MS: 463.1 (M+1)⁺.

### Compound 167

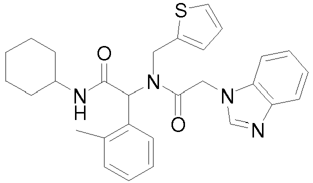

¹H NMR (300 MHz, CDCl₃): δ 7.86-6.44 (m, 14H), 5.34-4.82 (m, 4H), 3.82 (m, 1H), 2.29 (s, 3H), 1.91-0.87 (m, 10H); MS: 501.2 (M+1)⁺.

### Compound 175

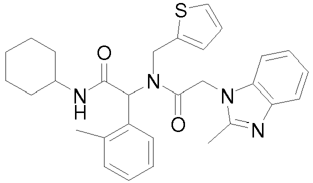

¹H NMR (300 MHz, MeOD-d4): δ 7.55-6.67 (m, 11H), 6.39 (s, 1H), 5.44-4.87 (m, 4H), 3.73 (s, 1H), 2.45 (s, 3H), 2.14 (s, 3H), 1.83-1.59 (m, 5H), 1.39-1.15 (m, 5H); MS: 515.0 (M+1)⁺.

### Compound 252

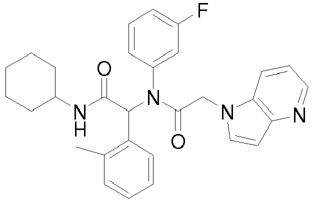

¹H NMR (400 MHz, DMSO-d6): δ 13.07 (s, 1H), 8.66-6.78 (m, 13H), 6.18 (s, 1H), 5.61-5.24 (m, 2H), 3.59 (s, 1H), 2.41 (s, 3H), 1.71-1.49 (m, 5H), 1.22-1.04 (m, 5H); MS: 499.2 (M+1)⁺.

### Compound 321

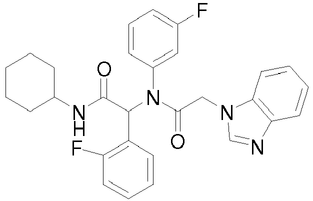

¹H NMR (400 MHz, DMSO-d6): δ 8.19-8.12 (m, 2H), 7.68-6.95 (m, 12H), 6.26 (s, 1H), 5.06 (d, 1H, J=16.8 MHz), 4.74 (d, 1H, J=20.0 MHz), 3.59 (m, 1H), 1.74-1.52 (m, 5H), 1.25-0.92 (m, 5H); MS: 503.1 (M+1)⁺.

### Compound 324

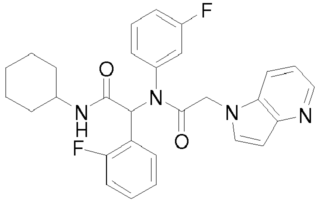

¹H NMR (400 MHz, DMSO-d6): δ 8.38-8.19 (m, 4H), 7.38-6.93 (m, 9H), 6.52 (s, 1H), 6.27 (s, 1H), 5.45-5.03 (m, 2H), 3.59 (m, 1H), 1.75-1.51 (m, 5H), 1.23-0.93 (m, 5H); MS: 503.2 (M+1)⁺.

### Compound 240

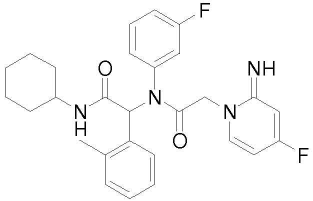

¹H NMR (400 MHz, DMSO-d6): δ 8.84 (br, 2H), 8.15-8.06 (m, 2H), 7.25-6.72 (m, 10H), 6.13 (s, 1H), 4.88-4.78 (m, 2H), 3.59 (m, 1H), 2.40 (s, 3H), 1.72-1.50 (m, 5H), 1.34-0.87 (m, 5H); MS: 493.2 (M+1)⁺.

### Compound 253

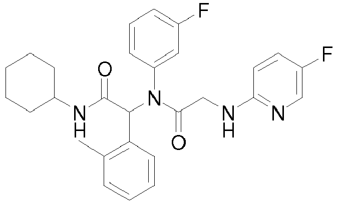

¹H NMR (400 MHz, DMSO-d6): δ 8.04 (br, 1H), 7.85 (m, 2H), 7.37-6.65 (m, 10H), 6.22 (s, 1H), 3.85 (m, 1H), 3.55 (m, 2H), 2.37 (s, 3H), 1.72-1.50 (m, 5H), 1.34-1.07 (m, 5H); MS: 493.2 (M+1)⁺.

### Compound 162

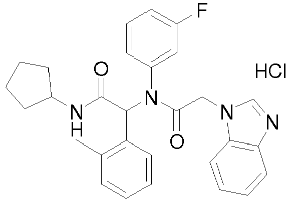

¹H NMR (400 MHz, DMSO-d6): 89.46(s, 1H), 8.13-6.77(m, 13H), 6.19(s, 1H), 5.41-5.12(m, 2H), 4.03(m, 1H), 2.42(s, 3H), 1.79(m, 2H), 1.56-1.26(m, 6H); MS: 485.6 (M+1)⁺.

### Compound 266

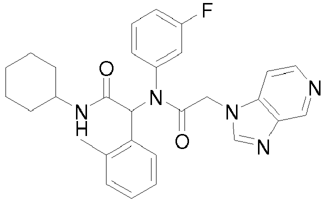

¹H-NMR (300 MHz, CDCl₃), δ 8.64-8.51 (m, 2H), 7.82-7.68 (m, 3H), 7.12-6.77 (m, 6H), 6.39 (s, 1H), 5.89 (s, 1H), 4.81-5.19 (m, 2H), 3.75 (s, 1H), 2.32 (s, 3H), 1.85-1.44 (m, 4H), 1.33-0.96 (m, 6H); MS: 499.2 (M+1)⁺.

### Compound 377

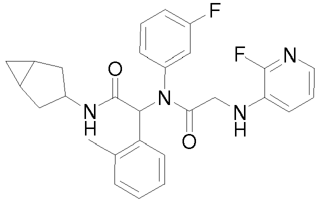

¹H NMR (400 MHz, DMSO-d6): 7.92-7.81 (2H, br), 7.36 (d, 1H, *J* = 4.4), 7.13-7.01 (m, 5H), 6.90-6.83 (m, 2H), 6.73-6.67 (m, 2H), 6.20 (s, 1H), 5.84 (s, 1H), 4.19 (s, 1H), 4.18 (d, 1H, *J* = 4.4), 3.83 (dd, 1H, *J* = 16.8, 4.8), 3.46 (d, 1H, *J* = 16.0), 3.33 (s, 1H), 2.37 (s, 3H),2.19-2.13 (m, 2H), 2.10 (s, 1H), 1.63 (q, 2H, *J* = 13.6), 1.24-1.20 (m, 3H); MS: 490.7 (M+1)⁺.

### Compound 378

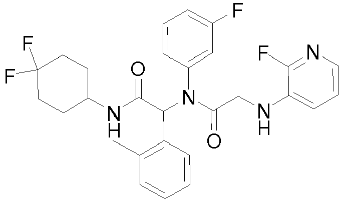

¹H NMR (400 MHz, DMSO-d6): 8.16 (br, 1H), 7.84 (br, 1H), 7.36 (d, 1H, *J* = 4.8), 7.14-7.02 (m, 5H), 6.90-6.84 (m, 2H), 6.75 (d, 1H, *J* = 8.4), 6.22 (s, 1H), 5.84 (t, 1H, *J* = 5.2), 3.84-3.79 (m, 2H), 3.49 (d, 1H, *J* = 12.4), 2.39 (s, 3H), 1.92-1.80 (m, 6H), 151-1.49 (m, 1H), 1.36-1.31 (m, 1H); MS: 528.7 (M+1)⁺.

### Compound 381

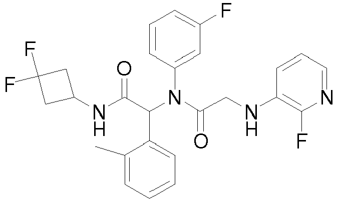

¹H NMR (400 MHz, DMSO-d6): δ 8.65 (s, 1H), 7.77-7.35 (m, 2H), 7.15-7.03 (m, 5H), 6.90-6.67 (m, 4H), 6.21(s, 1H), 5.81 (m, 1H), 4.08(m, 1H), 3.82-3.76 (m, 1H), 3.46 (m, 1H), 2.92 (m, 2H), 2.38 (m, 5H); MS: 500.9 (M+1)⁺.
**Example 4. Preparation of Compound 202 and it HCl Salt.** Compound 202 was also prepared by Scheme 2, using the following protocol. The corresponding HCl salt was prepared from Compound 202 following the protocol set forth in Example 1, step B.

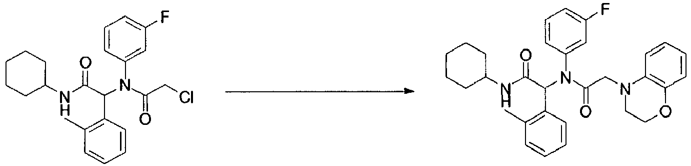

To a solution of Compound 118 (1.3 g, 3.1 mmol) in toluene (50 ml) was added Et₃N (1.9 g, 18.7 mmol) and 3,4-Dihydro-2H-benzo[1,4]oxazine (422mg, 3.1mmol). The mixture was refluxed overnight under N₂ atmosphere. The resulting mixture was concentrated and DCM (20 ml) was added. The organic liquid was washed with water, brine, dried over Na₂SO₄, filtered and the solvent was concentrated in vacuo. The residue was purified by prep-HPLC to give desired product as a white solid (70mg, 4.37% yield). ¹H NMR (400 MHz, DMSO-d6): δ 7.95 (s, 1H), 7.87 (dr, 1H), 7.14-7.12 (d, 1H, J=6.8), 7.06-6.99 (m, 2H), 6.88-6.85 (m, 1H), 6.72-6.63 (m, 4H), 6.52-6.48 (m, 1H), 6.38-6.36 (d, 1H, J=8), 6.19 (s, 1H), 4.12-4.09 (m, 2H), 3.92-3.87 (d, 1H, J=17.2), 3.67-3.59 (m, 2H), 3.36-3.34 (m, 2H), 2.36 (s, 3H), 1.73-1.51 (m, 5H), 1.27-0.93 (m, 5H); LC-MS: purity > 95%, MS: 516.3 (M⁺+1).
HCl Salt:
¹H NMR (300 MHz, DMSO-d6): δ7.93(br, 2H), 7.13-6.18(m, 11H), 4.09(m, 1H), 3.86-3.55(m, 3H), 3.22(m, 2H), 2.63(m, 2H), 2.36(s, 3H), 1.72-1.50(m, 7H), 1.32-0.89(m, 5H); MS: 514.3 (M+1)⁺.

The following compounds were also synthesized via Scheme 2 following the general procedure set forth above for Compound 202. The corresponding HCl salt was synthesized following the general procedure set forth in Example 1, step B.

### Compound 242

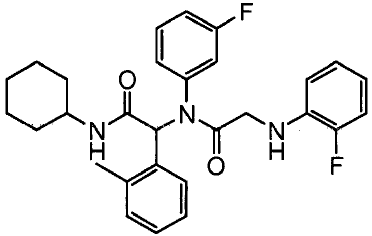

¹H NMR (400 MHz, DMSO-d6): δ 8.01-7.99 (m, 1H), 7.12-6.84 (m, 7H), 6.74-6.71 (m, 1H), 6.57-6.55 (m, 1H), 6.45-6.35 (m, 1H), 6.23 (s, 1H), 5.39-5.37 (m, 1H), 3.77-3.70 (m, 1H), 3.64-3.60 (m, 1H), 3.46-3.34 (m, 1H), 2.37 (s, 3H), 1.75-1.50 (m, 5H), 1.29-0.85 (m, 5H); MS: 492.2 (M+1)⁺.

### Compound 265

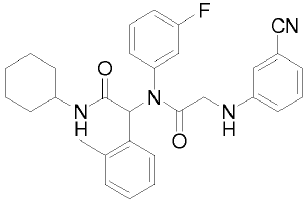

¹H NMR (300MHz, CDCl3): δ 7.19-7.13 (m, 4H), 7.00-6.91 (m, 3H), 6.76 (d, 1H, J=5.7 MHz), 6.69-6.66 (m, 1H), 6.55 (s, 1H), 6.39 (s, 1H), 5.25 (d, 1H, J=5.7 MHz), 5.03 (m, 1H), 3.86 (m, 1H), 3.56 (d, 2H, J=3.3 MHz), 2.40 (s, 3H), 1.97-1.87 (m, 2H), 1.68-1.55 (m, 3H), 1.36-1.10 (m, 5H); MS: 499.1 (M+1)⁺.

### Compound 278

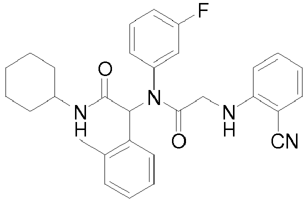

¹H NMR (400MHz, CDCl3): δ 7.19-7.13 (m, 4H), 7.00-6.89 (m, 3H), 6.76 (d, 1H, J=6.8 MHz), 6.69-6.66 (m, 1H), 6.55 (s, 1H), 6.39 (s, 1H), 5.25 (d, 1H, J=7.6 MHz), 5.03-5.02 (m, 1H), 3.87 (m, 1H), 3.58-3.57 (d, 2H, J=4.4 MHz), 2.40 (s, 3H), 1.99-1.80 (m, 2H), 1.69-1.55 (m, 2H), 1.36-1.02 (m, 6H); MS: 499.1 (M+1)⁺.
**Example 5. Preparation of Compound 161.** Compound 161 was prepared according to Scheme 2 using the following protocol.

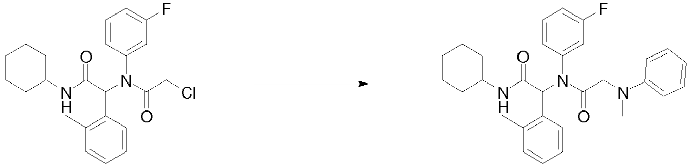

To a solution of Compound 118 (200 mg, 0.48 mmol) in DMF (4 ml) was added Et₃N (0.4 ml, 2.87 mmol) and Methyl-phenyl-amine (103 mg, 0.96 mmol). The mixture was stirred overnight at room temperature. Water (20 ml) was added and was then extracted with DCM (3x10 ml). The combined organic layer was washed with water, brine, dried over Na₂SO₄, filtered and concentrated in vacuo. The residue was purified by prep-HPLC to give desired product as a white solid (10.7 mg, 4.58% yield). ¹H NMR (300 MHz, DMSO-d6): δ 7.94-7.92 (d, 1H, J = 6.6), 7.15-6.51 (m, 12H), 6.18 (s, 1H), 3.97-3.91 (d, 1H, J = 17.1), 3.71-3.58 (m, 2H), 2.89 (s, 3H), 2.36 (s, 3H), 1.73-1.50 (m, 5H), 1.26-0.99 (m, 5H); MS: 488.2 (M+1)⁺.

The following compounds were also synthesized via Scheme 2 following the general procedure set forth above for Compound 161. The corresponding HCl salt was synthesized following the general procedure set forth in Example 1, step B.

### Compound 182

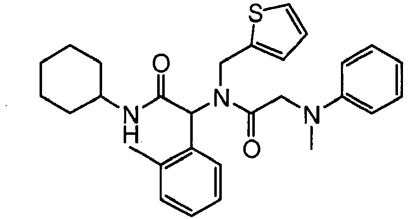

¹H NMR (300 MHz, DMSO-d6): δ 8.16-7.94 (m, 1H), 7.30-6.53 (m, 10H), 6.40-6.38 (d, 1H, J=10.8), 6.24-5.66 (m, 1H), 4.99-4.70 (m, 1H), 4.36-4.06 (m, 2H), 3.61-3.56 (m, 1H), 3.00-2.92 (m, 3H), 2.21-1.99 (m, 3H), 1.76-1.52 (m, 5H), 1.23-0.85 (m, 5H); MS: 490.2 (M+1)⁺.

### Compound 183

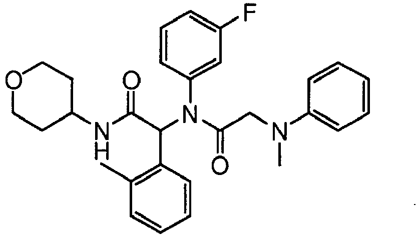

¹H NMR (300 MHz, DMSO-d6): δ 8.09-8.07 (d, 1H, J=8), 7.82 (br, 1H), 7.15-6.98 (m, 6H), 6.89-6.84 (m, 1H), 6.72-6.51 (m, 5H), 6.19 (s, 1H), 3.98-3.92 (d, 1H, J=22.8), 3.81-3.66 (m, 4H), 2.89 (s, 3H), 2.36 (s, 3H), 1.70-1.66 (d, 2H, J=14.8), 1.38-1.12 (m, 4H); MS: 490.2 (M+1)⁺. **Example 6. Preparation of Compound 189.** Compound 189 was synthesized according to Scheme 3 using the following protocol

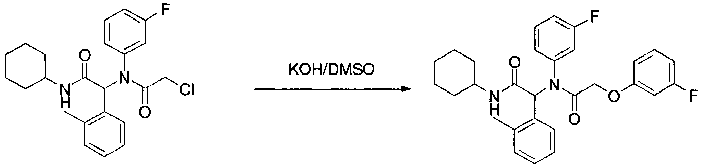

To a suspension of KOH (105 mg, 1.87 mmol) in dry DMSO (5 ml) was added 3-Fluorophenol (106 mg, 0.94 mmol) and Compound 118 (260 mg, 0.62 mmol). The reaction mixture was stirred at room temperature for 3 hours. The resulting mixture was quenched by H₂O (15 ml) and then extracted with EtOAc (2x10 ml). The combined organic layer was washed with NaHCO₃ solution, brine, dried over Na₂SO₄, filtered and the solvent was evaporated under vacuum. The residue was purified via silica gel chromatography to give the desired product as a white solid (122.5 mg, 40% yield). ¹H NMR (300 MHz, DMSO-d6): δ 8.03-8.00 (d, 1H, J=6.9), 7.31-6.62 (m, 11H), 6.21 (s, 1H), 4.69-4.23 (m, 2H), 3.62-3.61 (m, 1H), 2.36 (s, 3H), 1.76-1.56 (m, 5H), 1.29-1.00 (m, 5H); MS: 493.2 (M+1)⁺.

The following compounds were also synthesized via Scheme 2 following the general procedure set forth above for Compound 189. The corresponding HCl salt was synthesized following the general procedure set forth in Example 1, step B.

### Compound 136

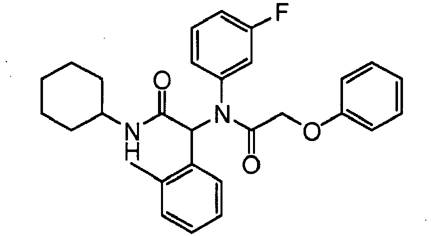

¹H NMR (300 MHz, DMSO-d6): 68.02-8.00(d, 1H, J=6.9), 7.27-6.73(m, 11H), 6.22(s, 1H), 4.63-4.20(m, 2H), 3.64-3.61(m, 1H), 2.37(s, 3H), 1.75-1.55 (m, 5H), 1.29-1.00 (m, 5H); MS: 475.2(M+1)⁺.

### Compound 194

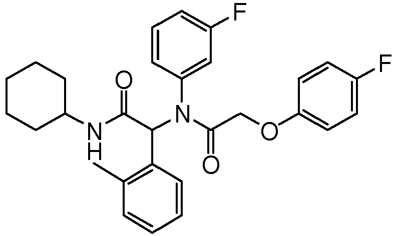

¹H NMR (300 MHz, DMSO-d6): δ 8.01-7.99 (d, 1H, J=7.8), 7.10-6.71 (m, 10H), 6.19 (s, 1H), 4.61-4.17 (m, 2H), 3.62-3.59 (m, 1H), 2.34 (s, 3H), 1.75-1.49 (m, 5H), 1.28-1.00 (m, 5H); MS: 493.1 (M+1)⁺.

### Compound 196

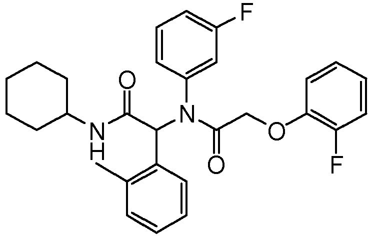

¹H NMR (300 MHz, DMSO-d6): δ 8.02-8.01 (d, 1H, J = 7.2), 7.21-6.73 (m, 10H), 6.21 (s, 1H), 4.74-4.34 (m, 2H), 3.62-3.60 (m, 1H), 2.36 (s, 3H), 1.75-1.49 (m, 5H), 1.25-0.95 (m, 5H); MS: 493.2 (M+1)⁺.

### Compound 197 and its HCl Salt

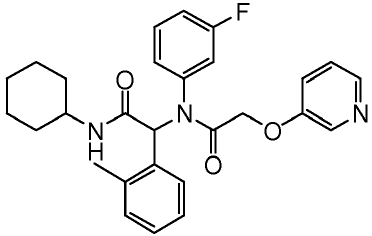

¹H NMR (300 MHz, DMSO-d6): δ 8.17-8.14 (m, 2H), 8.02-8.00 (m, 1H), 7.31-6.74 (m, 8H), 6.21 (s, 1H), 4.75-4.31 (m, 2H), 3.63-3.61 (m, 1H), 2.36 (s, 3H), 1.75-1.50 (m, 5H), 1.28-0.96 (m, 5H); MS: 476.2 (M+1)⁺.
HCl Salt:
¹H NMR (300 MHz, DMSO-d6): δ8.50(m, 2H), 8.06-7.77(m, 3H), 7.15-6.74(m, 6H), 6.17(s, 1H), 4.96-4.50(m, 2H), 3.62(m, 1H), 2.36(s, 3H), 1.72-1.50(m, 5H), 1.34-1.00(m, 5H); MS: 476.2 (M+1)⁺.

### Compound 198

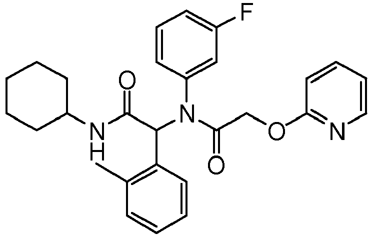

¹H NMR (300 MHz, DMSO-d6): δ 8.10-8.08 (m, 1H), 7.99-7.97 (m, 1H), 7.73-7.67 (m, 1H), 7.15-6.70 (m, 8H), 6.21(s, 1H), 4.73-4.43 (m, 2H), 3.63-3.61 (m, 1H), 2.39 (s, 3H), 1.75-1.50 (m, 5H), 1.28-0.96 (m, 5H); MS: 476.2 (M+1)⁺.

### Compound 199

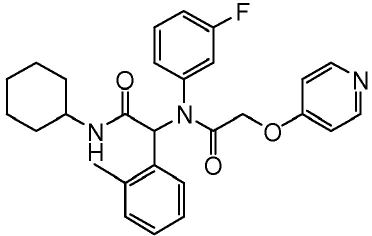

¹H NMR (300 MHz, DMSO-d6): δ 8.04-8.02 (m, 1H), 7.47-7.43 (m, 1H), 7.14-7.67 (m, 6H), 6.18 (s, 1H), 6.04-6.01 (m, 2H), 4.62-4.35 (m, 2H), 3.62-3.61 (m, 1H), 2.38 (s, 3H), 1.73-1.50 (m, 5H), 1.28-0.96 (m, 5H); MS: 476.2 (M+1)⁺.

### Compound 260

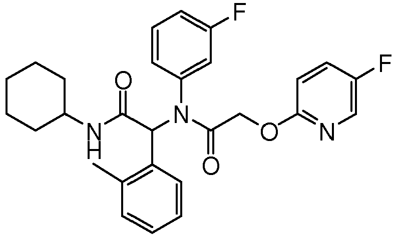

¹H NMR (300 MHz, DMSO-d6): δ 8.08-8.07 (d, 1H,J=2.4), 8.01 (s, 1H), 7.87 (br, 1H), 7.72-7.67 (m, 1H), 7.15-6.69 (m, 8H), 6.21 (s, 1H), 4.71-4.44 (m, 2H), 3.61-3.59 (m, 1H), 2.36 (s, 3H), 2.28 (s, 4H), 1.74-1.51 (m, 5H), 1.28-0.94 (m, 5H); MS: 494.1 (M+1)⁺.
**Example 7. Preparation of Compound 331.** Compound 331 was prepared using the following protocol. The 2-[(2-Chloro-acetyl)-(3-fluoro-phenyl)-amino]-N-(4,4-difluoro-cyclohexyl)-2-o-tolyl-acetamide used in the protocol set forth below was prepared according to Scheme 4. That chloroacetyl compound was converted to Compound 331 was prepared according to Scheme 3. 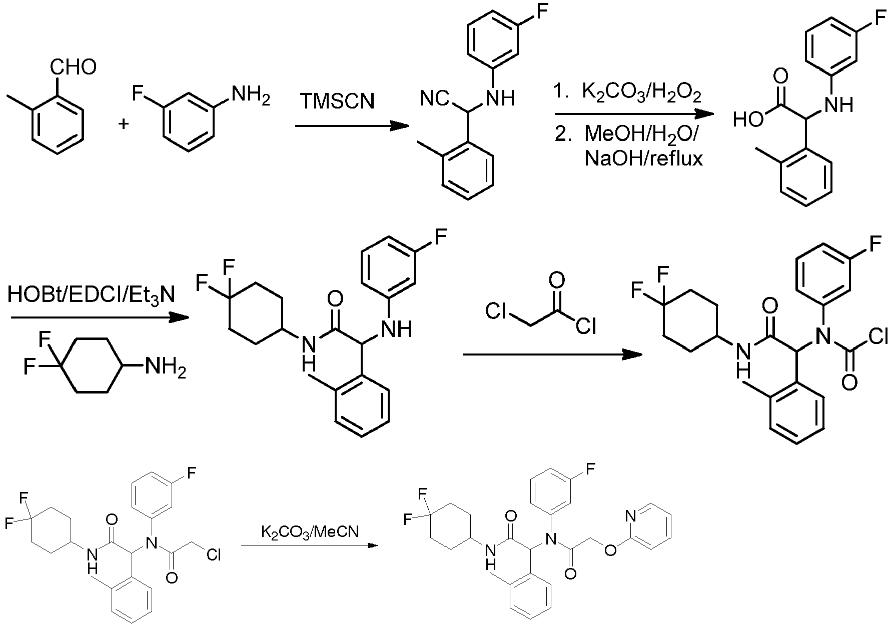
***Step A: (3-Fluoro-phenylamino)-o-tolyl-acetonitrile.*** A mixture of 2-Methyl-benzaldehyde (0.6 g, 5 mmol) and 3-Fluoro-phenylamine (0.56 g, 5 mmol) was stirred overnight at room temperature followed by the addition of TMSCN (0.6 g, 6 mmol). The reaction mixture was stirred for another 8 hours. Et₂O (20 ml) was added and the solid was collected by filtration and dried in vacuo to give the (3-Fluoro-phenylamino)-o-tolyl-acetonitrile, which was used directly without further purification (0.9 g, 77% yield). ¹H NMR (300 MHz, CDCl₃): δ 7.70 (d, 1H, J = 6.9), 7.37-7.18 (m, 4H), 6.59-6.46 (m, 3H), 5.43 (d, 1H, J = 7.8), 3.95 (d, 1H, J = 7.8), 2.38 (s, 3H); MS: 214.1 (M-26)⁺.
***Step B: (3-Fluoro-phenylamino)-o-tolyl-acetic acid.*** To a mixture of (3-Fluoro-phenylamino)-o-tolyl-acetonitrile (0.48 g, 2 mmol) and K₂CO₃ (0.14 g, 1 mol) in DMSO (2.5 ml) was added H₂O₂ (30%, 0.34 g) at 0°C. The mixture was warmed to room temperature and stirred for 2 hours. The precipitate was collected by filtration, washed with cold water and dried in vacuo. The residue was dissolved in a mixture of MeOH/H₂O (4:1, 5 ml) and NaOH (0.24 g, 6 mmol) was then added. This reaction mixture was refluxed for 5 hour and concentrated. Water (30 ml) was added. The resulting mixture was extracted with EtOAc (25ml) and the water phase adjust to pH=4 with conc. HCl, extracted with DCM (3x20 ml). The combined DCM layer was washed with brine, dried over Na₂SO₄, filtered and the solvent was evaporated in vacuo to give the (3-Fluoro-phenylamino)-o-tolyl-acetic acid (0.4 g, 80% yield), which was used directly for the next step. ¹H NMR (300 MHz, CDCl₃): δ 7.40 (d, 1H, J = 7.2), 7.37-7.21 (m, 4H), 7.05 (m, 1H), 6.40-6.18 (m, 3H), 5.26 (s, 1H), 2.53 (s, 3H); MS: 214.1 (M-45)⁺.
***Step C: N-(4,4-Difluoro-cyclohexyl)-2-(3-fluoro-phenylamino)-2-o-tolyl-acetamide.*** To a solution of (3-Fluoro-phenylamino)-phenyl-acetic acid (259 mg, 1 mmol) in DCM (5 ml) was added HOBt (162 mg, 1.2 mmol), EDCI (240 mg, 1.2 mmol), Et₃N (0.5 ml) and 4,4-Difluoro-cyclohexylamine (170 mg, 1.52 mmol) at 0°C. The reaction mixture was heated to 40°C for 48 hours. After cooling to room temperature, 30 ml of water was added. The organic layer was separated and the water phase was extracted with DCM (3x10 ml). The combined organic layer was washed with NaHCO₃, brine, dried over Na₂SO₄, filtered and the solvent was evaporated in vacuo. The residue was washed with Et₂O to give the N-(4,4-Difluoro-cyclohexyl)-2-(3-fluoro-phenylamino)-2-o-tolyl-acetamide, which was used directly without further purification (280 mg, 68% yield).
***Step D: 2-[(2-Chloro-acetyl)-(3-fluoro-phenyl)-amino]-N-(4,4-difluoro-cyclohexyl)-2-o-tolyl-acet amide.*** To a mixture of N-(4,4-Difluoro-cyclohexyl)-2-(3-fluoro-phenylamino)-2-o-tolyl-acetamide (280 mg, 0.74 mmol)) in toluene (5 ml) was added chloro-acetyl chloride (100 mg, 0.9 mmol) dropwise at 0°C. The reaction mixture was heated to 100°C for 2 hours and then cooled to room temperature. 10 ml of ethyl acetate was added and the solvent was washed with NaHCO₃ solution, brine, dried over Na₂SO₄, filtered and the solvent was evaporated in vacuo. The residue was washed with Et₂O to give the 2-[(2-Chloro-acetyl)-(3-fluoro-phenyl)-amino]-N-(4,4-difluoro-cyclohexyl)-2-o-tolyl-acet amide (230 mg, 68% yield). ¹H NMR (400 MHz, CDCl₃): δ 7.18-7.11 (m, 3H), 6.94-6.88 (m, 2H), 6.75 (d, 1H), 6.32(s, 1H), 5.33 (d, 1H), 3.97(br, 1H), 3.86 (q, H), 2.39 (s, 3H), 2.09-1.79 (m, 6H), 1.56-1.39 (m, 2H); MS: 452.8 (M+1)⁺.
***Step E: Compound 331.*** A mixture of 2-[(2-Chloro-acetyl)-(3-fluoro-phenyl)-amino]-N-(4,4-difluoro-cyclohexyl)-2-o-tolyl acetamide (100 mg, 0.22 mmol), K₂CO₃ (90 mg, 0.66 mmol) and pyridin-2-ol (42 mg, 0.44 mol) in MeCN (5 ml) was heated to 40°C and stirred overnight. The resulting mixture was evaporated in vacuo. The residue was suspended in water (25 ml) and extracted with DCM (3x10 ml). The combined organic layer was washed with NaHCO₃ solution, brine, dried over Na₂SO₄, filtered and evaporated in vacuo. The crude product was purified by TLC (DCM/MeOH=20/1) to give the desired product (20 mg, 17% yield). ¹H-NMR(CDCl3, 400MHz),δ 8.08 (d, 1H), 7.56 (m, 1H), 7.26-6.82 (m, 9H), 6.32 (s, 1H), 5.50 (d, 1H), 4.6 (dd, 2H), 3.96 (br, 1H), 2.41 (s, 3H), 2.07-1.59 (m, 6 H), 1.51-1.25 (m, 2H); MS: 512.2 (M+1)⁺

The following compounds were also synthesized from the appropriate chloroacetyl compound e following the general procedure set forth above in step E.

### Compound 351

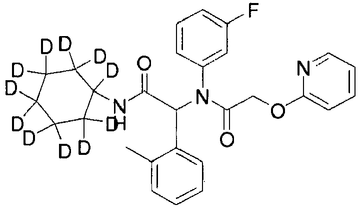

¹H NMR(400 MHz, CDCl₃),δ8.07 (dd, 1H, J=4.8, 12), 7.56 (td, 1H, J=6.8, 1.6), 7.18-7.10 (m, 3H), 6.93-6.83 (m, 5H), 5.39(s, 1H), 4.74 (d, 1H, J=14.8), 4.55 (d, 1H, J=15.2), 2.41 (s, 3H); MS: 487.3 (M+1)⁺.

### Compound 354

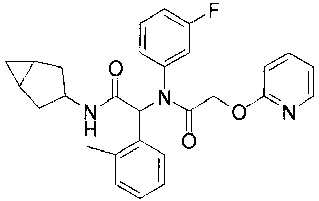

¹H NMR (400 MHz, DMSO-d6): δ 8.10-7.68 (m, 4H), 7.15-6.64 (m, 9H), 6.20 (s, 1H), 4.70 (d, 1H, J=14.4 MHz), 4.43 (d, 1H, J=15.2 MHz), 4.16 (m, 1H), 2.38 (s, 3H), 2.15-2.08 (m, 2H), 1.62-1.49 (m, 2H), 1.21-1.09 (m, 2H), 0.36-0.34 (m, 1H), 0.00--0.03 (m, 1H); MS: 474.2 (M+1) ⁺.

The following compounds were synthesized according to Scheme 4 (and steps A-D, above), using the appropriate R¹ amine and chloroacetyl derivative of R⁴.

### Compound 186

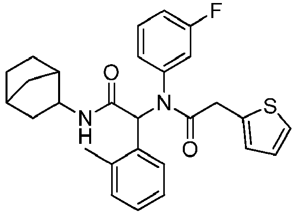

¹H NMR (300 MHz, DMSO-d6): δ 8.09 (m, 1H), 7.77-6.50 (m, 11H), 6.32 (s, 1H), 4.01-3.89 (m, 1H), 3.65-3.56 (m, 2H), 2.36 (s, 3H), 2.11-0.75 (m, 10H); MS: 477.2 (M+1)⁺.

### Compound 200

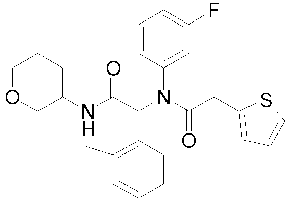

¹H NMR (400 MHz, DMSO-d6): δ 8.15 (s, 1H), 7.74 (s, 1H), 7.36-6.28 (m, 11H), 3.76-2.87 (m, 7H), 2.31 (s, 3H), 1.86-1.23 (m, 4H); MS: 467.1 (M+1)⁺.

### Compound 178

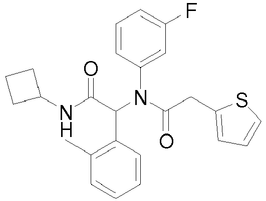

¹H NMR (400 MHz, DMSO-d6): δ 8.35 (s, 1H), 7.46(s, 1H), 7.36-7.34 (m, 1H), 7.12-6.44 (m, 9H), 4.25-4.23 (m, 1H), 3.69-3.52 (m, 2H), 2.35 (s, 3H), 2.19-2.12 (m, 2H), 1.92-1.88 (m, 1H), 1.71-1.57 (m, 3H); MS: 437.1 (M+1)⁺.

### Compound 159

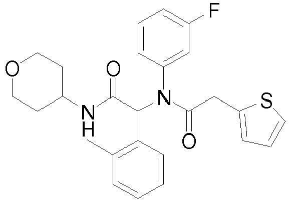

¹H NMR(300 MHz, DMSO-d6): δ 8.13 (d, 1H, J = 5.4), 7.70-6.50 (m, 11H), 6.25 (s, 1H), 3.84-3.49 (m, 5H), 3.32 (m, 2H), 2.34 (s, 3H), 1.74 (m, 2H), 1.43 (m, 1H), 1.23 (m, 1H); MS: 467.2 (M+1)⁺.

### Compound 211

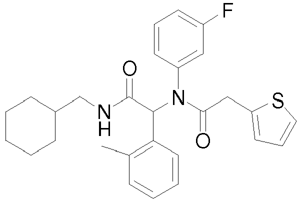

¹H NMR (300 MHz, DMSO-d6): δ 8.12 (s, 1H), 7.76-6.66 (m, 11H), 6.27 (s, 1H), 3.69-3.51 (m, 2H), 3.08-3.03 (m, 1H), 2.34 (s, 3H), 1.59-0.81 (m, 12H); MS: 479.2 (M+1)⁺.

### Compound 190

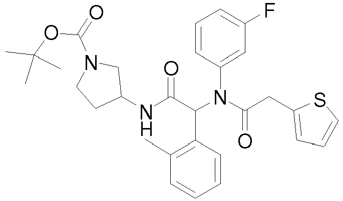

¹H NMR (300 MHz, CDCl₃): δ 7.16-6.72 (m, 9H), 6.37 (s, 1H), 5.59 (m, 1H), 4.51 (m, 1H), 3.66 (m, 3H), 3.34-3.18 (m, 3H), 2.34 (s, 3H), 2.08 (m, 1H), 1.72 (m, 1H), 1.43 (s, 9H); MS: 569.3 (M+18)⁺, 452.2 (M-100)⁺.
**Example 8. Preparation of Compound 341.** Compound 341 was prepared according to Scheme 5, using the following protocol 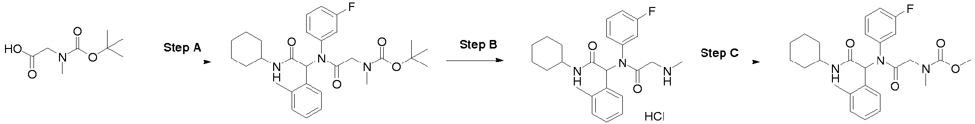
***Step A: {[(Cyclohexylcarbamoyl-o-tolyl-methyl)-(3-fluoro-phenyl)-carbamoyl]-methyl}-methyl-carbamic acid tert-butyl ester.*** The title compound was synthesized via Scheme 1, as described in Step A of Example 1. ¹H NMR (400 MHz, CDCl3): δ 7.16-7.07 (m, 3.5H), 6.91-6.76 (m, 3.5H), 5.49 (d, 0.5H), 5.29 (d, 0.5H), 4.05 (d, 0.5), 3.95-3.80 (br, 1H), 3.73 (d, 0.5 H), 3.56-3.44 (m, 1H), 2.90 (d, 3H), 0.29 (d, 3H), 1.97-1.89 (m, 2H), 1.71-1.57 (m, 4H), 1.44 (s, 9H), 1.37-1.32 (br, 2H), 1.16-1.01 (m, 4H); MS: 511.9 (M+1)⁺.
***Step B: N-Cyclohexyl-2-[(3-fluoro-phenyl)-(2-methylamino-acetyl)-amino]-2-o-tolyl-acetamide (hydrochloride).*** A mixture of {[(Cyclohexylcarbamoyl-o-tolyl-methyl)-(3-fluoro-phenyl)-carbamoyl]-methyl}-methyl-carbamic acid tert-butyl ester (150 mg, 0.29 mmol) in HCl/Et₂O (30% w/w, 5 ml) was stirred for 5 hours at room temperature. The resulting mixture was evaporated in vacuo to afford the desired product, which was used directly without further purification (135 mg, 100% yield).
***Step C: Compound 341.*** To a mixture of N-Cyclohexyl-2-[(3-fluoro-phenyl)-(2-methylamino-acetyl)-amino]-2-o-tolyl-acetamide (hydrochloride, 132 mg, 0.29 mmol) and Et₃N (85 mg, 0.6 mmol) in DCM (5 ml) was added methyl chloroformate (30 mg, 0.3 mmol) at 0°C. The reaction was stirred for 3 hours at the same temperature. 10 ml of water was added and the mixture was extracted with DCM (3x5 ml). The combined organic layer was washed with saturated NaHCO₃ solution, brine, dried over Na₂SO₄, filtered and the solvent was evaporated in vacuo. The residue was purified by TLC to give the pure product (30 mg, 22% yield). ¹H NMR (400MHz , CDCl3) δ 7.17-7.07 (m, 3H), 6.89-6.76 (m, 4H), 6.42 (s, 0.5H), 6.39 (s, 0.5H), 5.53 (d, 0.5H, J=7.6), 5.29 (d, 0.5H, J=8.4), 4.01-3.79 (3, 3H), 3.65-3.48 (m, 4H), 2.96 (s, 3H), 2.39 (s, 3H), 1.97-1.90 (br, 2H), 1.72-1.68 (br, 1H), 1.63-1.58 (br, 1H), 1.36-1.25 (br, 3H), 1.17-1.11 (m, 3H); MS: 470.2 (M+1)⁺

The following compounds were synthesized according to Scheme 5, following the above protocol.

### Compound 334

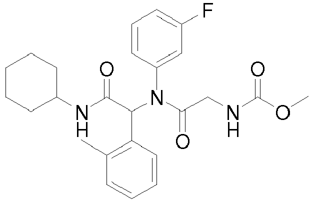

1H NMR (400 MHz, CDCl3), δ 7.16-6.74 (m, 6H), 6.34 (s, 1H), 5.54 (s, 1H), 5.54-5.26 (m, 1H), 3.88-3.64 (m, 6H), 2.38 (s, 3H), 1.98-1.62 (m, 4H), 1.42-0.98 (m, 6H); MS: 456.2 (M+1)⁺.

### Compound 352

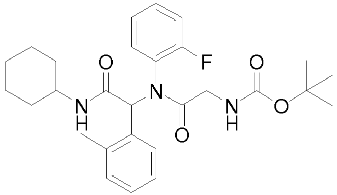

¹H NMR (400 MHz, DMSO-d6): δ 8.08 (s, 1H), 7.94-7.92 (d, 1H, J=6.8), 7.18-7.04 (m, 3H), 6.98-6.64 (m, 5H), 6.23 (s, 1H), 3.58-3.56 (m, 1H), 3.34-3.32 (m, 1H), 3.25-3.21 (m, 1H), 2.29 (s, 3H), 1.75-1.48 (m, 5H), 1.32 (s, 9H), 1.28-0.89 (m, 5H); MS: 498.2 (M+1)⁺.

### Compound 357

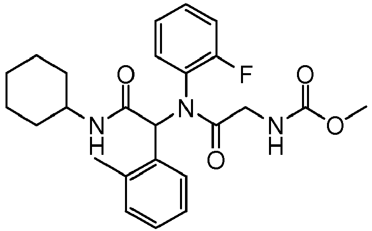

¹H NMR (400 MHz, DMSO-d6): δ 8.15-8.11 (m, 1H), 7.99-7.97 (d, 1H, J=7.6), 7.29-6.90 (m, 6H), 6.79-6.75 (m, 1H), 6.68-6.66 (d, 1H, J = 7.2), 6.27 (s, 1H), 3.61-3.40 (m, 5H), 3.30 (s, 1H), 2.34 (s, 3H), 1.78-1.52 (m, 5H), 1.28-0.91 (m, 5H); MS: 456.1 (M+1)⁺.

### Compound 353

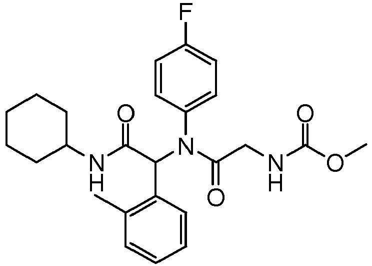

¹H NMR (400 MHz, DMSO-d6): δ 7.94-7.92 (d, 2H, J = 7.2), 7.15-6.98 (m, 4H), 6.82-6.78 (m, 2H), 6.63-6.56 (m, 2H), 6.17 (s, 1H), 3.58-3.45 (m, 5H), 3.25-3.19 (m, 1H), 2.32 (s, 3H), 1.73-1.48 (m, 5H), 1.25-0.88 (m, 5H); MS: 456.2 (M+1)⁺.

### Compound 358

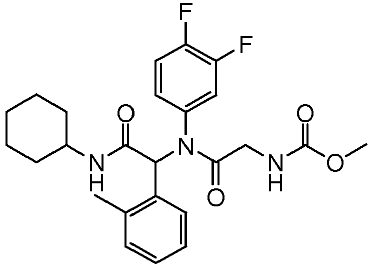

¹H NMR (400 MHz, DMSO-d6): δ 8.21-8.19 (m, 1H), 7.01-7.99 (d, 1H, J=7.6), 7.30-6.97 (m, 5H), 6.84-6.80 (m, 1H), 6.66-6.64 (d, 1H, J=7.2), 6.26 (s, 1H), 3.62-3.39 (m, 5H), 3.34-3.32 (m, 1H), 2.36 (s, 3H), 1.77-1.52 (m, 5H), 1.28-0.92 (m, 5H); MS: 474.0 (M+1)⁺.

### Compound 369

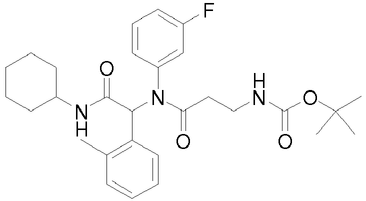

¹H NMR (400 MHz, DMSO-d6): δ7.80-7.72 (br, 1.7H), 7.10-7.08 (d, 2H), 7.02-6.94 (m, 2H), 6.84 (t, J = 8, 1H), 6.69 (d, J = 7.6, 1H), 6.61 (s, 1H), 6.22 (s, 1H), 3.62 (m, 1H), 3.13-2.50 (m, 2H), 2.34 (s, 3H), 2.27-2.23 (m, 1.5H), 2.04-2.00 (br, 1.3H), 1.78-1.52 (m, 5.5H), 1.52-1.11 (m, 12H); MS: 512.1 (M+1)⁺.

### Compound 374

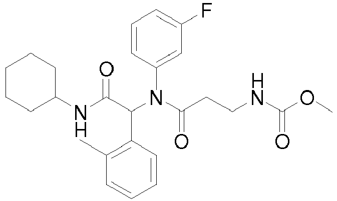

¹H NMR (400 MHz, DMSO-d6): δ7.80 (br, 1H), 7.72 (br, 0.8H), 7.09-7.06 (d, 2H), 7.02-6.94 (m, 3H), 6.84 (t, 1H), 6.70 (d, 1H), 6.22 (s, 1H), 3.63 (m, 1H), 3.46 (s, 3H), 3.20-3.08 (m, 2H), 2.34 (s,3H), 2.30-2.24 (m, 1H), 2.06-2.01 (m, 1H), 1.77-1.52 (m, 6H), 1.29-1.23 (br, 1H), 1.19-0.94 (m, 3H); MS: 470.1 (M+1)⁺.

### Compound 372

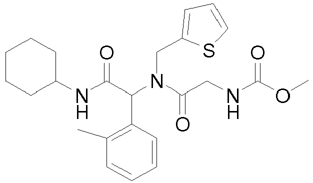

¹H NMR (300 MHz, DMSO-d6): δ 8.16-8.02 (m, 1H), 7.36-7.08 (m, 6H), 6.81-6.62 (m, 1.5H), 6.30 (s, 0.5H), 5.87 (s, 0.5H), 5.62 (s, 0.5H), 4.96-4.85 (m, 1H), 4.72 (d, J = 13.2, 0.5H), 4.44 (d, J = 13.2, 0.5H), 4.09-4.03 (m, 1H), 3.84-3.80 (m, 1H), 3.69-3.58 (m, 4H), 2.24 (s, 1.5H), 2.05 (s, 1.5H), 1.82-1.57 (m, 5H), 1.37-1.00 (m, 6H); MS: 458.0 (M+1)⁺.

### Compound 306

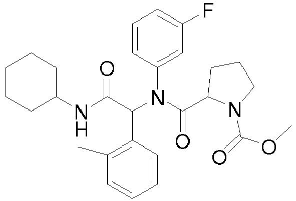

¹H NMR (400 MHz, DMSO-d6): δ 8.02-7.94 (m, 1H), 7.79-7.32 (m, 1H), 7.39-6.48 (m, 7H), 6.24 (s, 1H), 4.02 (m, 1H), 3.61-3.58 (m, 4H), 3.40-3.30 (m, 2H), 2.37 (s, 3H), 1.79-1.52 (m, 7H), 1.29-1.06 (m, 7H); MS: 496.1 (M+1)⁺.
**Example 9. Preparation of Compounds 225, 226, 236 and 241.** The title compounds were prepared according to the following Scheme 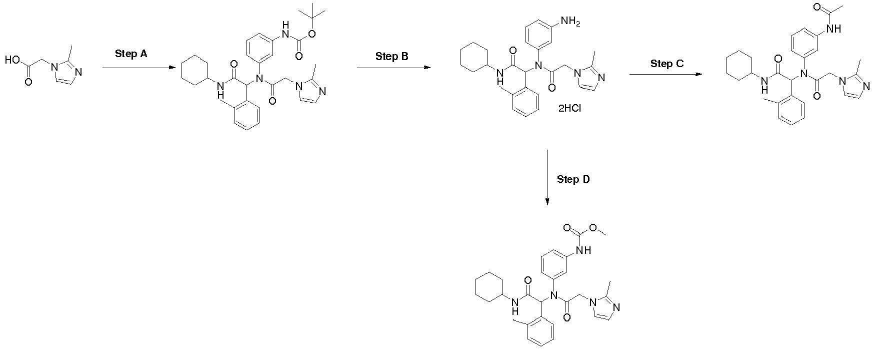
***Step A: Compound 224.*** Compound 224 was synthesized according to Scheme 1 and following the protocol set forth in Example 1, Step A. ¹H-NMR (300MHz , DMSO-d6), δ 9.48-9.25 (m, 1H), 7.99 (m, 1H), 7.53-7.30 (m, 4H), 7.08-6.47 (m, 5H), 6.10 (s, 1H), 4.98-4.62 (m, 2H), 3.59 (m, 1H), 2.45 (s, 3H), 2.36 (s, 3H), 1.73-1.46 (m, 14H), 1.25-1.22 (m, 5H); MS: 560.3 (M+1)⁺. ***Step B: Compound 226.*** Compound 226 was prepared following the protocol set forth in Example 8, step B. ¹H-NMR (300MHz , DMSO-d6), δ 14.43 (m, 1H), 7.98 (s, 1H), 7.72-7.53 (m, 2H), 7.23-6.71 (m, 6H), 6.12 (s, 1H), 5.00-4.66 (m, 2H), 3.59 (m, 1H), 2.47 (s, 3H), 2.37 (s, 3H), 1.72-1.50 (m, 4H), 1.24-1.23 (m, 6H); MS: 460.3 (M+1)+.
***Step C: Compound 236.*** To a mixture of the HCl salt of Compound 226 in DCM (5 ml) was added acetyl chloride (20 mg, 0.24 mmol) at 0°C. The reaction was stirred for 3 hours and the resulting mixture was washed with water, brine, dried over Na₂SO₄, filtered and the solvent was evaporated in vacuo. The residue was purified by TLC (DCM/MeOH=15/1) to give pure product (30 mg, 31% yield). ¹H NMR (400 MHz, MeOD-d4): δ 7.95 (s, 1H), 7.57-6.69 (m, 10H), 6.22 (s, 1H), 4.58-4.42 (m, 2H), 3.65-3.61 (m, 1H), 2.34 (s, 3H), 2.15 (s, 3H), 2.02-1.95 (m, 3H), 1.79-1.49 (m, 5H), 1.28-0.95 (m, 5H); MS: 502.3 (M+1)⁺.
***Step D: Compound 241.*** Compound 241 was synthesized following the protocol set forth in Example 8, step C. ¹H NMR (400 MHz, MeOD-d4): δ 8.04 (m, 1H), 7.62-6.45 (m, 10H), 6.34 (s, 1H), 4.76-4.61 (m, 2H), 3.76-3.73 (m, 4H), 2.46 (s, 3H), 2.33 (s, 3H), 1.91-1.63 (m, 5H), 1.40-1.07 (m, 5H); MS: 518.3 (M+1)⁺.
**Example 10. Preparation of Compound 328.** Compound 328 was prepared according to the following scheme 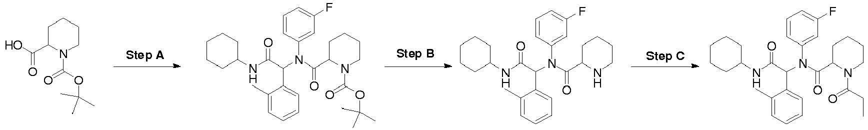
***Step A: (SR, RS)-2-[(Cyclohexylcarbamoyl-o-tolyl-methyl)-(3-fluoro-phenyl)-carbamoyl]-piperidine-1-carboxylic acid tert-butyl ester.*** Step A was carried out following Scheme 1 and the protocol set forth in Example 1, Step A and yielded two pairs of enantiomers separated via chromatography.
   (*SR*, *RS*)-2-[(Cyclohexylcarbamoyl-o-tolyl-methyl)-(3-fluoro-phenyl)-carbamoyl]-piperidine-1-carboxylic acid tert-butyl ester. (PE/EtOAc=5/1; Rf1 = 0.35). ¹H NMR (400 MHz, DMSO-d6): δ 8.02 (br, 1H), 7.82 (d, 1H), 7.10-6.82 (m, 8H), 4.45-4.45 (q, 1H), 3.78 (br, 0.5H), 3.635 (br, 1.5H), 3.45 (br, 0.5H), 2.30 (s, 1H), 1.75-1.42 (m, 7H), 1.42-1.02 (m, 18H) ; MS: 552.1 (M+1)⁺. (*RS, RS*)-2-[(Cyclohexylcarbamoyl-o-tolyl-methyl)-(3-fluoro-phenyl)-carbamoyl]-piperidine-1-carboxylic acid tert-butyl ester (PE/EtOAc=5/1; Rf1 = 0.3). ¹H NMR (400 MHz, DMSO-d6): δ 7.92-7.52 (m, 2H), 7.45-6.59 (m, 6H), 6.54-6.19 (m, 2H), 4.37-4.45 (m, 1H), 3.78-3.61 (m, 2H), 3.29-3.25 (m, 1H), 2.34 (s, 3H), 3.175-1.51 (m, 7H), 1.39-0.51 (m, 18H); MS: 552.1 (M+1)⁺.
***Step B1: (SR, RS)Piperidine-2-carboxylic acid (cyclohexylcarbamoyl-o-tolyl-methyl)-(3-fluoro-phenyl)-amide (hydrochloride).*** The title compound was synthesized from (*SR, RS*)-2-[(Cyclohexylcarbamoyl-o-tolyl-methyl)-(3-fluoro-phenyl)-carbamoyl]-piperidine-1-carboxylic acid tert-butyl ester via the protocol set forth in Example 8, step B. ¹H NMR (400 MHz, DMSO-d6): δ 9.12 (br, 1H), 8.11 (q, 1H), 7.75 (d, 1H), 7.34 (m, 0.4H), 7.16 (m, 0.4H), 7.07-6.73 (m, 6H), 6.28 (d, 1H), 6.16 (br, 2H), 3.64 (d, 1H), 3.15 (d, 1H), 1.78 (br, 1H), 2.35 (d, 3H), 1.75-1.56 (m, 9H), 1.46-1.05 (m, 7H); MS: 452.1 (M+1)⁺.
***Step B2: (RS, RS)Piperidine-2-carboxylic acid (cyclohexylcarbamoyl-o-tolyl-methyl)-(3-fluoro-phenyl)-amide (hydrochloride).*** The title compound was synthesized from (*RS, RS*)-2-[(Cyclohexylcarbamoyl-o-tolyl-methyl)-(3-fluoro-phenyl)-carbamoyl]-piperidine-1-carboxylic acid tert-butyl ester via the protocol set forth in Example 8, step B also via the protocol set forth in Example 8, step B. ¹H NMR (400 MHz, DMSO-d6): δ 8.48 (br, 1H), 8.06 (br, 1H), 7.83 (br, 1H), 7.18 (br, 1H), 7.09-7.07 (br, 2.66H), 6.86 (t, 1H), 6.61 (d, 1H), 6.16 (br, 2H), 3.63 (br, 1H), 3.54 (d, 1H), 3.08 (d, 1H), 2.73 (br, 1H), 2.37 (s, 3H), 1.84-1.43 (m, 9H), 1.28-0.90 (m, 6H); MS: 452.1 (M+1)⁺.
***Step C: Compound 328.*** To *(SR, RS)*-piperidine-2-carboxylic acid (cyclohexylcarbamoyl-o-tolyl-methyl)-(3-fluoro-phenyl)-amide (200 mg, 0.41 mmol) in DCM (10 ml) was added propionyl chloride (50 mg, 0.53 mmol) at 0°C. The reaction was stirred for 3 hours at the same temperature. The resulting mixture was washed with water, brine, dried over Na₂SO₄, filtered and the solvent was evaporated in vacuo. The residue was purified by TLC (DCM/MeOH=15/1) to give pure product (60 mg, 29% yield); ¹H NMR (400 MHz, DMSO-d6): δ 8.03-7.77 (m, 2H), 7.26-6.72 (m, 7H), 6.26-6.23 (d, 1H, *J*=13.6 MHz), 4.86-4.79 (m, 1H), 3.68-3.53 (m, 3H), 2.41-2.27 (m, 5H), 1.75-0.93 (m, 19H); MS: 508.2 (M+1)⁺.

The following compounds were also synthesized according to the Scheme set forth in this Example.

### Compound 293 (from (SR, RS)-2-[(Cyclohexylcarbamoyl-o-tolyl-methyl)-(3-fluoro-phenyl)-carbamoyl]-piperidine-1-carboxylic acid tert-butyl ester)

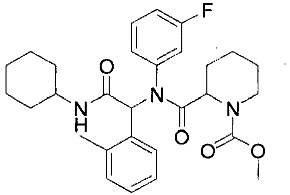

¹H NMR (400 MHz, DMSO-d6): δ 8.05-7.79 (m, 2H), 7.29-6.30 (m, 8H), 4.60-4.53 (m, 1H), 3.72-3.46 (m, 6H), 2.29 (s, 3H), 1.75-0.99 (m, 16H); MS: 510.1 (M+1)⁺.
**Example 11: Preparation of Stereospecific Compounds of Formula A where R⁴ is an Optionally Substituted Piperidin-2-yl.** Compounds of Formula A where R⁴ is optionally substituted piperidin-2-yl were prepared according to the following scheme exemplified for specific compounds. 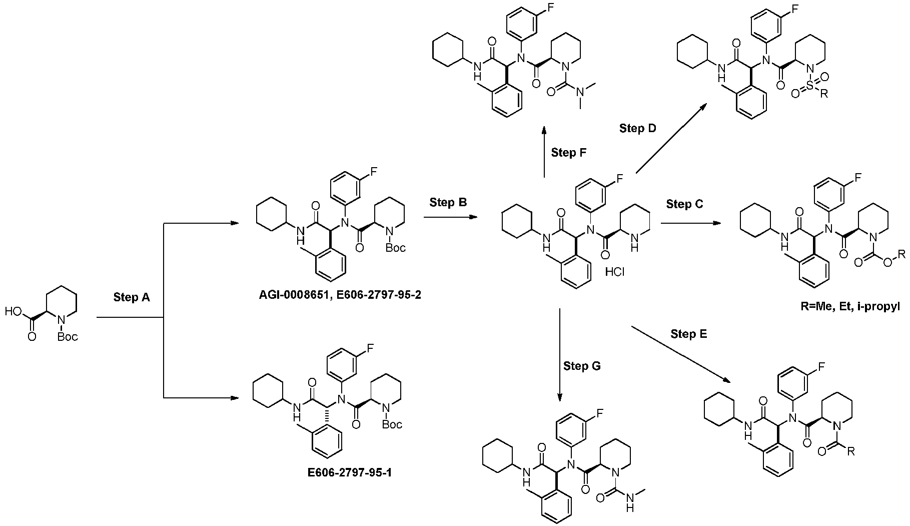
***Step A: Compound 332.*** Step A was carried out according to Scheme 1 using the protocol set forth in Example 1, Step A and both Compound 332 and its isomer (*R*, *R*)-2-[(Cyclohexylcarbamoyl-o-tolyl-methyl)-(3-fluoro-phenyl)-carbamoyl]-piperidine-1-carboxylic acid tert-butyl ester. These two isomers were separated via chromatography (PE/EtOAc=5/1; Rf1=0.35, Rf2=0.3). Compound 332 was the isomer with higher polarity. ¹H NMR (300 MHz, DMSO-d6): δ7.92-7.78 (m, 2H), 7.28-6.08 (m, 8H), 6.21 (s, 1H), 4.66-4.50 (m, 1H), 3.75-3.56 (m, 2H), 2.38-2.29 (m, 3H), 1.75-1.51 (m, 9H), 1.39 (m, 9H), 1.31-0.94 (m, 9H); MS: 552.3 (M+1)⁺.
***Step B: (S,R)-Piperidine-2-carboxylic acid (cyclohexylcarbamoyl-o-tolyl-methyl)-(3-fluoro-phenyl)-amide (hydrochloride) (Compound 337).*** The title compound was synthesized via the general protocol set forth in Example 8, step B. ¹H NMR (300 MHz, DMSO-d6): δ 8.08 (s, 1H), 7.85-7.82 (br, 1H), 7.20-6.60 (m, 5H), 6.23-6.21 (br, 1H), 6.14 (s,1H), 3.62-3.60 (m, 1H), 3.45-3.42 (m, 1H), 3.08-3.05 (m, 1H), 2.37 (s, 3H), 1.83-1.42 (m, 9H), 1.31-0.95 (m, 7H); MS: 452.2 (M+1)⁺.
***Step C: (S, R)-2-[(Cyclohexylcarbamoyl-o-tolyl-methyl)-(3-fluoro-phenyl)-carbamoyl]-piperidine-1-carboxylic acid methyl ester (R*=*methyl).*** The title compound was synthesized via the general protocol set forth in Example 8, step C.

### Step D: Compound 346

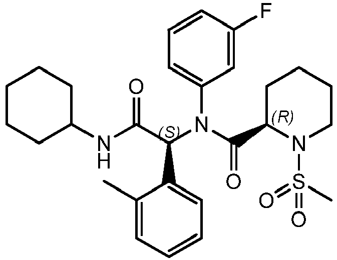

To Compound 337 (hydrochloride; 150 mg, 0.31 mmol) in DCM (5 ml) was added methanesulfonyl chloride (45 mg, 0.4 mmol) at 0°C. The reaction mixture was stirred for 3 hours. The resulting mixture was washed with brine, dried over Na₂SO₄, filtered and the solvent was evaporated in vacuo. The residue was purified by TLC (DCM/MeOH=20/1) to give the pure product (80 mg, 48% yield). ¹H NMR (300 MHz, DMSO-d6): δ 7.96-7.78 (m, 2H), 7.20-6.20 (m, 7H), 6.11 (s, 1H), 4.30-4.24 (m, 1H), 3.82-3.77 (m, 1H), 3.59-3.54 (m, 1H), 3.47-3.44 (m, 1H), 2.87 (s, 3H), 2.29 (s, 3H), 1.84-1.51 (m, 8H), 1.42-0.95 (m, 9H); MS: 530.2 (M+1)⁺.

### Step E: Compound 347.

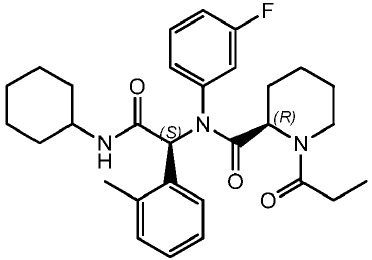

Compound 347 was synthesized from Compound 337 via the protocol set forth in Example 10, step C. ¹H NMR (300 MHz, DMSO-d6): δ 8.03 (s, 1H), 7.85-7.76 (m, 1H), 7.30-6.72 (m, 6H), 6.35-6.34 (br, 1H), 6.29(s, 1H), 5.13-5.04 (m, 1H), 4.47-4.27 (m, 1H), 3.69-3.59 (m, 2H), 2.45-2.40 (m, 3H), 2.67-1.61 (m, 11H), 1.37-1.02 (m, 8H), 0.91(s, 3H); MS: 508.2 (M+1)⁺.

### Step F: Compound 365

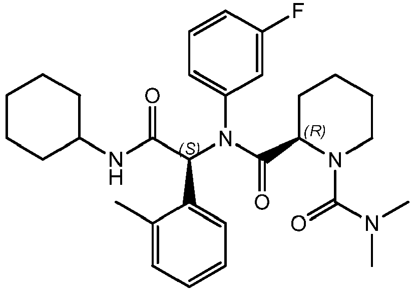

To Compound 337 (hydrochloride; 150 mg, 0.31 mmol) in DCM (10 ml) was added dimethylcarbamyl chloride (100 mg, 0.93 mmol) and Et₃N (95 mg, 0.93 mmol). The reaction was stirred over night at room temperature. The resulting mixture was washed with water, brine, dried over Na₂SO₄, filtered and the solvent was evaporated in vacuo. The residue was purified by TLC (PE/EtOAc=1/1) to give the desired product (100 mg, 62% yield). ¹H NMR (400 MHz, DMSO-d6): δ 7.99-7.98 (d, 1H, J=8), 7.63 (s, 1H), 7.14-6.99 (m, 4H), 6.84-6.80 (m, 1H), 6.56 (s, 1H), 6.26 (s, 1H), 3.77 (s, 1H),3.66-3.63 (m, 1H), 3.53-3.48 (m, 1H), 2.89 (s, 1H), 2.72 (s, 6H), 2.29 (s, 3H), 1.84-1.38 (m, 9H), 1.36-0.87 (m, 7H); MS:521.1 (M-1)⁻.

### Step G: Compound 364

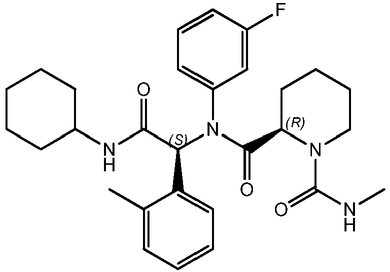

To a mixture of Et₃N (160 mg, 1.6 mmol) and Compound 337 (380 mg, 0.78 mmol) in THF (20 ml) was added a solution of triphosgene (230 mg, 0.78 mmol) in THF (20 ml). After stirring for 10 minutes, methylamine (1 M in THF, 1.3 ml, 1.3 mmol) was added in one portion. The reaction was stirred for 1.5 hours at room temperature. Water (50 ml) was added. The resulting mixture was extracted with EtOAc (2x20 ml). The combined organic layer was washed with brine, dried over Na₂SO₄, filtered and the solvent was evaporated in vacuo. The residue was purified via flash chromatography column eluted with DCM/MeOH (30/1) to give the desired product (40 mg, 10% yield); ¹H NMR (400 MHz, DMSO-d6): δ7.96-7.94 (d, 1H, J=7.6), 7.67(s, 1H), 7.17-6.83 (m, 4H), 6.59-6.57 (d, 1H, J=7.6), 6.34 (s, 1H), 6.19 (s, 1H), 4.51 (s, 1H),3.61-3.56 (m, 1H), 3.46-3.41 (m, 2H), 2.54 (s, 3H), 2.45(s, 3H), 1.76-1.44 (m, 9H), 1.30-0.84 (m, 8H); MS: 509.2 (M+1)⁺.

The following analogs were synthesized via the general procedures set forth in this Example

### Compound 343

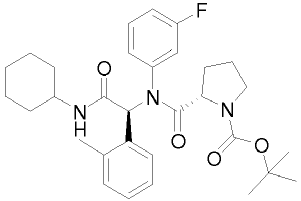

¹H NMR (400 MHz, DMSO-d6): δ 8.04-7.96 (m, 1H), 7.80-7.74 (m, 1H), 7.33-6.27 (m, 8H), 4.09-3.94 (m, 1H), 3.61 (m, 1H), 3.40-3.26 (m, 2H), 2.37 (d, 3H, *J*=6 MHz), 1.74-0.94 (m, 23H); MS: 538.3 (M+1)⁺.

### Compound 340

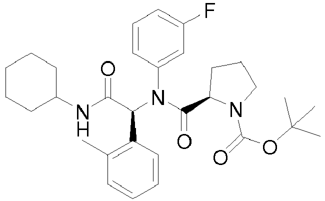

¹H NMR (400 MHz, DMSO-d6): δ8.03-7.94 (m, 2H), 7.15-6.69(m, 6H), 6.29-6.20(m, 2H), 3.93-3.92(t, 1H), 3.60-3.58(t, 1H), 3.37-3.25(m, 2H), 2.37-2.33(m, 3H), 2.08-0.95(m, 23H); MS: 538.3 (M+1)⁺.

### Compound 376

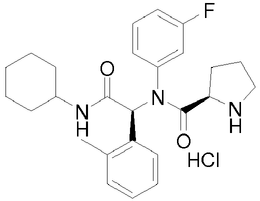

¹H NMR (400 MHz, DMSO-d6): δ 8.06-8.05 (d, J = 0.8, 1H), 7.85 (s, 1H), 7.16-6.82 (m, 8H), 6.66-6.12 (m, 2H), 3.62-3.58 (m, 2H), 3.33-3.29 (m, 1H), 3.10-2.81 (m, 1H), 2.45 (s, 3H), 1.77-1.52 (m, 8H), 1.29-0.47 (m, 6H); MS: 437.8 (M+1)⁺.

### Compound 338

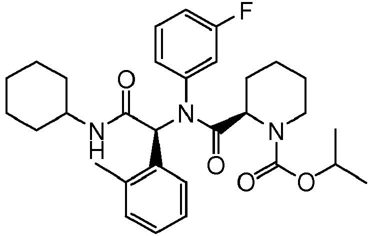

¹H NMR (300 MHz, DMSO-d6): δ 7.92-7.78 (m, 2H), 7.28-6.12 (m, 8H), 4.74-4.49 (m, 2H), 3.79-3.41 (m, 3H), 2.38 (s, 3H), 1.75-1.52 (m, 7H), 1.39-0.96 (m, 15H); MS: 538.3 (M+1)⁺.

### Compound 345

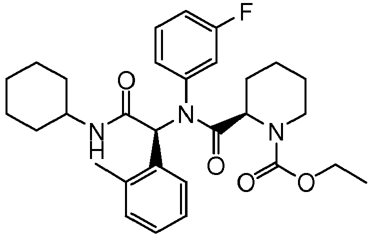

¹H NMR (300 MHz, DMSO-d6): δ 7.93-7.78 (m, 2H), 7.20-6.13 (m, 8H), 4.66-4.46 (m, 1H), 3.95-3.93 (m, 2H), 3.78-3.74 (m, 1H), 3.59-3.57 (m, 1H), 3.41-3.39 (m, 1H), 2.36 (s, 3H), 1.78-1.45 (m, 9H), 1.31-0.95 (m, 10H); MS: 524.3 (M+1)⁺.

### Compound 359

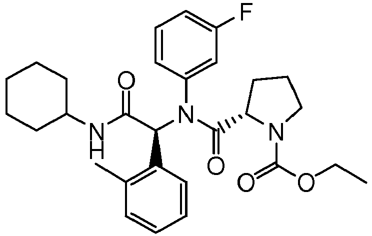

¹H NMR (300 MHz, DMSO-d6): δ8.03-8.01 (m, 1H), 7.81-7.78 (m, 1H), 7.20-6.66(m, 7H), 6.25(s, 1H), 4.07-4.00(m, 3H), 3.63-3.61(m, 1H), 3.38-3.37(m, 1H), 3.32-3.30(m, 1H), 2.36(s, 3H), 1.83-1.52(m, 9H), 1.30-0.95(m, 9H); MS: 510.1(M+1)⁺.

### Compound 336

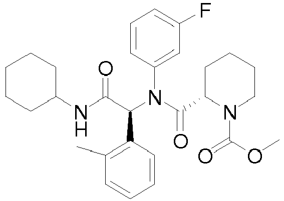

¹H NMR (400 MHz, DMSO-d6): δ 8.05-7.81 (m, 2H), 7.28-6.28 (m, 8H), 4.60-4.50 (m, 1H), 3.73-3.59 (m, 6H), 2.29 (s, 3H), 1.75-0.83 (m, 16H); MS: 510.2 (M+1)⁺.

### Compound 339

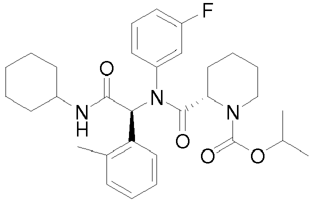

¹H NMR (400 MHz, DMSO-d6): δ 8.06-7.80 (m, 2H), 7.30-6.50 (m, 7H), 6.34 (s, 1H), 4.79-4.60 (m, 2H), 3.81-3.45 (m, 3H), 2.28 (s, 3H), 1.78-1.33 (m, 7H), 1.27-1.10 (m, 15H); MS: 538.3 (M+1)⁺.

### Compound 348

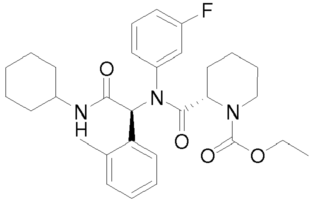

¹H NMR (400 MHz, DMSO-d6): δ 8.05-7.97 (m, 1H), 7.80-7.78 (m, 1H), 7.32-6.42 (m, 7H), 6.31 (s, 1H), 4.60-4.53 (m, 1H), 4.06-4.01 (m, 2H), 3.80-3.51 (m, 2H), 3.47-3.39 (m, 1H), 2.29(s, 3H), 1.75-1.52 (m, 8H), 1.44-0.96 (m, 11H); MS: 524.3 (M+1)⁺.

### Compound 355

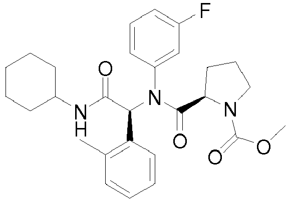

¹H NMR (300 MHz, DMSO-d6): δ 7.96-6.15 (m, 10H), 3.97-3.88 (m, 1H), 3.63-3.57 (m, 4H), 3.32-3.25 (, 2H), 2.35-2.08 (m, 3H), 1.94-1.49 (m, 9H), 1.28-0.85 (m, 5H); MS:496.2 (M+1)⁺.

### Compound 360

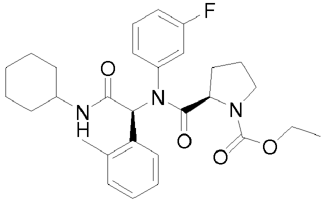

¹H NMR (400 MHz, DMSO-d6): δ 8.03-7.95 (m, 1H), 7.81-7.74 (m, 1H), 7.33-6.52 (m, 7H), 6.25 (s, 1H), 4.09-4.00 (m, 3H), 3.63-3.41 (d, 1H, J = 2.8), 3.41-3.29 (m, 2H), 2.36 (s, 3H), 1.87-1.52 (m, 9H), 1.29-0.98 (m, 8H); MS: 510.2 (M+1)⁺.

### Compound 356

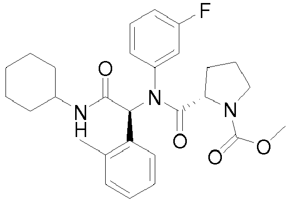

¹H NMR (400 MHz, DMSO-d6): δ 8.00-7. 94 (m, 1H), 7.79-7.74 (m, 1H), 7.35-6.48 (m, 7H), 4.05-4.02 (m, 1H), 3.61-3.58 (m, 4H), 3.39-3.30 (m, 2H), 2.37 (s, 3H), 1.84-1.52 (m, 9H), 1.29-0.96 (m, 5H); MS: 496.2 (M+1)⁺.

### Compound 350

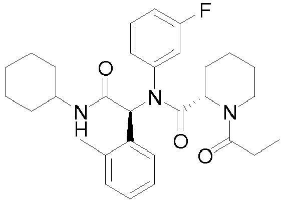

¹H NMR (400 MHz, DMSO-d6): δ 8.03-7.95 (m, 1H), 7.77-7.75 (m, 1H), 7.26-6.70 (m, 7H), 6.27-6.24 (m, 1H), 4.88-4.78 (m, 1H), 3.68-3.53 (m, 3H), 2.43-2.20 (m, 5H), 1.75-0.99 (m, 16H), 0.85 (t, 3H, *J*=7.4 Hz); MS: 508.3 (M+1)⁺.

### Compound 371

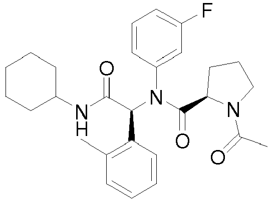

¹H NMR (400 MHz, DMSO-d6): δ 7.81-7.79 (d, J = 10.4 1H), 7.10-6.61 (m, 8H), 6.22 (s, 1H), 4.04-3.99 (d, J = 22.4, 1H), 3.64-3.33 (m, 3H), 2.33 (s, 3H), 1.99-1.53 (m, 12H), 1.32-0.63 (m, 5H); MS: 480.1 (M+1)⁺.

### Compound 370

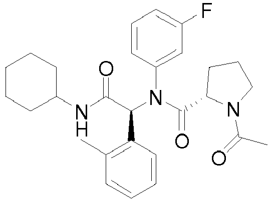

¹H NMR (400 MHz, DMSO-d6): δ 7.99-7.75 (m, 2H), 7.29-6.59 (m, 7H), 6.22 (s, 1H), 4.12-4.04 (m, 1H), 3.63-3.62 (m, 1H), 3.51-3.42 (m, 2H), 2.35 (s, 3H), 1.99 (s, 3H), 1.73-1.52 (m, 8H), 1.29-0.85 (m, 7H); MS: 480.1 (M+1)⁺.

### Compound 366

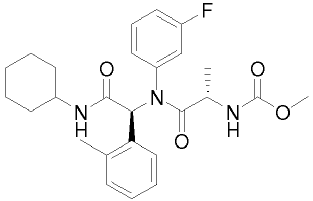

¹H NMR (400 MHz, CDCl₃): δ7.71 (br, 1H), 7.10 (br, 2H), 6.87-6.68 (m, 4H), 6.36-6.32 (br, 2H), 4.68-4.66 (m, 0.5H), 4.64-4.59 (br, 0.5H), 3.85-3.84 (br, 1H), 3.60 (s, 2H), 3.40-3.34 (br, 1H), 2.90-2.88 (br, 3H), 2.38 (s, 3H), 1.96-1.93 (br, 2H), 1.68-1.65 (br, 2H), 1.36-1.26 (br, 6H), 1.11-1.07 (br, 3H); MS: 484.1 (M+1)⁺.

### Compound 335

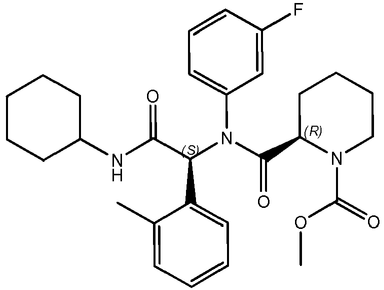

¹H NMR (300 MHz, DMSO-d6): δ 7.92 (s, 1H), 7.76-7.70 (br, 1H), 7.30-6.83 (m, 5H), 6.64-6.62 (d, 1H, J=5.7), 6.32 (br, 1H), 6.15(s, 1H), 4.68-4.62 (m, 1H), 3.73-3.70 (m, 1H), 3.59-3.58 (m, 1H), 3.47 (s, 3H), 2.35 (s, 3H), 1.80-1.45 (m, 9H), 1.31-0.95 (m, 7H); MS: 510.3 (M+1)⁺.

### Compound 396

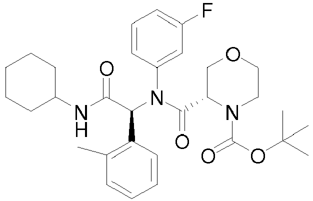

¹H NMR (400 MHz, DMSO-d6): δ 8.20-7.81(m, 2H), 7.36-6.39 (m, 8H), 4.40-4.25 (m, 1H), 3.82-3.51 (m, 5H), 3.28-3.21 (m, 2H), 2.33-2.32 (m, 3H), 1.77-0.94 (m, 19H); MS: 554.1 (M+1)⁺.

### Compound 395

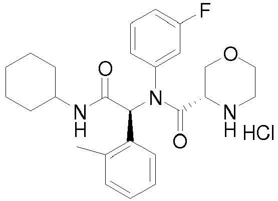

¹H NMR (400 MHz, DMSO-d6): δ 9.86 (m, 1H), 9.30 (s, 1H), 8.21-8.13 (m, 1H), 7.73-7.71 (m, 1H), 7.36-6.79 (m, 6H), 6.28-6.25 (m, 1H), 3.99 (m, 1H), 3.78-3.44 (m, 5H), 3.14 (m, 2H), 2.36-2.34 (d, 3H, *J* = 8.8), 1.76-1.74 (m, 4H), 1.30-1.11 (m, 6H); MS: 454.1 (M+1)⁺.

### Compound 349

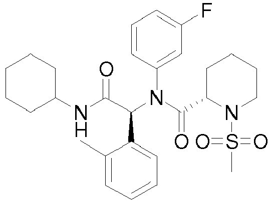

¹H NMR (400 MHz, DMSO-d6): δ 8.09-8.00 (m, 1H), 7.84-7.79 (m, 1H), 7.32-6.96 (m, 4H), 6.89-6.65 (m, 3H), 6.31-6.27 (m, 1H), 4.43-4.36 (m, 1H), 3.77-3.61 (m, 2H), 3.41-3.38 (m, 1H), 2.90 (s, 3H), 2.31 (s, 1H), 2.30 (s, 2H), 1.75-1.46 (m, 8H), 1.40-0.86 (m, 8H); MS: 530.2 (M+1)⁺.

### Compound 363

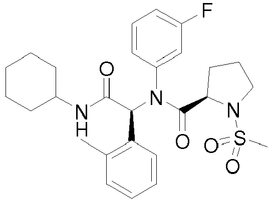

¹H NMR (400 MHz, DMSO-d6): δ 7.78-7.55 (m, 2H), 7.13-6.08 (m, 7H), 5.94 (s, 1H), 3.79 (s, 1H), 3.40-3.39 (m, 1H), 3.17-3.11 (m, 2H), 2.63 (s, 3H), 2.14 (s, 3H), 1.78-1.29 (m, 9H), 1.08-0.61 (m, 5H); MS: 516.2 (M+1)⁺.

### Compound 362

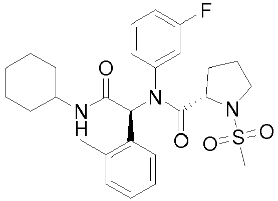

¹H NMR (400 MHz, DMSO-d6): δ 8.04-7.95 (m, 1H), 7.78-7.75 (d, 1H, J=10), 7.14-6.23 (m, 8H), 4.06-4.02 (m, 1H), 3.62 (s, 1H), 3.39-3.36 (m, 1H), 3.28-3.26 (m, 1H), 2.89-2.86 (m, 3H), 2.36-2.34 (d, 3H, J=6), 1.93-1.52 (m, 9H), 1.30-0.85 (m, 6H); MS: 516.0 (M+1)⁺.

### Compound 367

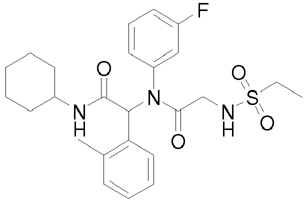

¹H NMR (400 MHz, MeOD-d4): δ 8.03 (m, 0.77H), 7.77 (m, 0.65H), 7.31 (br, 1H), 7.10-7.01 (m, 3H), 6.86 (m, 1H), 6.72 (m, 1H), 6.22 (s, 1H), 3.66-3.62 (m, 2H), 2.99-2.93(q, 2H), 2.36 (s, 3H), 1.79-1.52 (m, 4H), 1.29-0.98 (9H); MS: 490.2 (M+1)⁺.

### Compound 375

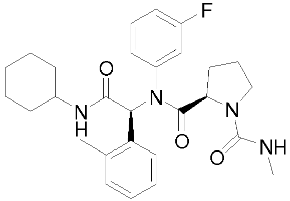

¹H NMR (400 MHz, DMSO-d6): δ 8.23-8.22 (d, J = 6.4 1H), 7.57-7.45 (m, 2H), 7.10-7.02 (m, 4H), 6.81 (s, 1H), 6.58 (s, 1H), 6.29-6.21 (m, 3H), 3.92-3.91 (m, 1H), 3.71-3.69 (m, 1H), 3.36-3.22 (m, 2H), 2.63-2.62 (d, J = 4.0, 3H), 2.28 (s, 3H), 2.08-1.33 (m, 9H), 1.29-0.51 (m, 5H); MS: 494.8 (M+1)⁺.

### Compound 385

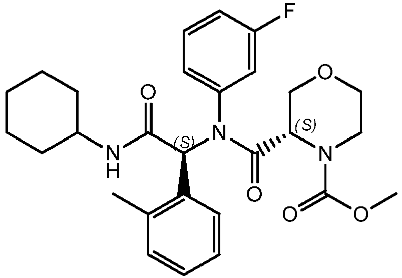

¹H NMR (400 MHz, DMSO-d6): δ 8.06-7.89 (m, 2H), 7.31-6.34 (m, 8H), 4.31-4.23 (m, 1H), 3.84-3.48 (m, 8H), 3.29-3.26 (m, 2H), 2.30 (s, 3H), 1.77-1.53 (m, 5H), 1.30-0.94 (m, 5H); MS: 512.0 (M+1)⁺.
**Example 12. Preparation of Compound 248.** Compound 248 was produced using the following protocol. 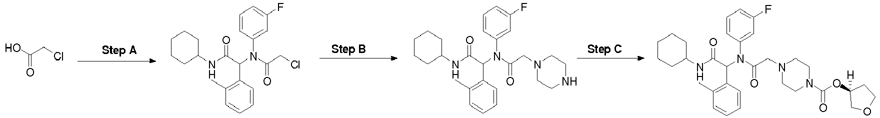
***Step A: 2-[(2-Chloro-acetyl)-(3-fluoro-phenyl)-amino]-N-cyclohexyl-2-o-tolyl-acetamide.*** The title compound was synthesized using Scheme 1 and the general procedure set forth in Example 1, step A.
***Step B: N-Cyclohexyl-2-[(3-fluoro-phenyl)-(2-piperazin-1-yl-acetyl)-amino]-2-o-tolyl-acetamide.*** The title compound was synthesized using Scheme 2 and the general procedure set forth in Example 2. ¹H NMR (300 MHz, DMSO-d6): δ9.12 (br, 2H), 8.02 (s, 1H), 7.11-6.71 (m, 6H), 6.23 (s, 1H), 3.64-3.62 (m, 1H), 3.08-3.03 (m, 2H), 2.89 (m, 4H), 2.59 (s, 4H), 2.35 (s, 3H), 1.78-1.54 (m, 5H), 1.31-0.98 (m, 6H); MS: 467.1 (M+1)⁺.
***Step C: Compound 248.*** To a mixture of Et₃N (40 mg, 0.39 mmol) and S-tetrahydro-furan-3-ol (35 mg, 0.39 mmol) in THF (10 ml) was added a solution of triphosgene (115 mg, 0.39 mmol) in THF (10 ml). After stirring for 10 minutes, N-Cyclohexyl-2-[(3-fluoro-phenyl)-(2-piperazin-1-yl-acetyl)-amino]-2-o-tolyl-acetamide (300 mg, 0.64 mmol) was added in one portion. The reaction was stirred for 1.5 hours at room temperature. Water (15 ml) was added. The resulting mixture was extracted with EtOAc (2x20 ml) and the combined organic layer was washed with brine, dried over Na₂SO₄, filtered and the solvent was evaporated in vacuo. The residue was purified via flash chromatography column eluted with DCM/MEOH (30/1) to give the desired product (280 33 mg, 15% yield); ¹H NMR (300 MHz, DMSO-d6): δ 7.99 (s, 1H), 7.74 (br, 1H), 7.21-6.56 (m, 7H), 6.22 (s, 1H), 5.09 (s, 1H), 3.77-3.63 (m, 5H), 3.24 (s, 4H), 3.00-2.84 (m, 2H), 2.33 (s, 3H), 2.28 (s, 4H), 2.10-1.52 (m, 7H), 1.30-0.96 (m, 5H); MS: 581.3 (M+1)⁺.

The following compounds were synthesized from N-Cyclohexyl-2-[(3-fluoro-phenyl)-(2-piperazin-1-yl-acetyl)-amino]-2-o-tolyl-acetamide via step C of this example

### Compound 249

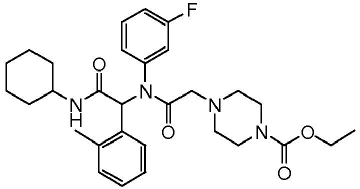

¹H NMR (300 MHz, DMSO-d6): δ 7.99 (s, 1H), 7.73 (br, 1H), 7.09-6.56 (m, 7H), 6.22 (s, 1H), 4.03-3.09 (m, 1H), 3.63-3.61 (m, 1H), 3.24 (s, 4H), 2.99-2.84 (m, 2H), 2.33(s, 3H), 2.28 (s, 4H), 1.78-1.52 (m, 5H), 1.29-0.95 (m, 8H); MS: 539.3 (M+1)⁺.

### Compound 250

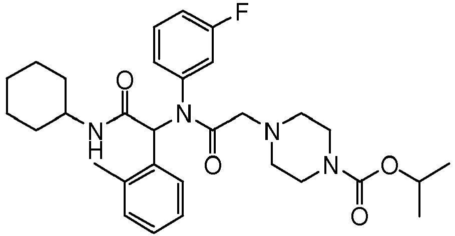

¹H NMR (300 MHz, DMSO-d6): δ 7.98 (s, 1H), 7.74 (br, 1H), 7.09-6.70 (m, 6H), 6.55 (br, 1H), 6.22 (s, 1H), 4.76-4.70 (m, 1H), 3.63-3.61 (m, 1H), 3.56 (s, 3H), 3.23 (s, 4H), 2.99-2.83 (m, 2H), 2.34 (s, 3H), 2.28 (s, 4H), 1.78-1.52 (m, 5H), 1.30-0.96 (m, 11H); MS: 553.3 (M+1)⁺.

### Compound 185 and its HCl Salt

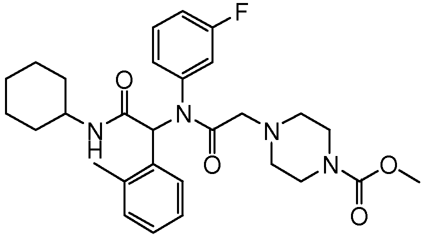

¹H NMR (300 MHz, DMSO-d6): δ 7.98 (s, 1H), 7.74 (br, 1H), 7.09-6.70 (m, 6H), 6.22 (s, 1H), 3.63-3.61 (m, 1H), 3.56 (s, 3H), 3.25 (s, 4H), 2.99-2.83 (m, 2H), 2.34(s, 3H), 2.28 (s, 4H), 1.78-1.52 (m, 5H), 1.30-0.96 (m, 5H); MS: 525.3 (M+1)⁺.
HCl Salt:
¹H NMR (400 MHz, DMSO-d6): δ10.25(s, 1H), 8.15(s, 1H), 7.87(m, 1H), 7.36-6.62(m, 8H), 6.22(s, 1H), 4.05-3.83 (m, 4H), 3.42-3.38(m, 7H), 3.00(m, 2H), 2.38(s, 3H), 1.72-1.50(m, 5H), 1.38-1.00(m, 5H); MS: 525.3 (M+1)⁺.

### Compound 208

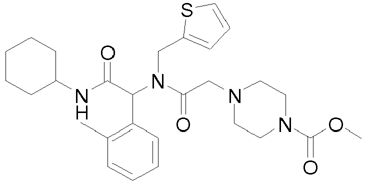

¹H NMR (300 MHz, DMSO-d6): δ 8.04-7.91 (m, 1H), 7.29-6.98 (m, 5H), 6.73-5.66 (m, 3H), 5.12-4.17 (m, 2H), 3.64-3.06 (m, 10H), 2.43-2.34 (m, 4H), 2.17 (s, 0.82H), 2.13 (s, 2.33H), 1.84-1.51 (m, 5H), 1.32-1.04 (m, 5H); MS: 527.2 (M+1)⁺.
**Example 13. Preparation of Compound 299.** Compound 299 was prepared according to the following protocol 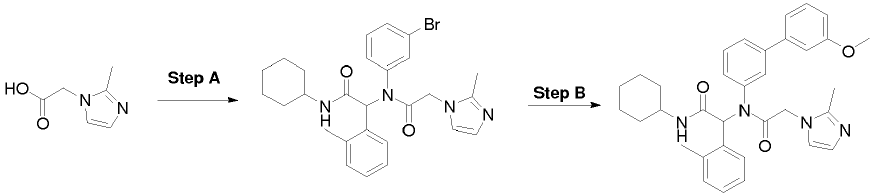
***Step A: 2-{(3-Bromo-phenyl)-[2-(2-methyl-imidazol-1-yl)-acetyl]-amino}-N-cyclohexyl-2-o-tolyl-acetamide.*** The title compound was synthesized via Scheme 1 and the protocol set forth in Example 1, step A. ¹H NMR (400 MHz, DMSO-d6): δ 8.22-8.00 (m, 2H), 7.38-6.88 (m, 7H), 6.73-6.66 (m, 2H), 6.19 (s, 1H), 4.66-4.34 (m, 2H), 3.60(s, 1H), 2.36 (s, 3H), 2.10 (s, 3H), 1.73-1.52 (m, 5H), 1.28-0.92 (m, 5H); MS: 523.1 (M+1)⁺.
***Step B: Compound 299.*** A mixture of 2-{(3-Bromo-phenyl)-[2-(2-methyl-imidazol-1-yl)-acetyl]-amino}-N-cyclo-hexyl-2-o-tolyl-acetamide (209 mg, 0.4 mmol), (3-methoxyphenyl) boronic acid (0.3 g, 2 mmol), K₂CO₃ (0.17g, 1.2 mmol) and Pd(dppf)Cl₂ (66m mg, 0.08 mmol) in DME (5ml) was stirred at 80 °C overnight under nitrogen atmosphere. The resulting mixture was filtered and the filtrate was concentrated. The residue was purified via flash chromatography to give desired product as a yellow powder (130 mg, 59%). ¹H NMR (400 MHz, DMSO-d6): δ 8.26-7.97 (m, 2H), 7.46-6.72 (m, 13H), 6.24 (s, 1H), 4.66-4.35 (m, 2H), 3.84-3.74 (m, 3H), 3.63-3.61 (m, 1H), 2.46-2.39 (m, 3H), 2.10 (s, 3H), 1.73-1.50 (m, 5H), 1.25-0.89 (m, 5H); MS: 551.1 (M+1)⁺.

The following compounds were synthesized according to the procedures set forth in this Example.

### Compound 286

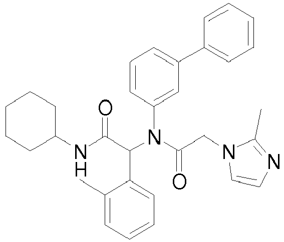

¹H NMR (400 MHz, CD3OD): δ 8.16-6.69 (m, 15H), 6.29 (d, 1H, *J*=9.6 Hz), 4.73-4.45 (m, 2H), 3.64-3.62 (m, 1H), 2.40 (d, 3H, *J*=24.4 Hz), 2.16 (s, 3H), 1.84-1.50 (m, 5H), 1.28-1.02 (m, 5H); MS: 521.2 (M+1)⁺.

### Compound 300

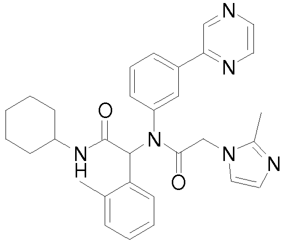

¹H NMR (400 MHz, DMSO-d6): δ 9.25-9.09 (m, 2H), 8.81(s, 1H), 8.35-8.03 (m, 2H), 7.64-7.23 (m, 2H), 7.18-6.71 (m, 7H), 6.26 (s, 1H), 4.76-4.45 (m, 2H), 3.63-3.61 (m, 1H), 2.45 (s, 2H), 2.41 (s, 1H), 2.17 (s, 3H), 1.78-1.55 (m, 5H), 1.28-0.98 (m, 5H); MS: 523.1 (M+1)⁺.

### Compound 292

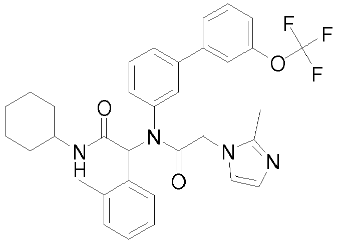

¹H NMR (400 MHz, DMSO-d6): δ 8.28-7.34 (m, 8H), 7.24-6.69 (m, 7H), 6.24 (d, 1H, *J*=6.8 Hz), 4.86-4.56 (m, 2H), 3.61-3.59 (m, 1H), 2.47-2.35 (m, 3H), 2.30 (s, 3H), 1.72-1.50 (m, 5H), 1.28-0.96 (m, 5H); MS: 605.1 (M+1)⁺.

### Compound 315

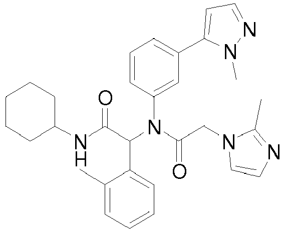

¹H NMR (400 MHz, DMSO-d6): δ 8.14-8.01 (m, 2H), 7.49-7.03 (m, 6H), 6.89-6.73 (m, 4H), 6.38-6.14 (m, 2H), 4.69-4.40 (m, 2H), 3.83-3.53 (m, 4H), 2.42-2.40 (m, 3H), 2.17 (s, 3H), 1.73-1.51 (m, 5H), 1.28-0.95 (m, 5H); MS: 525.1 (M+1)⁺.

### Example 14. Preparation of Compound 344.

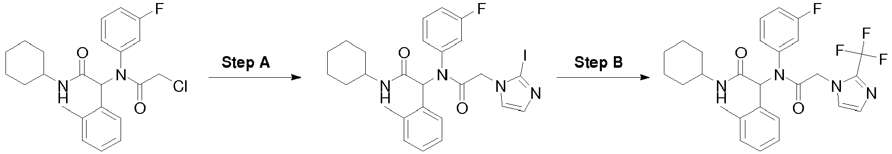

***Step A: N-Cyclohexyl-2-{(3-fluoro-phenyl)-[2-(2-iodo-imidazol-1-yl)-acetyl]-amino}-2-o-tolyl-acetamide.*** The title compound was synthesized using Scheme 2, and the protocol set forth in Example 2, step A. ¹H NMR (400 MHz, DMSO-d6): δ 8.03-7.93 (m, 2H), 7.28-7.06 (m, 5H), 6.90-6.48 (m, 4H), 6.22 (s, 1H), 4.48-4.45 (m, 2H), 3.61-3.60 (m, 1H), 2.33 (s, 3H), 1.76-1.51 (m, 5H), 1.28-0.93 (m, 5H); MS: 575.1 (M+1)⁺.
***Step B: Compound 344.*** A mixture of N-Cyclohexyl-2-{(3-fluoro-phenyl)-[2-(2-iodo-imidazol-1-yl)-acetyl]-amino}-2-o-tolyl-acetamide (144 mg, 0.25 mmol), KF (dry, 25 mg, 0.43 mol), CuI (75 mg, 0.39 mmol) and CF₃SiC(CH₃)₃ (60 mg, 0.43 mmol) in dry NMP (2.5 ml) was stirred at 50°C for 27 hours under N₂ atmosphere. The resulting mixture was cooled to room temperature and diluted with DCM (15 ml), washed with water, brine, dried over Na₂SO₄, filtered and the solvent was evaporated in vacuo. The residue was purified with prep-HPLC to give desired product as a solid (40 mg, 31 yield). ¹H NMR (400 MHz, DMSO-d6): δ 8.05-7.89 (m, 2H), 7.42-6.38 (m, 9H), 6.19 (s,1H), 4.76-4.74 (m,2H), 3.60-3.57 (m, 1H), 2.38 (s,3H), 1.74-1.51 (m, 5H), 1.28-0.93 (m, 5H); MS: 517.2 (M+1)⁺.

### Compound 373

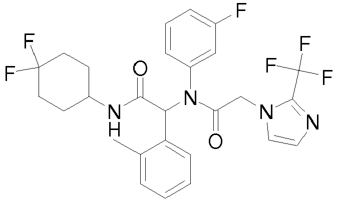

Compound 373 was synthesized via the procedure set forth in this example. ¹H NMR (400 MHz, DMSO-d6): δ 8.17-8.13 (m, 1H), 7.89-7.86 (m, 1H), 7.41-7.07 (m, 6H), 6.87 (t, 1H, J=7.6 Hz), 6.70-6.69 (m, 1H), 6.51-6.50 (m, 1H), 6.20 (s,1H), 4.82-4.76 (m,2H), 3.84 (s, 1H), 2.38 (s,3H), 2.01-1.76 (m, 6H), 1.51-1.43 (m, 1H), 1.31-1.23 (m, 1H); MS: 552.6 (M+1)⁺.

### Example 15. Preparation of Compound 326.

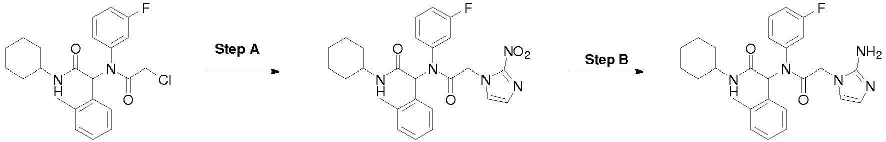

***Step A: N-Cyclohexyl-2-{(3-fluoro-phenyl)-[2-(2-nitro-imidazol-1-yl)-acetyl]-amino}-2-o-tolyl-acetamide.*** The title compound was synthesized using Scheme 2, and the protocol set forth in Example 2, step A.
***Step B: Compound 326.*** A suspension of N-Cyclohexyl-2-{(3-fluoro-phenyl)-[2-(2-nitroimidazol-1-yl)-acetyl]-amino}-2-o-tolyl-acetamide (110 mg, 0.22 mmol) and 10% Pd/C (30 mg) in MeOH (8 ml) was stirred under 1 atm of hydrogen gas at room temperature for 16 h. The solids were removed by filtration and the solvent was concentrated, purified by prep-HPLC to get 30 mg product (30 mg, 30% yield). ¹H NMR (300 MHz, DMSO-d6): δ 8.05-7.75 (m, 2H), 7.11-6.98 (m, 4H), 6.88-6.67 (m, 3H), 6.43 (d, 1H), 6.31 (d, 1H), 6.19(s, 1H), 5.07 (s, 2H), 4.39-4.35 d, 1H), 4.13-4.08 (d, 1H), 3.63-3.58 (m, 1H), 2.38 (s, 3H), 1.76-1.49 (m, 5H), 1.31-0.85(m, 5H); MS: 464.2 (M+1)⁺.

### Example 16. Preparation of Compound 319.

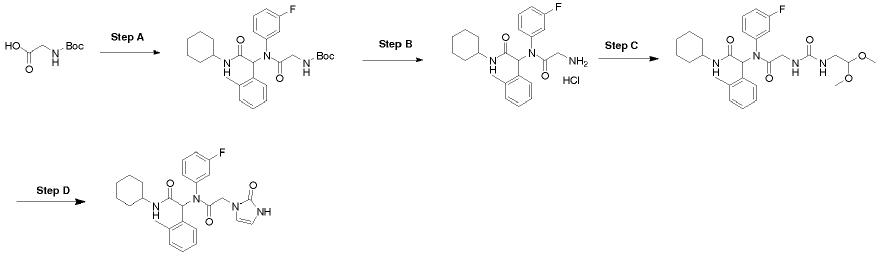

***Step A: Compound 368.*** The title compound was synthesized using Scheme 1 and the protocol set forth in Example 1, step A. ¹H NMR (400 MHz, MeOD-d4): δ 7.73 (br, 1H), 7.15 (d, J = 7.6, 1H), 7.09-7.09 (m, 1), 6.99-6.94 (m, 1H). 6.80-6.78 (m, 1H), 6.57 (br, 0.7H), 6.38 (s, 1H), 3.78-3.68 (m, 2H), 3.50-3.39 (d, 1H), 2.33 (s, 3H), 1.9-1.93 (m, 1H), 1.80-1.71 (m, 4H), 1.46-1.04 (m, 12H); MS: 498.1 (M+1)⁺.
***Step B: 2-[(2-Amino-acetyl)-(3-fluoro-phenyl)-amino]-N-cyclohexyl-2-o-tolyl-acetamide (hydrochloride).*** The title compound was synthesized using the protocol set forth in Example 8, step B. ¹H NMR (400 MHz, MeOD-d4): δ 8.1 (br, 1H), 7.66 (br, 1H), 7.03-6.70 (m, 6H), 6.29 (s, 1H), 4.16 (m, 1H), 3.43 (d, 1H), 3.26 (d, 1H), 2.34 (s, 3H), 1.69-1.64 (m, 1H), 1.52-1.50 (m, 3H), 1.29-1.00 (m, 3H), 0.80-0.76 (m, 3H); MS: 398.1 (M+1)⁺.
***Step C: N-Cyclohexyl-2-[{2-[3-(2,2-dimethoxy-ethyl)-ureido]-acetyl}-(3-fluoro-phenyl)-amino]-2-o-tolyl-acetamide.*** To a mixture of 2-[(2-Amino-acetyl)-(3-fluoro-phenyl)-amino]-N-cyclohexyl-2-o-tolyl-acet-amide (433 mg, 1 mmol) and Et₃N (0.2 ml, 1.5 mmol) in DCM was added 2-Isocyanato-1,1-dimethoxy-ethane (231 mg, 1.3 mmol). The reaction mixture was stirred for 8 hours. The resulting mixture was washed with HCl (1 N), water, brine, dried over Na₂SO₄ and filtered. The solvent was evaporated in vacuo and the residue was washed with Et₂O to give the crude N-Cyclohexyl-2-[{2-[3-(2,2-dimethoxy-ethyl)-ureido]-acetyl}-(3-fluorophenyl)-amino]-2-o-tolyl-acetamide, which was used directly without further purification (350 mg, 66% yield). ¹H NMR (400 MHz, DMSO-d6): δ 8.12-7.78 (m, 2H), 7.35-6.20 (m, 10H), 4.41 (m, 1H), 3.66 (m, 2H), 3.30-3.24 (m, 7H), 3.05 (m, 2H), 2.35 (s, 3H), 1.78-1.50 (m, 5H), 1.25-0.95 (m, 5H); MS: 529.1 (M+1)⁺.
***Step D: Compound 319.*** A mixture of N-Cyclohexyl-2-[{2-[3-(2,2-dimethoxy-ethyl)-ureido]-acetyl}-(3-fluoro-phenyl)-amino]-2-o-tolyl-acetamide (100 mg, 0.19 mmol) in AcOH (1 ml) and HCOOH (0.7 ml) was heated to 65°C for 1 hour. The resulting mixture was concentrated in vacuo and the residue was suspended in saturated NaHCO₃ (10 ml). The precipitate was collected by filtration and washed with Et₂O to give the desired product (25 mg, 28% yield). ¹H NMR (400 MHz, DMSO-d6): δ 9.90 (s, 1H), 8.04-7.81 (m, 2H), 7.05-6.57 (m, 7H), 6.28-6.19 (m, 3H), 4.02 (m, 2H), 3.58 (m, 1H), 2.33 (s, 3H), 1.70-1.50 (m, 5H), 1.34-1.02 (m, 5H); MS: 465.1 (M+1)⁺.

### Example 17. Preparation of Compound 163.

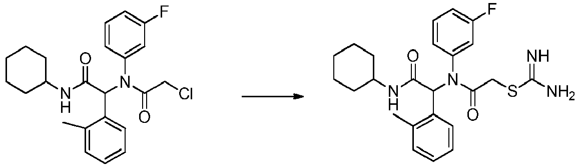

To a solution of 2-[(2-Chloro-acetyl)-(3-fluoro-phenyl)-amino]-N-cyclohexyl-2-o-tolyl-acetamide (208 mg, 0.50 mmol) in 1, 2-dichloroethane (10 ml) was added thiourea (54 mg, 0.71 mmol). The reaction mixture was stirred at 80°C for 8h and then cooled to room temperature. The precipitate was collected by filtration and purified by prep. HPLC to give the byproduct as a white solid (60 mg, yield =26%). ¹H NMR (300 MHz, DMSO-d6): δ 9.02 (br, 4H), 8.06-6.57 (m, 9H), 6.17 (s, 1H), 4.16-3.85 (m,2H), 3.63-3.60 (m, 1H), 2.43 (s,3H), 1.78-1.51 (m, 5H), 1.32-0.93 (m, 5H); MS: 457.2 (M+1)⁺.

### Example 18. Preparation of Compounds 263 and 212.

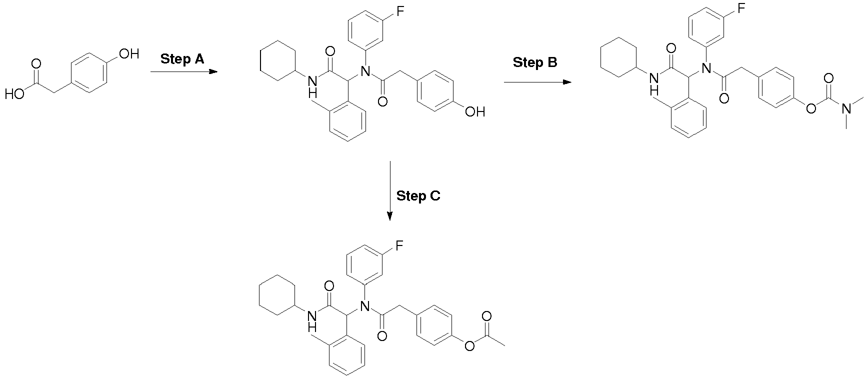

***Step A: Compound 217.*** N-Cyclohexyl-2-{ (3-fluoro-phenyl)-[2-(4-hydroxy-phenyl)-acetyl]-amino}-2-o-tolyl-acetamide (Compound 217) was synthesized according to Scheme 1, via the general procedure set forth in Example 1, step A. ¹H NMR (400 MHz, DMSO-d6): δ 9.21 (s, 1H), 7.96-7.71 (m, 2H), 7.09-6.23 (m, 12H), 3.62-3.57 (m, 1H), 3.33-3.21 (m, 2H), 2.32 (s, 3H), 1.76-1.52 (m, 5H), 1.29-0.93 (m, 5H); MS: 475.2 (M+1)⁺.
***Step B: Compound 263.*** A mixture of N-Cyclohexyl-2-{ (3-fluoro-phenyl)-[2-(4-hydroxy-phenyl)-acetyl]-amino}-2-o-tolyl-acetamide (100 mg, 0.21 mmol), Dimethylcarbamyl chloride (46 mg, 0.42 mmol), Et₃N (64 mg, 0.42 mmol) and DMAP (26 mg, 0.21 mol) in DCM (15 ml) was heated to 50°C for 10 hours. After cooling to room temperature, the resulting mixture was washed with brine, dried over Na₂SO₄, filtered and the solvent was evaporated in vacuo. The residue was purified by TLC (DCM/MeOH=20/1) to give the pure product (45 mg, 40% yield). ¹H NMR (400 MHz, DMSO-d6): δ 7.99-7.65 (m, 2H), 7.26-6.24 (m, 12H), 3.81-3.32 (m, 3H), 3.03 (s, 3H), 2.90 (s, 3H), 2.34 (s, 3H), 1.76-1.52 (m, 5H), 1.29-0.96 (m, 5H); MS: 546.1 (M+1)⁺.
***Step C: Compound 212.*** A mixture of N-Cyclohexyl-2-{(3-fluoro-phenyl)-[2-(4-hydroxy-phenyl)-acetyl]-amino}-2-o-tolyl-acetamide (200 mg, 0.42 mmol), Et₃N (260 mg, 0.84 mmol) and acetyl chloride (70 mg, 0.84 mmol) in DCM (15 ml) was stirred for 10 hours at room temperature. The resulting mixture was washed with brine, dried over Na₂SO₄, filtered and the solvent was evaporated in vacuo. The residue was purified by TLC (PE/EtOAc=2/1) to give the pure product (180 mg, 82% yield). ¹H NMR (400 MHz, DMSO-d6): δ 7.98-7.75 (m, 2H), 7.10-6.24 (m, 12H), 3.63-3.61 (m, 1H), 3.47-3.37 (m, 2H), 2.35 (s, 3H), 2.25 (s, 3H), 1.77-1.52 (m, 5H), 1.28-0.83 (m, 5H); MS: 517.3 (M+1)⁺.
The following compounds were synthesized via the general procedure set forth in this example.

### Compound 264

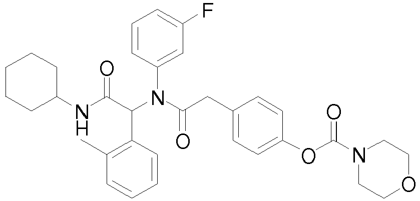

¹H NMR (400 MHz, DMSO-d6): δ 7.99-7.75 (m, 2H), 7.25-6.24 (m, 12H), 3.65-3.42 (m, 11H), 2.34 (s, 3H), 1.73-1.51 (m, 5H), 1.28-0.89 (m, 5H); MS: 588.1 (M+1)⁺.

### Compound 313

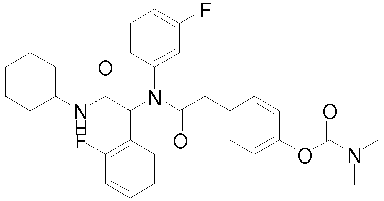

¹H NMR (400 MHz, DMSO-d6): δ 8.15 (m, 1H), 7.64-6.31 (m, 13H), 3.63 (m, 1H), 3.45-3.36 (m, 2H), 3.03 (s, 3H), 2.89 (s, 3H), 1.74-1.51 (m, 5H), 1.25-0.95 (m, 5H); MS: 550.1 (M+1)⁺.

### Example 19. Preparation of Compounds 215 and 216

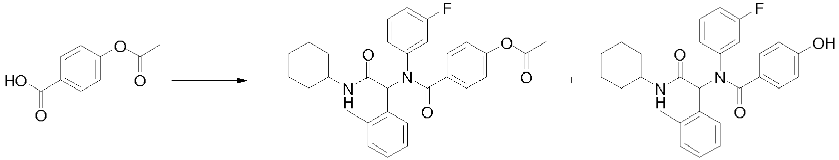

Acetic acid 4-[(cyclohexylcarbamoyl-o-tolyl-methyl)-(3-fluoro-phenyl)-carbamoyl]-phenyl ester (Compound 215) was synthesized via Scheme 1, following the general procedure set forth in Example 1, step A and some de-Ac byproduct (Compound 216) was isolated from the reaction.

### Compound 215

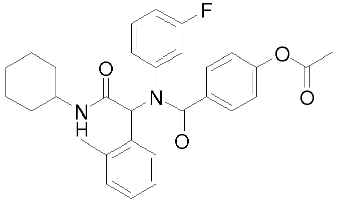

¹H NMR (300 MHz, DMSO-d6): δ 8.12 (d, 1H, *J*=8.1 MHz), 7.29-6.75 (m, 12H), 6.47 (s, 1H), 3.66 (m, 1H), 2.38 (s, 3H), 1.98 (s, 3H), 1.79-1.51 (m, 5H), 1.29-1.03 (m, 5H); MS: 503.2 (M+1)⁺.

### Compound 216

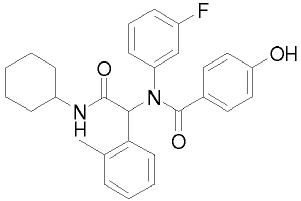

¹H NMR (400 MHz, DMSO-d6): δ 8.13 (d, 1H, *J*=7.6 MHz), 7.11-6.49 (m, 12H), 3.68-3.66 (m, 1H), 2.35 (s, 3H), 1.78-1.54 (m, 5H), 1.31-1.01 (m, 5H); MS: 461.2 (M+1)⁺.
**Example 20. Synthesis of 3-Isocyano-bicyclo[3.1.0]hexane.** The title intermediate was synthesized following the scheme below and used for synthesis of Compound 333 and Compound 377 following Scheme 1 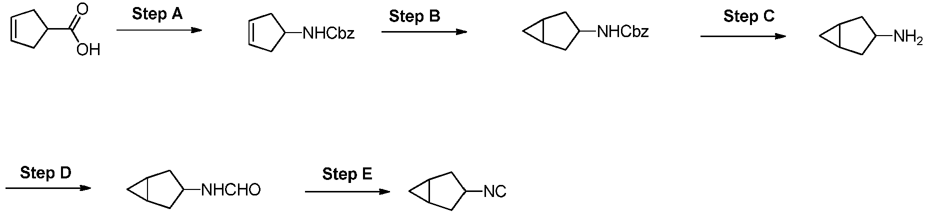
***Step A: For Cyclopent-3-enyl-carbamic acid benzyl ester.*** To a solution of Cyclopent-3-enecarboxylic acid (5 g, 44.6 mmol) in toluene (50 ml) was added a solution of DPPA (13.5 g, 49 mmol) and Et₃N (5.4 g, 53.5 mmol) in toluene (50 ml) dropwise at room temperature. The mixture was heated to reflux for 2 hours and then benzyl alcohol (7 ml, 66.9 mmol) was added. The reaction mixture was refluxed overnight then cooled to room temperature, washed with NaHCO₃ solution, brine, dried over Na₂SO₄, and filtered. The organic solvent was evaporated in vacuo and the residue was purified via flash chromatography column eluted with PE/EtOAc (from 50/1 to 5/1) to give the pure cyclopent-3-enyl-carbamic acid benzyl ester (5 g, 52% yield). ¹H NMR (400 MHz, DMSO-d6): δ 7.46 (d, *J* = 8.0, 1H), 7.36-7.30 (m, 5H), 5.66 (s, 2H), 5.00 (s, 2H), 4.11 (m, 1H), 2.59-2.49 (m, 2H), 2.19-2.14 (m, 2H)
***Step B: Bicyclo[3.1.0]hex-3-yl-carbamic acid benzyl ester.*** To a solution of Cyclopent-3-enyl-carbamic acid benzyl ester in DCM (30 ml) was added ZnEt₂ (1 M, 30.4 ml, 30.4 mmol) at 0°C under N₂ atmosphere. CH₂I₂ (2.5 ml, 30.4 mmol) was added dropwise under the same condition. The reaction mixture was warmed to room temperature and stirred for 4 hours. The resulting mixture was washed with brine, dried over Na₂SO₄, filtered and the solvent was concentrated. The residue was purified via flash chromatography column eluted with PE/EtOAc (from 50/1 to 5/1) to give the pure Bicyclo[3.1.0]hex-3-yl-carbamic acid benzyl ester (1.5 g, 46% yield). ¹H NMR (400 MHz, DMSO-d6): δ 7.37-7.31 (m, 5H), 7.50 (d, J = 4.6, 1H), 4.99 (s, 2H), 3.98-3.96(m, 1H), 1.59-1.55 (m, 2H), 1.23-1.14 (m, 2H), 0.50 (m, 1H), 0.27 (m, 1H).
***Step C: For Bicyclo[3.1.0]hex-3-ylamine.*** A solution of Bicyclo[3.1.0]hex-3-yl-carbamic acid benzyl ester (1.5 g, 6.5 mmol) in MeOH (20 ml) was hydrogenated with Pd/C (10%, 0.3 g) as a catalyst under atmospheric pressure for 2 hours. The resulting mixture was filtered and the filtrate was evaporated in vacuo to give the Bicyclo[3.1.0]hex-3-ylamine as a white solid which was used directly without further purification (0.45 g, 71% yield). ¹H NMR (400 MHz, CDCl₃): δ 4.35-3.64 (m, 3.8H), 2.23-2.18 (m, 2H), 1.53-1.50 (m, 2H), 1.23-1.13(m, 4H), 0.56-0.51 (m, 2H), 0.00 (br, 1H).
***Step D: N-Bicyclo[3.1.0]hex-3-yl-formamide.*** A mixture of Bicyclo[3.1.0]hex-3-ylamine (0.45 g, 4.6 mmol) in ethyl formate (2 ml) was reflux for 8 hours. The resulting mixture was evaporated in vacuo and the residue was purified via chromatography eluted with PE/EtOAc (from 20/1 to 2/1) to give the N-Bicyclo[3.1.0]hex-3-yl-formamide. (460 mg, 80% yield) ¹H NMR (400 MHz, DMSO-d6): δ 7.67-7.59 (m, 2H), 3.93 (m, 1H), 1.94-1.88 (m, 2H), 1.32-1.28(m, 2H), 1.03-1.00 (m, 2H), 0.30-0.26 (m, 1H), 0.00 (m, 1H).
***Step E: 3-Isocyano-bicyclo[3.1.0]hexane.*** A mixture of N-(Tetrahydro-pyran-4-yl)-formamide (0.46 g, 3.7 mmol), PPh₃ (1.06 g, 4 mmol), CCl₄ (0.57 g, 3.7 mmol), Et₃N (0.38 g, 3.7 mmol) in DCM (10 ml) was heated to 45°C for 8 hours. The resulting mixture was evaporated in vacuo and the residue was suspended in Et₂O (25 ml). The solid was filtered off and the solvent was concentrated and purified via flash chromatography column eluted with PE/EtOAc (from 100/1 to 20/1) to give the pure 3-Isocyano-bicyclo[3.1.0]hexane (0.1 g, 25% yield). ¹H NMR (400 MHz, CDCl₃): δ 4.01 (m, 1H), 2.22-2.17 (m, 2H), 2.08-2.04 (m, 2H), 0.66-0.60(m, 2H).
The following intermediates were synthesized via Steps D and E of the procedure set forth in this example and purified via flash chromatography eluted with Et₂O or PE to afford the desired product as an Et₂O or PE solution, which was concentrated under 1atm pressure and used directly.

### 1, 1-Difluoro-4-isocyano-cyclohexane (PE solution) used for synthesis of Compound 342

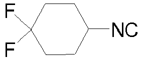

D₁₁-Isocyano-cyclohexane (Et₂O solution) used for synthesis of Compound 361 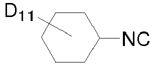 **Example 21. Synthesis of 4-Isocyano-tetrahydro-pyran.** The title compound was synthesized following the scheme below and used for synthesis of Compound 179 following Scheme 1. 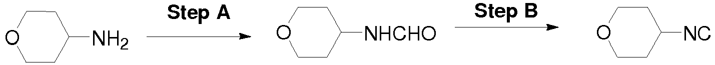
***Step A: N-(Tetrahydro-pyran-4-yl)-formamide.*** A mixture of Tetrahydro-pyran-4-ylamine (25 g, 247.5 mmol) in ethyl formate (25 g, 338 mmol) was reflux for 8 hours. The resulting mixture was evaporated in vacuo to give the crude N-(Tetrahydro-pyran-4-yl)-formamide, which was used directly without further purification (29 g, 90% yield). ¹H NMR (400 MHz, CDCl₃): δ 8.10 (br, 1H), 5.77 (br, 1H), 4.19-4.02 (m, 1H), 3.98-3.90 (m, 2H), 3.50-3.84 (m, 2H), 1.92-1.80 (m, 2H), 1.62-1.41 (m, 2H).
***Step B: For 4-Isocyano-tetrahydro-pyran.*** A mixture of N-(Tetrahydro-pyran-4-yl)-formamide (29 g, 224 mmol), PPh₃ (64.8 g, 247 mmol), CCl₄ (34.5 g, 224 mmol), Et₃N (22.6 g, 224 mmol) in DCM (300 ml) was heated to 45°C for 8 hours. The resulting mixture was evaporated in vacuo and the residue was suspended in Et₂O (250 ml). The solid was filtered off and the solvent was purified via flash chromatography column eluted with PE/EtOAc to give the 4-Isocyano-tetrahydro-pyran (15 g, 60% yield). ¹H NMR (400 MHz, CDCl₃): δ 3.90-3.82 (m, 3H), 3.57-3.50 (m, 2H), 1.95-1.91 (m, 2H), 1.84-1.77 (m, 2H).
**Example 22. Synthesis of 1,1-Difluoro-3-isocyano-cyclobutane.** The title compound was synthesized following scheme below and used for synthesis of Compound 379 according to Scheme 1. 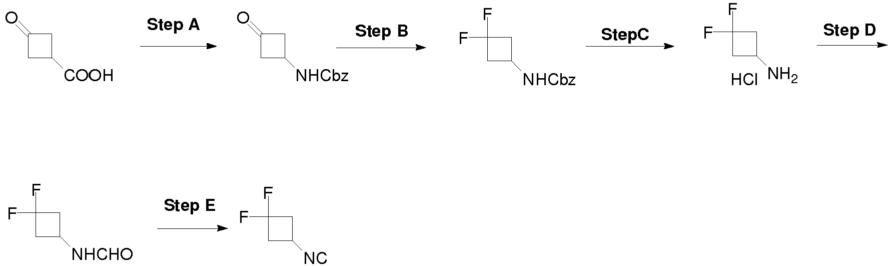
***Step A: (3-Oxo-cyclobutyl)-carbamic acid benzyl ester.*** A solution of 3-Oxo-cyclobutanecarboxylic acid (1.01 g, 8.8 mmol) and Et₃N (1.5ml, 10.5mmol) in THF/Toluene (1:1, 30ml) was treated with DPPA (1.9 ml, 8.8 mmol). The mixture was stirred for 3 hours at 60°C and then BnOH (1 ml, 9.7mmol) added. The reaction mixture was stirred for another 3 hours at the same temperature. The resulting mixture was concentrated under vacuum to remove most THF and then diluted with EtOAc (50 20 ml). This so-obtained mixture was washed with saturated NaHCO₃ solution, brine, dried over Na₂SO₄ and filtered. The solvent was evaporated and the residue was purified via chromatography eluted with PE/EtOAc (4:1) to give the desired product as a white solid (yield: 0.48g, 25% yield). ¹H NMR (400 MHz, CDCl₃): δ 7.35 (m, 5H), 5.12 (m, 3H), 4.33 (m, 1H), 3.41 (m, 2H), 3.07 (m, 2H).
***Step B: For (3,3-Difluoro-cyclobutyl)-carbamic acid benzyl ester.*** To a solution of (3-Oxo-cyclobutyl)-carbamic acid benzyl ester (0.3 g, 1.37 mmol) in CHCl₃ (3 ml) was added DAST (0.88 g, 5.48 mmol) dropwise. The reaction mixture was stirred overnight at room temperature and then quenched with saturated NaHCO₃ solution (25 ml). The resulting mixture was extracted with DCM (3x15 ml) and the combined organic layer was washed with water, brine, dried over Na₂SO₄, filtered and the solvent was evaporated in vacuo. The residue was purified by TLC (PE/EA =5:2) to give the desired product (0.23g, 69% yield). ¹H NMR (400 MHz, CDCl₃): δ 7.35 (m, 5H), 5.10 (s, 2H), 4.97 (br, 1H), 4.11 (m, 1H), 2.97 (m, 2H), 2.47 (m, 2H).
***Step C: 3,3-Difluoro-cyclobutylamine (hydrochloride).*** A mixture of (3,3-Difluoro-cyclobutyl)-carbamic acid benzyl ester (1.47 g, 6.1 mmol) and 10% Pd/C (1 g) in MeOH (20 ml) was stirred overnight under H₂ atmosphere (1 atm) at room temperature. The resulting mixture was filtered through a pad of celite and washed with MeOH. The filtration combined with 2ml of conc.HCl and evaporated in vacuo to afford the desired product (0.81g, 85% yield). ¹H NMR (400 MHz, DMSO-d6): δ 8.60 (m, 3H), 3.64 (m, 1H), 2.89 (m, 4H).
***Step D: N-(3,3-Difluoro-cyclobutyl)-formamide.*** The title compound was synthesized via general procedure set forth in Example 20, step D. ¹H NMR (400 MHz, DMSO-d6): δ 8.53 (br, 1H), 8.01 (s, 1H), 4.11 (m, 1H), 2.96-2.87(m, 2H), 2.63-2.51 (m, 2H).
***Step E: 1,1-Difluoro-3-isocyano-cyclobutane.*** The title compound was synthesized via general procedure set forth in Example 20, step E and purified via chromatography eluted with Et₂O to give the desired product as an Et₂O solution, which was concentrated under 1atm pressure and used directly. ¹H NMR (400 MHz, DMSO-6): δ 4.28 (m, 1H), 3.19-3.12 (m, 2H), 2.96-2.91 (m, 2H).
**Example 23. Synthesis of (4-Fluoro-benzyloxy)-acetic acid.** The title compound was synthesized following the scheme below and used for synthesis of Compound 214 according to Scheme 1 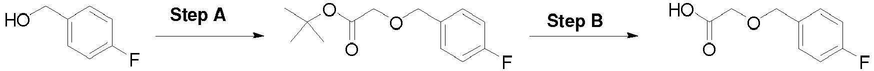
***Step A: (4-Fluoro-benzyloxy)-acetic acid tert-butyl ester.*** To a mixture of (4-Fluoro-phenyl)-methanol (0.6 g, 5.12 mmol) and Bu₄N⁺Cl⁻ (174 mg, 0.512 mmol) in toluene (100 ml) as added NaOH solution (50%, 100 ml) at 0°C. Bromo-acetic acid tert-butyl ester (2.0 g, 10.25 mmol) was added. The reaction mixture was warmed to room temperature and stirred overnight. The organic phase was separated, washed with water, brine, dried over Na2OS4, filtered and the solvent was evaporated in vacuo. The residue was purified via flash chromatography column eluted with PE/EtOAc (3/1) to give the (4-Fluoro-benzyloxy)-acetic acid tert-butyl ester as colorless oil (1.18 g, 96% yield) ¹H NMR (400 MHz, CDCl₃): δ 7.36-7.31 (m, 2H), 7.01 (t, 2H), 4.56 (s, 2H), 3.96 (s, 2H), 1.47 (s, 9H).
***Step B: (4-Fluoro-benzyloxy)-acetic acid.*** A solution of (4-Fluoro-benzyloxy)-acetic acid tert-butyl ester (1.1 g, 4.58 mmol) in TFA/DCM (1/1, 30 ml) was stirred for 3 hours at room temperature. The resulting mixture was evaporated in vacuo and the residue was washed with a mixture of EtOAc/hexane to give the (4-Fluoro-benzyloxy)-acetic acid as a solid, which was used directly without further purification (0.8 g, 94% yield).

The following carboxylic acid intermediates were synthesized via the general procedures set forth in this example.
(2-Fluoro-cyclohexyloxy)-acetic acid used for synthesis of Compound 235 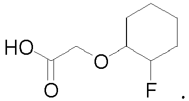 (Tetrahydro-pyran-4-yloxy)-acetic acid was used for synthesis of Compound 259 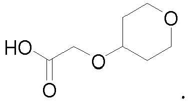 (S)-(Tetrahydro-furan-3-yloxy)-acetic acid was used for synthesis of Compound 251 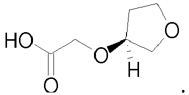 (2-Fluoro-benzyloxy)-acetic acid was used for synthesis of Compound 222 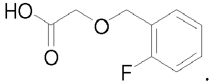 (Pyridin-4-ylmethoxy)-acetic acid was used for synthesis of Compound 229 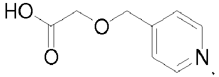 Cyclopentyloxy-acetic acid was used for synthesis of Compound 230 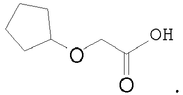 (Pyridin-3-ylmethoxy)-acetic acid was used for synthesis of Compound 233 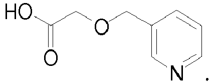 (3-Fluoro-benzyloxy)-acetic acid was used for synthesis of Compound 234 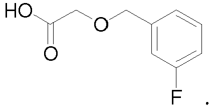 (Pyridin-2-ylmethoxy)-acetic acid was used for synthesis of Compound 281 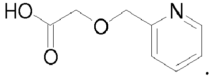 (Pyrazin-2-ylmethoxy)-acetic acid was used for synthesis of Compound 282 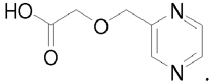 (4-Trifluoromethyl-pyridin-3-ylmethoxy)-acetic acid was used for synthesis of Compound 303 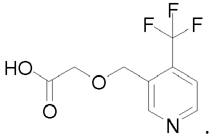 (6-Trifluoromethyl-pyridin-3-yloxy)-acetic acid was used for synthesis of Compound 274 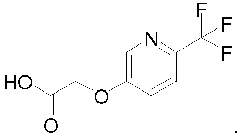 (Pyridazin-3-yloxy)-acetic acid was used for synthesis of Compound 273 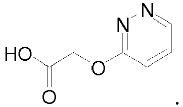 **Example 24. Synthesis of 3-Fluoro-pyridin-2-ylamino)-acetic acid.** The title compound was synthesized following the scheme below and used for the synthesis of Compound 361, Compound 342, Compound 333, Compound 301 and Compound 379. 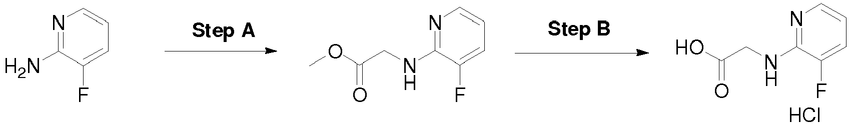
***Step A: (3-Fluoro-pyridin-2-ylamino)-acetic acid methyl ester.*** To a mixture of 40% glyoxal aqueous solution (1.5 ml) in MeOH (10 ml) was added a slurry of 3-Fluoro-pyridin-2-ylamine (1.17 g, 10.5 mmol) in HClO₄ (3 ml). The reaction mixture was heated to 70°C for 48 hours. The resulting mixture was adjusted to pH>8 with saturated NaHCO₃ solution after being cooled to room temperature. The basic solution was extracted with ethyl acetate (3x10 ml). The combined organic layer was washed with brine, dried over MgSO₄, filtered and the solvent was evaporated in vacuo. The residue was purified by flash column to give the (3-Fluoro-pyridin-2-ylamino)-acetic acid methyl ester as a white solid (600 mg, 31% yield). ¹H NMR (400 MHz, CDCl₃): δ 7.88 (d, 2H, J = 4.8), 7.17 (m, 1H), 6.59 (m, 1H), 5.12 (br, 1H), 4.26 (d, 2H, J = 6.4), 3.78 (s, 3H).
***Step B: (5-Fluoro-pyridin-2-ylamino)-acetic acid.*** A mixture of (3-Fluoro-pyridin-2-ylamino)-acetic acid methyl ester (280 mg, 1.5 mmol) in 5 N HCl (5 ml) was heated to reflux for 3 hours. The resulting mixture was evaporated in vacuo to give the (5-Fluoro-pyridin-2-ylamino)-acetic acid, which was used directly without further purification (300 mg, 97% yield). ¹H NMR (400 MHz, DMSO-d6): δ 7.82 (m, 2H), 7.68 (m, 1H), 6.75 (m, 1H), 4.13 (s, 2H).

The following carboxylic acid intermediates were synthesized via the general procedure set forth in this example
(5-Fluoro-pyridin-2-ylamino)-acetic acid (hydrochloride) was used for Compound 287 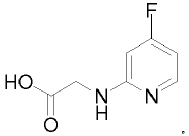 (Pyridin-2-ylamino)-acetic acid (hydrochloride) was used for Compound 316 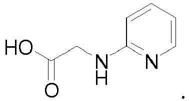 ¹H NMR (400 MHz, DMSO-d6): δ 13.65 (br, 2H), 8.94 (br, 1H), 7.94 (m, 2H), 7.17 (d, 1H, J = 7.2), 6.91 (t, 1H, J = 6.4).
**Example 25. Synthesis of 1H-Pyrrolo[2,3-b]pyridin-3-yl)-acetic acid (hydrochloride).** The title compound was synthesized following scheme below and used for synthesis of Compound 276, Compound 203 and Compound 213. 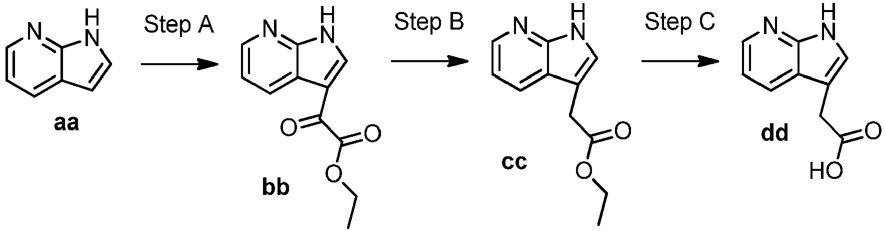
***Step A: Oxo-(1H-pyrrolo[2,3-b]pyridin-3-yl)-acetic acid ethyl ester (bb).*** To a solution of aluminum chloride (28.2 g, 0.212 mol) in DCM (50 ml) was added 7-azaindole (**aa**; 5.0 g, 0.042 mol) in one portion at room temperature (25°C) under N₂. After 1h at room temperature the resulting mixture was cooled to 0°C and a solution of chloro-oxo-acetic acid ethyl ester (28.9 g, 0.212 mol) in DCM (20 ml) was added dropwise for 1h. After stirring 30 min at the same temperature, the reaction was warmed to rt and stirred overnight. The reaction was cooled to 0°C and ethanol (100 ml) was added dropwise. After a period of 30 min at rt, the solvent was evaporated. DCM (250 ml) and saturated NaHCO₃ (300 ml) were added, the aqueous phase was extracted with DCM twice, the organics were combined, washed with brine, dried over Na₂SO₄, concentrated to give crude product. The crude product was washed with PE (20 ml), filtered and the filter cake was dried to give **bb** (2.1 g, 23% yield) as yellow solid. ¹H NMR (400 MHz, CDCl₃): δ 12.51 (s, 1H), 8.77-8.69 (m, 2H), 8.46-8.44 (m, 1H), 7.37-7.33 (m, 1H), 4.49-4.42 (q, 2H), 1.48-1.43 (t, 3H); MS: 219.0 (M+1)⁺.
***Step B: (1H-Pyrrolo[2,3-b]pyridin-3-yl)-acetic acid ethyl ester (cc).*** To a mixture of triethylsilane (2.0 g, 17.2 mmol) in TFA (20 ml) was added **bb** (1.0 g, 4.9 mmol) in one portion at room temperature. After a period of 16 h at 55°C, the solvent was removed and saturated NaHCO₃ was added, followed by DCM. The organic layer was collected, dried over Na₂SO₄ and concentrated to give **cc** (400 mg, 43% yield) as yellow solid without further purification for next step. ¹H NMR (400 MHz, CDCl₃): δ 12.81 (s, 1H), 8.42-8.37 (m, 2H), 7.45 (s, 1H), 7.37-7.32 (m, 1H), 4.18-4.16 (q, 2H), 3.81 (s, 2H), 1.28-1.24 (t, 3H); MS: 205.0 (M+1)⁺.
***Step C: (1H-Pyrrolo[2,3-b]pyridin-3-yl)-acetic acid (dd).*** A mixture of **cc** (0.4 g, 2.1 mmol) and LiOH·H₂O (0.35 g, 8.4 mmol) in THF/H₂O (10 ml, v/v=1:1) was stirred at rt for 1 h. 4 M HCl aq was added at 0°C until pH=5, the solvent was removed and the residue was washed with methanol, filtered and the organic layer was dried and concentrated to give crude **dd** (400 mg) without further purification for next step. ¹H NMR (400 MHz, DMSO-d6): δ 12.06 (s, 1H), 8.29-8.15 (m, 2H), 7.45-7.44 (m, 1H), 7.23-7.18 (m, 1H), 3.72 (s, 2H); MS: 177.1 (M+1)⁺.

The following carboxylic acid reagents were synthesized via the general procedure of this example
(1H-Pyrrolo[3,2-b]pyridin-3-yl)-acetic acid was used for synthesis of Compound 275 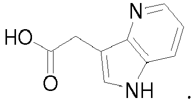 (1H-Pyrrolo[2,3-c]pyridin-3-yl)-acetic acid was used for synthesis of Compound 276 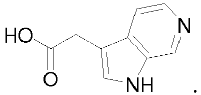 (1H-Pyrrolo[3,2-c]pyridin-3-yl)-acetic acid was used for synthesis of Compound 261 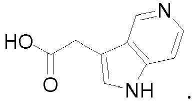 **Example 26. Synthesis of 2-Methyl-imidazol-1-yl)-acetic acid.** The title compound was synthesized following scheme below and used for synthesis of Compound 176 following Scheme 1. 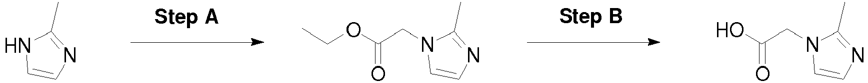
***Step A: (2-Methyl-imidazol-1-yl)-acetic acid ethyl ester.*** To a solution of 2-Methyl-1H-imidazole (20.52 g, 250 mmol) in THF (500 ml) was added K₂CO₃ (41.46 g, 300 mmol). The mixture was stirred at room temperature for 0.5 hour. Bromoacetic acid ethyl ester (27.6 ml, 250 mmol) was added and the mixture was stirred overnight at room temperature. The resulting mixture was filtered and the filtrate was evaporated in vacuo. The residue was purified via flash chromatography column eluted with PE/EtOAc (from 20/1 to 3/1) to give the (2-Methyl-imidazol-1-yl)-acetic acid ethyl ester as colorless oil (23.4 g, 56% yield). ¹H NMR (400 MHz, CDCl3): δ 6.93 (s, 1H), 6.83 (s, 1H), 4.58 (s, 2H), 4.25 (q, 2H, *J* = 6.8), 2.35 (s, 3H), 1.29 (t, 3H, *J* = 6.8).
***Step B: (2-Methyl-imidazol-1-yl)-acetic acid.*** A mixture of (2-Methyl-imidazol-1-yl)-acetic acid ethyl ester (23.4 g, 0.14 mol) and NaOH (12 g, 0.3 mol) in a mixture of H₂O (100 ml), THF (150 ml) and MeOH (150 ml) was stirred for 3 h at room temperature. The organic solvents were evaporated and the resulting aqueous solution was extracted with 10% MeOH/ DCM. The aqueous layer was acidified with conc. HCl to pH=4 and all solvent was evaporated. The residue was extracted with 40% MeOH/DCM and the solvent was evaporated in vacuo to give the (2-Methyl-imidazol-1-yl)-acetic acid as a white solid. ¹H NMR (400 MHz, DMSO-d6): δ 7.45 (d, 1H, *J* = 1.6), 7.39 (d, 1H, *J* = 1.6), 4.75 (s, 2H), 2.48 (s, 3H).
**Example 27. Synthesis of 5-Fluoro-pyridin-3-ylamino)-acetic acid.** The title compound was synthesized following scheme below and used for synthesis of Compound 310. 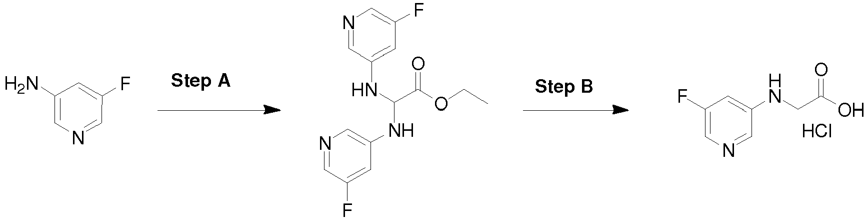
***Step A: Bis-(5-fluoro-pyridin-3-ylamino)-acetic acid ethyl ester.*** To a solution of 3-amino-5-fluropyridine (2.24 g, 20 mmol) in dry DMF (30 ml) was added Oxoacetic acid ethyl ester (2.04 g, 40 mmol) in toluene (30 ml). HCl/dioxane (3 M, 6.6 ml) was added dropwise below 15°C. The reaction mixture was stirred at 50°C for 70 hours and the solvent was removed under reduced pressure. The residue was neutralized with saturated aqueous NaHCO₃ to pH>8, extracted with DCM, purified through silica gel chromatography with MeOH/DCM (5%) to give the Bis-(5-fluoro-pyridin-3-ylamino)-acetic acid ethyl ester (1.0 g, 32% yield). ¹H NMR (400 MHz, DMSO-d6): δ 7.99 (s, 2H), 7.80 (s,2H), 7.18 (s, 2H, J = 7.2), 7.02 (d, 2H, J = 12), 5.63 (m, 1H), 4.20 (q, 2H, J = 6.8), 1.53 (t, 3H, J = 6.8).
***Step B: (5-Fluoro-pyridin-3-ylamino)-acetic acid (hydrochloride).*** A mixture of Bis-(5-fluoro-pyridin-3-ylamino)-acetic acid ethyl ester (1 g, 3.2 mmol) and Pd/C (5%, 0.9 g) in HCl (6N, 20 ml) was stirred overnight under H₂ atmosphere at room temperature. The resulting mixture was basified with 20% aqueous NaOH to pH>8 and extracted with ether. The aqueous phase was adjust to pH=4 and evaporated to dryness under reduced pressure. The residual solid was triturated in 20% MeOH/DCM, filtered and the filtrate was evaporated in vacuo to give the (5-Fluoro-pyridin-3-ylamino)-acetic acid (hydrochloride) which was used directly without further purification (0.5 g, 74% yield).
**Example 28. Synthesis of (1H-Indol-3-yl)-oxo-acetic acid.** The title compound was synthesized following scheme below and used for synthesis of Compound 262 following Scheme 1. 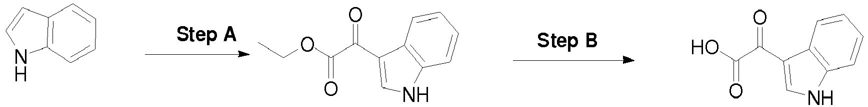
***Step A: (1H-Indol-3-yl)-oxo-acetic acid ethyl ester.*** The title compound was synthesized via general procedure set forth in Example 25, step A.
   ¹H NMR (400 MHz, DMSO-d6): δ 12.42 (br, 1H), 8.44 (s, 1H), 8.17 (d, 1H, J = 6.4), 7.57 (d, 1H, J = 6.4), 7.27 (m, 2H), 4.37 (q, 2H, J = 14, 5.1), 1.35 (t, 3H, J = 5.1).
***Step B: (1H-Indol-3-yl)-oxo-acetic acid.*** To a mixture of (1H-Indol-3-yl)-oxo-acetic acid ethyl ester (2.66 g, 12.2 mmol) in THF (300 ml) was added a solution of NaOH (1.0g, 24.2mmol) in water (20 ml). The reaction mixture was stirred for 2h at room temperature. The resulting mixture was concentrated in vacuo to remove most THF. The aqueous phase was acidified to PH=3 with conc.HCl and then the precipitate was collected by filtration, washed with water and dried to give the desired product (2.3 g, 100% yield). ¹H NMR (400 MHz, DMSO-d6): δ 13.86 (br, 1H), 12.36 (s, 1H), 8.42 (s, 1H), 8.17 (d, 1H, J = 6.4), 7.54 (d, 1H, J = 6.4), 7.27 (m, 2H), 4.37 (q, 2H, J = 14, 5.1), 1.35 (t, 3H, J = 5.1).
**Example 29. Synthesis of N-Pyridin-4-ylmethyl-oxalamic acid ethyl ester.** The title compound was synthesized following scheme below and used for synthesis of Compound 270 following Scheme 1. 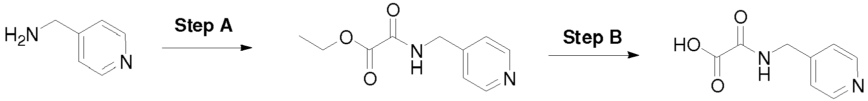
***Step A: N-Pyridin-4-ylmethyl-oxalamic acid ethyl ester.*** To a solution of 4-aminomethypyridine (7.5 g, 69.4 mmol) in dry THF (200 ml) was added ethyl chlorooxacetate (8.55 ml, 76.3 mmol) and Et3N (14.5 ml) at 0°C. The mixture was stirred for 3 hours at the same temperature and then concentrated. The residue was diluted with saturated aqueous NaHCO₃ (100 ml), extracted with EtOAc (3x45 ml) and the combined extracts were washed with brine, dried over Na2SO4 and filtered. The organic solvent was evaporated to dryness to give the N-Pyridin-4-ylmethyl-oxalamic acid ethyl ester as brown oil (12.6g, 87% yield). ¹H NMR (400 MHz, CDCl3): δ 8.57 m, 2H), 7.74 (br, 1H), 7.22 (m, 2H), 4.54 (d, 2H, J = 6.4, 14.4), 1.40 (t, 3H, J = 5.1).
***Step B: N-Pyridin-4-ylmethyl-oxalamic acid.*** The title compound was synthesized via general procedure set forth in Example 28, step B.
   ¹H NMR (400 MHz, DMSO-d6): δ 9.66 (s, 1H), 8.87 (m, 2H), 7.93 (m, 2H), 4.61 (d, 2H, J = 6).
**Example 30. Synthesis of 1-Methyl-1H-imidazol-2-yl)-oxo-acetic acid.** The title compound was synthesized following scheme below and used for synthesis of Compound 284 following Scheme 1. 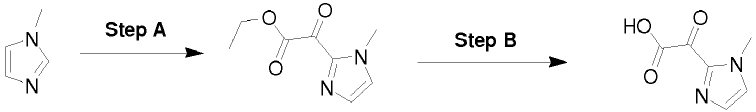
***Step A: (1-Methyl-1H-imidazol-2-yl)-oxo-acetic acid ethyl ester.*** To a solution of 1-methylimidazole (2.65g, 32.3mmol) in MeCN (30 ml) was added ethyl chlorooxacetate (4.41g, 32.3 mmol) dropwise at 0°C followed by Et3N (5.8 ml). The reaction mixture was stirred overnight and then filtered. The filtrate was evaporated to dryness, purified by flash chromatography eluted with PE/EtOAc (2/1) to give the pure (1-Methyl-1H-imidazol-2-yl)-oxoacetic acid ethyl ester (5.0 g, 85% yield). ¹H NMR (400 MHz, CDCl3): δ 7.31 (s, 1H), 7.17 (s, 1H), 4.47 (q, 2H, J= 6.8), 4.05 (s, 1H), 1.41 (t, 3H, J = 6.8)
***Step B: (1-Methyl-1H-imidazol-2-yl)-oxo-acetic acid.*** The title compound was synthesized via general procedure set forth in Example 28, step B
**Example 31. Synthesis of Oxo-(1H-pyrrolo[3,2-c]pyridin-3-yl)-acetic acid.** The title compound was synthesized following scheme below and used for synthesis of Compound 269 following Scheme 1.

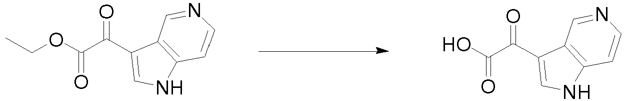

Oxo-(1H-pyrrolo[3,2-c]pyridin-3-yl)-acetic acid was synthesized via general procedure set forth in Example 25, step C. ¹H NMR (400 MHz, DMSO-d6): δ 13.71 (br, 1H), 9.53 (s, 1H), 9.00 (s, 1H), 8.63 (d, 1H, J = 6.4), 8.14 (d, 1H, J = 6.4).
**Example 32. Synthesis of 4,4-Difluoro-cyclohexylamine.** The title compound was synthesized following scheme below and used for synthesis of Compound 331, Compound 330, Compound 378 and Compound 373, all following Scheme 1. 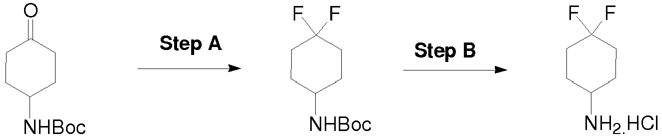
***Step A: (4, 4-Difluoro-cyclohexyl)-carbamic acid tert-butyl ester.*** To a solution of (4-Oxo-cyclohexyl)-carbamic acid tert-butyl ester (10 g, 47 mmol) in DCM (50 ml) was added DAST (12.8 g, 80 mmol) dropwise at 0°C. The reaction mixture was stirred overnight at room temperature. The resulting mixture was washed with NaHCO3 solution, brine, dried over Na2SO4, filtered and concentrated. The residue was re-crystallized with Et2O and PE to (4, 4-Difluoro-cyclohexyl)-carbamic acid tert-butyl ester as a solid (4.0 g, 32% yield). ¹H NMR (400 MHz, DMSO-d6): δ 6.91-6.89 (d, 1H), 3.44-3.43 (d, 1H), 1.99-1.74 (m, 6H), 1.49-1.36 (m, 11H).
***Step B: 4, 4-Difluoro-cyclohexylamine (hydrochloride).*** A mixture of (4, 4-Difluorocyclohexyl)-carbamic acid tert-butyl ester (4.0 g, 17 mmol) in Et2O/HCl (saturated, 50 ml) was stirred for 3 hours. The precipitate was collected by filtration and dried in vacuo to give the 4, 4-Difluoro-cyclohexylamine (hydrochloride) which was used directly without further purification (2.0 g, 67% yield). ¹H NMR (400 MHz, DMSO-d6): δ 8.30 (s, 3H), 3.19-3.18 (s, 1H), 2.06-1.85 (m, 6H), 1.65-1.59 (m, 2H).
**Example 33. Synthesis of 4-(1H-Tetrazol-5-yl)-phenylamine.** The title compound was synthesized following the scheme below and used for synthesis of Compound 285 via Scheme 1.

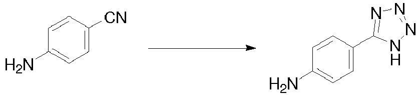

To a solution of 4-amino-benzonitrile (2.36 g, 20 mmol) in dry DMF (20 ml) was added NaN3 (1.6 g, 30 mmol) and NH4Cl (1.6 g, 30 mmol) and the reaction mixture was refluxed overnight. After cooling to room temperature, the resulting mixture was diluted with 40 ml of water and extracted with EtOAc (3x30 ml). The organic layer was washed with brine, dried over Na2SO4, filtered and evaporated to give the 4-(1H-Tetrazol-5-yl)-phenylamine (1.5 g, 54% yield). ¹H NMR (400 MHz, DMSO-d6): δ 16.26 (s, 1H), 7.70-7.68 (d, 2H, J = 8.4), 6.70-6.67 (d, 2H, J = 8.4), 5.79-5.76 (s, 2H).
**Example 34. Synthesis of 4-[1,3,4]Oxadiazol-2-yl-phenylamine.** The title compound was synthesized following the scheme below and used for synthesis of Compound 290 via Scheme 1 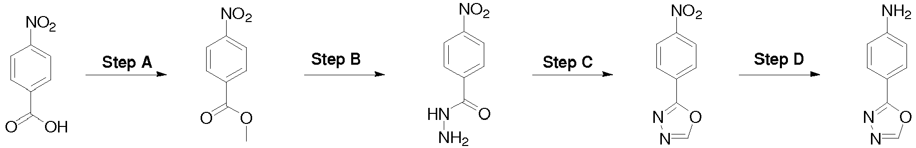
***Step A: 4-Nitro-benzoic acid methyl ester.*** To a solution of the p-nitrobenzoic acid (8 g, 50 mmol) in MeOH (100 ml) was added conc.H2SO4 (10 ml) dropwise. The reaction mixture was refluxed overnight and then concentrated under vacuum. The residue was dissolved in EtOAc (10 ml), washed with water , brine, dried with Na₂SO₄, filtered and the solvent was concentrated to give the 4-Nitro-benzoic acid methyl ester which was used directly without further purification (7.2 g, 84% yield).
***Step B: 4-Nitro-benzoic acid hydrazide.*** To a solution of 4-Nitro-benzoic acid methyl ester (3.6g, 20mmol) in MeOH (100 ml) was added Hydrazine hydrate (2.0 g, 40 mmol). The reaction mixture was stirred overnight at room temperature. The resulting mixture was evaporated under vacuum to give the 4-Nitro-benzoic acid hydrazide which was used directly without further purification (2.9 g, 81% yield).
***Step C: 2-(4-Nitro-phenyl)-[1,3,4]oxadiazole.*** A mixture of 4-Nitro-benzoic acid hydrazide (1.8 g, 10 mmol) in Orthoformic acid triethylester (30 ml) was refluxed overnight. The resulting mixture was concentrated under reduced pressure and the residue was washed with Et2O to give the pure 2-(4-Nitro-phenyl)-[1,3,4]oxadiazole (1.2 g, 78% yield). ¹H NMR (400 MHz, DMSO-d6): δ 8.60 (s, 1H), 8.42-8.40 (d, 2H, J =9.2), 8.32-8.30 (d, 2H, J = 8.8).
***Step D: For 4-[1,3,4]Oxadiazol-2-yl-phenylamine.*** A mixture of 2-(4-Nitro-phenyl)-[1,3,4]oxadiazole (1.2 g, 6 mmol) in MeOH (10 ml) was hydrogenated overnight under atmospheric pressure with 10% Pd/C (400 mg) as a catalyst at room temperature. The resulting mixture was filtered. The filtration was concentrated and purified by flash chromatography eluted with PE/EtOAc (from 30/1 to 2/1) to give the pure 4-[1,3,4]Oxadiazol-2-yl-phenylamine (0.8 g, 71% yield). ¹H NMR (400 MHz, DMSO-d6): δ 10.16 (s, 1H), 8.25-8.23 (d, 2H, J = 9.2), 7.97-7.95 (d, 2H, J = 8.8), 6.09 (s, 2H).
**Example 35. Synthesis of 4-[1,2,4]Oxadiazol-3-yl-phenylamine.** The title compound was synthesized following the scheme below and used for synthesis of Compound 291 via Scheme 1. 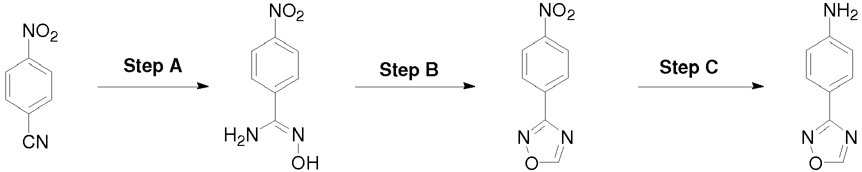
***Step A: N-Hydroxy-4-nitro-benzamidine.*** Hydroxylamine hydrochloride (18 g, 200 mol) and K₂CO₃ (5.53 g, 400 mmol) were added to a solution of 4-Nitro-benzonitrile (8 g, 55mmol) in EtOH (200 ml). The reaction mixture was refluxed overnight and the hot mixture was filtered. The filtrate was collected and concentrated in vacuo to provide N-Hydroxy-4-nitro-benzamidine which was used directly without purification (9.4 g, 87% yield).
***Step B: For 3-(4-Nitro-phenyl)-[1,2,4]oxadiazole.*** A mixture of N-Hydroxy-4-nitro-benzamidine (5.2 g, 30 mmol) in Orthoformic acid triethyl ester (50 ml) was refluxed overnight. The resulting mixture was concentrated in vacuo and the residue was washed with Et₂O to give the 3-(4-Nitro-phenyl)-[1,2,4]oxadiazole which was pure enough to be used directly (4.6 g, 92% yield). ¹H NMR (400 MHz, DMSO-d6): δ 8.87 (s, 1H), 8.39-8.32 (m, 4H).
***Step C: For 4-[1,2,4]Oxadiazol-3-yl-phenylamine.*** A mixture of 3-(4-Nitro-phenyl)-[1,2,4]oxadiazole (2.3 g, 16 mmol) in MeOH (20 ml) was hydrogenated overnight under atmospheric pressure with 10% Pd/C (400 mg) as a catalyst at room temperature. The resulting mixture was filtered. The filtered was concentrated and purified by flash chromatography eluted with PE/EtOAc (from 30/1 to 2/1) to give the pure 4-[1,2,4]Oxadiazol-3-yl-phenylamine (1.5 g, 77% yield). ¹H NMR (400 MHz, DMSO-d6): δ 9.13 (s, 1H), 7.68-7.66 (d, 2H, J = 8.4), 6.69-6.67(d, 2H, J = 8.4), 5.95 (s, 2H).
**Example 36. Synthesis of 3,4-Dihydro-2H-benzo[1,4]oxazine.** The title compound was synthesized following the scheme below and used for synthesis of Compound 202 via Scheme 1 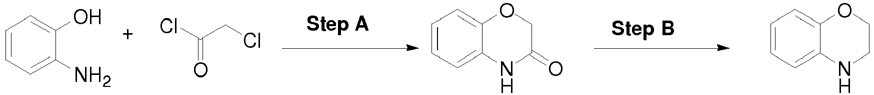
***Step A: For 4H-Benzo[1,4]oxazin-3-one.*** To a mixture of 2-aminophenol (5.45 g, 49.98 mmol), TEBA (11.4 g, 50.00 mmol) and NaHCO₃ (16.8 g, 200.00 mmol) in CHCl₃ (30 ml) was added a solution of 2-chloroacetyl chloride (8.16 g, 72.21 mmol) in CHCl₃ (5 ml) dropwise at 0°C. The reaction mixture was stirred for another 1 h at the same temperature and then heated to 55°C for 10 hours with stirring. The resulting mixture was concentrated under vacuum and then 50 ml of water was added. The precipitate was collected, purified by re-crystallization to give the 4H-Benzo[1,4]oxazin-3-one as a white solid (3.6 g , 48% yield). ¹H NMR (300MHz, DMSO-d6): δ 6.97-6.86 (m, 4H), 4.55 (s, 2H).
***Step B: 3,4-Dihydro-2H-benzo[1,4]oxazine.*** To a mixture of LAH (3.6 g, 94.74 mmol) in THF (80 ml) was added a solution of 4H-Benzo[1,4]oxazin-3-one (5.7 g, 38.22 mmol) in THF (21 ml) dropwise at room temperature. The reaction mixture was refluxed overnight. The resulting mixture was cooled to 0°C and then quenched by the adding 3.6 ml of H₂O, followed by 10.8 ml 15% NaOH solution. The precipitate was filtered off and the solvent was extracted with EtOAc (2x50 ml). The organic layer was washed with brine, dried over Na₂SO₄, filtered and concentrated to give the 3,4-dihydro-2H-benzo[b][1,4]oxazine as red oil which was pure enough to be used directly (1.5 g , 50% yield). ¹H NMR (300 MHz, DMSO-d6): δ 6.67-6.41 (m, 4H), 5.68 (s, 1H), 4.11-4.07 (m, 2H), 3.27-3.24 (m, 2H).
**Example 37. Synthesis of 3-(1-Methyl-1H-pyrazol-4-yl)-phenylamine.** The title compound was synthesized following the scheme below and used for synthesis of Compound 223 via Scheme 1. 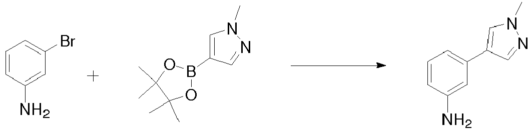 3-Bromo-phenylamine (0.83 g, 4.8mmol) was dissolved in 30 ml of dry toluene with stirring, and 15 ml of EtOH was added. Then a solution of Na₂CO₃ (3.3 g, 31.2 mmol) in water (15 ml) was added followed by 4-(4,5-Dimethyl-[1,3,2]dioxaborolan-2-yl)-1-methyl-1H-pyrazole (1.0 g, 4.8 mmol) and Pd(PPh₃)₄ (0.28 g, 0.24 mmol). The reaction mixture was heated to reflux with stirring overnight. The resulting mixture was cooled to room temperature, filtered and the solution was extracted with EtOAc (3x30 ml). The organic layer was washed with brine, dried over Na₂SO₄, filtered and concentrated. The residue was purified by flash gel chromatography eluted with PE/EtOAc (from 2/1 to 1/3) to give 3-(1-Methyl-1H-pyrazol-4-yl)-phenylamine as a white solid (0.56 g, 67% yield).
**Example 38. Synthesis of 6,7-Dihydro-5H-[1]pyrindin-6-ylamine.** The title compound was synthesized following the scheme below and used for synthesis of Compound 318 via Scheme 1. 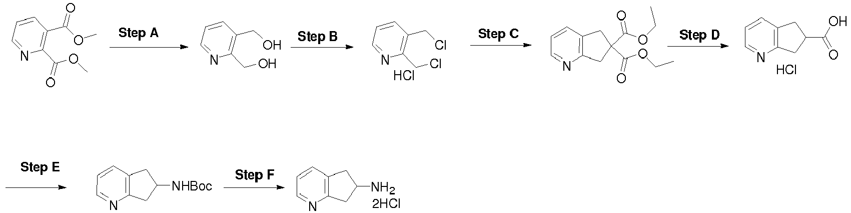
***Step A: For (3-Hydroxymethyl-pyridin-2-yl)-methanol.*** To a solution of pyridine-2,3-dicarboxylic acid dimethyl ester (35 g, 179 mmol) in EtOH (400 ml) was added NaHB4 (35 g, 921 mmol) portionwise. The reaction mixture was refluxed overnight and the resulting mixture was filtered and the filtrate was evaporated to give the crude product. The residue was purified by flash chromatography eluted with DCM/MeOH/Et3N (form 51/1/0.2 to 100/1/0.5) to give the pure (3-Hydroxymethyl-pyridin-2-yl)-methanol as brown oil (6 g, 24% yield). ¹H NMR (400 MHz, CDCl3): δ8.42(d, 1H, J = 4), 7.74(d, 1H, J = 7.6), 7.27-7.22(m, 1H), 4.75 (s, 2H), 4.66 (s, 2H), 4.19(br, 2H).
***Step B: 2,3-Bis-chloromethyl-pyridine.*** To a mixture of (3-Hydroxymethyl-pyridin-2-yl)-methanol (5.5 g, 43 mmol) in DCM (50 ml) was added SOCl2 (5 ml) at 0°C. The reaction was stirred for 2 hours at 75°C and then evaporated in vacuo to give the crude 2,3-Bis-chloromethyl-pyridine (hydrochloride) which was used directly without further purification (6 g, 71% yield). ¹H NMR (400 MHz, DMSO-d6): δ15.86 (br, 0.6H), 8.69(d, 1H), 7.69-7.66 (m, 1H), 5.05 (s, 2H), 5.02 (s, 2H).
***Step C: For 5,7-Dihydro-[1]pyrindine-6,6-dicarboxylic acid diethyl ester.*** To 100 ml of EtOH was added Na (1.6 g, 68 mmol) portionwise. After the solid was dissolved, Malonic acid diethyl ester (4.94 g, 30.86 mmol) was added, followed by a solution of 2,3-Bis-chloromethyl-pyridine (hydrochloride, 5.4 g, 30.86 mol) in EtOH (100 ml). The reaction mixture was refluxed overnight. The resulting mixture was concentrated and diluted with water (100 ml). The so-obtained mixture was extracted with EtOAc (3x30 ml) and the organic layer was washed with NaHCO3 solution, brine, dried over Na2SO4, filtered and the solvent was evaporated in vacuo. The residue was purified by flash chromatography eluted with (PE/EtOAc from 50/1 to 10/1) to give the pure 5,7-Dihydro-[1]pyrindine-6,6-dicarboxylic acid diethyl ester as colorless oil. (2.9 g, 35% yield). ¹H NMR (400 MHz, CDCl3): δ8.38 (d, 1H), 7.49 (d, 1H), 7.09-7.06 (m, 1H), 4.25-4.20 (q, 4H), 3.70 (s, 2H), 3.60 (s, 2H), 1.27 (t, 3H).
***Step D: For 6,7-Dihydro-5H-[1]pyrindine-6-carboxylic acid.*** A mixture of 5,7-Dihydro-[1]pyrindine-6,6-dicarboxylic acid diethyl ester (2 g, 7.6 mmol) in conc. HCl (200 ml) was refluxed for 2 hours and then evaporated in vacuo to give the crude 6,7-Dihydro-5H-[1]pyrindine-6-carboxylic acid (hydrochloride) as a black solid which was used directly without further purification 1.6 g, 100% yield). ¹H NMR (400 MHz, DMSO-d6): δ8.64 (d, 1H), 834(d, 1H), 7.76 (m, 1H), 3.55-3.28 (m, 5H).
***Step E: For (6,7-Dihydro-5H-[1]pyrindin-6-yl)-carbamic acid tert-butyl ester.*** To a solution of crude 6,7-Dihydro-5H-[1]pyrindine-6-carboxylic acid (hydrochloride, 0.66 g, 3.32 mmol), Et3N (1.7 g, 16.6 mmol) and t-BuOH (15 ml) in dioxane (15ml) was added DPPA (1.05 g, 4.32 mmol) dropwise. The reaction mixture was heated to 100°C and stirred overnight. The resulting mixture was concentrated and dissolved in EtOAc (50 ml). The organic layer was washed with NaHCO3, brine, dried over Na2SO4, filtered and the solvent was evaporated in vacuo. The residue was purified by flash chromatography eluted with (PE/EtOAc 5/1) to give the (6,7-Dihydro-5H-[1]pyrindin-6-yl)-carbamic acid tert-butyl ester (0.35 g, 35% yield). ¹H NMR (400 MHz, DMSO-d6): δ8.28 (d, 1H), 7.56(d, 1H), 7.23 (d, 1H), 7.11 (q, 1H), 4.24 (m, 1H), 3.19-3.10 (m, 2 H), 2.86-2.75 (m, 2H), 1.39 (s, 9H).
***Step F: For 6,7-Dihydro-5H-[1]pyrindin-6-ylamine.*** A mixture of (6,7-Dihydro-5H-[1]pyrindin-6-yl)-carbamic acid tert-butyl ester (0.2 g, 0.85 mmol) in HCl/Et2O (3 M, 5 ml) was stirred overnight at room temperature, and then evaporated in vacuo to give the 6,7-Dihydro-5H-[1]pyrindin-6-ylamine (hydrochloride) as a solid (0.16 g, 100% yield). ¹H NMR (400 MHz, DMSO-d6): δ8.65 (d, 1H), 8.36(m, 1H), 7.783 (m, 1H), 3.66-3.26 (m, 5H).
**Example 39. Synthesis of 2-(1H-Indol-3-yl)-propionic acid.** The title compound was synthesized following scheme below and used for synthesis of Compound 283 via Scheme 1. 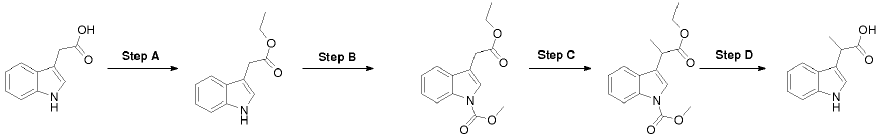
***Step A: For (1H-Indol-3-yl)-acetic acid ethyl ester.*** To a solution of (1H-Indol-3-yl)-acetic acid (5.0 g, 28.6 mmol) in EtOH (50 ml) was added SOCl2 (6.1 g, 51.4 mmol) dropwise at room temperature. The reaction mixture was refluxed overnight. The solution was cooled to room temperature and the solvent was removed to give (1H-Indol-3-yl)-acetic acid ethyl ester as brown solid (5.5 g, 95% yield). ¹H NMR (400 MHz, CDCl₃): δ 8.10 (s, 1H), 7.63-7.61 (d, 1H, *J*=8 Hz), 7.34-7.32 (d, 1H, *J*=8 Hz), 7.21-7.11 (m, 3H), 4.19-4.14 (q, 2H, *J*=7.2 Hz), 3.76 (s, 2H), 1.28-1.24 (t, 2H, *J* = 7.2); MS: 204.1 (M+1)⁺.
***Step B: 3-Ethoxycarbonylmethyl-indole-1-carboxylic acid methyl ester.*** To a solution of (1H-Indol-3-yl)-acetic acid ethyl ester (5.5 g, 27.1mmol) and TBAI (0.08g, 0.2mmol) in a mixture of 30% NaOH (80 ml) and DCM (80 ml) was added methyl chloroformate (3.8 g, 40.6mmol) at - 4°C for 15 minutes. The reaction was stirred at 0°C for 2 h. The two layer mixture was separated and the aqueous layer was extracted one time with DCM. The combined DCM layer was washed with brine and concentrated in vacuo, purified by flash chromatography eluted with PE/EtOAc (form 20/1 to 15/1) to give the 3-Ethoxycarbonylmethyl-indole-1-carboxylic acid methyl ester as a solid (5.0 g, 71% yield). ¹H NMR (400 MHz, CDCl₃): δ 8.18-8.16 (m, 1H), 7.60-7.53 (m, 2H), 7.37-7.25 (m, 2H), 4.20-4.15 (q, 2H, *J* = 6.8), 4.02 (s, 3H), 3.70 (s, 2H), 1.28-1.24 (t, 2H, *J*=7.2 Hz); MS: 262.1 (M+1)⁺.
***Step C: 3-(1-Ethoxycarbonyl-ethyl)-indole-1-carboxylic acid methyl ester.*** To a solution of 3-Ethoxycarbonylmethyl-indole-1-carboxylic acid methyl ester (2.0g, 7.7 mmol) in dry THF (10 ml) was added LDA (15 ml, in THF, 11.5 mmol) dropwise at -78°C for 30 min under N₂. Then the solution was stirred at -78°C for another 1 h, a solution of Iodomethane (1.6 g, 11.5mmol) in dry THF (5 ml) was added dropwise at -78°C. After stirring at -78°C for 1.5 h, the reaction was quenched with saturated NH₄Cl solution at room temperature, extracted with EtOAc (2x30 ml). The organic layer was washed with brine, dried over Na₂SO₄, filtered and concentrated in vacuo. The residue was purified by flash chromatography eluted with PE/EtOAc (20/1) to give the 3-(1-Ethoxycarbonyl-ethyl)-indole-1-carboxylic acid methyl ester as a white solid (0.4 g, 19% yield). ¹H NMR (400 MHz, CDCl₃): δ 8.18-8.17 (m, 1H), 7.62-7.55 (m, 2H₀, 7.36-7.24 (m, 2H), 4.18-4.11 (m, 2H), 4.03 (s, 3H), 3.96-3.91 (m, 1H), 1.61-1.59 (d, 3H), 1.24-1.20 (t, 2H, *J* = 7.2); MS: 276.1 (M+1)⁺.
***Step D: 2-(1H-Indol-3-yl)-propionic acid.*** A solution of KOH (575 mg, 8.7 mmol) in water (10 ml) was added to a solution of 3-(1-Ethoxycarbonyl-ethyl)-indole-1-carboxylic acid methyl ester (400 mg, 1.45 mmol) in methanol (40 ml) at room temperature. The mixture was stirred at 70°C for 1 h and concentrated. The residual oil was adjusted to pH=1 with aq.HCl (1 M) and the precipitate was filtered off. The water phase was extracted with EtOAc (2x, 30 ml) and the organic layer was washed with brine, dried over Na₂SO₄, filleted and concentrated to give 2-(1H-Indol-3-yl)-propionic acid as clear oil (250 mg, 89% yield). ¹H NMR (400 MHz, DMSO-d6): δ 12.12 (s, 1H), 10.93 (s, 1H), 7.56-7.55 (d, 1H, *J*=7.6 Hz), 7.35-7.33 (d, 1H, *J* = 8.0), 7.21-7.20 (d, 1H, *J*=2.4 Hz), 7.08-7.05 (t, 1H, *J* = 6.8, *J* = 8.0), 6.99-6.95 (t, 1H, *J*=7.2, *J* = 7.6), 3.87-3.85 (m, 1H), 1.47-1.45 (d, 3H, *J* = 7.2); MS: 190.1 (M+1)⁺.
**Example 40. Synthesis of Indol-1-yl-acetic acid.** The title compound was synthesized following scheme below and used for synthesis of Compound 271 via Scheme 1. 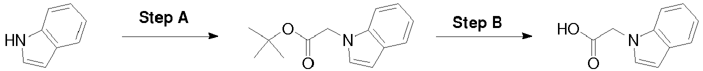
***Step A: Indol-1-yl-acetic acid tert-butyl ester.*** Indol-1-yl-acetic acid tert-butyl ester was synthesized via general procedure 19 (step A), except for the alcohol was replaced by indole. ¹H NMR (400 MHz, CDCl₃): δ 7.631 (d, 1H, *J* = 8), 7.25-7.21 (m, 2H), 7.13-7.08 (m, 2H), 6.55 (d, 1H, *J* = 3.2), 4.74 (s, 2H), 1.43 (s, 9H).
***Step B: Indol-1-yl-acetic acid.*** To a stirred of indol-1-ylacetic acid tert-butyl ester (2 g, 8.6 mmol) in MeOH (12 ml) was added KOH (4 g, 71.4 mmol) and water (0.4 ml). The reaction mixture was stirred at room temperature for 16 hours, and then diluted with water (100 ml). The resulting mixture was extracted with Et₂O (25 ml) and the organic layer was discarded. The aqueous phase was acidified to pH 3-4 with HCl (6 N) and extracted with Et₂O (3x15 ml). The combined organic layer was dried over Na₂SO₄, filtered and concentrated to produce indol-1-ylacetic acid, which was used directly without further purification (1.2g, 79.7% yield).
**Example 41. Synthesis of Benzenesulfonylamino-acetic acid.** The title compound was synthesized following scheme below and used for synthesis of Compound 10, Compound 28 and Compound 29 via Scheme 1.

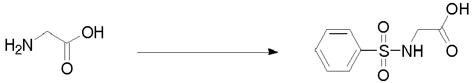

A mixture of glycine (7.51 g, 100 mmol) and benzenesulfonyl chloride (12.9 ml, 100 mmol) in NaOH solution (1 M, 272 ml, 272 mmol) was heated to 70°C for 2 hours. The resulting mixture was cooled to 5°C and then adjust to pH=6.5. The precipitate was collected by filtration and dried in vacuo to give the pure Benzenesulfonylamino-acetic acid (10.5 g, 48% yield). ¹H NMR (300 MHz, H₂O): δ 7.78 (d, 2H,), 7.62-7.53 (m, 3H), 3.69 (s, 2H).
**Example 42. Synthesis of (4-Cyano-phenylamino)-acetic acid.** The title compound was synthesized following scheme below and used for synthesis of Compound 227 and Compound 228 via Scheme 1.

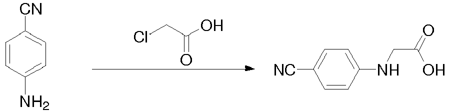

A suspension of 4-Amino-benzonitrile (1.0 g, 8.5mmol) and chloro-acetic acid (1.6 g, 16.9 mmol) in water (30 ml) was refluxed for 4 h. The resulting mixture was cooled to room temperature. The precipitate was collected by filtration and washed with EtOAc to give the pure (4-Cyano-phenylamino)-acetic acid as a white solid (300 mg, 20% yield). ¹H NMR (400 MHz, DMSO-d6): δ 12.73 (s, 1H), 7.47-7.45 (d, 2H, *J*=8.8 Hz), 6.92 (m, 1H), 6.65-6.63 (d, 2H, *J* = 8.8), 3.91-3.89 (d, 2H, *J* = 6.0); MS: 177.1 (M+1)⁺.
**Example 43. Synthesis of [1,2,3]Triazol-1-yl-acetic acid.** The title compound was synthesized following the scheme below and used for synthesis of Compound 329 via Scheme 1. 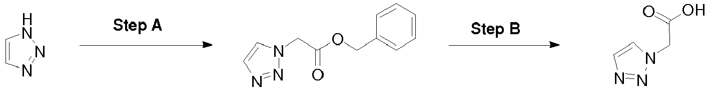
***Step A: [1,2,3]Triazol-1-yl-acetic acid benzyl ester.*** A mixture of 1H-[1,2,3]Triazole (2.07 g, 30 mmol), CbzCl (6.9 g, 30 mmol) and DIEA (5.1 ml, 30 mmol) in DCM (40 ml) was stirred overnight at room temperature. 150 ml of Et₂O was added. The precipitate was filtered off and the filtrate was concentrated. The residue was purified via flash chromatography column eluted with DCM/PE (19/1) to give the pure [1,2,3]Triazol-1-yl-acetic acid benzyl ester (1 g, 32% yield). ¹H NMR (400 MHz, DMSO-d6): δ 8.16 (s, 1H), 7.77 (s, 1H), 7.40-7.35 (m, 5H), 5.54-5.50 (s, 2H), 5.29-5.10 (d, 2H).
***Step B: [1,2,3]Triazol-1-yl-acetic acid.*** A mixture of [1,2,3]Triazol-1-yl-acetic acid benzyl ester (1 g, 4.6 mmol) in MeOH was hydrogenated overnight under 50 psi pressure with PdOH/C (20%, 92 mg) as a catalyst. The catalyst was filtered off and the solvent was concentrated under vacuum to give the crude [1,2,3]Triazol-1-yl-acetic acid as a solid which was used directly without further purification (560 mg, 95% yield). ¹H NMR (400 MHz, DMSO-d6): δ 13.37 (s, 1H), 8.13-8.11 (m, 1H), 7.77-7.74 (d, 1H), 5.31-5.23 (d, 2H).
**Example 44. Synthesis of Benzotriazol-1-yl-acetic acid.** The title compound was synthesized following the scheme below and used for synthesis of Compound 205, Compound 5, Compound 157 and Compound 151 via Scheme 1.

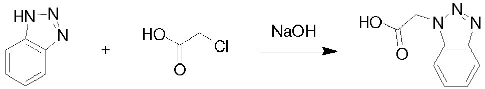

To a solution of chloroacetic acid (2.37 g, 25 mmol) and NaOH (2.0 g, 50 mmol) in H₂O was added benzotriazole (3.0 g, 25 mmol) in one portion. The reaction mixture was stirred for 30 minutes at room temperature and then heated to reflux for 2 hours. The resulting mixture was cooled to 0°C, adjust to pH=3 with HCl (0.5 M). The precipitate was collected by filtration, washed with water and dried in vacuo to give the Benzotriazol-1-yl-acetic acid which was pure enough to be used directly (3.1 g, 70% yield). ¹H NMR (300 MHz, DMSO-d6): δ 8.02 (d, 2H, J = 8.1), 7.74 (d, 1H, J = 8.1), 7.50-7.36 (m, 2H), 5.35 (s, 3H).

### Example 45. Preparation of Compound 386.

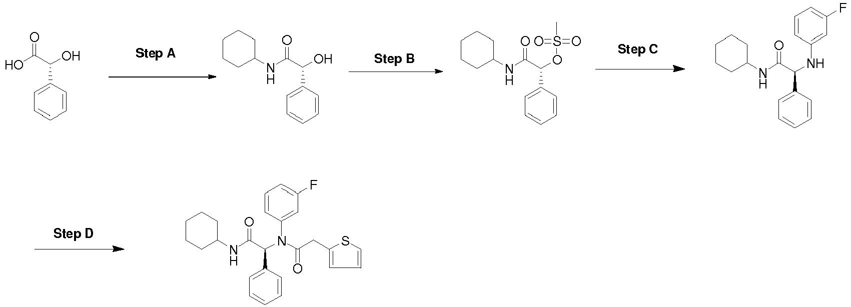

***Step A: (R)-N-Cyclohexyl-2-hydroxy-2-phenyl-acetamide.*** To a stirred solution of D-Mandelic acid (34 g, 223.68 mmol) in DMF (200 ml) was added HOBT (45.2g, 335.5 mmol), EDCI (68.4 g, 357.9 mmol) at 0°C. Cyclohexylamine (88 g, 894.7 mmol) was added slowly. The reaction mixture was stirred overnight at room temperature. Water (500 ml) was added to the reaction mixture below 5°C. The resulting mixture was extracted with ethyl acetate (2x1.5 L) and the combined organic layer was washed with brine, dried over Na₂SO₄, filtered and the solvent was evaporated in vacuo. The residue was purified via column chromatography to give the (R)-N-Cyclohexyl-2-hydroxy-2-phenyl-acetamide (38 g, 73.1% yield, ee%=100%). ¹H NMR (300 MHz, DMSO-d6): δ7.69-7.67 (m, 1H), 7.40-7.25 (m, 5H), 6.07-6.05 (m, 1H), 4.87-4.86 (m, 1H), 3.32 (s, 1H), 1.67-1.53 (m, 5H), 1.26-1.21 (m, 5H); MS: 234.2 (M+1)⁺.
***Step B: (R)-Methanesulfonic acid cyclohexylcarbamoyl-phenyl-methyl ester.*** To a solution of (R)-N-Cyclohexyl-2-hydroxy-2-phenyl-acetamide (38 g, 163 mmol) in pyridine (100 ml) was added MsCl (20.5 g, 179 mmol) dropwise at 0°C. The reaction mixture was stirred for another 1.5 hours at the same temperature and was then concentrated under vacuum. The residue was dissolved in EtOAc (200 ml), washed with water, brine, dried over Na₂SO₄, filtered and the solvent was evaporated in vacuo to give the (*R*)-Methanesulfonic acid cyclohexylcarbamoyl-phenyl-methyl ester which was used directly without further purification (20 g, 39.4% yield). ¹H NMR (300 MHz, DMSO-d6): δ 8.30-8.28 (m, 1H), 7.54-7.36 (m, 5H), 5.87 (s, 1H), 3.54 (s, 1H), 3.18 (s,3H),1.76-1.52 (m, 5H), 1.26-1.09 (m, 5H); MS: 312.1 (M+1)⁺.
***Step C: (S)-N-Cyclohexyl-2-(3-fluoro-phenylamino)-2-phenyl-acetamide.*** A mixture of *(R)-*Methanesulfonic acid cyclohexylcarbamoyl-phenyl-methyl ester (20 g, 64.3 mmol), DIEA (24.8 g, 192.9 mmol) and 3-fluoro-phenylamine (7.13 g, 64.3 mmol) in DMF (80 ml) was heated to 80°C for 4 hours. The resulting mixture was cooled to room temperature and water was (150 ml) was added. This mixture was extracted with EtOAc (2x200 ml). The combined organic layer was washed with water, brine, dried over Na₂SO₄, filtered and the solvent was evaporated in vacuo. The residue was purified via flash chromatography column eluted with DCM/ MeOH (from 20/1 to 1/1) to give the (*S*)⁻N-Cyclohexyl-2-(3-fluoro-phenylamino)-2-phenyl-acetamide (6 g, 28.6% yield, ee%=100%). ¹H NMR (300 MHz, DMSO-d6): δ 8.27-8.13 (m, 1H), 7.51-7.00 (m, 6H), 6.50-6.27 (m,3H), 4.98 (s, 1H), 3.55 (s, 1H), 1.76-1.50 (m, 5H), 1.27-1.03 (m, 5H); MS: 327.1 (M+1)⁺.
***Step D: Compound 386.*** To a mixture of (*S*)-N-Cyclohexyl-2-(3-fluoro-phenylamino)-2-phenyl-acetamide (120 mg, 0.37 mmol) and NaHCO₃ (154 mg, 1.84 mmol) in THF (6 ml) was added 2-(thiophen-2-yl)acetyl chloride (236 mg, 1.48 mmol) dropwise at 0°C. The reaction mixture was warmed to room temperature and stirred overnight. Water (20 ml) was added and the resulting mixture was extracted with DCM (3x10 ml). The combined organic layer was washed with saturated NaHCO₃ solution, brine, dried over Na₂SO₄, filtered and the solvent was evaporated in vacuo. The residue was purified by prep-HPLC to give the desired product (35 mg, 21% yield, ee% = 99%). ¹H NMR (300 MHz, DMSO-d6): δ 8.03-8.00 (d, 1H), 7.35-7.33 (d, 1H), 7.14-6.72 (m, 10H), 6.07 (s, 1H), 3.59-3.56 (m, 3H), 1.70-1.55 (m, 5H), 1.30-0.97 (m, 5H); MS: 451.2 (M+1)⁺.

### Example 46: Preparation of Compounds 387-389.

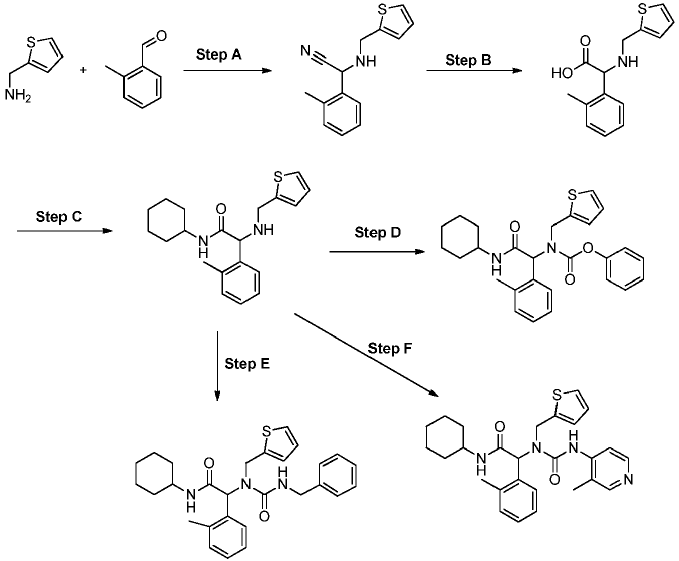

***Step A: [(Thiophen-2-ylmethyl)-amino]-o-tolyl-acetonitrile.*** [(Thiophen-2-ylmethyl)-amino]-o-tolyl-acetonitrile was synthesized via a procedure similar to that described in Example 7, step A.
***Step B: [(Thiophen-2-ylmethyl)-amino]-o-tolyl-acetic acid.*** [(Thiophen-2-ylmethyl)-amino]-o-tolyl-acetic acid was synthesized via a procedure similar to that described in Example 7, step B. ¹H NMR (300 MHz, DMSO-d₆): δ 7.34-7.31 (m, 2H), 7.08-7.01 (m, 3H), 9.94-6.85 (m, 2H), 4.10 (s, 1H), 3.80-3.64 (m, 2H), 3.61-3.60 (m, 1H), 2.31(s, 1H).
***Step C: N-Cyclohexyl-2-[(thiophen-2-ylmethyl)-amino]-2-o-tolyl-acetamide.*** N-Cyclohexyl-2-[(thiophen-2-ylmethyl)-amino]-2-o-tolyl-acetamide was synthesized via a procedure similar to that described in Example 7, step C. ¹H NMR (300 MHz, DMSO-d₆): δ 7.84-7.8 2(d, 1H, J = 10.8), 7.41-7.40 (d, 1H, J = 1.2), 7.30-7.27 (m, 3H), 6.95-6.92 (m, 2H), 4.32-4.30 (d, 1H, J = 8.7), 3.85-3.82 (m, 2H), 3.61-3.58 (m, 1H), 2.88-2.85 (m, 1H), 2.26(s, 3H), 1.77-1.52 (m, 5H), 1.30-1.10 (m, 5H).
***Step D: Compound 387.***

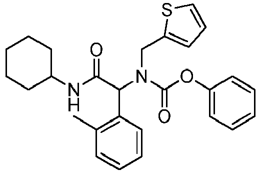

To a mixture of N-cyclohexyl-2-[(thiophen-2-ylmethyl)-amino]-2-o-tolyl-acetamide (170 mg, 0.5 mmol) in dioxane (5 ml) was added NaHCO3 (294 mg, 3.5 mmol) and phenylchloroformate (156 mg, 1 mmol). The reaction mixture was refluxed overnight and then quenched with water (20 ml) after being cooling to room temperature. The resulting mixture was extracted with DCM (3X15 ml). The combined organic layers were washed with brine, dried over Na₂SO₄, filtered and the solvent was evaporated in vacuo. The residue was purified by TLC (PE/EtOAc = 8/1) to give the desired product (133 mg, 66% yield). ¹H NMR (400 MHz, DMSO-d₆): δ 8.10-8.08 (d, 1H, J = 7.2), 7.46-7.43 (m, 2H), 7.30-7.20 (m, 5H), 7.15-7.10 (m, H), 6.65-6.63 (m, 1H), 5.97-5.92 (m, 2H), 4.92-4.56 (m, 2H), 3.44-3.34 (m, 1H), 2.11-2.03 (m, 3H), 1.78-1.54 (m, 5H), 1.30-1.56 (m, 5H); MS: 463.2 (M+1)⁺.

### Step E: Compound 388.

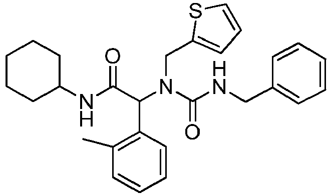

A mixture of N-cyclohexyl-2-[(thiophen-2-ylmethyl)-amino]-2-o-tolyl-acetamide (100 mg, 0.29 mmol) and isocyanatomethyl-benzene (69.6 mg, 0.58 mmol) in DMF (2 ml) was stirred overnight at room temperature. The precipitate was collected by filtration and washed with ether to give the desired product as white solid (63 mg, 46.8% yield). ¹H NMR (400 MHz, DMSO-d₆): δ 7.80 (d, 1H), 7.26-7.07 (m, 10H), 6.76-6.67 (m, 2H), 6.40 (d, 1H), 6.01 (s, 1H), 4.80 (d, 1H), 4.45 (d, 1H), 4.40 (m, 1H), 4.20 (m, 1H), 3.58 (m, 1H), 2.18 (s, 3H), 1.75-1.52 (m, 5H), 1.27-0.98 (m, 5H); MS: 476.2 (M+1)⁺.

### Step F: Compound 389.

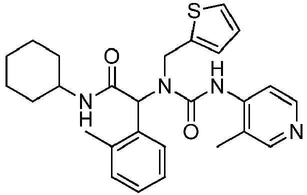

A mixture of N-cyclohexyl-2-[(thiophen-2-ylmethyl)-amino]-2-o-tolyl-acetamide (86 mg, 0.25 mmol), (3-methyl-pyridin-4-yl)-carbamic acid phenyl ester (114 mg, 0.5 mmol) and DMAP (39 mg, 0.32 mmol) in MeCN (4 ml) was heated to 60 for 10 min and then cooled to room temperature. The precipitate was collected by filtration to give the pure product (52 mg, 43% yield). ¹H NMR (300 MHz, DMSO-d₆): δ 8.19-8.03 (m, 4H), 7.65 (d, 1H, *J*=5.7 Hz), 7.32 (dd, 1H, *J* = 4.7, 1.4), 7.24-7.15 (m, 4H), 6.85-6.82 (m, 2H), 5.99 (s, 1H), 5.16 (d, 1H, *J* = 17.1), 4.57 (d, 1H, *J* = 16.8), 3.64-3.61 (m, 1H), 2.30 (s, 3H), 1.84 (s, 3H), 1.79-1.52 (m, 5H), 1.29-1.04 (m, 5H); MS: 477.2 (M+1)⁺

The following compounds were synthesized from via procedures similar to those described in Example 46.

### Compound 390

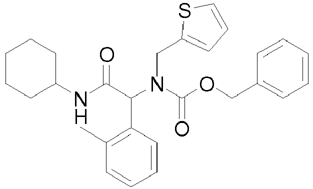

¹H NMR (400 MHz, DMSO-d₆): δ 8.22-8.00 (d, 1H, J = 7.2), 7.35-7.31 (m, 5H), 7.26-7.05 (m, 5H), 6.60 (s, 1H), 5.89-5.82 (m, 2H), 5.27-5.16 (m, 2H), 4.77-4.30 (m, 2H), 3.64-3.61 (m, 1H), 2.03-1.96 (m, 3H, J = 27.2), 1.76-1.53 (m, 5H), 1.29-1.10 (m, 5H); MS: 477.2 (M+1)⁺.

### Compound 391

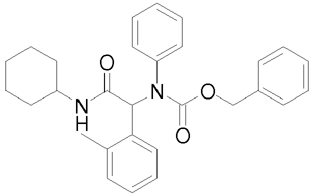

¹H NMR (400 MHz, CDCl₃): δ 7.30-6.88 (m, 14H), 6.02 (s, 1H), 5.34 (d, 1H), 5.19 (m, 2H), 3.86 (m, 1H), 2.35 (s, 3H), 1.93-1.25 (m, 5H), 1.13-0.91 (m, 5H); MS: 457.2 (M+1)⁺.

### Compound 392

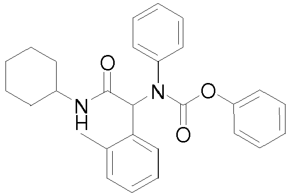

¹H NMR (400 MHz, DMSO-d₆): δ 8.02 (d, 1H), 7.40-7.33 (m, 4H), 7.23-7.00 (m, 8H), 6.89-6.81 (m, 2H), 6.06(s, 1H), 2.45 (s, 3H), 1.74-1.52 (m, 5H), 1.29-0.98 (m, 5H); MS: 443.2 (M+1)⁺.

### Example 47: Preparation of Compound 393

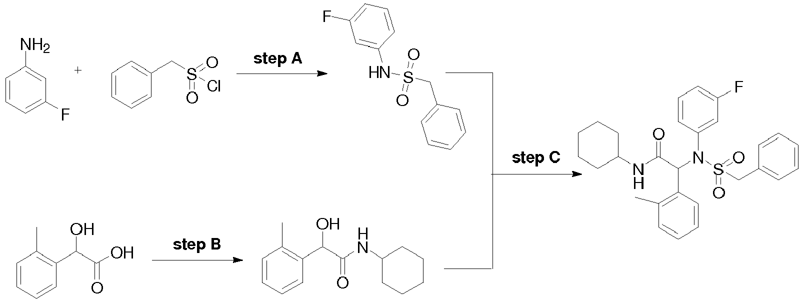

***Step A: N-(3-Fluoro-phenyl)-C-phenyl-methanesulfonamide.*** To a solution of 3-Fluoro-phenylamine (1.15 g, 10.4 mmol) and TEA (1.6 g, 31.2 mmol) in DCM (10 ml) was added Phenyl-methanesulfonyl chloride (1 g, 7 mmol) dropwise at 0°C. The reaction mixture was stirred overnight at room temperature, concentrated and purified by chromatography to get the desired product (1 g, 36% yield). ¹H NMR (400 MHz, CDCl₃): δ 7.39-7.23 (m, 6H), 6.94-6.82 (m, 3H), 6.61 (brs, 1H), 4.35 (s, 2H).
***Step B: N-Cyclohexyl-2-hydroxy-2-o-tolyl-acetamide.*** To a stirred solution of hydroxy-o-tolyl-acetic acid (500 mg, 3 mmol) in DMF (5 ml) was added HOBt (610 mg, 4.5 mmol), EDCI (922 mg, 4.8 mmol) at 0°C. Cyclohexylamine (1.2 g, 12 mmol) was added slowly. The reaction mixture was stirred overnight at room temperature and then poured into 20 ml of ice-water. The precipitate was collected by filtration, dried and triturated with ether to get the desired product (300 mg, 40% yield).

### Step C: Compound 393

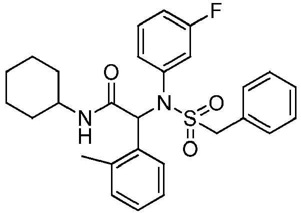

To a solution of triphenylphosphine (110 mg, 0.42 mmol) in THF (6 ml) was added DIAD (85 mg, 0.42 mmol) dropwise at 0°C. After a slurry forms, a solution of N-cyclohexyl-2-hydroxy-2-o-tolyl-acetamide (111 mg, 0.42 mmol) in THF (2 ml) was added, followed by a solution of N-(3-fluoro-phenyl)-C-phenyl-methanesulfonamide (62 mg, 0.42 mmol) in THF (2 ml). The reaction mixture was allowed to warm to room temperature and stirred overnight. The resulting mixture was concentrated and purified by chromatography to get the desired product (65 mg, 31% yield). ¹H NMR (400 MHz, CDCl₃): δ 7.38-7.05 (m, 10H), 6.89-6.85 (m, 2H), 6.70 (d, 1H), 6.28 (s, 1H), 5.26 (d, 1H), 4.90 (d, 1H), 4.42 (d, 1H), 3.89 (m, 1H), 2.49 (s, 3H), 2.04-1.55 (m, 5H), 1.42-1.03 (m, 5H); MS: 495.2 (M+1)⁺.

### Example 48: In Vitro Assays for IDH1 R132H Inhibitors

Assays were conducted in a volume of 76 µl assay buffer (150 mM NaCl, 10 mM MgCl₂, 20 mM Tris pH 7.5, 0.03% bovine serum albumin) as follows in a standard 384-well plate: To 25 ul of substrate mix (8 uM NADPH, 2 mM aKG), 1 µl of test compound was added in DMSO. The plate was centrifuged briefly, and then 25 µl of enzyme mix was added (0.2 µg/ml IDH1 R132H) followed by a brief centrifugation and shake at 100 RPM. The reaction was incubated for 50 minutes at room temperature, then 25 µl of detection mix (30 µM resazurin, 36 µg/ml) was added and the mixture further incubated for 5 minutes at room temperature. The conversion of resazurin to resorufin was detected by fluorescent spectroscopy at Ex544 Em590 c/o 590.

The compounds of Formula I set forth in Table 1 and the compounds set forth in Table 2 were tested in this assay and the results set forth below in Table 4A and 4B. As used in Table 4A and 4B, "A" refers to an inhibitory activity against IDH1 R132H with an IC₅₀ ≤ 0.1 µM; "B" refers to an inhibitory activity against IDH1 R132H with an IC₅₀ between 0.1 µM and 1 µM; "C" refers to an inhibitory activity against IDH1 R132H with an IC₅₀ between 1 µM and 10 µM; "D" refers to an inhibitory activity against IDH1 R132H with an IC₅₀ between 10 µM and 100 µM; "E" refers to an inhibitory activity against IDH1 R132H with an IC₅₀ ≥ 100 µM.

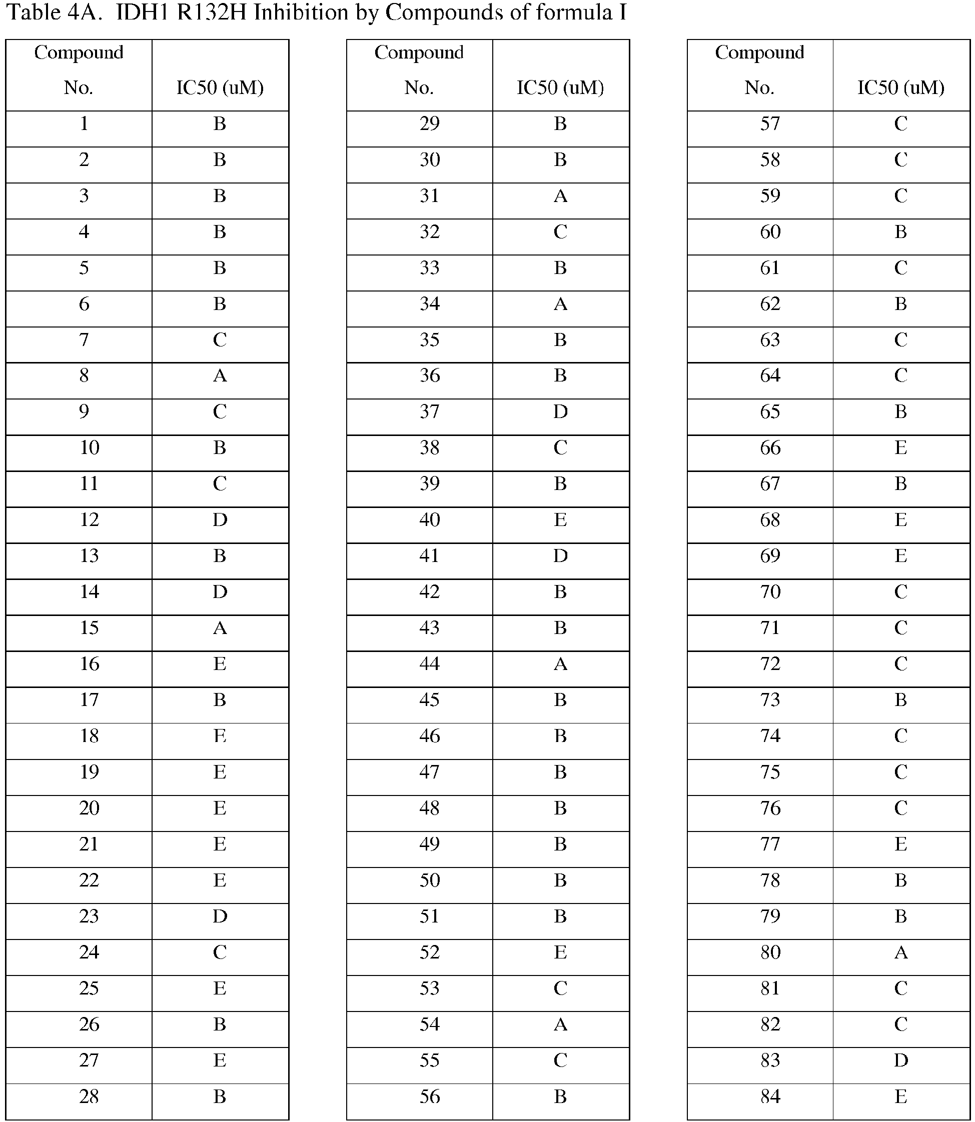

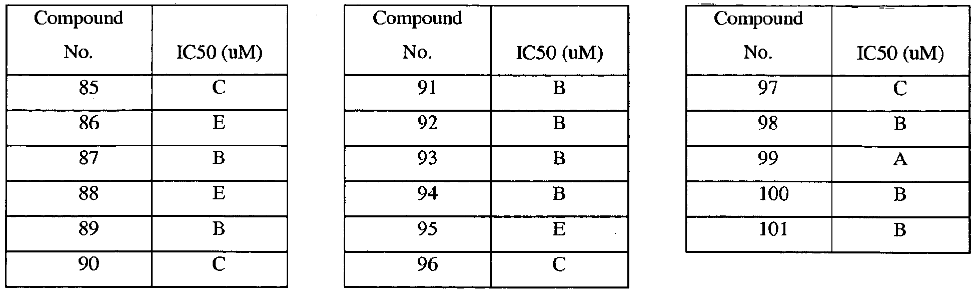

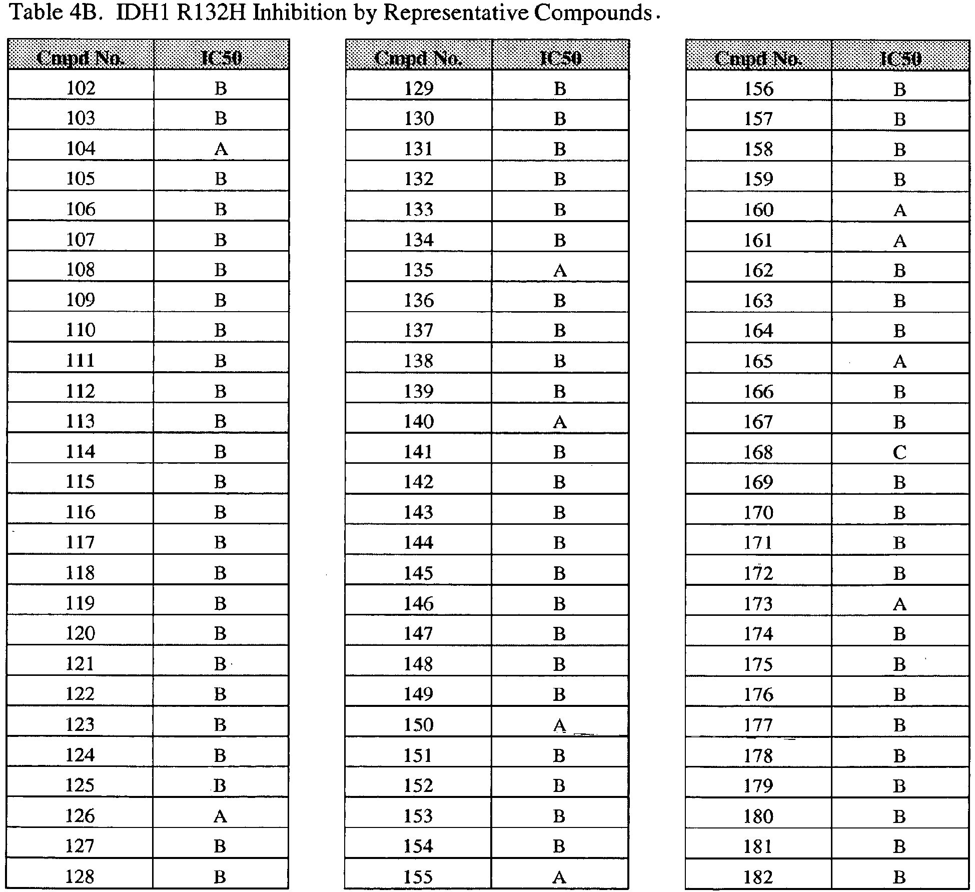

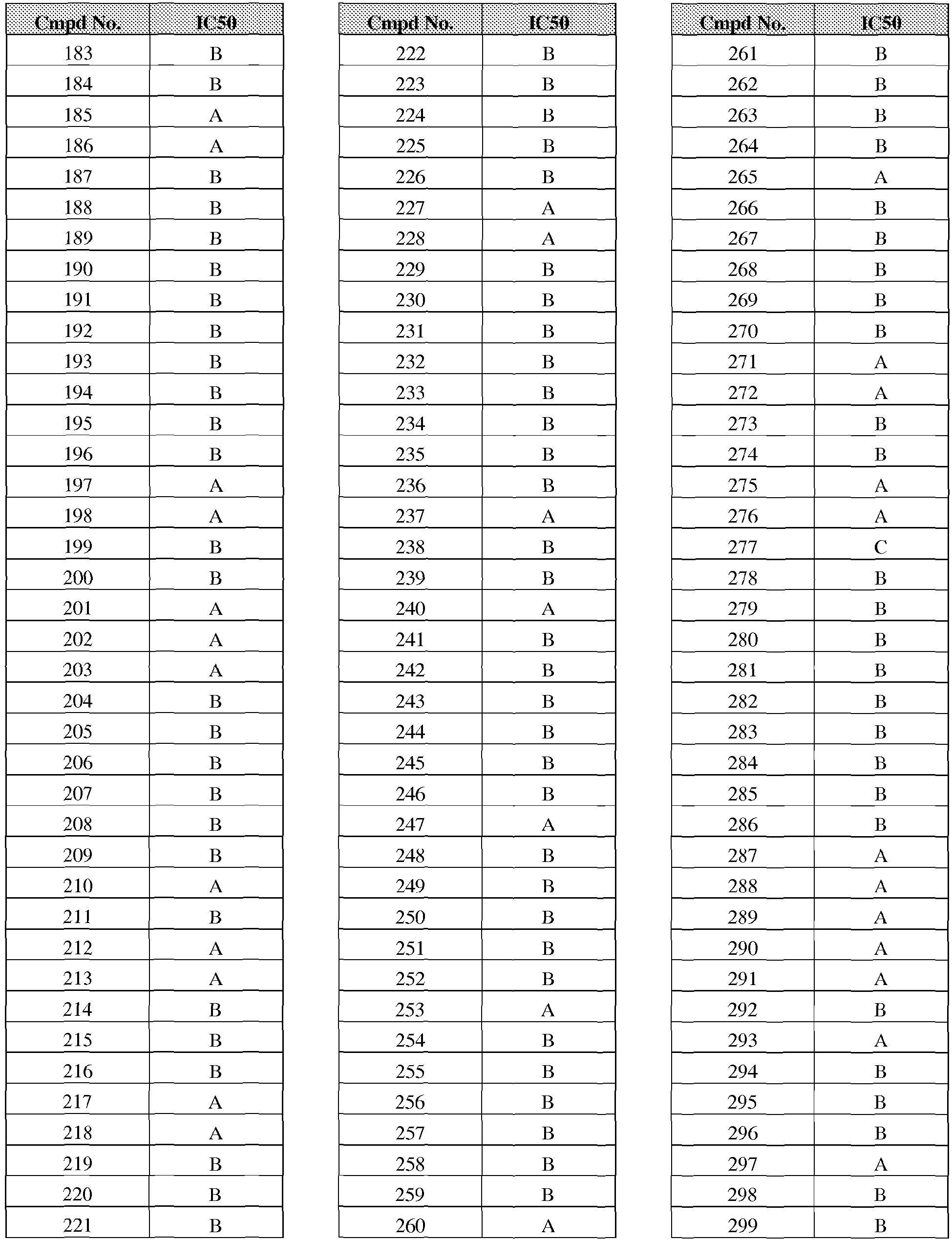

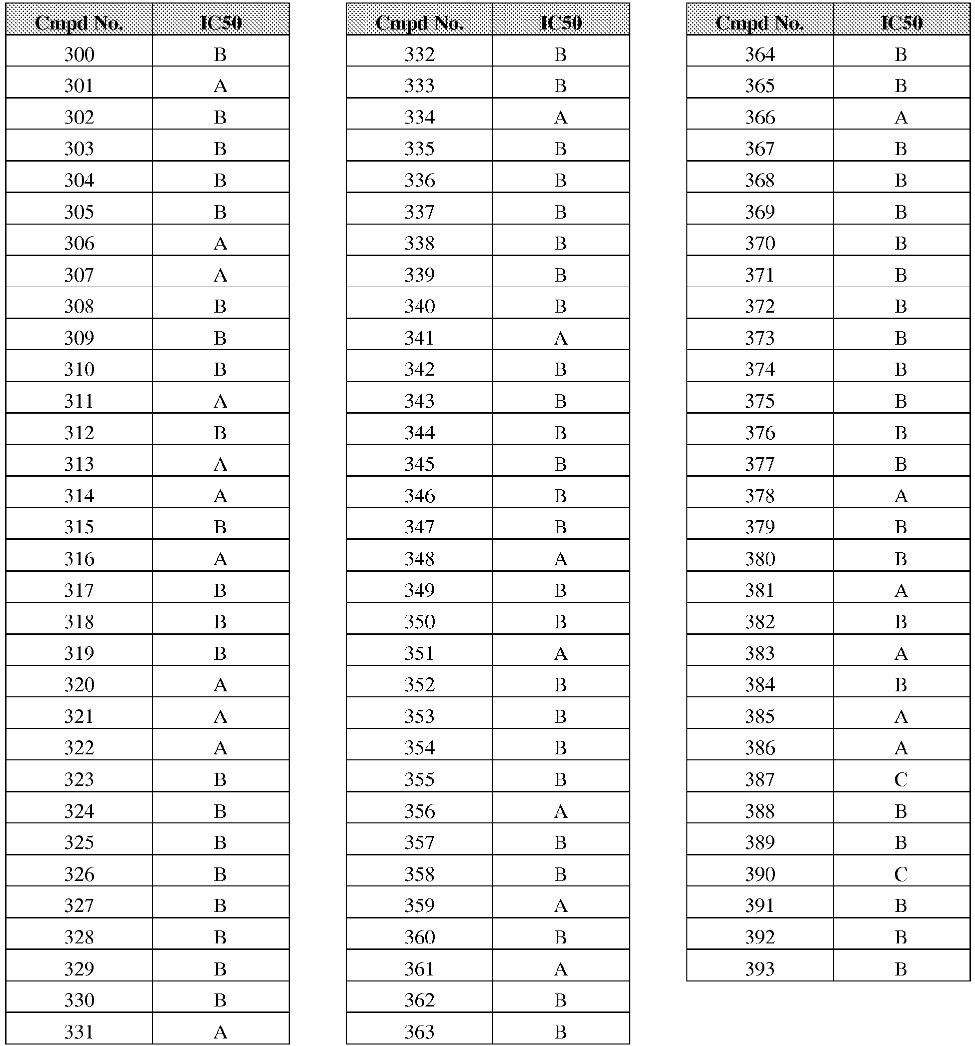

**Example 49: Cellular Assays for IDH1 R132H Inhibitors.** Cells (HT1080 or U87MG) were grown in T125 flasks in DMEM containing 10% FBS, 1x penicillin/streptomycin and 500ug/mL G418 (present in U87MG cells only). They were harvested by trypsin and seeded into 96 well white bottom plates at a density of 5000 cell/well in 100 ul/well in DMEM with 10% FBS. No cells were placed in columns 1 and 12. Cells were incubated overnight at 37°C in 5% CO₂. The next day test compounds were made up at 2x the final concentration and 100ul were added to each cell well. The final concentration of DMSO was 0.2% and the DMSO control wells were plated in row G. The plates were then placed in the incubator for 48 hours. At 48 hours, 100ul of media was removed from each well and analyzed by LC-MS for 2-HG concentrations. The cell plate was placed back in the incubator for another 24 hours. At 72 hours post compound addition, 10 mL/plate of Promega Cell Titer Glo reagent was thawed and mixed. The cell plate was removed from the incubator and allowed to equilibrate to room temperature. Then 100ul of Promega Cell Titer Glo reagent was added to each well of media. The cell plate was then placed on an orbital shaker for 10 minutes and then allowed to sit at room temperature for 20 minutes. The plate was then read for luminescence with an integration time of 500ms.

The IC₅₀ for inhibition of 2-HG production (concentration of test compound to reduce 2HG production by 50% compared to control) in these two cell lines for various compounds
is set forth in Tables 5A (HT1080 cells) and 5B (U87MG cells) below. As used in Tables 5A and 5B "A" refers to an IC₅₀ for inhibition of 2-HG production ≤ 0.25 µM; "B" refers to an IC₅₀ for inhibition of 2-HG production between 0.25 µM and 1 µM; "C" refers to an IC₅₀ for inhibition of 2-HG production between 1 µM and 5 µM; "D" refers to an IC₅₀ for inhibition of 2-HG production ≥ 5 µM.

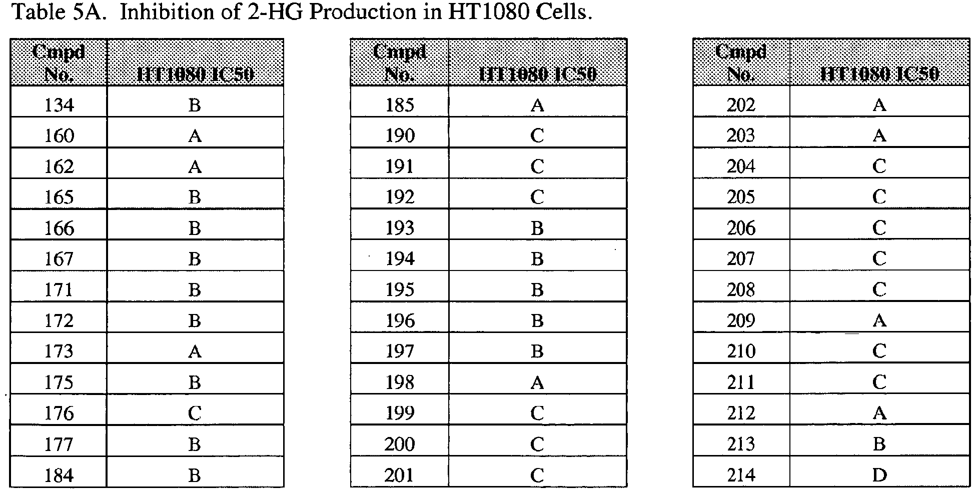

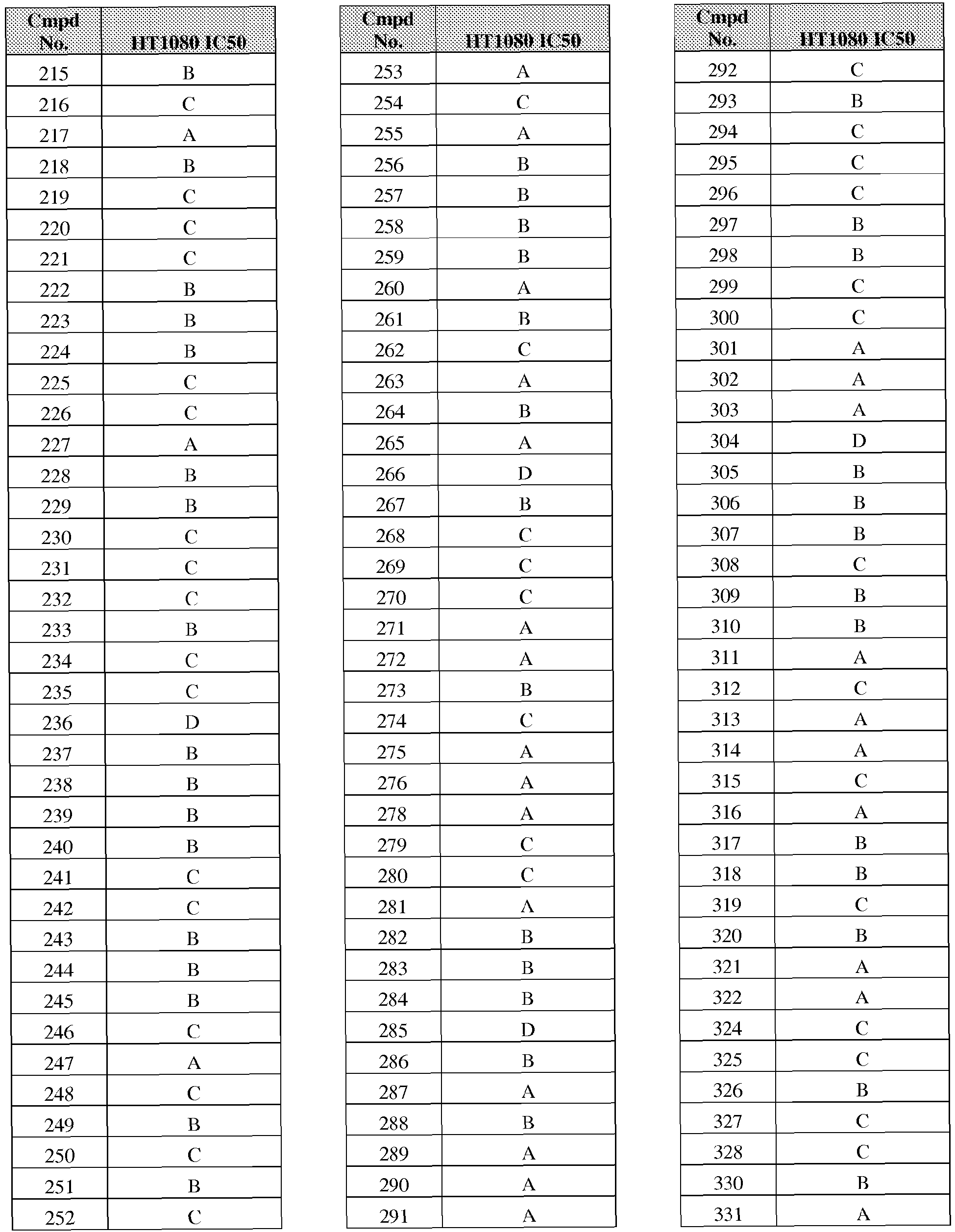

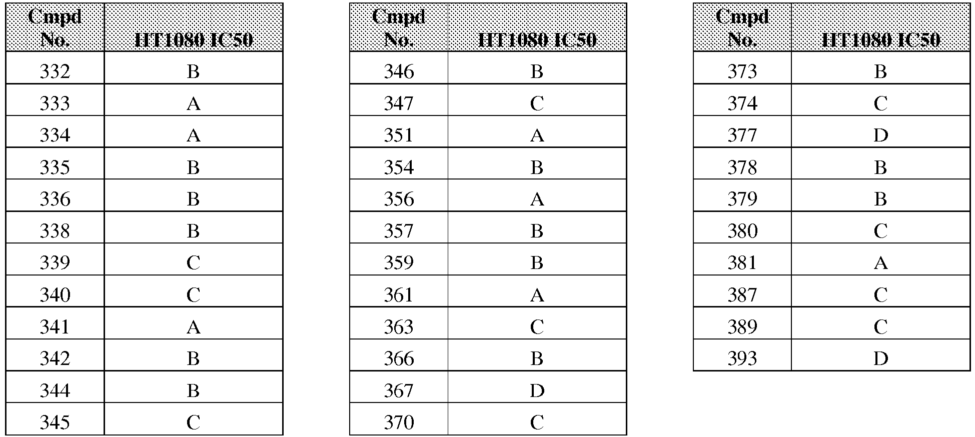

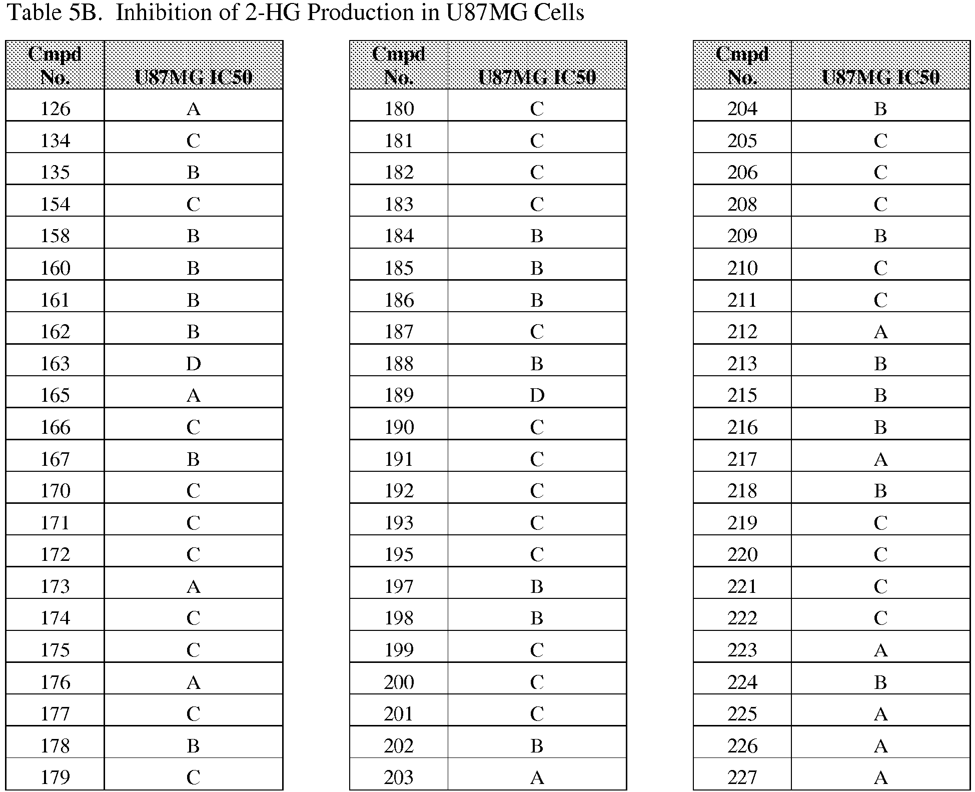

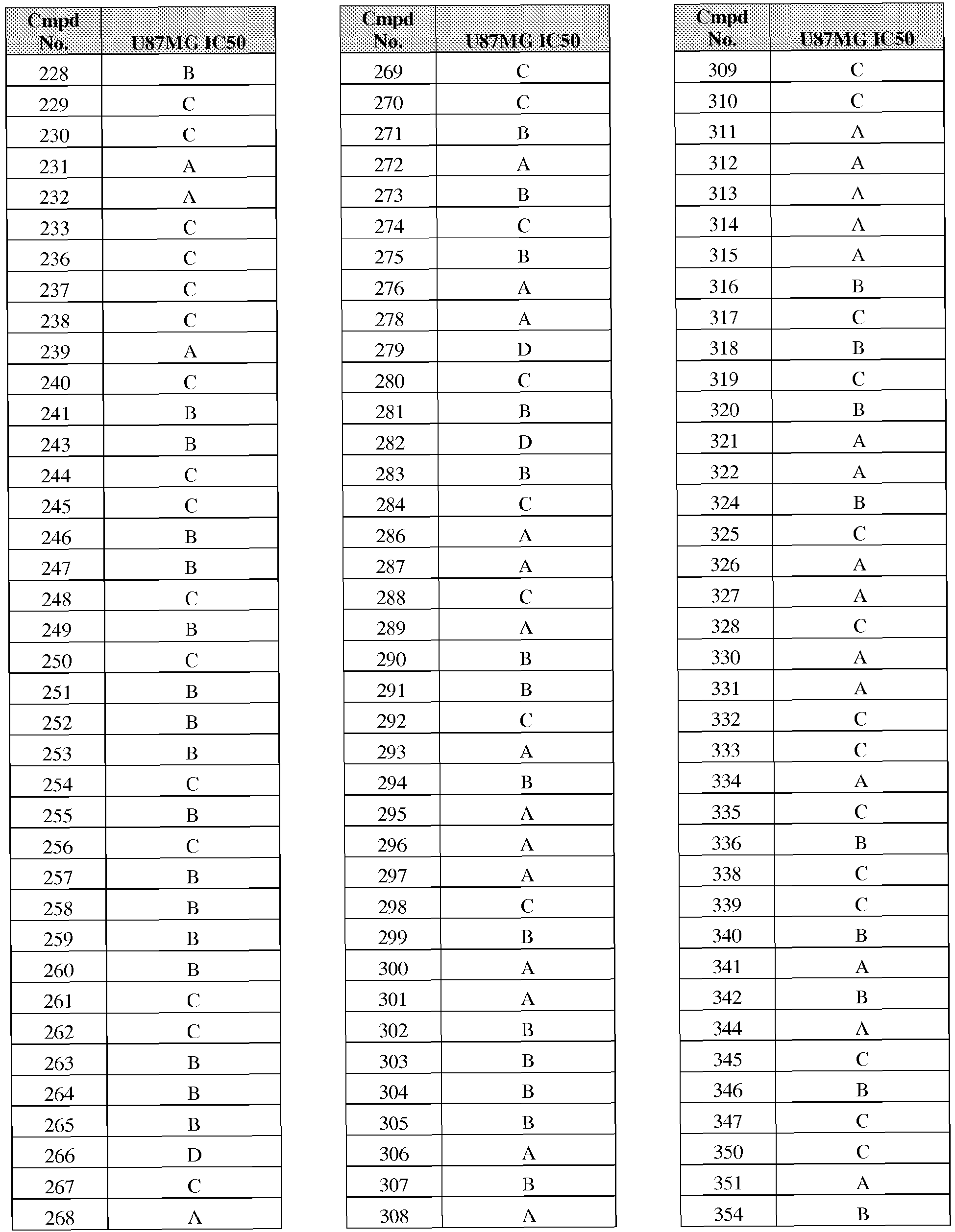

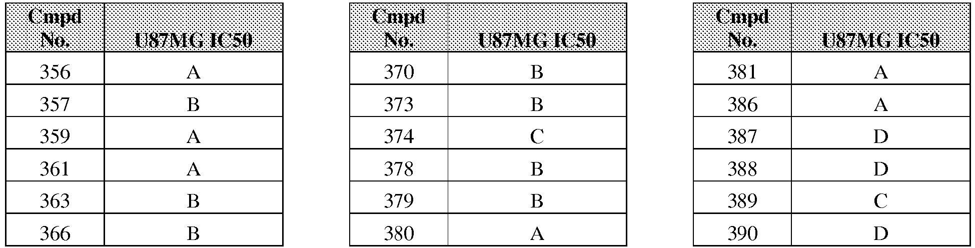

## Claims

1. A compound of Formula II: 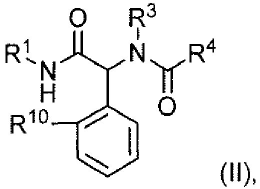 or a pharmaceutically acceptable salt thereof, wherein:
R¹ is selected from cyclohexyl, cyclopentyl, cycloheptyl, 3,3-difluorocyclobutyl, 4,4-difluorocyclohexyl, and bicyclo[2.2.1]heptanyl;
R³ is selected from 3-fluorophenyl, 3-methylphenyl, 3-chlorophenyl, and thien-2-ylmethyl;
R⁴ is selected from 1-(methylmethoxycarbonylamino)ethyl, 1,2,3,4-tetrahydroquinolin-1-yl, 1-ethoxycarbonylpiperidin-2-yl, 1-ethoxycarbonylpyrrolidin-2-yl, 1H-benzimidazol-1-ylmethyl, 1H-indazol-3-ylmethyl, indolin-1-ylmethyl, 1H-indol-3-ylmethyl, 1H-indol-5-ylmethyl, 1H-pyrrolo[2,3-b]pyridine-3-ylmethyl, 1H-pyrrolo[3,2-b]pyridin-3-ylmethyl, 1-methoxycarbonylpiperidin-2-yl, 1-methoxycarbonylpyrrolidin-2-yl, 2-fluoropyridin-3-ylaminomethyl, 2-imino-4-fluoropyridin-1-ylmethyl, 2-methoxyphenylaminomethyl, 2-methyl-1H-benzimidazol-1-ylmethyl, 2-methylimidazol-1-ylmethyl, 2-trifluoromethyl-1H-imidazol-1-yl, 3-cyanophenylaminomethyl, 3-fluoropyridin-2-ylaminomethyl, 3-methoxyphenylaminomethyl, 4-(1,3,4-oxadiazole-2-yl)phenylaminomethyl, 4-(dimethylaminocarbonyloxy)phenylmethyl, 4,5-dichloroimidazol-1-ylmethyl, 4-cyanophenylaminomethyl, 4-fluorophenylaminomethyl, 4-fluoropyridin-2-ylaminomethyl, 4-hydroxyphenylmethyl, 4-methoxycarbonylmorpholin-3-yl, 4-methoxycarbonylpiperazin-1-ylmethyl, 4-methoxyphenylaminomethyl, 4-methylcarbonyloxyphenylmethyl, 5-fluoropyridin-2-aminomethyl, 5-fluoropyridin-2-oxymethyl, 6-fluoropyridin-3-ylaminomethyl, benzomorpholin-4-ylmethyl, methoxycarbonylaminomethyl, methylmethoxycarbonylaminomethyl, methylphenylaminomethyl, phenylaminomethyl, pyridin-2-oxymethyl, pyridin-2-ylaminomethyl, pyridin-2-yloxymethyl, pyridin-3-oxymethyl, pyridin-3-ylmethyl, pyridin-4-ylmethyl, thiazol-4-ylmethyl, and thien-2-ylmethyl; and
R¹⁰ is selected from methyl, hydrogen, fluoro, chloro, and bromo, wherein: when R¹ is cyclopentyl or cyclohexyl, and R³ is thien-2-ylmethyl, then R⁴ is other than thien-2-ylmethyl, 1H-benizimidazol-1-ylmethyl, 1H-indol-3-ylmethyl, or 1H-benzotriazol-1-ylmethyl;
when R¹ is cyclopentyl, R¹⁰ is hydrogen, and R³ is 3-fluorophenyl, 3-methylphenyl, or 3-chlorophenyl, then R⁴ is other than thien-2-ylmethyl;
when R¹ is cyclopentyl, R¹⁰ is methyl and R³ is 3-fluorophenyl, then R⁴ is other than thien-2-ylmethyl or 1H-benzotriazol-1-ylmethyl;
when R¹ is cyclopentyl, R¹⁰ is fluoro and R³ is 3-methylphenyl, then R⁴ is other than thien-2-ylmethyl or 1H-benzotriazol-1-ylmethyl;
when R¹ is cyclopentyl, R¹⁰ is fluoro and R³ is 3-fluorophenyl, then R⁴ is other than thien-2-ylmethyl;
when R¹ is cyclohexyl, R¹⁰ is hydrogen, and R³ is 3-methylphenyl, or 3-chlorophenyl, then R⁴ is other than thien-2-ylmethyl; and
when R¹ is cyclohexyl, R¹⁰ is hydrogen, and R³ is 3-fluorophenyl, then R⁴ is other than 1H-benzotriazol-1-ylmethyl.

2. The compound of claim 1 or a pharmaceutically acceptable salt thereof, wherein R³ is 3-fluorophenyl.

3. The compound of claim 1 or a pharmaceutically acceptable salt thereof, wherein the compound is selected from:
| |
|---|
| 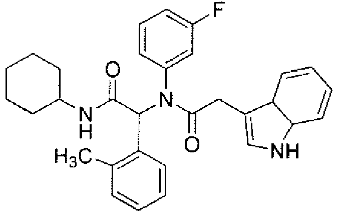 |
| 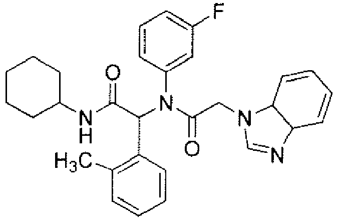 |
| 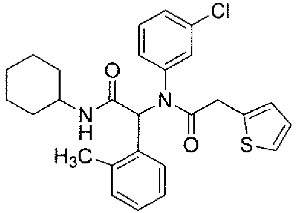 |
| 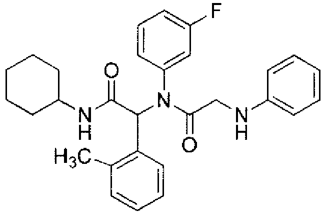 |
| 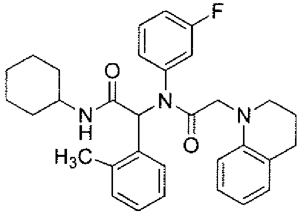 |
| 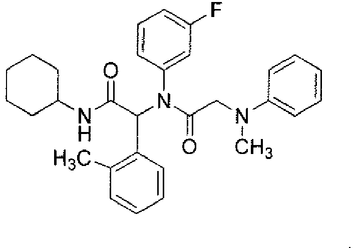 |
| 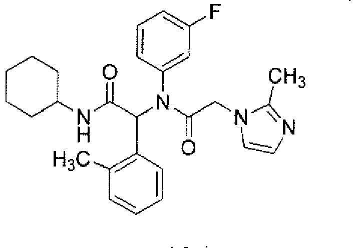 |
| 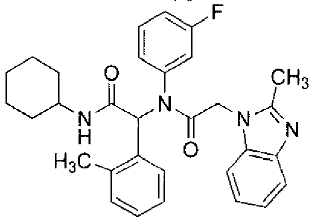 |
| 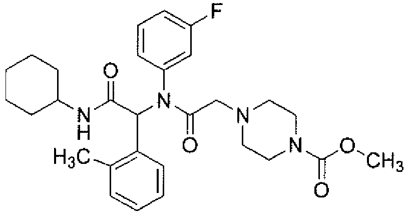 |
| 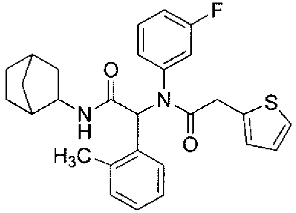 |
| 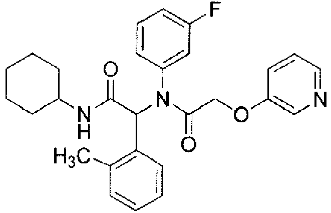 |
| 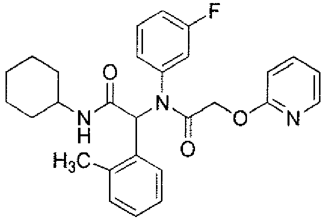 |
| 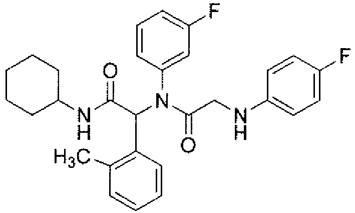 |
| 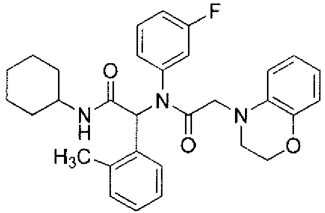 |
| 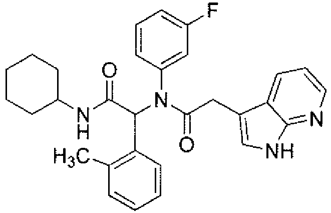 |
| 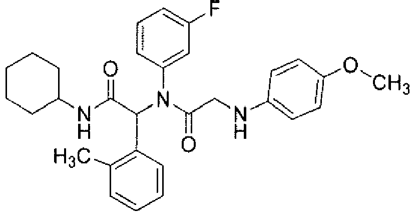 |
| 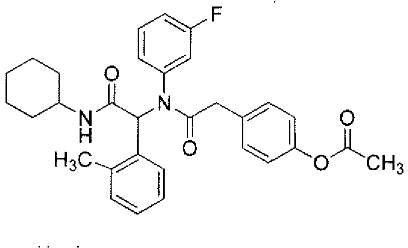 |
| 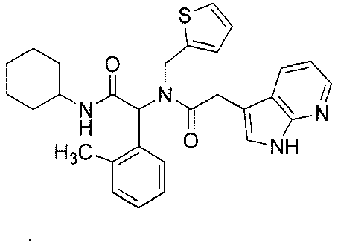 |
| 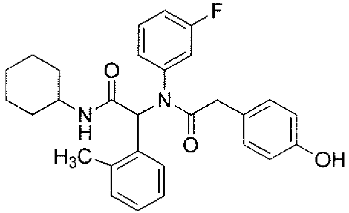 |
| 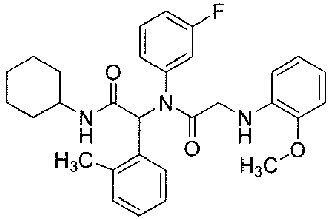 |
| 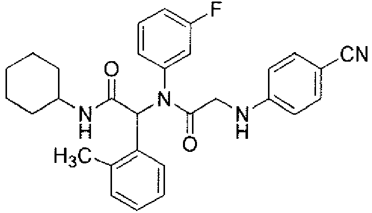 |
| 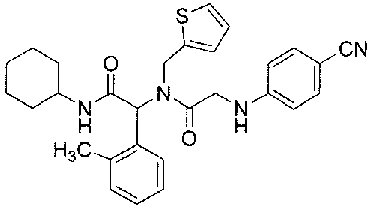 |
| 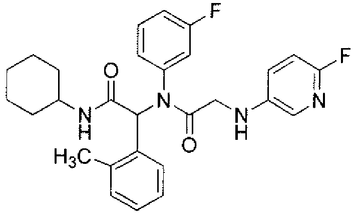 |
| 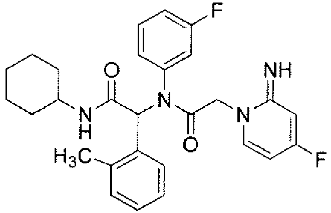 |
| 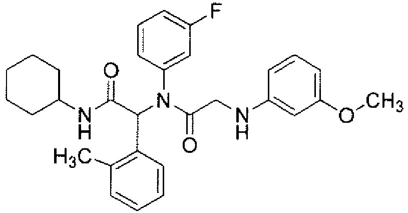 |
| 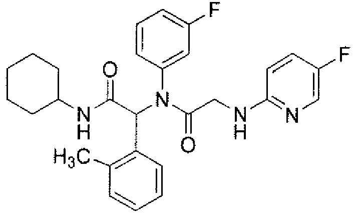 |
| 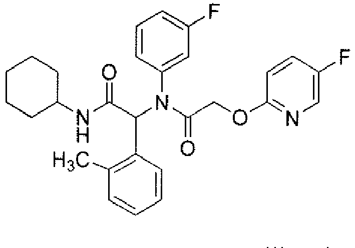 |
| 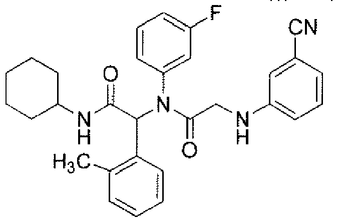 |
| 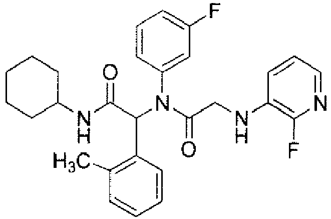 |
| 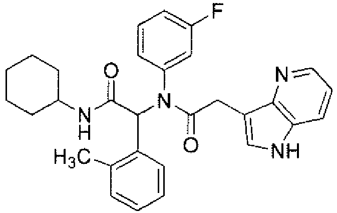 |
| 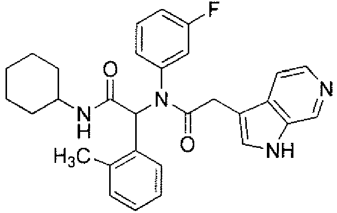 |
| 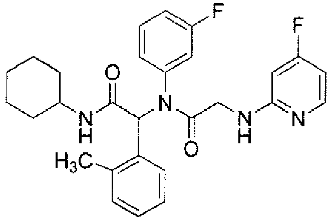 |
| 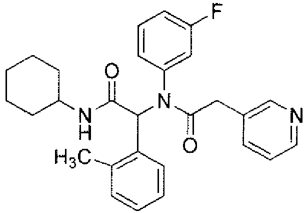 |
| 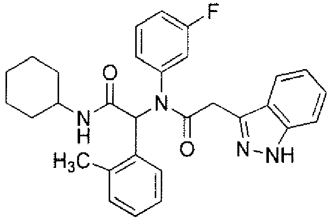 |
| 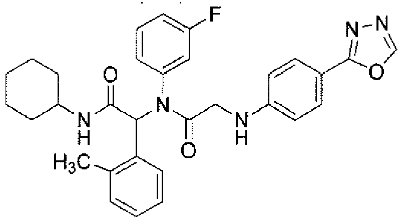 |
| 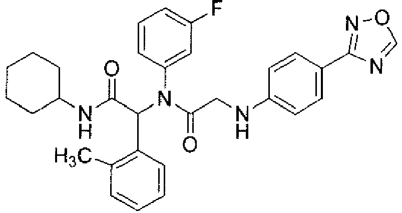 |
| 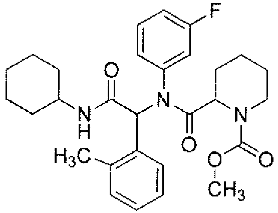 |
| 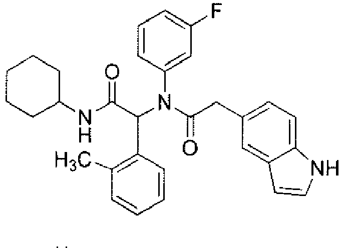 |
| 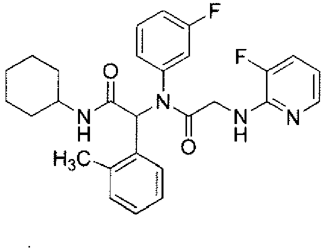 |
| 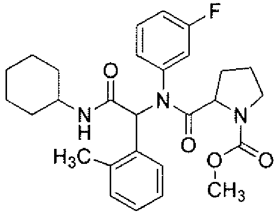 |
| 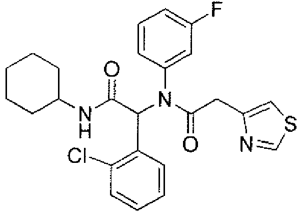 |
| 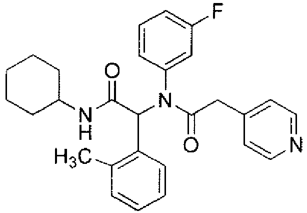 |
| 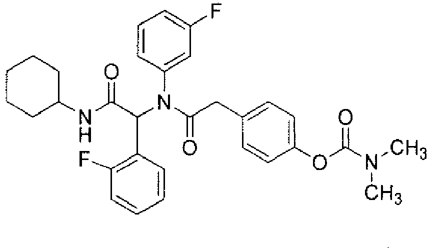 |
| 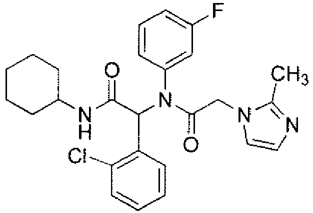 |
| 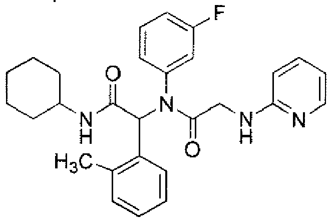 |
| 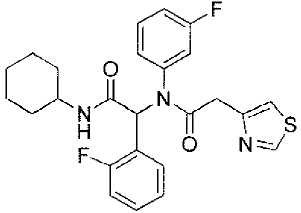 |
| 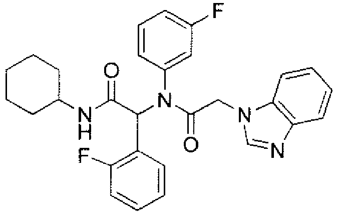 |
| 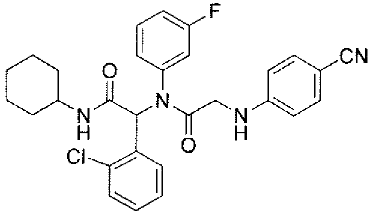 |
| 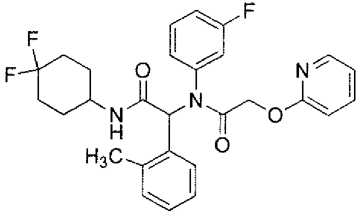 |
| 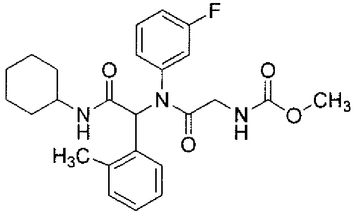 |
| 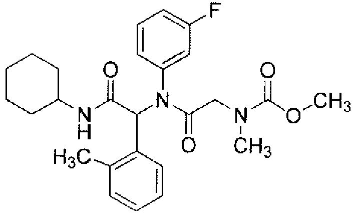 |
| 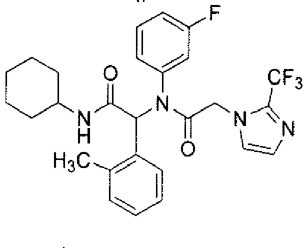 |
| 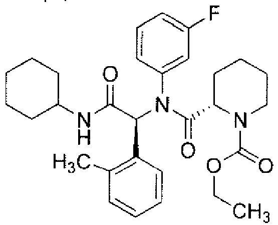 |
| 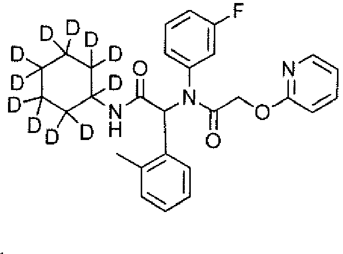 |
| 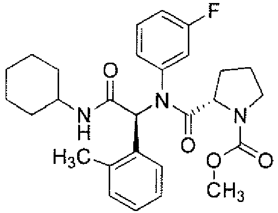 |
| 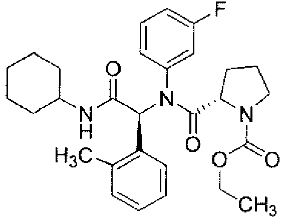 |
| 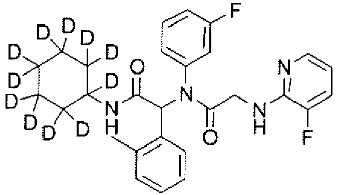 |
| 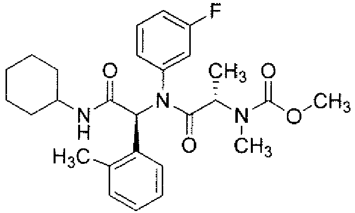 |
| 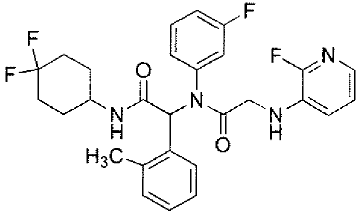 |
| 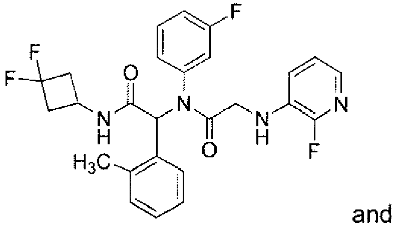 |
| 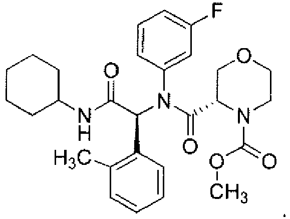 |

4. A compound or a pharmaceutically acceptable salt thereof for use in a method of treating a cancer having an R132X IDH1 mutation, the method comprising administering to a subject a therapeutically effective amount of a compound, wherein the compound is a compound of any one of claims 1-3.

5. The compound or a pharmaceutically acceptable salt thereof for use of claim 4, wherein the R132X IDH1 mutation is an R132H IDH1 mutation.

6. The compound or a pharmaceutically acceptable salt thereof for use of claim 4, wherein the R132X IDH1 mutation is an R132C IDH1 mutation.

7. The compound or a pharmaceutically acceptable salt thereof for use of claim 4, wherein a sample from a subject is evaluated in vitro for the presence of an elevated level of 2HG.

8. The compound or a pharmaceutically acceptable salt thereof for use of claim 4, wherein efficacy of treatment of cancer comprises monitoring in vitro the level of 2HG in a sample from a subject during treatment.

9. The compound or a pharmaceutically acceptable salt thereof for use of claim 4, wherein efficacy of treatment of cancer comprises monitoring in vitro the level of 2HG in a sample from a subject following termination of treatment.

10. A pharmaceutical composition comprising a compound of any one of claims 1 to 3 or a pharmaceutically acceptable salt thereof; and a pharmaceutically acceptable carrier.

## Patentansprüche

1. Verbindung der Formel II: 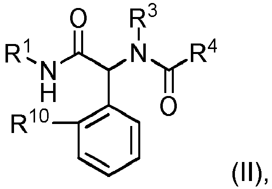 oder ein pharmazeutisch verträgliches Salz davon, wobei:
R¹ ausgewählt ist aus Cyclohexyl, Cyclopentyl, Cycloheptyl, 3,3-Difluorcyclobutyl, 4,4-Difluorcyclohexyl und Bicyclo[2.2.1]heptanyl;
R³ ausgewählt ist aus 3-Fluorphenyl, 3-Methylphenyl, 3-Chlorphenyl und Thien-2-ylmethyl;
R⁴ ausgewählt ist aus 1-(Methylmethoxycarbonylamino)ethyl, 1,2,3,4-Tetrahydroquinolin-1-yl, 1-Ethoxycarbonylpiperidin-2-yl, 1-Ethoxycarbonylpyrrolidin-2-yl, 1H-Benzimidazol-1-ylmethyl, 1H-Indazol-3-ylmethyl, Indolin-1-ylmethyl, 1H-Indol-3-ylmethyl, 1H-Indol-5-ylmethyl, 1H-Pyrrolo[2,3-b]pyridin-3-ylmethyl, 1H-Pyrrolo[3,2-b]pyridin-3-ylmethyl, 1-Methoxycarbonylpiperidin-2-yl, 1-Methoxycarbonylpyrrolidin-2-yl, 2-Fluorpyridin-3-ylaminomethyl, 2-Imino-4-fluorpyridin-1-ylmethyl, 2-Methoxyphenylaminomethyl, 2-Methyl-1H-benzimidazol-1-ylmethyl, 2-Methylimidazol-1-ylmethyl, 2-Trifluormethyl-1H-imidazol-1-yl, 3-Cyanophenylaminomethyl, 3-Fluorpyridin-2-ylaminomethyl, 3-Methoxyphenylaminomethyl, 4-(1,3,4-Oxadiazol-2-yl)phenylaminomethyl, 4-(Dimethylaminocarbonyloxy)phenylmethyl, 4,5-Dichlorimidazol-1-ylmethyl, 4-Cyanophenylaminomethyl, 4-Fluorphenylaminomethyl, 4-Fluorpyridin-2-ylaminomethyl, 4-Hydroxyphenylmethyl, 4-Methoxycarbonylmorpholin-3-yl, 4-Methoxycarbonylpiperazin-1-ylmethyl, 4-Methoxyphenylaminomethyl, 4-Methylcarbonyloxyphenylmethyl, 5-Fluorpyridin-2-aminomethyl, 5-Fluorpyridin-2-oxymethyl, 6-Fluorpyridin-3-ylaminomethyl, Benzomorpholin-4-ylmethyl, Methoxycarbonylaminomethyl, Methylmethoxycarbonylaminomethyl, Methylphenylaminomethyl, Phenylaminomethyl, Pyridin-2-oxymethyl, Pyridin-2-ylaminomethyl, Pyridin-2-yloxymethyl, Pyridin-3-oxymethyl, Pyridin-3-ylmethyl, Pyridin-4-ylmethyl, Thiazol-4-ylmethyl und Thien-2-ylmethyl; und
R¹⁰ ausgewählt ist aus Methyl, Wasserstoff, Fluor, Chlor und Brom, wobei:
wenn R¹ Cyclopentyl oder Cyclohexyl ist, und R³ Thien-2-ylmethyl ist, dann ist R⁴ etwas anderes als Thien-2-ylmethyl, 1H-Benizimidazol-1-ylmethyl, 1H-Indol-3-ylmethyl oder 1H-Benzotriazol-1-ylmethyl;
wenn R¹ Cyclopentyl ist, R¹⁰ Wasserstoff ist und R³ 3-Fluorphenyl, 3-Methylphenyl oder 3-Chlorphenyl ist, dann ist R⁴ etwas anderes als Thien-2-ylmethyl;
wenn R¹ Cyclopentyl ist, R¹⁰ Methyl ist und R³ 3-Fluorphenyl ist, dann ist R⁴ etwas anderes als Thien-2-ylmethyl oder 1H-Benztriazol-1-ylmethyl;
wenn R¹ Cyclopentyl ist, R¹⁰ Fluor ist und R³ 3-Methylphenyl ist, dann ist R⁴ etwas anderes als Thien-2-ylmethyl oder 1H-Benzotriazol-1-ylmethyl;
wenn R¹ Cyclopentyl ist, R¹⁰ Fluor ist und R³ 3-Fluorphenyl ist, dann ist R⁴ etwas anderes als Thien-2-ylmethyl;
wenn R¹ Cyclohexyl ist, R¹⁰ Wasserstoff ist und R³ 3-Methylphenyl oder 3-Chlorphenyl ist, dann ist R⁴ etwas anderes als Thien-2-ylmethyl; und
wenn R¹ Cyclohexyl ist, R¹⁰ Wasserstoff ist und R³ 3-Fluorphenyl ist, dann ist R⁴ etwas anderes als 1H-Benztriazol-1-ylmethyl.

2. Verbindung nach Anspruch 1 oder ein pharmazeutisch verträgliches Salz davon, wobei R³ 3-Fluorphenyl ist.

3. Verbindung nach Anspruch 1 oder ein pharmazeutisch verträgliches Salz davon, wobei die Verbindung ausgewählt ist aus:
| |
|---|
| 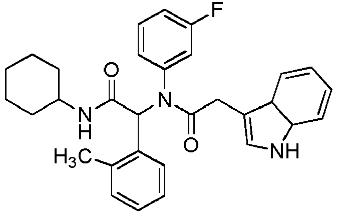 |
| 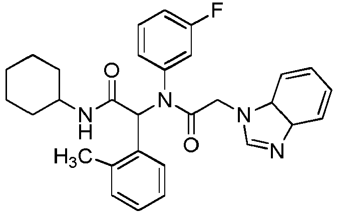 |
| 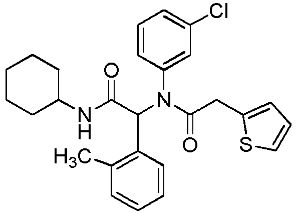 |
| 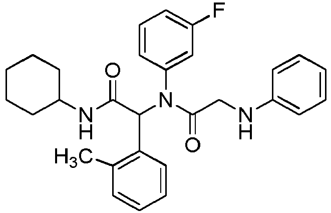 |
| 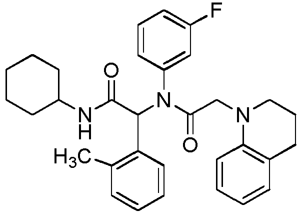 |
| 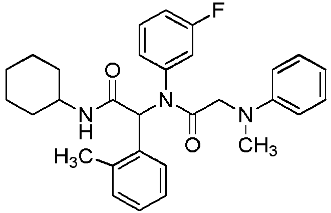 |
| 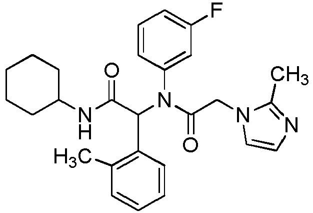 |
| 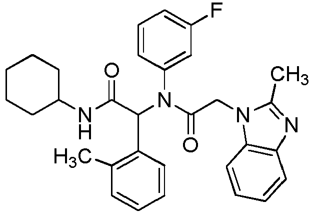 |
| 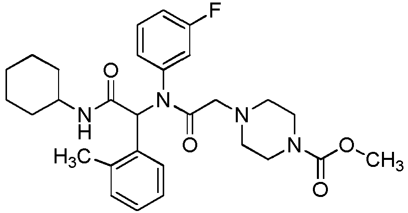 |
| 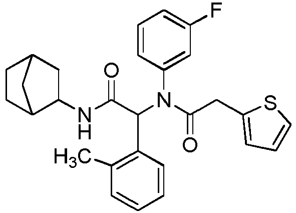 |
| 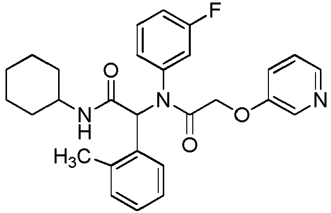 |
| 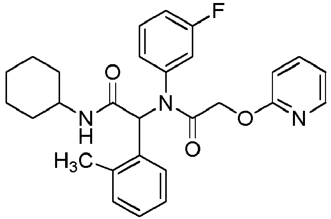 |
| 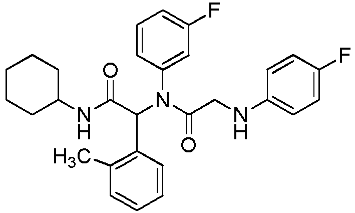 |
| 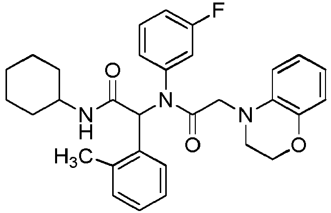 |
| 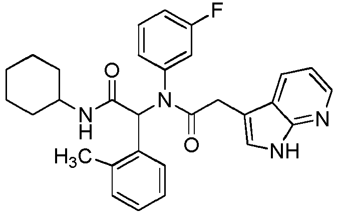 |
| 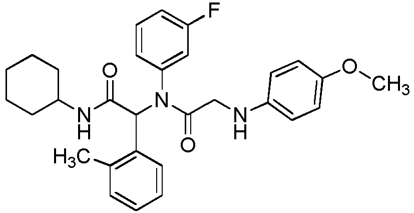 |
| 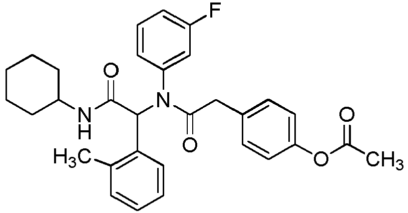 |
| 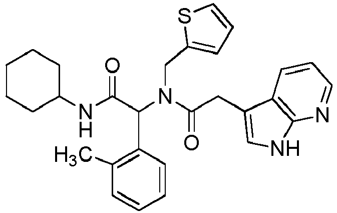 |
| 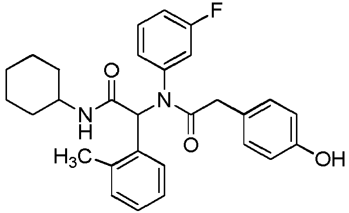 |
| 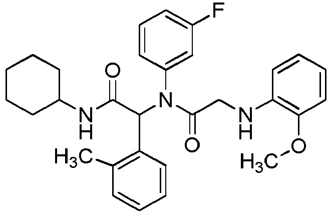 |
| 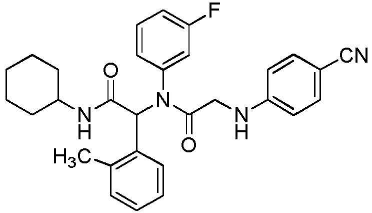 |
| 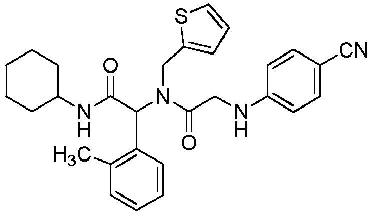 |
| 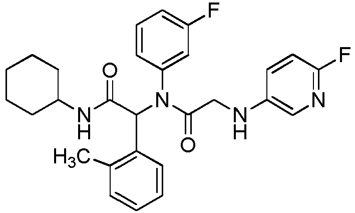 |
| 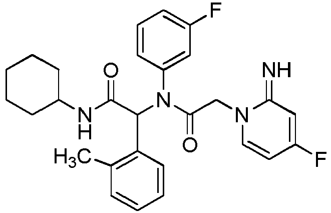 |
| 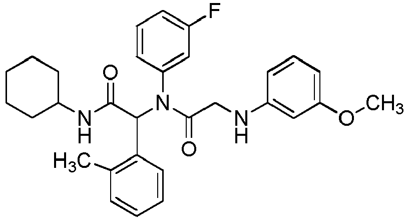 |
| 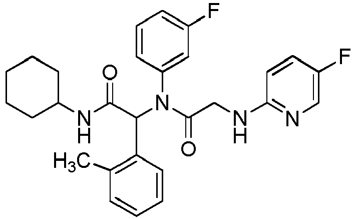 |
| 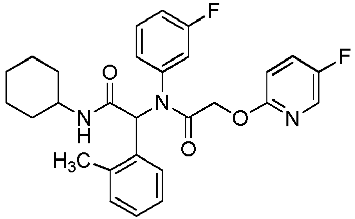 |
| 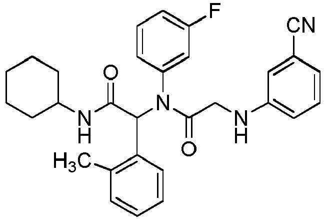 |
| 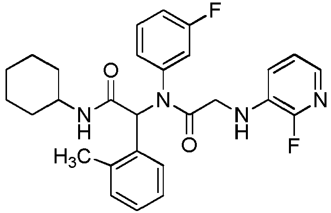 |
| 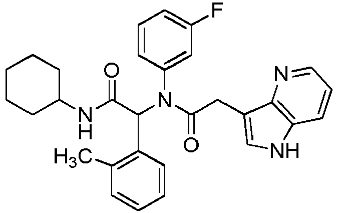 |
| 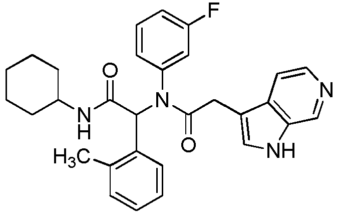 |
| 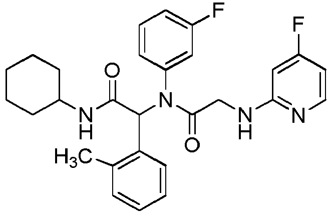 |
| 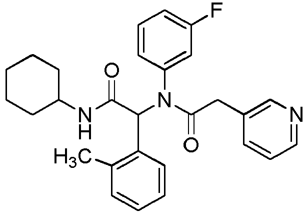 |
| 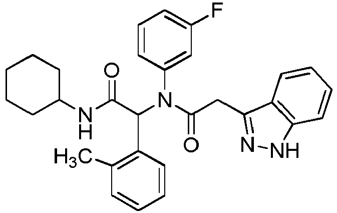 |
| 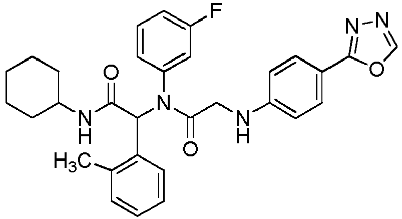 |
| 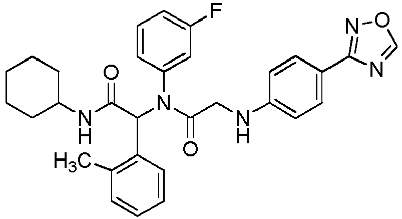 |
| 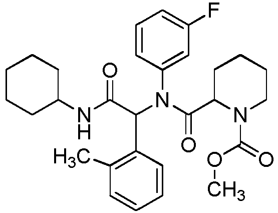 |
| 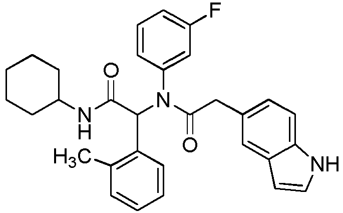 |
| 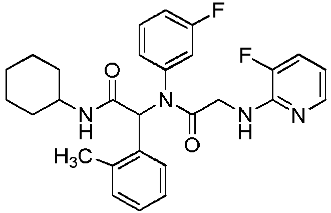 |
| 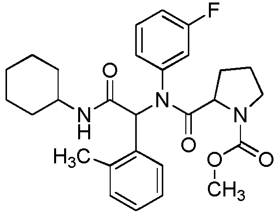 |
| 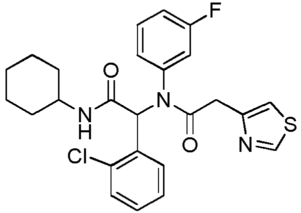 |
| 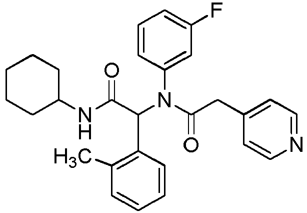 |
| 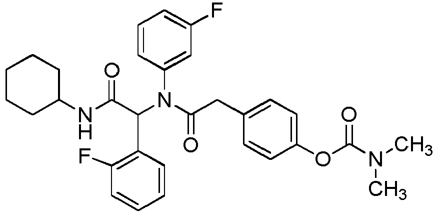 |
| 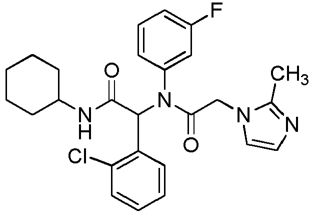 |
| 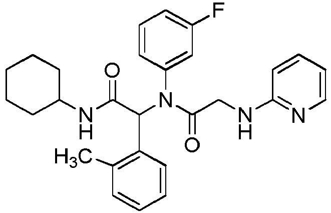 |
| 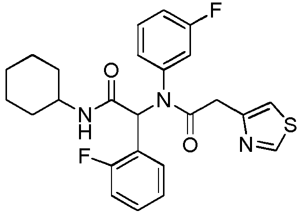 |
| 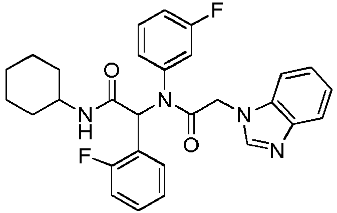 |
| 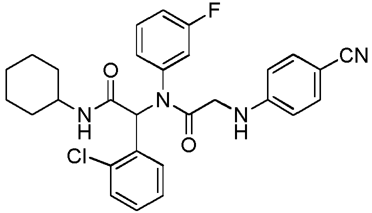 |
| 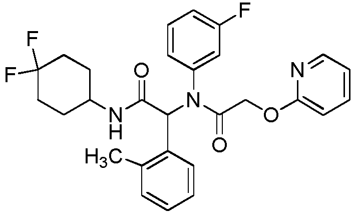 |
| 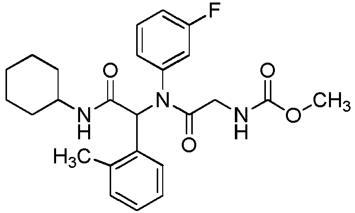 |
| 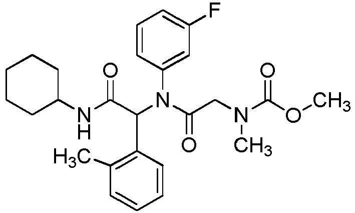 |
| 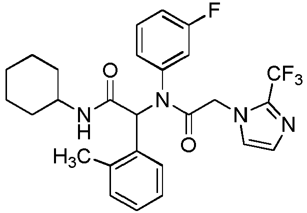 |
| 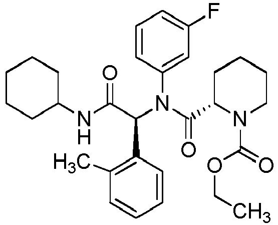 |
| 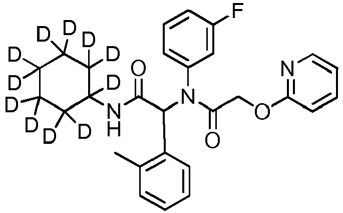 |
| 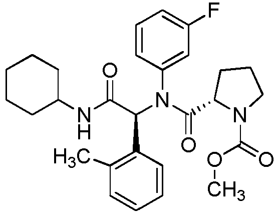 |
| 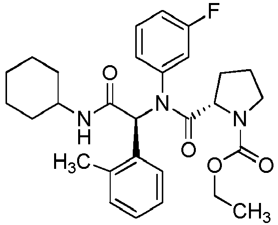 |
| 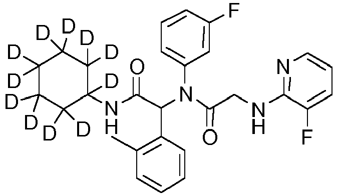 |
| 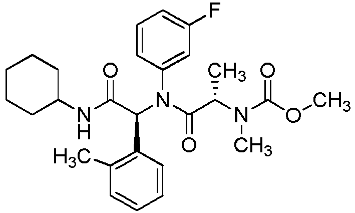 |
| 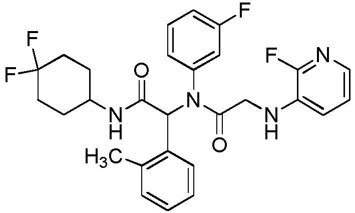 |
| 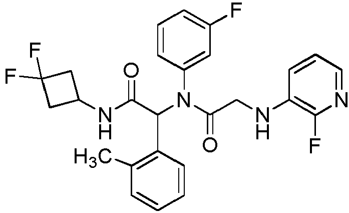 |
| und |
| 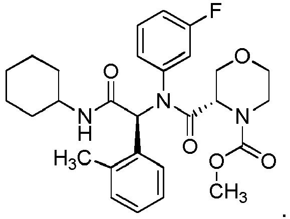 |

4. Verbindung oder ein pharmazeutisch verträgliches Salz davon zur Verwendung in einem Verfahren zur Behandlung eines Krebses mit einer R132X-IDH1-Mutation, das Verfahren umfassend Verabreichen an ein Subjekt einer therapeutisch effektiven Menge einer Verbindung, wobei die Verbindung eine Verbindung nach irgendeinem der Ansprüche 1-3 ist.

5. Verbindung oder ein pharmazeutisch verträgliches Salz davon zur Verwendung nach Anspruch 4, wobei die R132X-IDH1-Mutation eine R132H-IDH1-Mutation ist.

6. Verbindung oder ein pharmazeutisch verträgliches Salz davon zur Verwendung nach Anspruch 4, wobei die R132X-IDH1-Mutation eine R132C-IDH1-Mutation ist.

7. Verbindung oder ein pharmazeutisch verträgliches Salz davon zur Verwendung nach Anspruch 4, wobei eine Probe von einem Subjekt auf das Vorhandensein eines erhöhten Levels von 2HG in vitro untersucht wird.

8. Verbindung oder ein pharmazeutisch verträgliches Salz davon zur Verwendung nach Anspruch 4, wobei die Effizienz der Behandlung des Krebses die in-vitro-Überwachung des Levels von 2HG in einer Probe aus einem Subjekt während der Behandlung umfasst.

9. Verbindung oder ein pharmazeutisch verträgliches Salz davon zur Verwendung nach Anspruch 4, wobei die Effizienz der Behandlung des Krebses die in-vitro-Überwachung des Levels von 2HG in einer Probe aus einem Subjekt nach Beendigung der Behandlung umfasst.

10. Pharmazeutische Zusammensetzung umfassend eine Verbindung nach irgendeinem der Ansprüche 1 bis 3 oder ein pharmazeutisch verträgliches Salz davon; und einen pharmazeutisch verträglichen Träger.

## Revendications

1. Composé selon la Formule II : 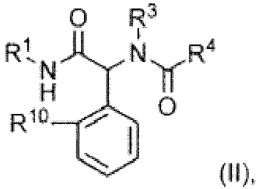 ou son sel pharmaceutiquement acceptable, dans lequel :
R¹ est sélectionné parmi un groupe cyclohexyle, cyclopentyle, cycloheptyle, 3,3-difluorocyclobutyle, 4,4-difluorocyclohexyle, et bicyclo[2.2.1]heptanyle ;
R³ est sélectionné parmi un groupe 3-fluorophényle, 3-méthylphényle, 3-chlorophényle, et thién-2-ylméthyle ;
R⁴ est sélectionné parmi un groupe 1-(méthyl-méthoxycarbonylamino)éthyle, 1,2,3,4-tétrahydroquinolin-1-yle, 1-éthoxycarbonylpipéridin-2-yle, 1-éthoxycarbonyl-pyrrolidin-2-yle, 1H-benzimidazol-1-ylméthyle, 1H-indazol-3-ylméthyle, indolin-1-ylméthyle, 1H-indol-3-ylméthyle, 1H-indol-5-ylméthyle, 1H-pyrrolo[2,3-b]pyridin-3-ylméthyle, 1H-pyrrolo[3,2-b]pyridin-3-ylméthyle, 1-méthoxycarbonylpipéridin-2-yle, 1-méthoxycarbonylpyrrolidin-2-yle, 2-fluoropyridin-3-ylaminométhyle, 2-imino-4-fluoropyridin-1-ylméthyle, 2-méthoxyphénylaminométhyle, 2-méthyl-1H-benzimidazol-1-yl-méthyle, 2-méthylimidazol-1-ylméthyle, 2-trifluorométhyl-1H-imidazol-1-yle, 3-cyanophénylaminométhyle, 3-fluoro-pyridin-2-ylaminométhyle, 3-méthoxyphénylaminométhyle, 4-(1,3,4-oxadiazole-2-yl)phénylaminométhyle, 4-(diméthylamino-carbonyloxy)phénylméthyle, 4,5-dichloroimidazol-1-yl-méthyle, 4-cyanophénylaminométhyle, 4-fluorophénylamino-méthyle, 4-fluoropyridin-2-ylaminométhyle, 4-hydroxyphényl-méthyle, 4-méthoxycarbonylmorpholin-3-yle, 4-méthoxy-carbonylpipérazin-1-ylméthyle, 4-méthoxyphénylaminométhyle, 4-méthylcarbonyloxyphénylméthyle, 5-fluoropyridin-2-amino-méthyle, 5-fluoropyridin-2-oxyméthyle, 6-fluoropyridin-3-ylaminométhyle, benzomorpholin-4-ylméthyle, méthoxycarbonyl-aminométhyle, méthylméthoxycarbonylaminométhyle, méthyl-phénylaminométhyle, phénylaminométhyle, pyridin-2-oxy-méthyle, pyridin-2-ylaminométhyle, pyridin-2-yloxyméthyle, pyridin-3-oxyméthyle, pyridin-3-ylméthyle, pyridin-4-yl-méthyle, thiazol-4-ylméthyle, et thién-2-ylméthyle ; et
R¹⁰ est sélectionné parmi un groupe méthyle, un hydrogène, un groupe fluoro, chloro et bromo, et dans lequel :
lorsque R¹ est un groupe cyclopentyle ou cyclohexyle, et R³ est un groupe thién-2-ylméthyle, alors R⁴ est autre qu'un groupe thién-2-ylméthyle, 1H-benzimidazol-1-ylméthyle, 1H-indol-3-ylméthyle, ou 1H-benzotriazol-1-ylméthyle ;
lorsque R¹ est un groupe cyclopentyle, R¹⁰ est un hydrogène, et R³ est un groupe 3-fluorophényle, 3-méthylphényle, ou 3-chlorophényle, alors R⁴ est autre qu'un groupe thién-2-ylméthyle ;
lorsque R¹ est un groupe cyclopentyle, R¹⁰ est un groupe méthyle et R³ est un groupe 3-fluorophényle, alors R⁴ est autre qu'un groupe thién-2-ylméthyle ou 1H-benzo-triazol-1-ylméthyle ;
lorsque R¹ est un groupe cyclopentyle, R¹⁰ est un groupe fluoro et R³ est un groupe 3-méthylphényle, alors R⁴ est autre qu'un groupe thién-2-ylméthyle ou 1H-benzo-triazol-1-ylméthyle ;
lorsque R¹ est un groupe cyclopentyle, R¹⁰ est un groupe fluoro et R³ est un groupe 3-fluorophényle, alors R⁴ est autre qu'un groupe thién-2-ylméthyle ;
lorsque R¹ est un groupe cyclohexyle, R¹⁰ est un hydrogène, et R³ est un groupe 3-méthylphényle ou 3-chlorophényle, alors R⁴ est autre qu'un groupe thién-2-ylméthyle ; et
lorsque R¹ est un groupe cyclohexyle, R¹⁰ est un hydrogène, et R³ est un groupe 3-fluorophényle, alors R⁴ est autre qu'un groupe 1H-benzotriazol-1-ylméthyle.

2. Composé selon la revendication 1 ou son sel pharmaceutiquement acceptable, dans lequel R³ est un groupe 3-fluorophényle.

3. Composé selon la revendication 1 ou son sel pharmaceutiquement acceptable, le composé étant sélectionné parmi :
| |
|---|
| 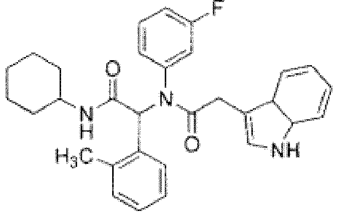 |
| 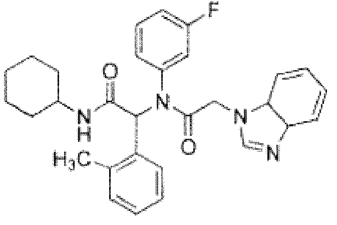 |
| 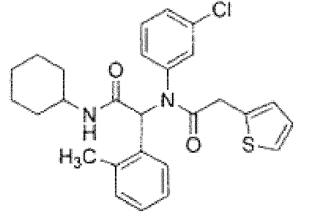 |
| 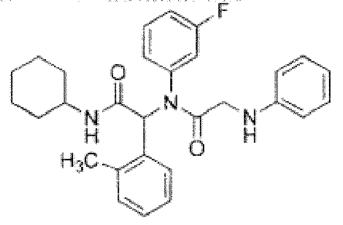 |
| 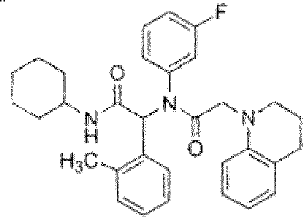 |
| 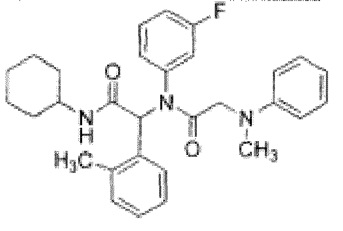 |
| 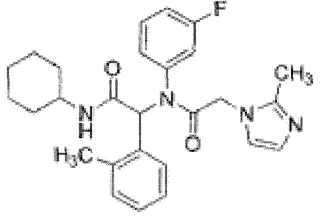 |
| 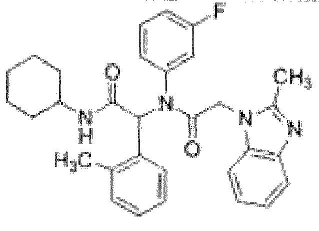 |
| 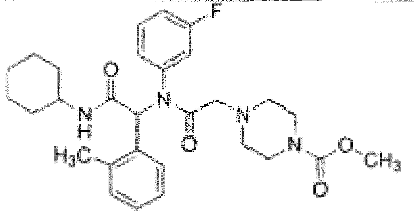 |
| 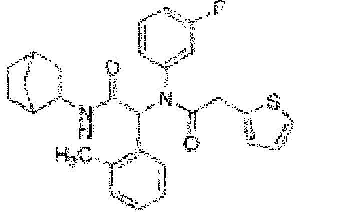 |
| 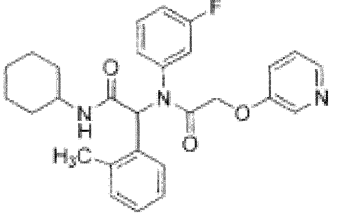 |
| 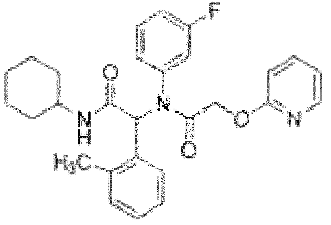 |
| 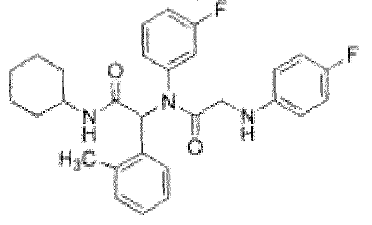 |
| 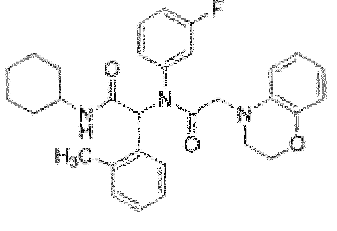 |
| 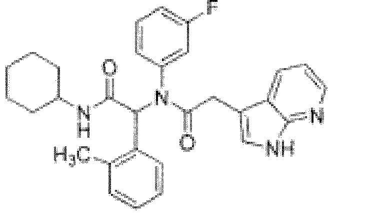 |
| 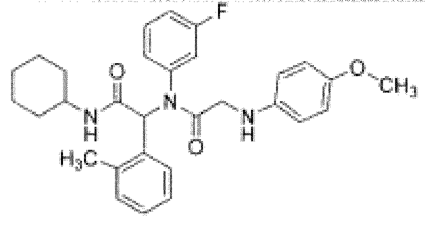 |
| 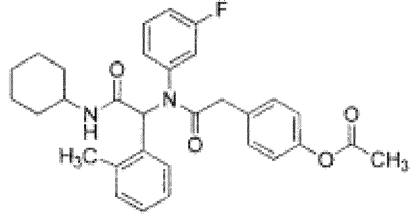 |
| 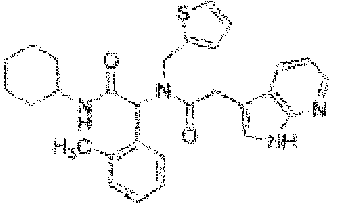 |
| 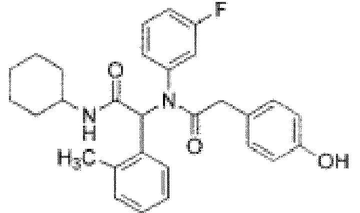 |
| 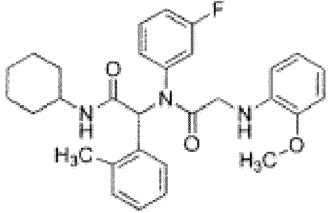 |
| 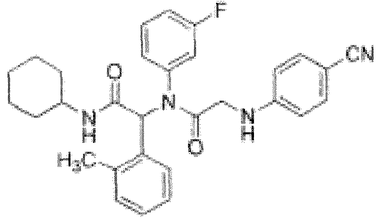 |
| 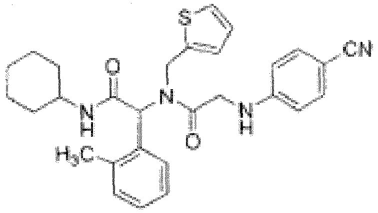 |
| 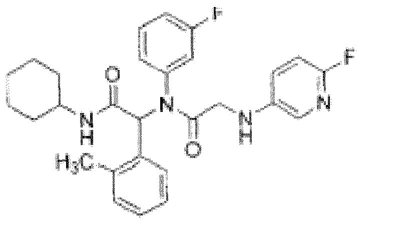 |
| 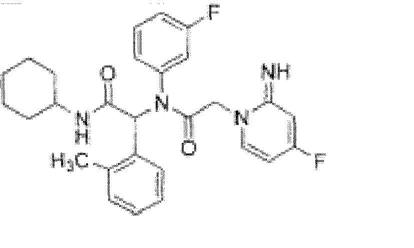 |
| 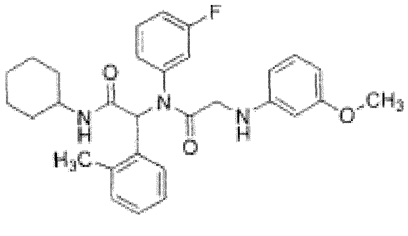 |
| 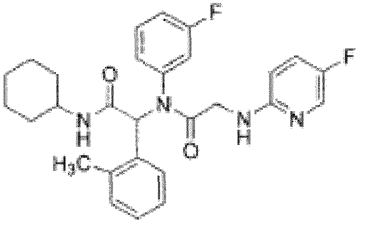 |
| 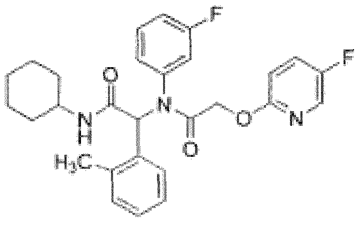 |
| 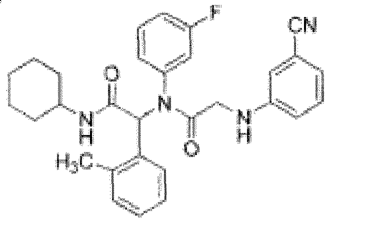 |
| 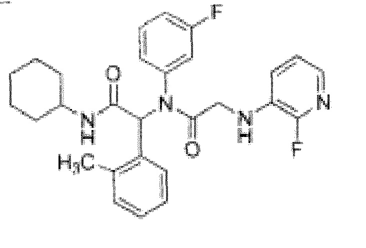 |
| 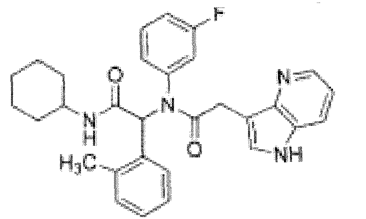 |
| 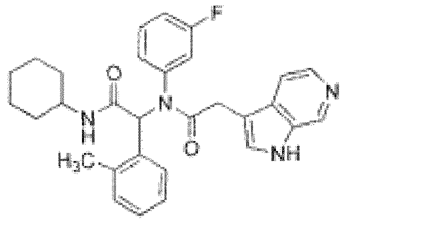 |
| 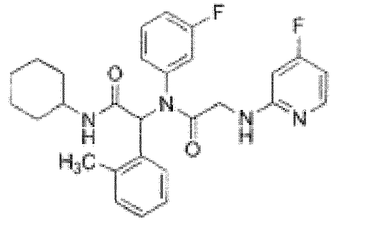 |
| 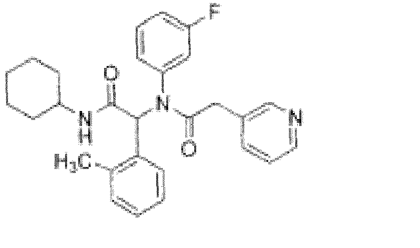 |
| 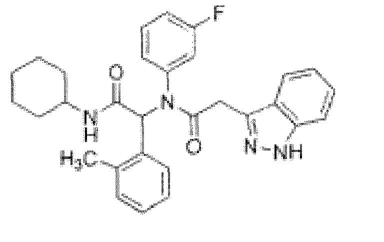 |
| 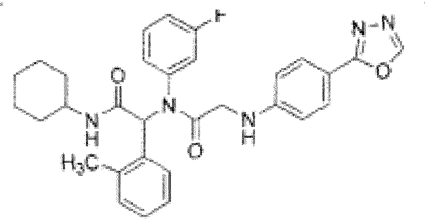 |
| 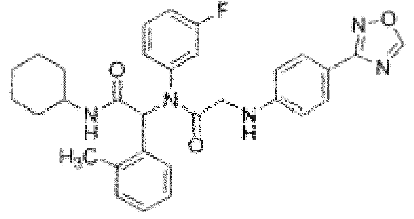 |
| 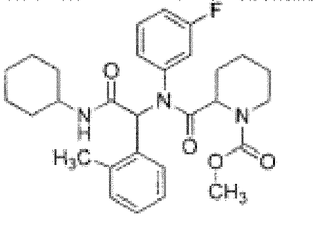 |
| 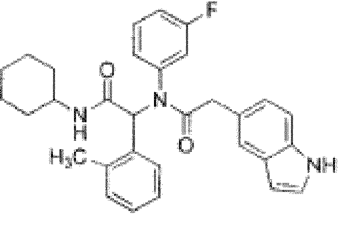 |
| 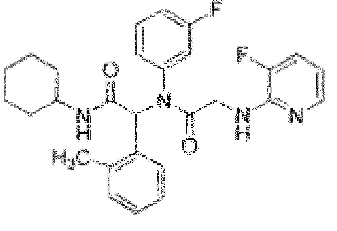 |
| 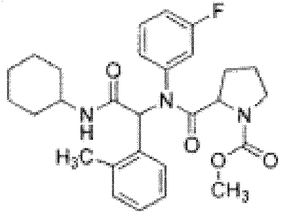 |
| 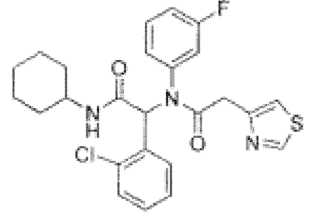 |
| 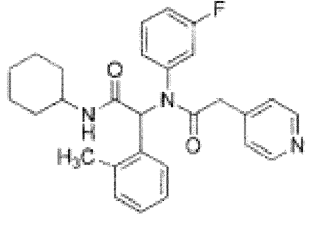 |
| 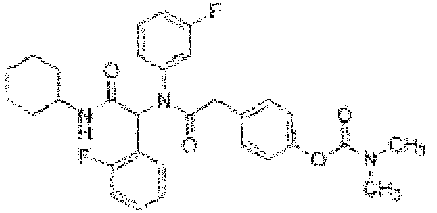 |
| 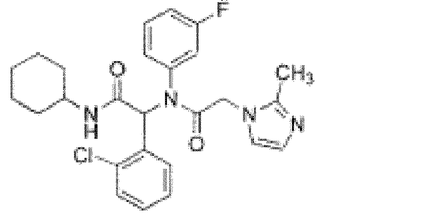 |
| 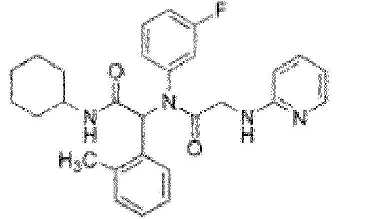 |
| 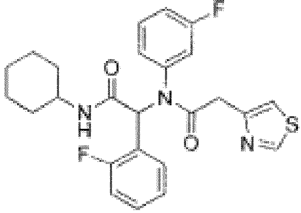 |
| 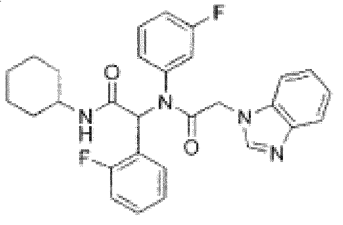 |
| 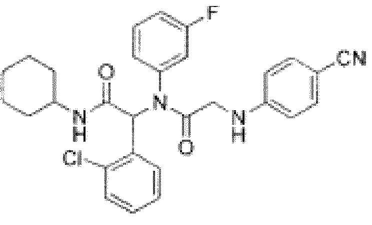 |
| 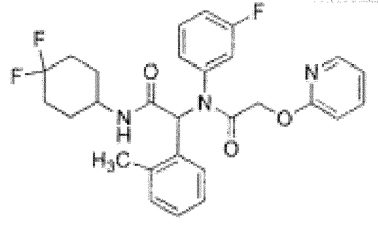 |
| 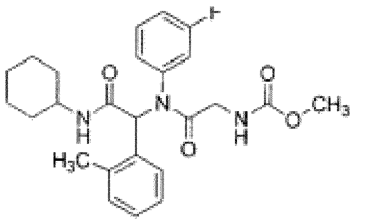 |
| 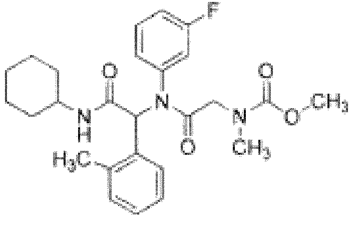 |
| 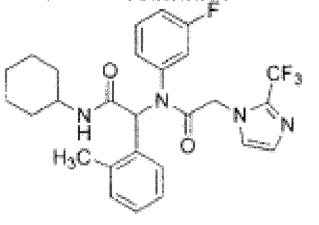 |
| 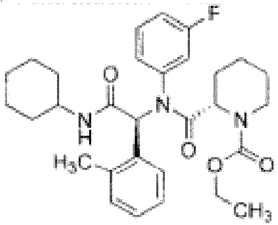 |
| 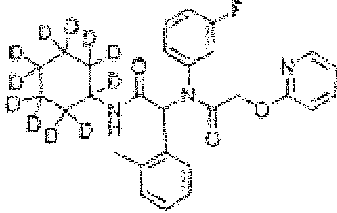 |
| 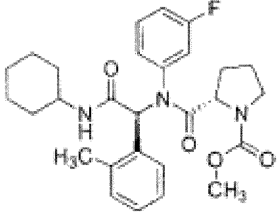 |
| 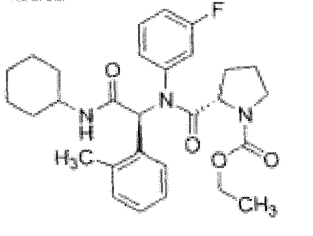 |
| 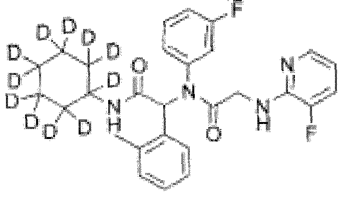 |
| 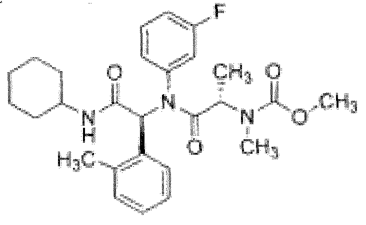 |
| 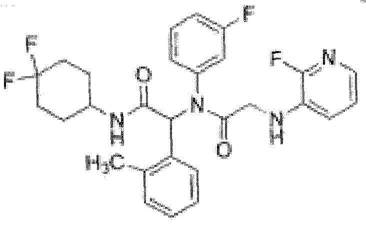 |
| 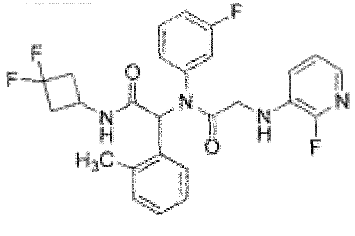 et |
| 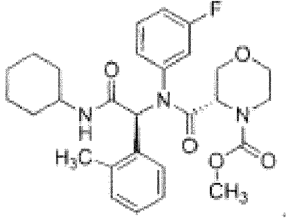 |

4. Composé ou son sel pharmaceutiquement acceptable, destiné à être utilisé dans un procédé de traitement d'un cancer présentant une mutation d'un R132X de l'IDH1, le procédé consistant à administrer à un sujet une quantité thérapeutiquement efficace d'un composé, le composé étant un composé défini selon l'une quelconque des revendications 1 à 3.

5. Composé ou son sel pharmaceutiquement acceptable destiné à être utilisé selon la revendication 4, dans lequel la mutation d'un R132X de l'IDH1 est une mutation du R132H de l'IDH1.

6. Composé ou son sel pharmaceutiquement acceptable destiné à être utilisé selon la revendication 4, dans lequel la mutation d'un R132X de l'IDH1 est une mutation du R132C de l'IDH1.

7. Composé ou son sel pharmaceutiquement acceptable destiné à être utilisé selon la revendication 4, dans lequel un échantillon provenant d'un sujet est évalué in vitro pour rechercher la présence d'un taux élevé de 2HG.

8. Composé ou son sel pharmaceutiquement acceptable destiné à être utilisé selon la revendication 4, dans lequel l'efficacité du traitement du cancer consiste à surveiller in vitro le taux de 2HG dans un échantillon provenant d'un sujet pendant le traitement.

9. Composé ou son sel pharmaceutiquement acceptable destiné à être utilisé selon la revendication 4, dans lequel l'efficacité du traitement du cancer consiste à surveiller in vitro le taux de 2HG dans un échantillon provenant d'un sujet après la fin du traitement.

10. Composition pharmaceutique comprenant un composé selon l'une quelconque des revendications 1 à 3 ou son sel pharmaceutiquement acceptable ; et un support pharmaceutiquement acceptable.
